(19) Europäisches Patentamt / European Patent Office / Office européen des brevets

(11) EP 4 299 557 A1

(12) EUROPEAN PATENT APPLICATION
published in accordance with Art. 153(4) EPC

(43) Date of publication:
**03.01.2024 Bulletin 2024/01**

(21) Application number: **22759827.3**

(22) Date of filing: **25.02.2022**

(51) International Patent Classification (IPC):
***C07C 69/612*** *(2006.01)* ***A01N 25/00*** *(2006.01)*
***A01N 37/36*** *(2006.01)* ***A01N 37/44*** *(2006.01)*
***A01N 43/10*** *(2006.01)* ***A01N 43/40*** *(2006.01)*
***A01N 43/56*** *(2006.01)* ***A01N 43/78*** *(2006.01)*
***A01N 47/02*** *(2006.01)* ***A01N 65/12*** *(2009.01)*
***A01P 3/00*** *(2006.01)* ***A01P 7/02*** *(2006.01)*
***A01P 7/04*** *(2006.01)* ***A61K 31/216*** *(2006.01)*
***A61K 31/381*** *(2006.01)* ***A61K 31/415*** *(2006.01)*
***A61K 31/427*** *(2006.01)* ***A61K 31/4418*** *(2006.01)*
***A61P 31/04*** *(2006.01)* ***A61P 33/14*** *(2006.01)*
***C07C 69/618*** *(2006.01)*

(52) Cooperative Patent Classification (CPC):
**A01N 25/00; A01N 37/36; A01N 37/44; A01N 43/10; A01N 43/40; A01N 43/56; A01N 43/78; A01N 47/02; A01N 65/12; A01P 3/00; A01P 7/02; A01P 7/04; A61K 31/216; A61K 31/381; A61K 31/415;** (Cont.)

(86) International application number:
**PCT/JP2022/008031**

(87) International publication number:
**WO 2022/181793 (01.09.2022 Gazette 2022/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.02.2021 JP 2021029651**
**29.07.2021 JP 2021124464**

(71) Applicant: **Sumitomo Chemical Company Limited**
**Tokyo 103-6020 (JP)**

(72) Inventor: **ARAI, Keisuke**
**Takarazuka-shi, Hyogo 665-8555 (JP)**

(74) Representative: **Vossius & Partner**
**Patentanwälte Rechtsanwälte mbB**
**Siebertstraße 3**
**81675 München (DE)**

# (54) PHENYLACETIC ACID DERIVATIVE, USE THEREFOR, AND PRODUCTION INTERMEDIATE THEREOF

(57) The present invention provides a compound represented by formula (I) wherein a combination of X and L represents a combination wherein X represents a nitrogen atom and L represents an oxygen atom or NH, etc., n is 1 or 2, E represents a C6-C10 aryl group, etc., $R^1$ and $R^2$ are identical to or different from each other and each represents a C1-C3 chain hydrocarbon group which may be optionally substituted with one or more halogens, etc.]
which has an excellent control efficacy against pests, or its N-oxide or salts of the same.

EP 4 299 557 A1

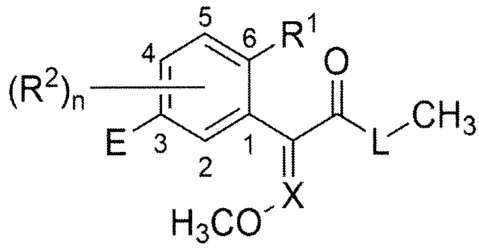

(I)

(52) Cooperative Patent Classification (CPC): (Cont.)
**A61K 31/427; A61K 31/4418; A61P 31/04; A61P 33/14; C07C 69/612; C07C 69/618; C07C 69/65; C07C 251/38; C07C 251/48; C07C 251/52; C07D 213/26; C07D 213/61; C07D 231/12; C07D 277/22; C07D 333/28**

## Description

TECHNICAL FIELD

This application claims priority to and the benefit of Japanese Patent Application No. 2021-029651 filed February 26, 2021, and Japanese Patent Application No. 2021-124464 filed July 29, 2021, the entire contents of which are incorporated herein by reference.

The present invention relates to phenylacetic acid derivatives, use and production intermediate of the same.

BACKGROUND ART

Patent Literature 1 describes phenylacetic acid derivatives.

CITATION LIST

PATENT LITERATURE

Patent Literature 1: EP 0422597 A2

SUMMARY OF THE INVENTION

(PROBLEMS TO BE SOLVED BY INVENTION)

An object of the present invention is to provide a compound having excellent efficacy for controlling pests.

(MEANS TO SOLVE PROBLEMS)

The present inventor has intensively studied to find out a compound having an excellent efficacy for controlling a pest, and as a result, found that a compound represented by the following formula (I) has an excellent efficacy for controlling pests.

That is, the present invention encompasses the followings.

[1] A compound represented by formula (I):

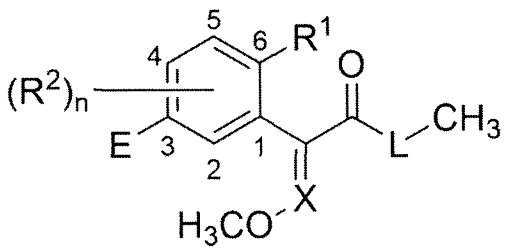

(I)

[wherein

a combination of X and L represents

a combination wherein X represents a nitrogen atom, and L represents an oxygen atom or NH; or
a combination wherein X represents CH, and L represents an oxygen atom,

n is 1 or 2,
E represents a C6-C10 aryl group which may be optionally substituted with one or more substituents selected from Group A, a five- to ten- membered aromatic heterocyclic group which may be optionally substituted with one or more substituents selected from Group B, a C3-C7 cycloalkenyl group which may be optionally substituted with one or more substituents selected from Group C, $R^3$-O-N=C($R^4$)-, $R^5$-C≡C-, or $R^6$O-,
$R^1$ and $R^2$ are identical to or different from each other and each represents a C1-C3 chain hydrocarbon group which may be optionally substituted with one or more halogen atoms, a methoxy group, a cyclopropyl group, or a halogen atom,

$R^3$ represents a C1-C6 chain hydrocarbon group which may be optionally substituted with one or more substituents selected from Group G, a C3-C6 cycloalkyl group which may be optionally substituted with one or more substituents selected from Group E, a C6-C10 aryl group, a five- to ten-membered aromatic heterocyclic group {the C6-C10 aryl group, and the five- to ten- membered aromatic heterocyclic group may be optionally substituted with one or more substituents selected from Group F}, or $R^7CH_2$-,
$R^4$ represents a C1-C3 chain hydrocarbon group which may be optionally substituted with one or more halogen atoms, or a hydrogen atom,
$R^5$ represents a C1-C6 chain hydrocarbon group which may be optionally substituted with one or more substituents selected from Group D, a C3-C6 cycloalkyl group which may be optionally substituted with one or more substituents selected from Group E, a phenyl group, or a five- to six-membered aromatic heterocyclic group {the phenyl group, and the five- to six- membered aromatic heterocyclic group may be optionally substituted with one or more substituents selected from Group F},
$R^6$ represents a methyl group which is substituted with one or more substituents selected from Group G, a C2-C6 chain hydrocarbon group which may be optionally substituted with one or more substituents selected from Group D, or a C3-C4 cycloalkyl group which may be optionally substituted with one or more substituents selected from Group E,
$R^7$ represents a phenyl group, or a five- to six-membered aromatic heterocyclic group {the phenyl group, and the five- to six- membered aromatic heterocyclic group may be optionally substituted with one or more substituents selected from Group F},
Group A is a group consisting of a C1-C6 chain hydrocarbon group, a C1-C3 alkylthio group {the C1-C6 chain hydrocarbon group, and the C1-C3 alkylthio group may be optionally substituted with one or more substituents selected from Group G}, a C3-C6 cycloalkyl group which may be optionally substituted with one or more substituents selected from Group E, $OR^{10}$, $C(O)R^8$, $C(O)OR^8$, $C(O)NR^8R^9$, $NR^8R^9$, $C(R^{11})=N-OR^{12}$, a phenyl group, a five- to six- membered aromatic heterocyclic group {the phenyl group, and the five-to six- membered aromatic heterocyclic group may be optionally substituted with one or more substituents selected from Group F}, a halogen atom, a cyano group, and a nitro group,
$R^8$ and $R^9$ are identical to or different from each other and each represents a C1-C6 chain hydrocarbon group which may be optionally substituted with one or more substituents selected from Group G, a C3-C6 cycloalkyl group which may be optionally substituted with one or more substituents selected from Group E, a phenyl group, a five- to six-membered aromatic heterocyclic group {the phenyl group, and the five- to six- membered aromatic heterocyclic group may be optionally substituted with one or more substituents selected from Group F}, or a hydrogen atom,
$R^{10}$ represents a C1-C6 chain hydrocarbon group which may be optionally substituted with one or more substituents selected from Group G, a C3-C6 cycloalkyl group which may be optionally substituted with one or more substituents selected from Group E, a phenyl group, or a five- to six-membered aromatic heterocyclic group {the phenyl group, and the five- to six- membered aromatic heterocyclic group may be optionally substituted with one or more substituents selected from Group F},
$R^{11}$ represents a C1-C3 chain hydrocarbon group which may be optionally substituted with one or more halogen atoms, or a hydrogen atom,
$R^{12}$ represents a C1-C3 chain hydrocarbon group which may be optionally substituted with one or more substituents selected from Group D, a C3-C6 cycloalkyl group which may be optionally substituted with one or more substituents selected from Group E, a C6-C10 aryl group, and a five- to ten- membered aromatic heterocyclic group {the C6-C10 aryl group, and the five- to ten- membered aromatic heterocyclic group may be optionally substituted with one or more substituents selected from Group F},
Group B is a group consisting of a C3-C6 cycloalkyl group which may be optionally substituted with one or more substituents selected from Group E, a C1-C6 chain hydrocarbon group, a C1-C3 alkylthio group {the C1-C6 chain hydrocarbon group, and the C1-C3 alkylthio group may be optionally substituted with one or more substituents selected from Group G}, a phenyl group, a five- to six- membered aromatic heterocyclic group {the phenyl group, and the five- to six-membered aromatic heterocyclic group may be optionally substituted with one or more substituents selected from Group F}, $OR^{13}$, $C(O)R^8$, $C(O)OR^8$, $C(O)NR^8R^9$, $NR^8R^9$, $C(R^{11})=N-OR^{12}$, a halogen atom, a cyano group, and a nitro group,
$R^{13}$ represents a C3-C6 cycloalkyl group which may be optionally substituted with one or more substituents selected from Group E, a C1-C6 chain hydrocarbon group {the C1-C6 chain hydrocarbon group may be optionally substituted with one or more substituents selected from a C3-C6 cycloalkyl group, and a C1-C3 alkoxy group}, a phenyl group, a five- to six- membered aromatic heterocyclic group {the phenyl group, and the five- to six-membered aromatic heterocyclic group may be optionally substituted with one or more substituents selected from Group F}, or a trifluoromethyl group,
Group C is a group consisting of a C1-C4 chain hydrocarbon group, a C1-C3 alkoxy group {the C1-C4 chain

hydrocarbon group, and the C1-C3 alkoxy group may be optionally substituted with one or more halogen atoms}, a halogen atom, a cyano group, an oxo group, and (=$NOR^{13}$),

Group D is a group consisting of a C1-C6 alkoxy group, a C1-C6 alkylthio group {the C1-C6 alkoxy group and the C1-C6 alkylthio group may be optionally substituted with one or more substituents selected from Group G}, a C3-C6 cycloalkyl group, a three- to eight- membered non-aromatic heterocyclic group {the C3-C6 cycloalkyl group, and the three- to eight-membered non-aromatic heterocyclic group may be optionally substituted with one or more substituents selected from Group E}, a C6-C10 aryl group, a five- to ten- membered aromatic heterocyclic group {the C6-C10 aryl group, and the five- to ten- membered aromatic heterocyclic group may be optionally substituted with one or more substituents selected from Group F}, a halogen atom, a cyano group, a nitro group, and a hydroxy group,

Group E is a group consisting of a C1-C3 chain hydrocarbon group, a C1-C3 alkoxy group, a C3-C6 cycloalkyl group {the C1-C3 chain hydrocarbon group, the C1-C3 alkoxy group, and the C3-C6 cycloalkyl group may be optionally substituted with one or more halogen atoms}, an oxo group, a halogen atom, a cyano group, and (=$NOR^4$),

Group F is a group consisting of a C1-C3 chain hydrocarbon group, a C3-C6 cycloalkyl group, a C1-C3 alkoxy group, a C1-C3 alkylthio group {the C1-C3 chain hydrocarbon group, the C3-C6 cycloalkyl group, the C1-C3 alkoxy group, and the C1-C3 alkylthio group may be optionally substituted with one or more substituents selected from Group G}, a halogen atom, a cyano group, a nitro group, and a hydroxy group,

Group G is a group consisting of a C3-C6 cycloalkyl group, a C1-C3 alkoxy group, and a halogen atom] (hereinafter, referred to as "Present compound N" or "Compound N of the present invention"), or its N-oxide or salts thereof (hereinafter, the compound represented by formula (I), or its N-oxide or salts thereof are referred to as "Present compound" or "Compound of the present invention").

[2] The compound according to [1], its N-oxide or salts thereof, wherein

n is 1,

$R^1$ and $R^2$ are identical to or different from each other and each represents a C1-C3 alkyl group which may be optionally substituted with one or more halogen atoms, a methoxy group, or a halogen atom,

$R^2$ binds at the 4-position or the 5-position,

E represents a phenyl group, a five- to six- membered aromatic heterocyclic group {the phenyl group, and the five-to six- membered aromatic heterocyclic group may be optionally substituted with one or more substituents selected from Group H}, $R^3$-O-N=C($R^4$)-, $R^5$-C≡C-, $R^6$O-, or a C5-C6 cycloalkenyl group,

$R^3$ represents a C1-C6 chain hydrocarbon group which may be optionally substituted with one or more substituents selected from Group G, a C3-C6 cycloalkyl group which may be optionally substituted with one or more halogen atoms, or $R^7CH_2$-,

$R^4$ represents a C1-C3 chain hydrocarbon group, or a hydrogen atom,

$R^5$ represents a C1-C6 chain hydrocarbon group, or a C3-C6 cycloalkyl group {the C1-C6 chain hydrocarbon group, and the C3-C6 cycloalkyl group may be optionally substituted with one or more halogen atoms},

$R^6$ represents a C2-C6 chain hydrocarbon group which may be optionally substituted with one or more substituents selected from Group I, or a C3-C4 cycloalkyl group,

$R^7$ represents a phenyl group or a five- to six- membered aromatic heterocyclic group {the phenyl group, and the five-to six- membered aromatic heterocyclic group may be optionally substituted with one or more substituents selected from Group J},

Group H is a group consisting of a C1-C3 chain hydrocarbon group which may be optionally substituted with one or more halogen atoms, a C1-C3 alkoxy group, a trifluoromethoxy group, a C3-C4 cycloalkyl group, a halogen atom, a cyano group, and a nitro group,

Group I is a group consisting of a C3-C6 cycloalkyl group, a C1-C3 alkoxy group, a phenyl group {the C1-C3 alkoxy group and the phenyl group may be optionally substituted with one or more halogen atoms}, and a halogen atom,

Group J is a group consisting of a C1-C3 chain hydrocarbon group, a C1-C3 alkoxy group {the C1-C3 chain hydrocarbon group, and the C1-C3 alkoxy group may be optionally substituted with one or more halogen atoms}, a C3-C6 cycloalkyl group, a halogen atom, a cyano group, and a nitro group.

[3] The compound according to [2], or its N-oxide or salts thereof, wherein E represents a phenyl group, a pyridyl group, a thienyl group, a thiazolyl group, a pyrazolyl group {the phenyl group, the pyridyl group, the thienyl group, the thiazolyl group, and the pyrazolyl group may be optionally substituted with one or more substituents selected from Group H}, $R^5$-C≡C-, $R^6$O-, or a C5-C6 cycloalkenyl group.

[4] An agricultural composition which comprises the compound according to any one of [1] to [3] or salts thereof,

and an inert carrier.

[5] A composition which comprises one or more ingredients selected from the group consisting of the following Groups (a), (b), (c) and (d), as well as the compound according to any one of [1] to [3] or its N-oxide or salts thereof:

Group (a): a group consisting of insecticidal ingredients, miticidal ingredients, and nematicidal ingredients;
Group (b): fungicidal ingredients;
Group (c): plant growth modulating ingredients; and
Group (d): repellent ingredients.

[6] A method for controlling pests which comprises applying an effective amount of the compound according to any one of [1] to [3] or its N-oxide or salts thereof or an effective amount of the composition according to [5] to a plant or soil.

[7] A method for controlling soybean rust fungus having an amino acid substitution of F129L on mitochondrial cytochrome b protein, which comprises applying an effective amount of the compound according to any one of [1] to [3], or its N-oxide or salts thereof, or an effective amount of the composition according to [5] to a soybean or soil where the soybean grows.

[8] Use of the compound according to any one of [1] to [3], or its N-oxide or salts thereof, or the composition according to [5] for controlling a pest.

[9] A seed or vegetative reproductive organ carrying an effective amount of the compound according to any one of [1] to [3] or its N-oxide or salt thereof or an effective amount of the composition according to [5].

[10] A compound represented by formula (II):

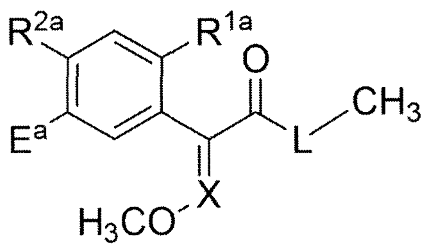

(II)

[wherein

a combination of X and L represents

a combination wherein X represents a nitrogen atom, and L represents an oxygen atom or NH; or
a combination wherein X represents CH, and L represents an oxygen atom,

$E^a$ represents $R^{12a}C(O)$-, $R^{12a}C(=N-OH)$-, a hydroxy group, $B(OH)_2$, a 4,4,5,5-tetramethyl-1,3,2-dioxaborolan-2-yl group, or a halogen atom,
$R^{1a}$ and $R^{2a}$ are identical to or different from each other and each represents a methyl group, or a halogen atom, and
$R^{12a}$ represents a C1-C3 alkyl group, or a hydrogen atom] (hereinafter, referred to as "Intermediate compound A").

[EFFECT OF INVENTION]

The present invention can control pests.

MODE FOR CARRYING OUT THE INVENTION

The substituents as used herein are explained as follows.

The term "halogen atom" represents a fluorine atom, a chlorine atom, a bromine atom, or an iodine atom.

When a substituent has two or more halogen atoms, the halogen atoms may be identical to or different from each other.

When a substituent has two or more groups or atoms selected from particular groups (for example, a group selected from a C1-C3 alkyl group and a halogen atom), each of the groups or the atoms may be identical to or different from each other.

The expression of "which may be optionally substituted with one or more substituents selected from Group X

(wherein X means any one of A, B, C, D, E, F, G, H, I, J, or K)" represents that when two or more of the substituents selected from Group X are existed, the substituents may be identical to or different from each other.

The expression "CX-CY" as used herein represents that the number of carbon atoms is from X to Y. For example, the expression "C1-C6" represents that the number of carbon atoms is from 1 to 6.

The term "chain hydrocarbon group" represents an alkyl group, an alkenyl group, or an alkynyl group.

Examples of the "alkyl group" include methyl group, ethyl group, propyl group, isopropyl group, 1,1-dimethylpropyl group, 1,2-dimethylpropyl group, butyl group, sec-butyl group, tert-butyl group, pentyl group, hexyl group, and decyl group.

Examples of the "alkenyl group" include vinyl group, 1-propenyl group, 2-propenyl group, 1-methyl-1-propenyl group, 1-methyl-2-propenyl group, 1,2-dimethyl-1-propenyl group, 3-butenyl group, 4-pentenyl group, 5-hexenyl group, and 9-decenyl group.

Examples of the "alkynyl group" include ethynyl group, 1-propynyl group, 2-propynyl group, 1-methyl-2-propynyl group, 1,1-dimethyl-2-propynyl group, 2-butynyl group, 4-pentynyl group, 5-hexynyl group, and 9-decynyl group.

Examples of the "alkoxy group" include methoxy group, ethoxy group, propoxy group, isopropoxy group, butoxy group, tert-butoxy group, pentyloxy group, and hexyloxy group.

Examples of the "alkylthio group" includes methylthio group, ethylthio group, propylthio group, isopropylthio group, butylthio group, tert-butylthio group, pentylthio group, and hexylthio group.

Examples of the "cycloalkyl group" include cyclopropyl group, cyclobutyl group, cyclopentyl group, and cyclohexyl group.

Examples of the "cycloalkenyl group" include cyclopentenyl group, and cyclohexenyl group.

Examples of the "aryl group" include phenyl group, indenyl group, indanyl group, naphthyl group, and tetrahydronaphthyl group.

Examples of the "aromatic heterocyclic group" include pyrrolyl group, furyl group, thienyl group, pyrazolyl group, imidazolyl group, triazolyl group, tetrazolyl group, oxazolyl group, isoxazolyl group, thiazolyl group, isothiazolyl group, oxadiazolyl group, thiadiazolyl group, pyridyl group, pyridazinyl group, pyrimidinyl group, pyrazinyl group, triazinyl group, tetrazinyl group, indolyl group, indazolyl group, benzimidazolyl group, imidazopyridyl group, benzothiophenyl group, benzofuranyl group, quinolyl group, isoquinolyl group, quinazolinyl group, and quinoxalinyl group.

The term(s) as described herein is/are explained.

The term of "soybean rust fungus having an amino acid substitution of F129L on mitochondrial cytochrome b protein" represents soybean rust fungus (scientific name: *Phakopsora pachyrhizi*) which shows a resistance against QoI fungicide by having a mutation in the mitochondrial cytochrome b gene encoding mitochondrial cytochrome protein and as a result of the mutation, causing amino acid substitution of F129L.

The present compound and the intermediate compound A may be existed as one or more stereoisomers. Examples of the stereoisomer include enantiomer, diastereoisomer, atropisomer, and geometric isomer. Each stereoisomer, and stereoisomer mixture(s) in an arbitrary ratio thereof are included in the present invention.

Examples of the geometric isomer include the below-mentioned structures.

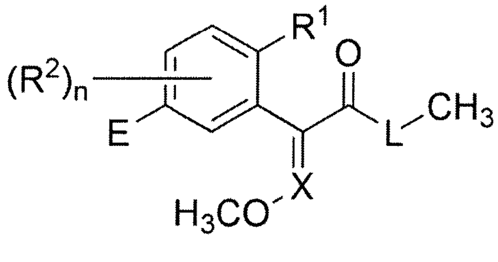

( I )

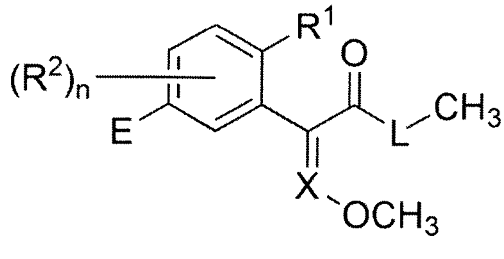

( I–Z )

The present compound N or its N-oxide may form an acid additional salts thereof such as hydrochloride salt, sulfate, nitrate, phosphate, acetate, and benzoate by mixing it with an acid such as hydrochloric acid, sulfuric acid, nitric acid, phosphoric acid, acetic acid, and benzoic acid.

Embodiments of the compound N of the present invention include the following compounds.

[Embodiment 1] The compound N of the present invention wherein E represents $R^3$-O-N=C($R^4$)-.

[Embodiment 2] The compound according to the Embodiment 1 wherein $R^3$ represents a C1-C6 chain hydrocarbon group {the C1-C6 chain hydrocarbon group may be optionally substituted with one or more substituents selected from C3-C4 cycloalkyl group and a halogen atom}, or $R^7CH_2$-, $R^4$ represents a methyl group or a hydrogen atom,

and $R^7$ represents a phenyl group or a five- to six- membered aromatic heterocyclic group {the phenyl group, and the five- to six- membered aromatic heterocyclic group may be optionally substituted with one or more substituents selected from Group K},

Group K is a group consisting of a C1-C3 chain hydrocarbon group which may be optionally substituted with one or more halogen atoms, and a halogen atom.

[Embodiment 3] The compound N of the present invention wherein E represents $R^5$-C≡C-.

[Embodiment 4] The compound according to the Embodiment 3 wherein $R^5$ represents a C1-C6 chain hydrocarbon group or a C3-C6 cycloalkyl group.

[Embodiment 5] The compound N of the present invention wherein E represents $R^6$O-.

[Embodiment 6] The compound according to the Embodiment 5 wherein $R^6$ represents a C2-C6 chain hydrocarbon group which may be optionally substituted with one or more substituents selected from Group I, or a C3-C4 cycloalkyl group.

[Embodiment 7] The compound N of the present invention wherein E represents a phenyl group which may be optionally substituted with one or more substituents selected from Group A.

[Embodiment 8] The compound N of the present invention wherein E represents a phenyl group which may be optionally substituted with one or more substituents selected from Group H.

[Embodiment 9] The compound N of the present invention wherein E represents a five- to six- membered aromatic heterocyclic group which may be optionally substituted with one or more substituents selected from Group B.

[Embodiment 10] The compound N of the present invention wherein E represents a five- to six- membered aromatic heterocyclic group which may be optionally substituted with one or more substituents selected from Group H.

[Embodiment 11] The compound N of the present invention wherein E represents a pyridyl group, a thienyl group, a thiazolyl group, or a pyrazolyl group {the pyridyl group, the thienyl group, the thiazolyl group, and the pyrazolyl group may be optionally substituted with one or more substituents selected from Group H}.

[Embodiment 12] The compound N of the present invention wherein E represents a pyridyl group, a thienyl group, a thiazolyl group, or a pyrazolyl group {the pyridyl group, the thienyl group, the thiazolyl group, and the pyrazolyl group may be optionally substituted with one or more substituents selected from Group K}.

[Embodiment 13] The compound N of the present invention wherein E represents a C5-C6 cycloalkenyl group.

[Embodiment 14] The compound N of the present invention wherein E represents a phenyl group {the phenyl group may be optionally substituted with one or more substituents selected from Group H}, a pyridyl group, a thienyl group, a thiazolyl group, a pyrazolyl group {the pyridyl group, the thienyl group, the thiazolyl group, and the pyrazolyl group may be optionally substituted with one or more substituents selected from Group K}, a C5-C6 cycloalkenyl group, $R^3$-O-N=C($R^4$)-, $R^5$-C≡C-, or $R^6$O-, $R^3$ represents a C1-C6 chain hydrocarbon group {the C1-C6 chain hydrocarbon group may be optionally substituted with one or more substituents selected from a C3-C4 cycloalkyl group and a halogen atom}, or $R^7CH_2$-, $R^4$ represents a methyl group or a hydrogen atom, $R^5$ represents a C1-C6 chain hydrocarbon group or a C3-C6 cycloalkyl group, $R^6$ represents a C2-C6 chain hydrocarbon group which may be optionally substituted with one or more substituents selected from Group I, or a C3-C4 cycloalkyl group, and $R^7$ represents a phenyl group or a five- to six-membered aromatic heterocyclic group {the phenyl group, and the five- to six- membered aromatic heterocyclic group may be optionally substituted with one or more substituents selected from Group K}.

[Embodiment 15] The compound N of the present invention wherein E represents a phenyl group {the phenyl group may be optionally substituted with one or more substituents selected from Group A}, a five- to six- membered aromatic heterocyclic group {the five- to six- membered aromatic heterocyclic group may be optionally substituted with one or more substituents selected from Group H}, a C3-C7 cycloalkenyl group which may be optionally substituted with one or more substituents selected from Group C, $R^5$-C≡C-, or $R^6$O-, and $R^5$ represents a C1-C6 chain hydrocarbon group or a C3-C6 cycloalkyl group {the C1-C6 chain hydrocarbon group, and the C3-C6 cycloalkyl group may be optionally substituted with one or more halogen atoms}.

[Embodiment 16] The compound N of the present invention wherein E represents a phenyl group {the phenyl group may be optionally substituted with one or more substituents selected from Group H}, a pyridyl group, a thienyl group, a thiazolyl group, a pyrazolyl group {the pyridyl group, the thienyl group, the thiazolyl group, and the pyrazolyl group may be optionally substituted with one or more substituents selected from Group K}, a C5-C6 cycloalkenyl group, a $R^5$-C=C-, or $R^6$O-, $R^5$ represents a C1-C6 chain hydrocarbon group or a C3-C6 cycloalkyl group, and $R^6$ represents a C2-C6 chain hydrocarbon group which may be optionally substituted with one or more substituents selected from Group I, or a C3-C4 cycloalkyl group.

[Embodiment 17] The compound N of the present invention wherein E represents a phenyl group {the phenyl group may be optionally substituted with one or more substituents selected from Group A}, a five- to six- membered aromatic heterocyclic group {the five- to six- membered aromatic heterocyclic group may be optionally substituted with one or more substituents selected from Group H}, $R^5$-C≡C-, or $R^6$O-, and $R^5$ represents a C1-C6 chain hydrocarbon group or a C3-C6 cycloalkyl group {the C1-C6 chain hydrocarbon group, and the C3-C6 cycloalkyl group may be optionally substituted with one or more halogen atoms}.

[Embodiment 18] The compound N of the present invention wherein E represents a phenyl group {the phenyl group may be optionally substituted with one or more substituents selected from Group H}, a pyridyl group, a thienyl group, a thiazolyl group, a pyrazolyl group {the pyridyl group, the thienyl group, the thiazolyl group, and the pyrazolyl group may be optionally substituted with one or more substituents selected from Group K}, $R^5$-C≡C-, or $R^6$O-, $R^5$ represents a C1-C6 chain hydrocarbon group or a C3-C6 cycloalkyl group, and $R^6$ represents a C2-C6 chain hydrocarbon group which may be optionally substituted with one or more substituents selected from Group I, or a C3-C4 cycloalkyl group.

[Embodiment 19] The compound N of the present invention wherein E represents a phenyl group {the phenyl group may be optionally substituted with one or more substituents selected from Group A}, a five- to six- membered aromatic heterocyclic group {the five- to six- membered aromatic heterocyclic group may be optionally substituted with one or more substituents selected from Group H}, or $R^5$-C≡C-, and $R^5$ represents a C1-C6 chain hydrocarbon group or a C3-C6 cycloalkyl group {the C1-C6 chain hydrocarbon group, and the C3-C6 cycloalkyl group may be optionally substituted with one or more halogen atoms}.

[Embodiment 20] The compound N of the present invention wherein E represents a phenyl group {the phenyl group may be optionally substituted with one or more substituents selected from Group H}, a pyridyl group, a thienyl group, a thiazolyl group, a pyrazolyl group {the pyridyl group, the thienyl group, the thiazolyl group, and the pyrazolyl group may be optionally substituted with one or more substituents selected from Group K}, or $R^5$-C≡C-, and $R^5$ represents a C1-C6 chain hydrocarbon group or a C3-C6 cycloalkyl group.

[Embodiment 21] The compound N of the present invention wherein E represents a phenyl group {the phenyl group may be optionally substituted with one or more substituents selected from Group A}, a five- to six- membered aromatic heterocyclic group {the five- to six- membered aromatic heterocyclic group may be optionally substituted with one or more substituents selected from Group H}, or $R^6$O-.

[Embodiment 22] The compound N of the present invention wherein E represents a phenyl group {the phenyl group may be optionally substituted with one or more substituents selected from Group H}, a pyridyl group, a thienyl group, a thiazolyl group, a pyrazolyl group {the pyridyl group, the thienyl group, the thiazolyl group, and the pyrazolyl group may be optionally substituted with one or more substituents selected from Group K}, or $R^6$O-, and $R^6$ represents a C2-C6 chain hydrocarbon group which may be optionally substituted with one or more substituents selected from Group I, or a C3-C4 cycloalkyl group.

[Embodiment 23] The compound N of the present invention wherein E represents a phenyl group {the phenyl group may be optionally substituted with one or more substituents selected from Group A}, $R^5$-C≡C-, or $R^6$O-, $R^5$ represents a C1-C6 chain hydrocarbon group or a C3-C6 cycloalkyl group {the C1-C6 chain hydrocarbon group, and the C3-C6 cycloalkyl group may be optionally substituted with one or more halogen atoms.

[Embodiment 24] The compound N of the present invention wherein E represents a phenyl group {the phenyl group may be optionally substituted with one or more substituents selected from Group H}, $R^5$-C≡C-, or $R^6$O-, $R^5$ represents a C1-C6 chain hydrocarbon group or a C3-C6 cycloalkyl group, and $R^6$ represents a C2-C6 chain hydrocarbon group which may be optionally substituted with one or more substituents selected from Group I, or a C3-C4 cycloalkyl group.

[Embodiment 25] The compound N of the present invention wherein E represents a phenyl group {the phenyl group may be optionally substituted with one or more substituents selected from Group A}, or a five- to six- membered aromatic heterocyclic group {the five- to six- membered aromatic heterocyclic group may be optionally substituted with one or more substituents selected from Group H}.

[Embodiment 26] The compound N of the present invention wherein E represents a phenyl group {the phenyl group may be optionally substituted with one or more substituents selected from Group H}, a pyridyl group, a thienyl group, a thiazolyl group, or a pyrazolyl group {the pyridyl group, the thienyl group, the thiazolyl group, and the pyrazolyl group may be optionally substituted with one or more substituents selected from Group K}.

[Embodiment 27] The compound N of the present invention wherein E represents a phenyl group {the phenyl group may be optionally substituted with one or more substituents selected from Group A}, or $R^5$-C≡C-, $R^5$ represents a C1-C6 chain hydrocarbon group or C3-C6 cycloalkyl group {the C1-C6 chain hydrocarbon group, and the C3-C6 cycloalkyl group may be optionally substituted with one or more halogen atoms}.

[Embodiment 28] The compound N of the present invention wherein E represents a phenyl group {the phenyl group may be optionally substituted with one or more substituents selected from Group H}, or $R^5$-C≡C-, $R^5$ represents a C1-C6 chain hydrocarbon group or a C3-C6 cycloalkyl group.

[Embodiment 29] The compound N of the present invention wherein E represents a phenyl group {the phenyl group may be optionally substituted with one or more substituents selected from Group A}, or $R^6$O-.

[Embodiment 30] The compound N of the present invention wherein E represents a phenyl group {the phenyl group may be optionally substituted with one or more substituents selected from Group H}, or $R^6$O-, and $R^6$ represents a C2-C6 chain hydrocarbon group which may be optionally substituted with one or more substituents selected from Group I, or a C3-C4 cycloalkyl group.

[Embodiment 31] The compound N of the present invention wherein X represents CH, and L represents an oxygen atom.

[Embodiment 32] The compound according to the Embodiment 1 wherein X represents CH, and L represents an oxygen atom.

[Embodiment 33] The compound according to the Embodiment 2 wherein X represents CH, and L represents an oxygen atom.

[Embodiment 34] The compound according to the Embodiment 3 wherein X represents CH, and L represents an oxygen atom.

[Embodiment 35] The compound according to the Embodiment 4 wherein X represents CH, and L represents an oxygen atom.

[Embodiment 36] The compound according to the Embodiment 5 wherein X represents CH, and L represents an oxygen atom.

[Embodiment 37] The compound according to the Embodiment 6 wherein X represents CH, and L represents an oxygen atom.

[Embodiment 38] The compound according to the Embodiment 7 wherein X represents CH, and L represents an oxygen atom.

[Embodiment 39] The compound according to the Embodiment 8 wherein X represents CH, and L represents an oxygen atom.

[Embodiment 40] The compound according to the Embodiment 9 wherein X represents CH, and L represents an oxygen atom.

[Embodiment 41] The compound according to the Embodiment 10 wherein X represents CH, and L represents an oxygen atom.

[Embodiment 42] The compound according to the Embodiment 11 wherein X represents CH, and L represents an oxygen atom.

[Embodiment 43] The compound according to the Embodiment 12 wherein X represents CH, and L represents an oxygen atom.

[Embodiment 44] The compound according to the Embodiment 13 wherein X represents CH, and L represents an oxygen atom.

[Embodiment 45] The compound according to the Embodiment 14 wherein X represents CH, and L represents an oxygen atom.

[Embodiment 46] The compound according to the Embodiment 15 wherein X represents CH, and L represents an oxygen atom.

[Embodiment 47] The compound according to the Embodiment 16 wherein X represents CH, and L represents an oxygen atom.

[Embodiment 48] The compound according to the Embodiment 17 wherein X represents CH, and L represents an oxygen atom.

[Embodiment 49] The compound according to the Embodiment 18 wherein X represents CH, and L represents an oxygen atom.

[Embodiment 50] The compound according to the Embodiment 19 wherein X represents CH, and L represents an oxygen atom.

[Embodiment 51] The compound according to the Embodiment 20 wherein X represents CH, and L represents an oxygen atom.

[Embodiment 52] The compound according to the Embodiment 21 wherein X represents CH, and L represents an oxygen atom.

[Embodiment 53] The compound according to the Embodiment 22 wherein X represents CH, and L represents an oxygen atom.

[Embodiment 54] The compound according to the Embodiment 23 wherein X represents CH, and L represents an oxygen atom.

[Embodiment 55] The compound according to the Embodiment 24 wherein X represents CH, and L represents an oxygen atom.

[Embodiment 56] The compound according to the Embodiment 25 wherein X represents CH, and L represents an oxygen atom.

[Embodiment 57] The compound according to the Embodiment 26 wherein X represents CH, and L represents an oxygen atom.

[Embodiment 58] The compound according to the Embodiment 27 wherein X represents CH, and L represents an oxygen atom.

[Embodiment 59] The compound according to the Embodiment 28 wherein X represents CH, and L represents an oxygen atom.

[Embodiment 60] The compound according to the Embodiment 29 wherein X represents CH, and L represents an oxygen atom.

[Embodiment 61] The compound according to the Embodiment 30 wherein X represents CH, and L represents an oxygen atom.

[Embodiment 62] The compound according to any one of the Embodiments 1 to 61 or the compound N of the present invention wherein $R^1$ represents a C1-C3 alkyl group which may be optionally substituted with one or more halogen atoms, a methoxy group, or a halogen atom.

[Embodiment 63] The compound according to any one of the Embodiments 1 to 61 or the compound N of the present invention wherein $R^1$ represents a methyl group, a methoxy group, or a halogen atom.

[Embodiment 64] The compound according to any one of the Embodiments 1 to 61 or the compound N of the present invention wherein $R^1$ represents a methyl group.

[Embodiment 65] The compound according to any one of the Embodiments 1 to 61 or the compound N of the present invention wherein $R^2$ represents a C1-C3 alkyl group which may be optionally substituted with one or more halogen atoms, a methoxy group, or a halogen atom.

[Embodiment 66] The compound according to any one of the Embodiments 1 to 61 or the compound N of the present invention wherein $R^2$ represents a methyl group, a methoxy group, or a halogen atom.

[Embodiment 67] The compound according to any one of the Embodiments 1 to 61 or the compound N of the present invention wherein $R^2$ represents a methyl group.

[Embodiment 68] The compound according to any one of the Embodiments 1 to 61 or the compound N of the present invention wherein $R^1$ and $R^2$ are identical to or different from each other and each represents a C1-C3 alkyl group which may be optionally substituted with one or more halogen atoms, a methoxy group, or a halogen atom.

[Embodiment 69] The compound according to any one of the Embodiments 1 to 61 or the compound N of the present invention wherein $R^1$ represents a methyl group, a methoxy group, or a halogen atom, $R^2$ represents a C1-C3 alkyl group which may be optionally substituted with one or more halogen atoms, a methoxy group, or a halogen atom.

[Embodiment 70] The compound according to any one of the Embodiments 1 to 61 or the compound N of the present invention wherein $R^1$ represents a methyl group, and $R^2$ represents a C1-C3 alkyl group which may be optionally substituted with one or more halogen atoms, a methoxy group, or a halogen atom.

[Embodiment 71] The compound according to any one of the Embodiments 1 to 61 or the compound N of the present invention wherein $R^1$ and $R^2$ are identical to or different from each other and each represents a methyl group, a methoxy group, or a halogen atom.

[Embodiment 72] The compound according to any one of the Embodiments 1 to 61 or the compound N of the present invention wherein $R^1$ represents a methyl group, and $R^2$ represents a methyl group, a methoxy group, or a halogen atom.

[Embodiment 73] The compound according to any one of the Embodiments 1 to 61 or the compound N of the present invention wherein $R^1$ and $R^2$ represent a methyl group.

[Embodiment 74] The compound according to any one of the Embodiments 1 to 61 or the compound N of the present invention wherein n is 1.

[Embodiment 75] The compound according to Embodiment 68 wherein n is 1.

[Embodiment 76] The compound according to Embodiment 69 wherein n is 1.

[Embodiment 77] The compound according to Embodiment 70 wherein n is 1.

[Embodiment 78] The compound according to Embodiment 71 wherein n is 1.

[Embodiment 79] The compound according to Embodiment 72 wherein n is 1.

[Embodiment 80] The compound according to Embodiment 73 wherein n is 1.

[Embodiment 81] The compound according to any one of the Embodiments 1 to 61 or the compound N of the present invention wherein n is 1, and $R^2$ binds at the 4-position or the 5-position.

[Embodiment 82] The compound according to the Embodiments 68 wherein n is 1, and $R^2$ binds at the 4-position or the 5-position.

[Embodiment 83] The compound according to the Embodiments 69 wherein n is 1, and $R^2$ binds at the 4-position or the 5-position.

[Embodiment 84] The compound according to the Embodiments 70 wherein n is 1, and $R^2$ binds at the 4-position or the 5-position.

[Embodiment 85] The compound according to the Embodiments 71 wherein n is 1, and $R^2$ binds at the 4-position or the 5-position.

[Embodiment 86] The compound according to the Embodiments 72 wherein n is 1, and $R^2$ binds at the 4-position or the 5-position.

[Embodiment 87] The compound according to the Embodiments 73 wherein n is 1, and $R^2$ binds at the 4-position or the 5-position.

[Embodiment 88] The compound according to any one of the Embodiments 1 to 61 or the compound N of the present

invention wherein n is 1, and $R^2$ binds at the 4-position.

[Embodiment 89] The compound according to the Embodiment 68 wherein n is 1, and $R^2$ binds at the 4-position.

[Embodiment 90] The compound according to the Embodiment 69 wherein n is 1, and $R^2$ binds at the 4-position.

[Embodiment 91] The compound according to the Embodiment 70 wherein n is 1, and $R^2$ binds at the 4-position.

[Embodiment 92] The compound according to the Embodiment 71 wherein n is 1, and $R^2$ binds at the 4-position.

[Embodiment 93] The compound according to the Embodiment 72 wherein n is 1, and $R^2$ binds at the 4-position.

[Embodiment 94] The compound according to the Embodiment 73 wherein n is 1, and $R^2$ binds at the 4-position.

The compound N of the present invention wherein n is 1 and $R^4$ binds at the 4-position represents the compound represented by formula (I-4):

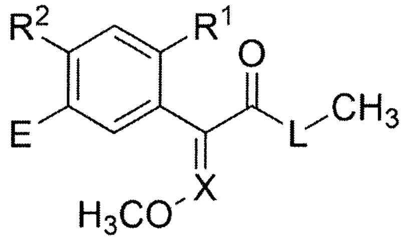

( I - 4 )

[wherein the symbols are the same as defined in [1]].

The compounds according to the Embodiments 88 to 94 can be described also by the formula (I-4).

Next, a process for preparing a compound of the present invention is explained.

Process A

A compound represented by formula (A1) (hereinafter, referred to as Compound (A1)) can be prepared by reacting a compound represented by formula (B1) (hereinafter, referred to as Compound (B1)) with a compound represented by formula (M1) (hereinafter, referred to as Compound (M1)) in the presence of a palladium catalyst and a base.

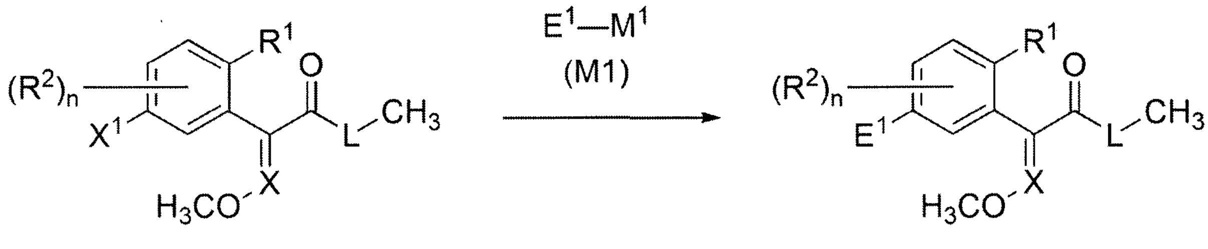


(B1) (A1)

[wherein, $E^1$ represents a C6-C10 aryl group, or a five- to ten- membered aromatic heterocyclic group {the C6-C10 aryl group, and the five- to ten- membered aromatic heterocyclic group each may be optionally substituted with one or more substituents selected from Group A}, $M^1$ represents $B(OH)_2$ or 4,4,5,5-tetramethyl-1,3,2-dioxaborolan-2-yl group, and $X_1$ represents a leaving group such as a chlorine atom, a bromine atom, an iodine atom and a trifuryloxy group, and the other symbols are the same as defined above].

The reaction is usually carried out in a solvent. Examples of the solvent to be used in the reaction include hydrocarbons such as hexane, toluene, and xylene (hereinafter, collectively referred to as hydrocarbons); methyl tert-butyl ether (hereinafter, referred to as MTBE), tetrahydrofuran (hereinafter, referred to as THF), ethers such as dimethoxyethane (hereinafter, collectively referred to as ethers); halogenated hydrocarbons such as chloroform and chlorobenzene (hereinafter, collectively referred to as halogenated hydrocarbons); amides such as dimethylformamide (hereinafter, referred to as DMF) and N-methyl pyrrolidone (hereinafter, referred to as NMP) (hereinafter, collectively referred to as amides); esters such as methyl acetate and ethyl acetate (hereinafter, collectively referred to as esters); nitriles such as acetonitrile and propionitrile (hereinafter, collectively referred to as nitriles); water, and mixed solvents of two or more kinds of the solvents.

Examples of the palladium catalyst to be used in the reaction include palladium catalysts such as tris(dibenzylideneacetone)dipalladium(0) (hereinafter, referred to as $Pd_2(dba)_3$), tetrakis(triphenylphosphine)palladium(0) (hereinafter, referred to as $Pd(PPh_3)_4$), and {1,1'-bis(diphenylphosphino)ferrocene}palladium(II) dichloride (hereinafter, re-

ferred to as $PdCl_2$(dppf)).

Examples of the bases to be used in the reaction include organic bases such as triethylamine and pyridine (hereinafter, referred to as organic bases); alkali metal carbonates such as sodium carbonate and potassium carbonate (hereinafter, referred to as alkali metal carbonates); alkali metal hydrocarbonates (such as sodium hydrocarbonates and potassium hydrocarbonates) (hereinafter, referred to as alkali metal hydrocarbonates); sodium fluoride and tripotassium phosphate.

In the reaction, as needed, a ligand may be used. Examples of the ligand to be used in the reaction include triphenylphosphine, Xantphos, 2,2'-bis(diphenylphoshino)-1,1'-binaphthyl, 1,1'-bis(diphenylphoshino)ferrocene, 2-dicyclohexylphosphino-2',4',6'-triisopropylbiphenyl, 2-dicyclohexylphosphino-2',6'-dimethoxybiphenyl, and 1,2-bis(diphenylphosphino)ethane. When the ligand is used in the reaction, the ligand is used within a range of 0.01 to 1 molar ratio(s) as opposed to 1 mole of the compound (B1).

In the reaction, the compound (M1) is used within a range of 1 to 10 molar ratio(s), and the palladium catalyst is used within a range of 0.01 to 1 molar ratio(s), and the base is used within a range of 1 to 10 molar ratio (s), as opposed to 1 mole of the compound (B1).

The reaction temperature is usually within a range of 0 to 150°C. The reaction period is usually within a range of 0.1 to 120 hours.

When the reaction is completed, water is added to the reaction mixtures, and the reaction mixtures are extracted with organic solvent(s), and the organic solvents are worked up (for example, drying and concentration) to isolate the compound (A1).

The compound (M1) is publicly known, or can be prepared according to a publicly known method.

Process B

The compound (A1) can be prepared by reacting a compound represented by formula (B2) (hereinafter, referred to as Compound (B2)) with a compound represented by formula (M2) (hereinafter, referred to as Compound (M2)) in the presence of a palladium catalyst and a base.

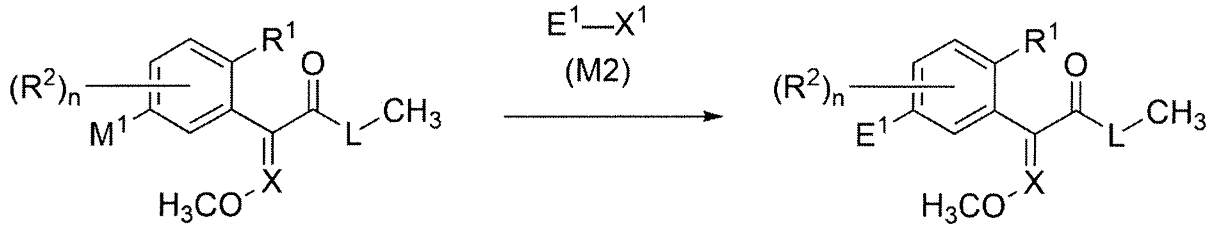


(B2) (A1)

[wherein the symbols are the same as defined above.]

The reaction can be carried out by using the compound (M2) in place of the Compound (B1) and by using the compound (B2) in place of the compound (M1) according to the Process A.

The compound (M2) is publicly known, or can be prepared according to a publicly known method.

Process C

A compound represented by formula (A2) (hereinafter, referred to as Compound (A2)) can be prepared by reacting the Compound (B1) with a compound represented by formula (M3) (hereinafter, referred to as Compound (M3)) in the presence of a metallic catalyst and a base.

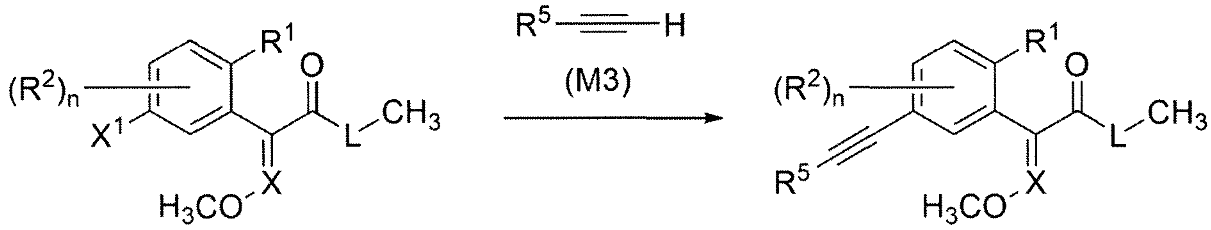


(B1)

(A2)

[wherein the symbols are the same as defined above.]

The reaction is usually carried out in a solvent. Examples of the solvents to be used in the reaction include hydrocarbons, ethers, halogenated hydrocarbons, amides, esters, nitriles, and mixed solvents of two or more kinds of these solvents.

Examples of the metallic catalysts to be used in the reaction include bis(triphenylphosphine)palladium(II) dichloride and copper(I) iodide.

Examples of the bases to be used in the reaction include organic bases.

In the reaction, the compound (M3) is used within a range of 1 to 10 molar ratio(s), and the metallic catalyst is used within a range of 0.01 to 1 molar ratio(s), and the base is used within a range of 1 to 10 molar ratio (s), as opposed to 1 mole of the compound (B1).

The reaction temperature is usually within a range of 0 to 150°C. The reaction period is usually within a range of 0.1 to 120 hours.

When the reaction is completed, water is added to the reaction mixtures, and the reaction mixtures are extracted with organic solvent(s), and the organic solvents are worked up (for example, drying and concentration) to isolate the compound (A2).

The compound (M3) is publicly known, or can be prepared according to a publicly known method.

Process D

A compound represented by formula (A3) (hereinafter, referred to as Compound (A3)) can be prepared by reacting a compound represented by formula (B3) (hereinafter, referred to as Compound (B3)) with a compound represented by formula (M4) (hereinafter, referred to as Compound (M4)) or salts thereof.

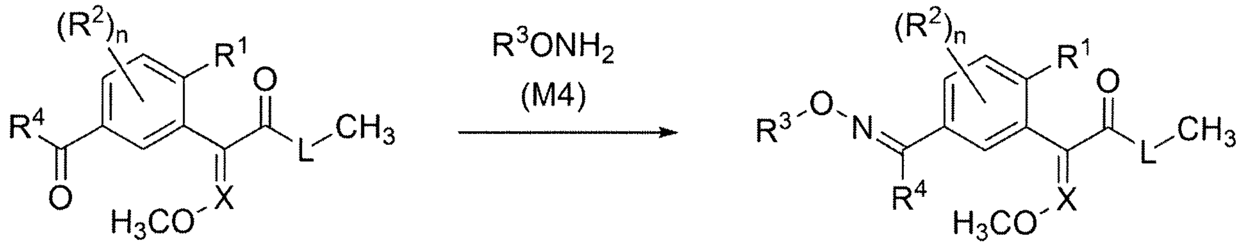


(B3)

(A3)

[wherein the symbols are the same as defined above.]

Examples of the salts of the compound (M4) include hydrochloride salts and sulfate salts thereof.

The reaction is usually carried out in a solvent. Examples of the solvents to be used in the reaction include hydrocarbons; ethers; halogenated hydrocarbons; amides; esters; nitriles; alcohols such as methanol and ethanol (hereinafter, collectively referred to as alcohols); and mixed solvents of two or more kinds of these solvents.

In the reaction, as needed, a base may be added. Examples of the bases to be used in the reaction include organic bases; alkali metal carbonates; alkali metal hydrocarbonates; sodium hydride and tripotassium phosphate. When the base is used in the reaction, the base is usually used within a range of 1 to 10 molar ratio(s), and the base is usually used within a range of 1 to 10 molar ratio(s), as opposed to 1 mole of the compound (B3).

In the reaction, the compound (M4) is usually used within a range of 1 to 10 molar ratio (s) as opposed to 1 mole of the compound (B3).

The reaction temperature is usually within a range of 0 to 150°C. The reaction period is usually within a range of 0.1 to 120 hours.

When the reaction is completed, water is added to the reaction mixtures, and the reaction mixtures are extracted with organic solvent(s), and the organic solvents are worked up (for example, drying and concentration) to isolate the Compound (A3).

Also the compound (A3) can be prepared according to the method described in WO 98/043949 A1.

The compound (M4) is publicly known, or can be prepared according to a publicly known method.

Process E

The compound (A3) can be prepared by reacting a compound represented by formula (B4) (hereinafter, referred to as Compound (B4)) with a compound represented by formula (M5) (hereinafter, referred to as Compound (M5)) in the presence of a base.

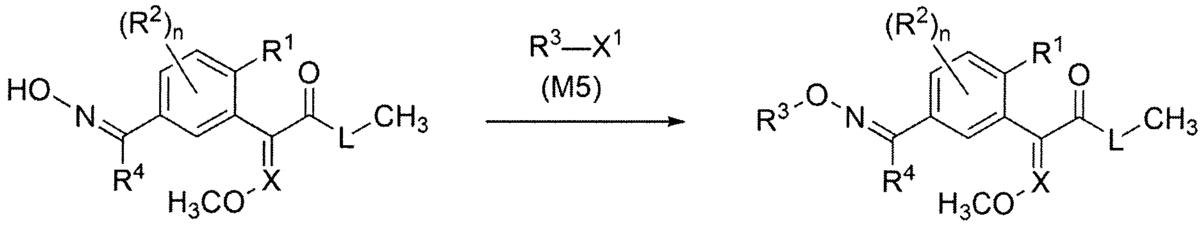


(B4) (A3)

[wherein the symbols are the same as defined above.]

The reaction is usually carried out in a solvent. Examples of the solvent to be used in the reaction include hydrocarbons, ethers, halogenated hydrocarbons, amides, esters, nitriles, and mixed solvents of two or more kinds of these solvents.

Examples of the bases include organic bases, alkali metal carbonates, alkali metal hydrocarbonates, sodium hydride, and tripotassium phosphate.

In the reaction, the compound (M5) is usually used within a range of 1 to 10 molar ratio (s), and the base is usually used within a range of 1 to 10 molar ratio (s), as opposed to 1 mole of the compound (B4).

The reaction temperature is usually within a range of -20 to 150°C. The reaction period is usually within a range of 0.1 to 48 hours.

When the reaction is completed, water is added to the reaction mixtures, and the reaction mixtures are extracted with organic solvent(s), and the organic solvents are worked up (for example, drying and concentration) to isolate the Compound (A3).

The Compound (M5) is publicly known, or can be prepared according to a publicly known method.

Process F

A compound represented by formula (A4) (hereinafter, referred to as Compound (A4)) can be prepared by reacting the Compound (B1) with a compound represented by formula (M6) (hereinafter, referred to as Compound (M6)) in the presence of a metallic catalyst and a base.

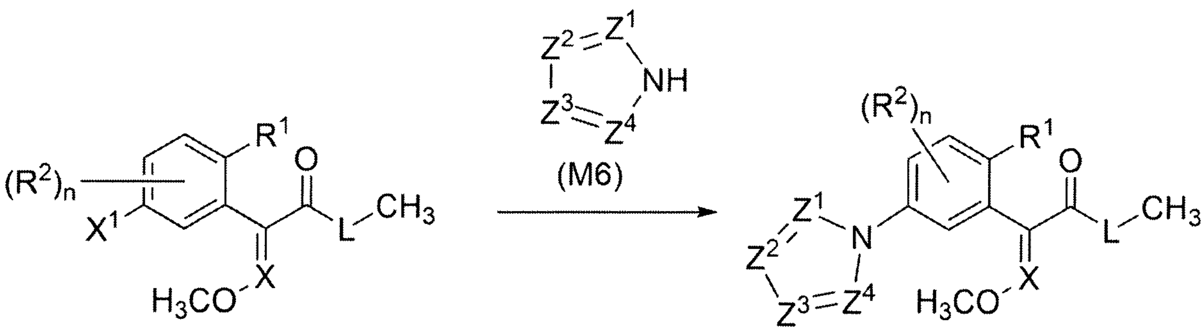


(B1) (A4)

[wherein $Z^1$ represents a nitrogen atom or $CR^{51}$, $Z^2$ represents a nitrogen atom or $CR^{52}$, $Z^3$ represents a nitrogen atom

or $CR^{53}$, $Z^4$ represents a nitrogen atom or $CR^{54}$ (with the proviso that the case where all of $Z^1$, $Z^2$, $Z^3$, and $Z^4$ are a nitrogen atom is excluded), $R^{51}$, $R^{52}$, $R^{53}$, and $R^{54}$ are identical to or different from each other and each represents a C3-C6 cycloalkyl group which may be optionally substituted with one or more substituents selected from Group E, a C1-C6 chain hydrocarbon group, a C1-C3 alkylthio group {the C1-C6 chain hydrocarbon group, and the C1-C3 alkylthio group may be optionally substituted with one or more substituents selected from Group G}, a phenyl group, a five- to six-membered aromatic heterocyclic group {the phenyl group, and the five- to six- membered aromatic heterocyclic group may be optionally substituted with one or more substituents selected from Group F}, $OR^{13}$, $C(O)R^8$, $C(O)OR^8$, $C(O)NR^8R^9$, $NR^8R^9$, $C(R^{11})$=N-$OR^{12}$, a halogen atom, a cyano group, a nitro group, or a hydrogen atom, and the other symbols are the same as defined above.]

The reaction is usually carried out in a solvent. Examples of the solvent to be used in the reaction include ethers, hydrocarbons, amides, water, and mixed solvents of two or more kinds of these solvents.

Examples of the metallic catalysts to be used in the reaction include copper catalysts such as copper(I) iodide, copper(I) bromide, copper(I) chloride, copper(I) oxide, copper(I) trifluoromethanesulfonate benzene complex, tetrakis(acetonitrile) copper(I) hexafluorophosphate, and copper(I) 2-thiophenecarboxylate; and nickel catalysts such as bis (1,5-cyclooctadiene)nickel (0) and nickel(II) chloride.

Examples of the bases include organic bases, alkali metal carbonates, alkali metal hydrocarbonates, sodium fluoride and tripotassium phosphate.

In the reaction, as needed, a ligand and/or an alkali metal halide may be used.

Examples of the ligand include triphenylphosphine, Xantphos, 2,2'-bis(diphenylphoshino)-1,1'-binaphthyl, 1,1'-bis(diphenylphoshino) ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, 1,10-phenanthroline, trans-1,2-cyclohexanediamine, trans-N,N'-dimethylcyclohexane-1,2-diamine, and N,N'-dimethylethylenediamine. When a ligand is used in the reaction, the ligand is usually used within a range of 0.01 to 1 molar ratio (s) as opposed to 1 mole of the compound (B1) .

Examples of the alkali metal halides include potassium fluoride, sodium fluoride, lithium chloride, and sodium chloride. When the alkali metal halide is used in the reaction, the alkali metal halide is usually used within a range of 0.1 to 5 molar ratios as opposed to 1 mole of the compound (B1).

In the reaction, the Compound (M6) is usually used within a range of 1 to 10 molar ratio(s), the metallic catalyst is usually used within a range of 0.01 to 2 molar ratios, and the base is usually used within a range of 1 to 10 molar ratio (s), as opposed to 1 mole of the compound (B1) .

The reaction temperature of the reaction is usually within a range of -20 to 200°C. The reaction period is usually within a range of 0.1 to 48 hours.

When the reaction is completed, water is added to the reaction mixtures, and the reaction mixtures are extracted with organic solvent(s), and the organic solvents are worked up (for example, drying and concentration) to isolate the compound (A4).

The Compound (M6) is publicly known, or can be prepared according to a publicly known method.

Process G

A compound represented by formula (A5) (hereinafter, referred to as Compound (A5)) can be prepared by reacting a compound represented by formula (B5) (referred to as Compound (B5)) with a compound represented by formula (M7) (hereinafter, referred to as Compound (M7)) in the presence of phosphines and azodiesters.

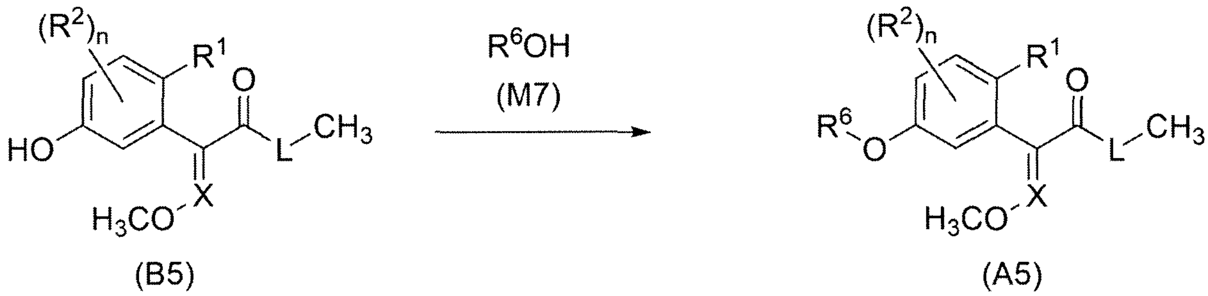


[wherein the symbols are the same as defined above.]

The reaction is usually carried out in a solvent. Examples of the solvent to be used in the reaction include hydrocarbons; ethers; halogenated hydrocarbons; amides; esters; nitriles; and mixed solvents of two or more kinds of these solvents.

Examples of the phosphines include triphenylphosphine and trimethylphosphine.

Examples of the azodiesters include diethyl azodicarboxylate, diisopropyl azodicarboxylate, and bis (2-methoxyethyl) azodicarboxylate.

In the reaction, the Compound (M7) is usually used within a range of 1 to 10 molar ratio(s), the phosphines is usually used within a range of 1 to 10 molar ratio(s), and the azodiesters is usually used within a range of 1 to 10 molar ratio(s), as opposed to 1 mole of the Compound (B5).

The reaction temperature of the reaction is usually within a range of 0 to 150°C. The reaction period is usually within a range of 0.1 to 48 hours.

When the reaction is completed, water is added to the reaction mixtures, and the reaction mixtures are extracted with organic solvent(s), and the organic solvents are worked up (for example, drying and concentration) to isolate the compound (A5).

The compound (M7) is publicly known, or can be prepared according to a publicly known method.

Process H

The compound (A5) can be prepared by reacting the compound (B5) with a compound represented by formula (M8) (hereinafter, referred to as Compound (M8)) in the presence of a base.

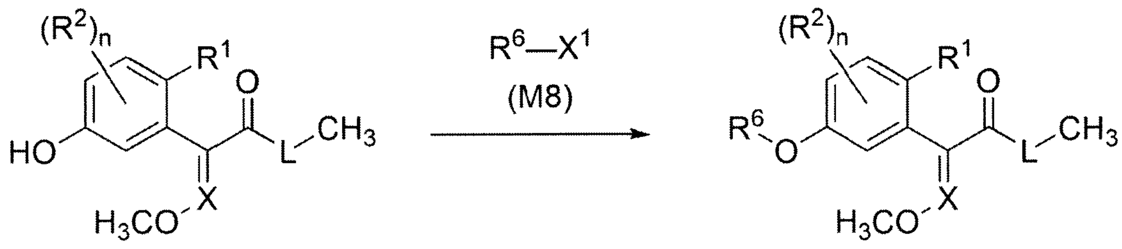


(B5) (A5)

[wherein the symbols are the same as defined above.]

The reaction is usually carried out in a solvent. Examples of the solvent to be used in the reaction include hydrocarbons, ethers, halogenated hydrocarbons, amides, esters, nitriles, and mixed solvents of two or more kinds of these solvents.

Examples of the base include organic bases, alkali metal carbonates, alkali metal hydrocarbonates, sodium hydride and tripotassium phosphate.

In the reaction, the compound (M8) is usually used within a range of 1 to 10 molar ratio (s), and the base is usually used within a range of 1 to 10 molar ratio (s), as opposed to 1 mole of the compound (B5).

The reaction temperature is usually within a range of -20 to 150°C. The reaction period is usually within a range of 0.1 to 48 hours.

When the reaction is completed, the reaction mixtures are extracted with organic solvent (s), and the organic layers are worked up (for example, drying and concentration) to isolate the compound (A5).

The compound (M8) is publicly known, or can be prepared according to a publicly known method.

Process I

A compound represented by formula (A6) (hereinafter, referred to as Compound (A6)) can be prepared by a step of reacting a compound represented by formula (B6) (hereinafter, referred to as Compound (B6)) with a compound represented by formula (M9) (hereinafter, referred to as Compound (M9)) in the presence of a base to obtain a compound represented by formula (B7)) (hereinafter, referred to as Compound (B7)) (hereinafter, the step is referred to as Step (I-1)), followed by a step of reacting the compound (B7) with a compound represented by formula (M10) (hereinafter, referred to as Compound (M10)) in the presence of a base (hereinafter, the step is referred to as Step (I-2)).

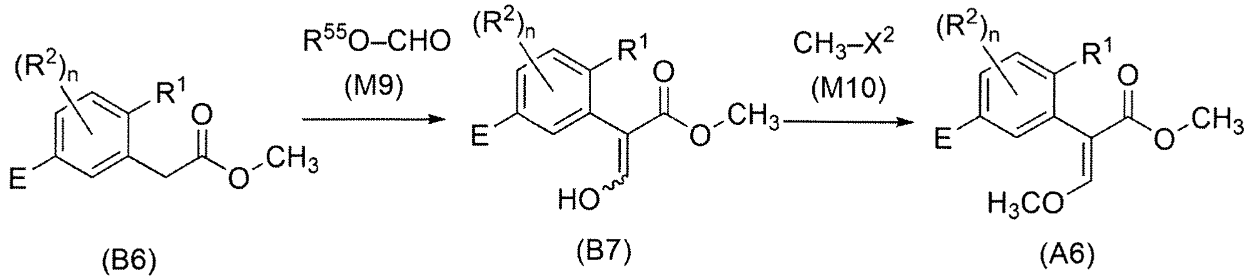


(B6) (B7) (A6)

[wherein $R^{55}$ represents a C1-C4 alkyl group, $X^2$ represents an iodine atom, a methoxysulfonyloxy group, a mesyloxy group, or a tosyloxy group, and the other symbols are the same as defined above.]

The Step (I-1) is usually carried out in a solvent. Examples of the solvent to be used in the reaction include ethers, amides, and mixed solvents of two or more kinds of these solvents.

Examples of the bases to be used in the reaction include sodium hydride.

In the reaction, the compound (M9) is usually used within a range of 1 to 10 molar ratio (s), and the base is usually used within a range of 0.5 to 5 molar ratio(s), as opposed to 1 mole of the compound (B6).

The reaction period is usually within a range of 5 minutes to 72 hours. The reaction temperature is usually within a range of -20 to 100°C.

When the reaction is completed, water is added to the reaction mixtures, and the reaction mixtures are extracted with organic solvent(s), and the organic layers are worked up (for example, drying and concentration) to obtain the compound (B7).

The compound (M9) is a commercially available compound, or can be prepared according to a publicly known method.

The Step (I-2) is usually carried out in a solvent. Examples of the solvent to be used in the reaction include hydrocarbons, ethers, halogenated hydrocarbons, amides, esters, nitriles, and mixed solvents of two or more kinds of these solvents.

Examples of the bases to be used in the reaction include organic bases, alkali metal carbonates, alkali metal hydrocarbonates, and sodium hydride.

In the reaction, the compound (M10) is usually used within a range of 1 to 10 molar ratio (s), and the base is usually used within a range of 1 to 20 molar ratio (s), as opposed to 1 mole of the compound (B7).

The reaction temperature is usually within a range of -20 to 100°C. The reaction period is usually within a range of 0.1 to 48 hours.

When the reaction is completed, water is added to the reaction mixtures, and the reaction mixtures are extracted with organic solvent(s), and the organic layers are worked up (for example, drying and concentration) to isolate the compound (A6).

The compound (M10) is a commercially available compound, or can be prepared according to a publicly known method.

Process K

A compound represented by formula (A7) (hereinafter, referred to as Compound (A7)) can be prepared by a step of reacting a compound represented by formula (B26) (hereinafter, referred to as Compound (B26)) with a compound represented by formula (M12) (hereinafter, referred to as Compound (M12)) in the presence of a base to obtain a compound represented by formula (B9)) (hereinafter, referred to as Compound (B9)) (hereinafter, the step is referred to as Step (K-1)), followed by a step of reacting the compound (B9) with the compound (B10) in the presence of a base (hereinafter, the step is referred to as Step (K-2)).

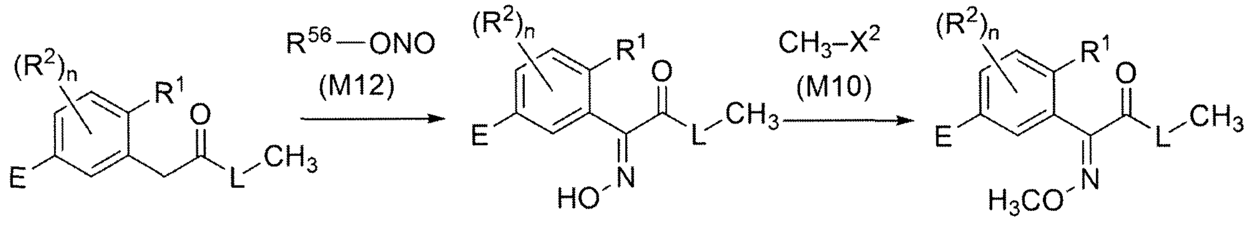


(B26) (B9) (A7)

[wherein $R^{56}$ represents a tert-butyl group or an isopentyl group, and the other symbols are the same as defined above.]

The Step (K-1) is usually carried out in a solvent. Examples of the solvent to be used in the reaction include ethers, amides, alcohols, and mixed solvents of two or more kinds of these solvents.

Examples of the base to be used in the reaction include sodium hydride; and alkali metal alkoxides such as sodium methoxide, sodium ethoxide, potassium t-butoxide (hereinafter, referred to as alkali metal alkoxides).

In the reaction, the compound (M12) is usualy used within a range of 1 to 10 molar ratio (s), and the base is usually used within a range of 1 to 5 molar ratio (s), as opposed to 1 mole of the compound (B6).

The reaction period is usually within a range of 5 minutes to 72 hours. The reaction temperature is usually within a range of -20 to 100°C.

When the reaction is completed, water is added to the reaction mixtures, and the reaction mixtures are extracted with organic solvent(s), and the organic layers are worked up (for example, drying and concentration) to obtain the compound (B9).

The compound (M12) is a commercially available compound, or can be prepared according to a publicly known method.

The step (K-2) can be carried out by using the compound (B9) in place of the compound (B7) according to the step (I-2) of the process I.

Process L

A compound represented by formula (A8) (hereinafter, referred to as Compound (A8)) can be prepared by reacting a compound represented by formula (B10) (hereinafter, referred to as Compound (B10)) with a compound represented by formula (M13) (hereinafter, referred to as Compound (M13)) in the presence of a catalyst and a base.

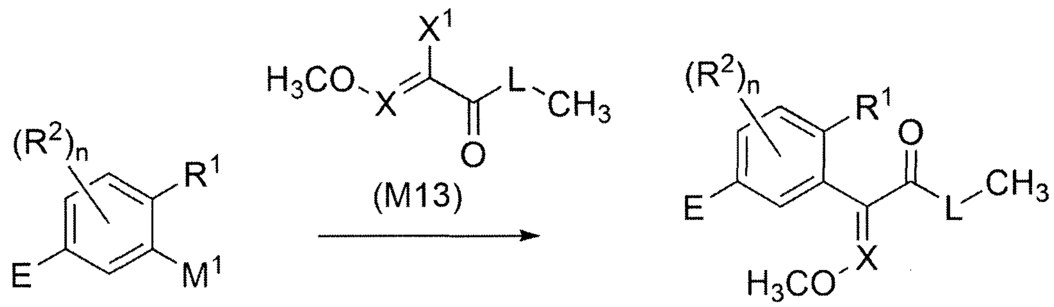

(M13)

(B10) (A8)

[wherein the symbols are the same as defined above.]

The reaction can be carried out by using the compound (M13) in place of the compound (B1), and using the compound (B10) in place of the compound (M1) according to the process A.

The compound (M13) is a publicly known compound, or can be prepared according to the publicly known method.

Process M

A N-oxide of the compound represented by formula (I) can be prepared by reacting the compound represented by formula (I) with an oxidizing agent. The reaction can be carried out according to the method described in U.S. 2018/0009778 A1 or WO 2016/121970 A1.

Reference Process 1

The compound (B7) can be prepared by a step of reacting the compound (B6) with a compound represented by formula (M11) (hereinafter, referred to as Compound (M11)) to obtain a compound represented by formula (B8) (hereinafter, referred to as Compound (B8)) (hereinafter, the step is referred to as Step (1-1)), followed by a step of subjecting the compound (M8) to a reaction in the presence of an acid to obtain the compound (B7) (hereinafter, the step is referred to as Step (1-2)).

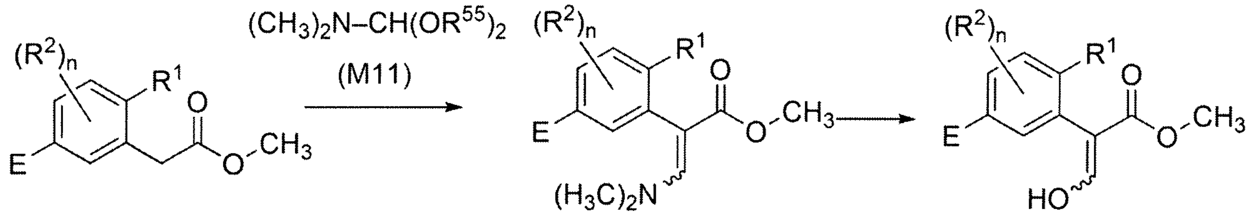


(B6) (B8) (B7)

[wherein the symbols are the same as defined above.]

The step (1-1) is usually carried out in a solvent. Examples of the solvent to be used in the reaction include hydrocarbons, halogenated hydrocarbons, ethers, amides, and mixed solvents of two or more kinds of these solvents.

In the reaction, the compound (M11) is usually used within a range of 1 to 10 molar ratio (s) as opposed to 1 mole of the compound (B6).

The reaction period is usually within a range of 5 minutes to 72 hours. The reaction temperature is usually within a range of -20 to 200°C.

When the reaction is completed, water is added to the reaction mixtures, and the reaction mixtures are extracted with organic solvent(s), and the organic layers are worked up (for example, drying and concentration) to obtain the compound (B8).

The compound (M11) is a commercially available compound, or can be prepared according to a publicly known method.

The step (1-2) is usually carried out in a solvent. Examples of the solvent to be used in the reaction include hydrocarbons, ethers, halogenated hydrocarbons, amides, esters, nitriles, alcohols, water, and mixed solvents of two or more kinds of these solvents.

Examples of the acid to be used in the reaction include hydrochloric acid and acetic acid.

In the reaction, the acid is usually used within a range of 1 to 10 molar ratio (s), and the base is usually used within a range of 1 to 20 molar ratio (s), as opposed to 1 mole of the compound (B8).

The reaction temperature is usually within a range of -20 to 100°C. The reaction period is usually within a range of 0.1 to 48 hours.

When the reaction is completed, water is added to the reaction mixtures, and the reaction mixtures are extracted with organic solvent(s), and the organic layers are worked up (for example, drying and concentration) to isolate the compound (B7).

Reference Process 2

A compound represented by formula (B16) (hereinafter, referred to as Compound (B16)) can be prepared by a step of reacting a compound represented by formula (B11) (hereinafter, referred to as Compound (B11)) with a compound represented by formula (M14) (hereinafter, referred to as Compound (M14)) in the presence of a catalyst and a base to obtain a compound represented by formula (B12) (hereinafter, referred to as compound (B12)) (hereinafter, the step is referred to as Step (2-1)), followed by a step of subjecting the compound (B12) to a reaction in the presence of base to obtain a compound represented by formula (B13) (hereinafter, referred to as Compound (B13)) (hereinafter, the step is referred to as Step (2-2)), and a step of reacting the compound (B13) with the compound (M11) to obtain a compound represented by formula (B14) (hereinafter, referred to as Compound (B14)) (hereinafter, the step is referred to as Step (2-3)), and a step of subjecting the compound (B14) to the reaction in the presence of an acid to obtain a compound represented by formula (B15) (hereinafter, the step is referred to as Step (2-4)), and a step of reacting the compound (B15) with the compound (M10) in the presence of a base (hereinafter, the step is referred to as Step (2-5)).

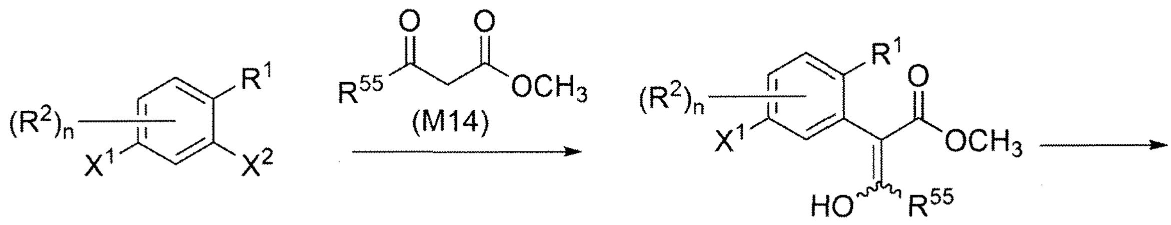


(B11) (B12)

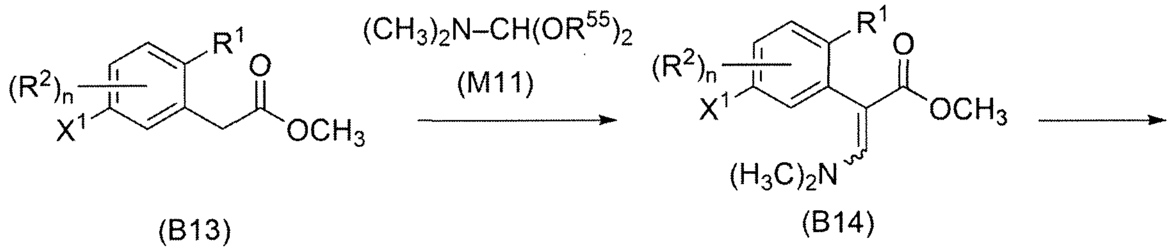


(B13) (B14)

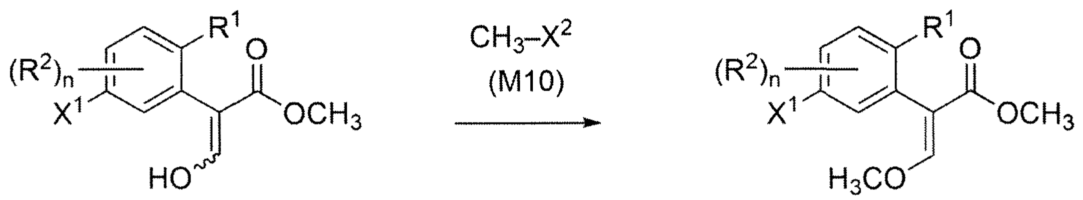


(B15) (B16)

[wherein the symbols are the same as defined above.]

The Step (2-1) is usually carried out in a solvent. Examples of the solvent to be used in the reaction include hydrocarbons, ethers, halogenated hydrocarbons, amides, esters, nitriles, water, and mixed solvents of two or more kinds of these solvents.

Examples of the catalysts to be used in the reaction include palladium catalyst such as palladium(II) acetate, $Pd_2(dba)_2$, $Pd(PPh_3)_4$, and $PdCl_2(dppf)$; and copper catalysts such as copper(I) iodide, copper(I) bromide, copper(I) chloride, copper(I) oxide, trifluoromethanesulfonate copper(I) benzene complex, tetrakis(acetonitrile) copper(I) hexafluorophosphate, and 2-thiophencarboxylate copper(I).

Examples of the base to be used in the reaction include organic bases; alkali metal carbonates; alkali metal hydrocarbonates; alkali metal phosphates such as tripotassium phosphate (hereinafter, referred to as alkali metal phosphates); and acetates such as sodium acetate (hereinafter, referred to as acetates).

In the reaction, as needed, a ligand may be used. Examples of the ligand to be used in the reaction include triphenylphosphine, Xantphos, 2,2'-bis(diphenylphoshino)-1,1'-binaphthyl, 1,1'-bis(diphenylphoshino)ferrocene, 2-dicyclohexylphosphino-2',4',6'-triisopropylbiphenyl, 2-dicyclohexylphosphino-2',6'-dimethoxybiphenyl, 2-di-tert-butylphoshino-2',4',6'-triisopropyl-1,1'-biphenyl, tri-tert-butylphosphine, 1,2-bis(diphenylphosphino)ethane, pyridine-2-carboxlic acid, (L)-proline, trans-1,2-cyclohexane diamine, trans-N,N'-dimethylcyclohexane-1,2-diamine, N,N'-dimethylethylenediamine, N,N-dimethylglycine hydrochloride salt. When the ligand is used in the reaction, the ligand is usually used within a range of 0.01 to 1 molar ratio(s) as opposed to 1 mole of the compound (B11).

In the reaction, the compound (M14) is usually used within a range of 0.1 to 10 molar ratio(s), the catalyst is usually used within a range of 0.01 to 1 molar ratio(s), and the base is usually used within a range of 1 to 10 molar ratio(s), as opposed to 1 mole of the compound (B11).

The reaction period is usually within a range of 5 minutes to 72 hours. The reaction temperature is usually within a range of -20 to 200°C.

When the reaction is completed, water is added to the reaction mixtures, and the reaction mixtures are extracted with organic solvent(s), and the organic layers are worked up (for example, drying and concentration) to isolate the compound (B12).

The compound (M14) is a publicly compound, or can be prepared according to a publicly known method.

The step (2-2) is usually carried out in a solvent. Examples of the solvent to be used in the reaction include

hydrocarbons, ethers, halogenated hydrocarbons, amides, esters, nitriles, alcohols, water, and mixed solvents of two or more kinds of these solvents.

Examples of the base to be used in the reaction include alkali metal phosphates and alkali metal alkoxides.

In the reaction, the base is usually used within a range of 1 to 20 as opposed to 1 mole of the compound (B12).

The step (2-3) can be carried out by using the compound (B13) in place of the compound (B6) according to the step (1-1) of the Reference process 1.

The step (2-4) can be carried out by using the compound (B14) in place of the compound (B8) according to the step (1-2) of the Reference process 1.

The step (2-5) can be carried out by using the compound (B15) in place of the compound (B7) according to the step (2-1) of the Process I.

Reference Process 3

A compound represented by formula (B18) (hereinafter, referred to as Compound (B18)) can be prepared by a step of reacting a compound represented by formula (B27) (hereinafter, referred to as Compound (B27)) with the compound (M12) in the presence of a base to obtain a compound represented by formula (B17) (hereinafter, referred to as Compound (B17)) (hereinafter, the step is referred to as Step (3-1)), followed by a step of reacting the compound (B17) with the compound (M10) in the presence of a base (hereinafter, the step is referred to as Step (3-2)).

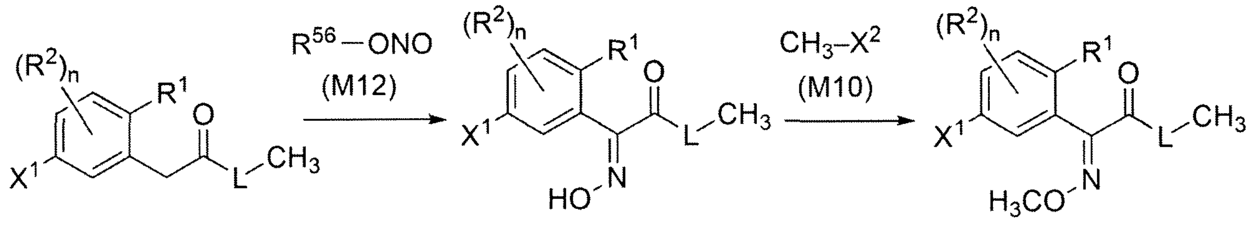


(B27) (B17) (B18)

[wherein the symbols are the same as defined above.]

The step (3-1) can be carried out by using the compound (B27) in place of the compound (B26) according to the step (K-1) of the Process K.

The step (3-2) can be carried out by using the compound (B17) in place of the compound (B9) according to the step (K-2) of the Process K.

Reference Process 4

The compound (B2) can be prepared by reacting the compound (B1) with diboronic acid or diboronate in the presence of a base and a palladium catalyst.

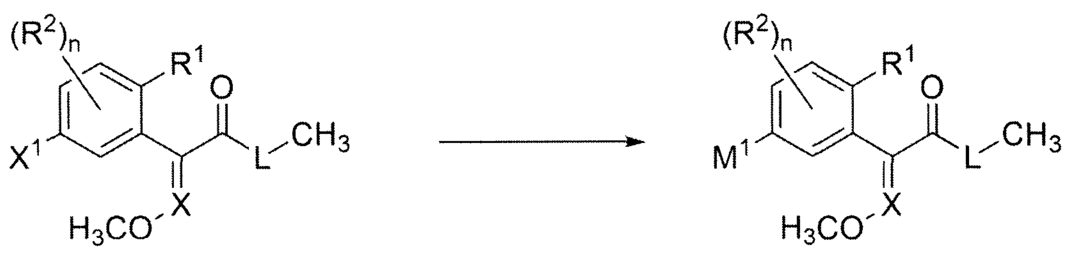

(B1) (B2)

[wherein the symbols are the same as defined above.]

The reaction is usually carried out in a solvent. Examples of the solvent to be used in the reaction include hydrocarbons; ethers; halogenated hydrocarbons; amides; esters; sulfoxides such as dimethyl sulfoxide (hereinafter, referred to as DMSO) (hereinafter, referred to as Sulfoxides); nitriles, and mixed solvents of two or more kinds of these solvents.

Examples of the base to be used in the reaction include organic bases, alkali metal carbonates, alkali metal

hydrocarbonates, acetates, and tripotassium phosphate.

Examples of the palladium catalyst include $PdCl_2$(dppf).

Examples of the diboronic acid or diboronate include bis(pinacolato)diboron, and tetrahydroxydiboron.

In the reaction, the diboronic acid or the diboronate is usually used within a range of 1 to 5 molar ratio(s), the base is usually used within a range of 1 to 5 molar ratio (s), and the palladium catalyst is usually used within a range of 0.01 to 0.5 molar ratios, as opposed to 1 mole of the compound (B1) .

The reaction temperature is usually within a range of 0 to 150°C. The reaction period is usually within a range of 0.1 to 48 hours.

When the reaction is completed, water is added to the reaction mixtures, and the reaction mixtures are extracted with organic solvent(s), and the organic layers are worked up (for example, drying and concentration) to isolate the compound (B2).

Reference Process 5

The compound (B5) can be prepared by oxidizing the compound (B2).

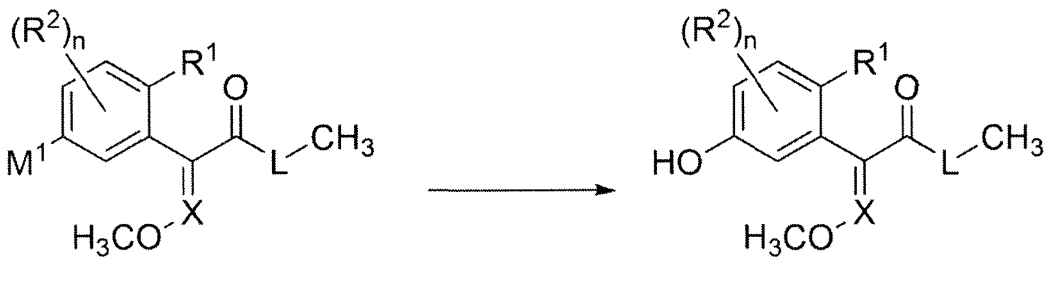

(B2) (B5)

[wherein the symbols are the same as defined above.]

The reaction is usually carried out in a solvent. Examples of the solvent to be used in the reaction include hydrocarbons, ethers, halogenated hydrocarbons, amides, esters, nitriles, alcohols, water, and mixed solvents of two or more kinds of these solvents.

Examples of the oxidizing agent to be used in the reaction include meta-chloroperoxybenzoic acid and hydrogen peroxide water.

When hydrogen peroxide water is used as an oxidizing agent, as needed, a base may be added.

Examples of the base include sodium hydroxide and potassium hydroxide.

In the reaction, the oxidizing agent is usually used within a range of 1 to 5 molar ratio(s) as opposed to 1 mole of the compound (B2).

When the base is used in the reaction, the base is usually used within a range of 0.1 to 5 molar ratio (s) as opposed to 1 mole of the compound (B2).

The reaction temperature is usually within a range of -20 to 120°C. The reaction period is usually within a range of 0.1 to 48 hours.

When the reaction is completed, water and a reducing agent such as sodium thiosulfate are added to the reaction mixtures, and the reaction mixtures are extracted with organic solvent(s), and the organic layers are worked up (for example, drying and concentration) to isolate the compound (B5).

Reference Process 6

The compound (B5) can be prepared by a step of reacting a compound represented by formula (B19) (hereinafter, referred to as Compound (B19)) with a diboronic acid or a diboronate in the presence of a base and a palladium catalyst to obtain a compound represented by formula (B20) (hereinafter, referred to as Compound (B20)) (hereinafter, the step is referred to as Step (6-1)), followed by a step of reacting the compound (B20) with the compound (M13) in the presence of a base and a metallic catalyst (hereinafter, the step is referred to as Step (6-2)).

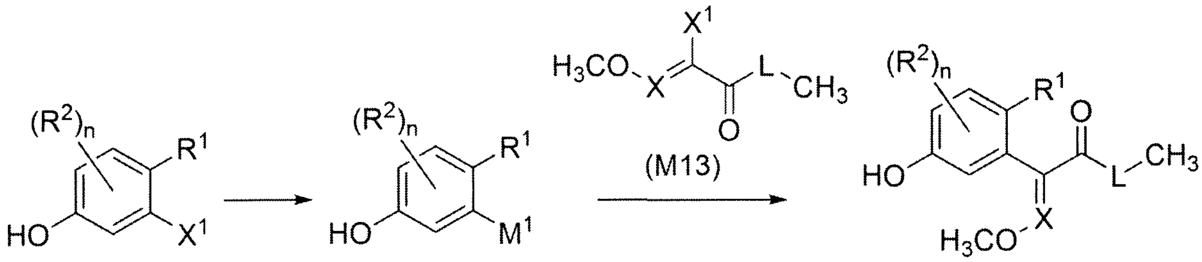

(M13)

(B19) (B20) (B5)

[wherein the symbols are the same as defined above.]

The step (6-1) can be carried out by using the compound (B19) in place of the compound (B1) according to the Reference process 4.

The compound (B19) is a publicly known compound, or can be prepared according to the publicly known method.

The step (6-2) can be carried out by using the compound (B20) in place of the compound (M1) and using the compound (M13) in place of the compound (B1) according to the Process A.

Reference Process 7

A compound represented by formula (B21) (hereinafter, referred to as compound (B21)) can be prepared by reacting the compound (B5) with a trifluoroacetic anhydride in the presence of a base.

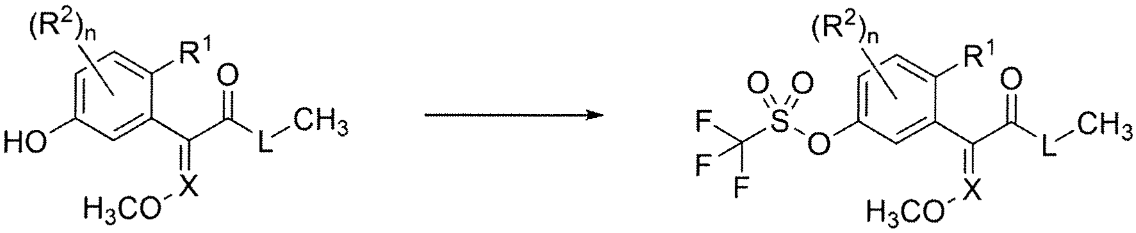

(B5) (B21)

[wherein the symbols are the same as defined above.]

The reaction is usually carried out in a solvent. Examples of the solvent to be used in the reaction include hydrocarbons, ethers, halogenated hydrocarbons, amides, esters, nitriles, and mixed solvents of two or more kinds of these solvents.

Examples of the base include organic bases.

In the reaction, the trifluoroacetic anhydride is usually used within a range of 1 to 10 molar ratio(s), and the base is usually used within a range of 1 to 10 molar ratio(s), as opposed to 1 mole of the Compound (B5).

The reaction temperature is usually within a range of -20 to 100°C. The reaction period is usually within a range of 0.1 to 48 hours.

Reference Process 8

The compound (B22) can be prepared by reacting the compound (B1) with a compound represented by formula (M15) (hereinafter, referred to as Compound (M15)).

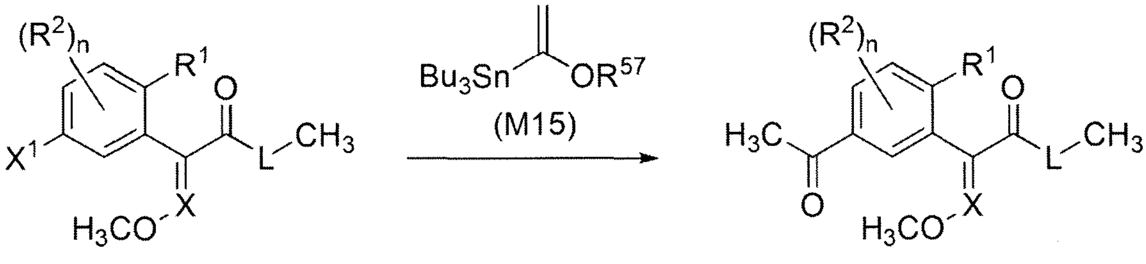


(B1) (B22)

[wherein $R^{57}$ represents a methyl group or an ethyl group, and the other symbols are the same as defined above.]

The reaction can be carried out, for example, according to the method described in WO 2016/123253 A1.

The compound (M15) is a commercially available compound, or can be prepared according to a publicly known method.

Reference Process 9

The compound (B4) can be prepared by reacting the compound (B3) with hydroxylamine or salts thereof.

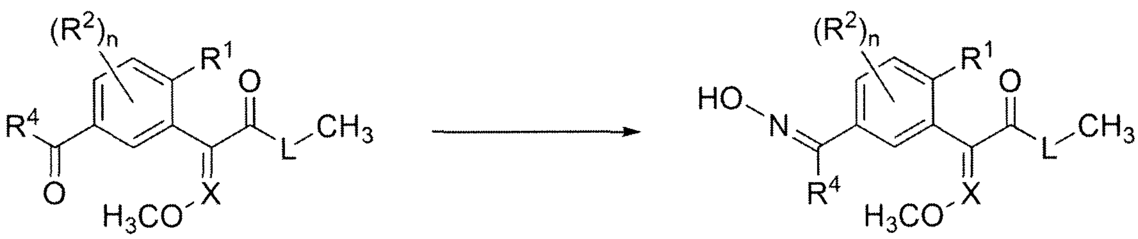

(B3) (B4)

[wherein the symbols are the same as defined above.]

Examples of a salt of the hydroxylamine include hydrochloride salt and sulfate.

The reaction can be carried out by using hydroxylamine or salts thereof in place of the compound (M4) according to the Process D.

Reference Process 10

A compound represented by formula (B23) can be prepared by reacting the compound (B1) with N-formylsaccharin in the presence of a palladium catalyst, a ligand, triethylsilane, and a base.

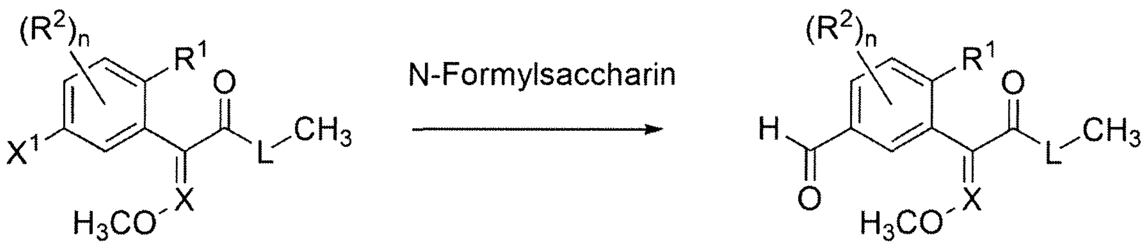


(B1) (B23)

[wherein the symbols are the same as defined above.]

The reaction can be carried out according to the method described in Angew. Chem. Int. Ed., 2013, 52, 8611-8615 and the others.

Reference Process 11

A compound represented by formula (B24) can be prepared by reacting the compound (B27) with the compound (M1) in the presence of a palladium catalyst and a base.

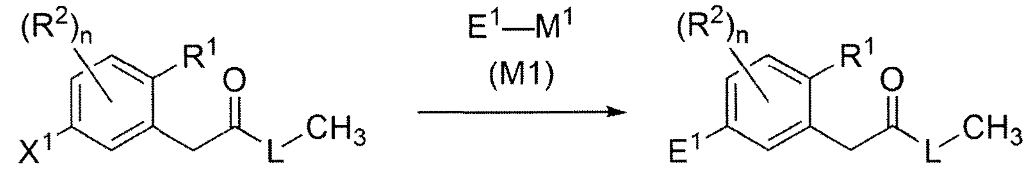


(B27) (B24)

[wherein the symbols are the same as defined above.]

The reaction can be carried out by using the compound (B27) in place of the compound (B1) according to the Process A.

Reference Process 12

A compound represented by formula (B25) can be prepared by reacting the compound (B27) with the compound (M3) in the presence of a metallic catalyst and a base.

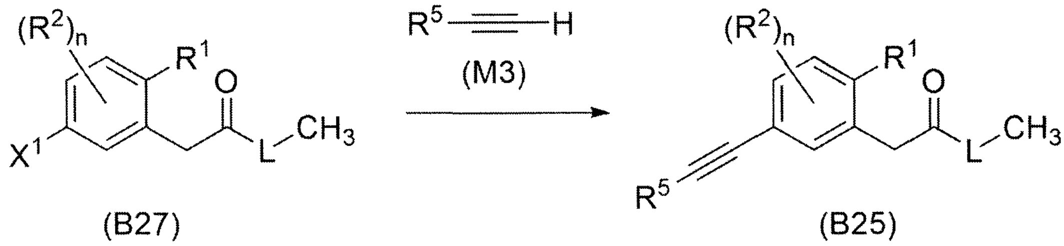


(B27) (B25)

[wherein the symbols are the same as defined above.]

The reaction can be carried out by using the compound (B27) in place of the compound (B1) according to the Process C.

The Present compound may be mixed with or used in combination with one or more ingredient(s) selected from the group consisting of the following Group (a), Group (b), Group (c), and Group (d) (hereinafter referred to as "Present ingredient").

When the Present compound is mixed with or used in combination with the Present ingredient, they are used simultaneously, separately, or at time intervals with each other.

When the Present compound is used simultaneously with the Present ingredient, the Present compound and the Present ingredient may be contained in separate formulations or contained in one formulation.

One aspect of the present invention provides a composition comprising one or more ingredient(s) selected from the group consisting of Group (a), Group (b), Group (c), and Group (d), and the Present compound (hereinafter referred to as "Composition A").

Group (a) is a group consisting of acetylcholinesterase inhibitors (for example, carbamate insecticides and organophosphate insecticides), GABA-gated chloride channel blockers (for example, phenylpyrazole insecticides), sodium channel modulators (for example, pyrethroid insecticides), nicotinic acetylcholine receptor competitive modulators (for example, neonicotinoid insecticides), nicotinic acetylcholine receptor allosteric modulators, glutamate-gated chloride channel allosteric modulators (for example, macrolide insecticides), juvenile hormone mimics, multisite inhibitors, chordotonal organ TRPV channel modulators, mite growth inhibitors, microbial disruptors of insect midgut membranes, inhibitors of mitochondrial ATP synthase, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, mitochondrial complexes I, II, III, and IV electron transport inhibitors, voltage-dependent sodium channel blockers, inhibitors of acetyl CoA carboxylase, ryanodine receptor modulators (for example, diamide insecticides), chordotonal organ modulators, and microbial insecticides, and other insecticidal active ingredients, miticidal active ingredients, and nematicidal active ingredients. These ingredients are described in the classification on the basis of action mechanism by IRAC.

Group (b) is a group consisting of nucleic acids synthesis inhibitors (for example, phenylamide fungicides and acylamino acid fungicides), cell division and cytoskeleton inhibitors (for example, MBC fungicides), respiration inhibitors (for example, QoI fungicides, and QiI fungicides), amino acids synthesis and protein synthesis inhibitors (for example, anilino-pyrimidine fungicides), signal transduction inhibitors, lipid synthesis and membrane synthesis inhibitors, sterol biosynthesis inhibitors (for example, DMI fungicides such as triazole fungicides), cell wall biosynthesis inhibitors, melanin synthesis inhibitors, plant defense inducers, fungicides with multi-site contact activity, microbial fungicides, and other fungicidal active ingredients. These ingredients are described in the classification on the basis of action mechanism by FRAC.

Group (c) is a group of plant growth regulatory ingredients (including mycorrhizal fungi and root nodule bacteria).

Group (d) is a group of repellent ingredients.

Hereinafter, examples of the combination of the Present ingredient and the Present compound are described. For example, "alanycarb + SX" indicates a combination of alanycarb and SX.

The abbreviation of "SX" indicates any one of the Present compound selected from the Compound groups SX1 to SX259 described in Examples. Also, all of the following Present ingredient are known ingredients, and may be obtained from commercially available formulations, or may be prepared by known methods. When the Present ingredient is a microorganism, it may also be available from a bacterial authority depository. Further, the number in parentheses represents the CAS RN (registered trademark).

Combinations of the Present ingredient in the above Group (a) and the Present compound:
abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alpha-cypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, bark of Celastrus angulatus + SX, bendiocarb + SX, benfluthrin + SX, benfuracarb + SX, bensultap + SX, benzoximate + SX, benzpyrimoxan + SX, beta-cyfluthrin + SX, beta-cypermethrin + SX, bifenazate + SX, bifenthrin + SX, bioallethrin + SX, bioresmethrin + SX, bistrifluron + SX, borax + SX, boric acid + SX, broflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cyclaniliprole + SX, cyclobutrifluram + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphenothrin + SX, cyproflanilide + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC(2-methyl-4,6-dinitrophenol) + SX, doramectin + SX, dried leaves of Dryopteris filix-mas + SX, emamectin-benzoate + SX, empenthrin + SX, endosulfan + SX, EPN(O-ethyl O-(4-nitrophenyl) phenylphosphonothioate) + SX, epsilon-metofluthrin + SX, epsilon-momfluorothrin + SX, esfenvalerate + SX, ethiofencarb + SX, ethion + SX, ethiprole + SX, ethoprophos + SX, etofenprox + SX, etoxazole + SX, extract of Artemisia absinthium + SX, extract of Azadirachta indica + SX, extract of Cassia nigricans + SX, extract of clitoria ternatea + SX, extract of Symphytum officinale + SX, extracts or simulated blend of Chenopodium ambrosioides + SX, extract of Tanacetum vulgare + SX, extract of Urtica dioica + SX, extract of Viscum album + SX, famphur + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, fluchlordiniliprole + SX, flucycloxuron + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, fluralaner + SX, fluvalinate + SX, fluxametamide + SX, formetanate + SX, fosthiazate + SX, furamethrin + SX, furathiocarb + SX, gamma-cyhalothrin + SX, GS-omega/kappa HXTX-Hv1a peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoprocarb + SX, isopropyl-O-(methoxyaminothiophosphoryl) salicylate + SX, isoxathion + SX, ivermectin + SX, kadethrin + SX, kappa-tefluthrin + SX, kappa-bifenthrin + SX, kinoprene + SX, lambda-cyhalothrin + SX, lenoremycin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, momfluorothrin + SX, monocrotophos + SX, moxidectin + SX, naled + SX, nicofluprole + SX, nicotine + SX, nicotine-sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pirimicarb + SX, pirimiphos-methyl + SX, prallethrin + SX, profenofos + SX, profluthrin + SX, propargite + SX, propetamphos + SX, propoxur + SX, propylene glycol alginate + SX, prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, ryanodine + SX, sarolaner + SX, selamectin + SX, sigma-cypermethrin + SX, silafluofen + SX, sodium borate + SX, sodium metaborate + SX, spidoxamat + SX, spinetoram + SX, spinosad + SX, spirodiclofen + SX, spiromesifen + SX, spiropidion + SX, spirotetramat + SX, sulfluramid + SX, sulfotep + SX, sulfoxaflor + SX, sulfur + SX, sulfuryl fluoride + SX, tartar emetic + SX, tau-fluvalinate + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioides near ambrosioides + SX, tetrachlorantraniliprole + SX, tetrachlorvinphos + SX, tetradifon + SX, tetramethrin + SX, tetramethylfluthrin + SX, tetraniliprole + SX, theta-cypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium + SX, tioxazafen + SX, tolfenpyrad + SX, tralomethrin + SX, transfluthrin + SX, triazamate + SX, triazophos + SX, trichlorfon + SX, trifluenfuronate + SX, triflumezopyrim + SX, triflumuron + SX, trimethacarb + SX, tyclopyrazoflor + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050-20-3) + SX, 3-methoxy-N-(5-{5-(trifluoromethyl)-5-[3-(trifluoromethyl)phenyl]-4,5-dihydro-1,2-oxazol-3-yl}indan-1-yl)propanamide (1118626-57-5) + SX, 2-({2-fluoro-4-methyl-5-[(2,2,2-trifluoroethyl)sulfinyl]phenyl}imino)-3-(2,2,2-trifluoroethyl)-1,3-thiazolidin-4-one (1445683-71-5) + SX, (2Z)-2-({2-fluoro-4-methyl-5-[(R)-(2,2,2-trifluoroethyl)sulfinyl]phenyl}imino)-3-(2,2,2-trifluoroethyl)-1,3-thiazolidin-4-one (2377084-09-6) + SX, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboxamide (1400768-21-9) + SX, N-[3-chloro-1-(pyridin-3-yl)-1H-pyrazol-4-yl]-2-(methanesulfonyl)propanamide (2396747-83-2) + SX, 1,4-dimethyl-2-[2-(pyridin-3-yl)-2H-indazol-5-yl]-1,2,4-triazolidine-3,5-dione (2171099-09-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl]phenyl}methyl)cyclopropanecarboxamide + SX, 7-fluoro-N-[1-(methylsulfanyl)-2-methylpropan-2-yl]-2-(pyridin-3-yl)-2H-indazole-4-carboxamide + SX, 7-fluoro-N-[1-(methanesulfinyl)-2-methylpropan-2-yl]-2-(pyridin-3-yl)-2H-indazole-4-carboxamide + SX, 7-fluoro-N-[1-(methanesulfonyl)-2-methylpropan-2-yl]-2-(pyridin-3-yl)-2H-indazole-4-carboxamide + SX, N-[1-(difluoromethyl)cyclopropyl]-2-(pyridin-3-yl)-2H-indazole-4-carboxamide + SX, 2,9-dihydro-9-(methoxymethyl)-2-(pyridin-3-yl)-10H-pyrazolo[3,4-f]pyrido[2,3-b][1,4]oxazepin-10-one (2607927-97-7) + SX, BT crop protein Cry1Ab + SX, BT crop protein Cry1Ac + SX, BT crop protein Cry1Fa + SX, BT crop protein Cry1A.105 + SX, BT crop protein Cry2Ab + SX, BT crop protein Vip3A + SX, BT crop protein mCry3A + SX, BT crop protein Cry3Ab + SX, BT crop protein Cry3Bb + SX, BT crop protein Cry34Ab1/Cry35Ab1 + SX, Adoxophyes orana granulosis virus strain BV-0001 + SX, Anticarsia gemmatalis mNPV + SX, Autographa californica mNPV + SX, Cydia pomonella GV strain V15 + SX, Cydia pomonella GV strain V22 + SX, Cryptophlebia leucotreta GV + SX, Dendrolimus punctatus cypovirus + SX, Helicoverpa armigera NPV strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua mNPV + SX, Spodoptera littoralis mNPV + SX, Spodoptera litura NPV + SX, Arthrobotrys dactyloides + SX, Bacillus firmus strain GB-126 + SX, Bacillus firmus strain I-1582 + SX, Bacillus firmus strain NCIM2637 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 serotype H5a5b + SX, Bacillus sphaericus strain ABTS1743 + SX, Bacillus thuringiensis strain AQ52 + SX, Bacillus thuringiensis strain BD#32 + SX, Bacillus thuringiensis strain CR-371 + SX, Bacillus thuringiensis subsp. Aizawai strain ABTS-1857 + SX, Bacillus thuringiensis subsp. Aizawai strain AM65-52 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype strain H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain CCT1306) + SX, Bacillus thuringiensis subsp. Kurstaki strain EG2348 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL002 + SX, Bacillus thuringiensis subsp. morrisoni + SX, Bacillus thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain

M-7 + SX, Bacillus thuringiensis var. 7216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain GHA + SX, Beauveria brongniartii + SX, Burkholderia rinojensis strain A396 + SX, Chromobacterium subtsugae strain PRAA4-1T + SX, Dactyllela ellipsospora + SX, Dectylaria thaumasia + SX, Hirsutella minnesotensis + SX, Hirsutella rhossiliensis + SX, Hirsutella thompsonii + SX, Lagenidium giganteum + SX, Lecanicillium lecanii strain KV01 + SX, Lecanicillium lecanii conidia of strain DAOM198499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX, Pasteuria nishizawae strain Pn1 + SX, Pasteuria penetrans + SX, Pasteuria usgae + SX, Pasteuria thornei + SX, Serratia entomophila + SX, Verticillium chlamydosporium + SX, Verticillium lecani strain NCIM1312 + SX, Wolbachia pipientis + SX.

Combination of the Present ingredient in the above Group (b) and the Present compound:

acibenzolar-S-methyl + SX, aldimorph + SX, ametoctradin + SX, aminopyrifen + SX, amisulbrom + SX, anilazine + SX, azaconazole + SX, azoxystrobin + SX, basic copper sulfate + SX, benalaxyl + SX, benalaxyl-M + SX, benodanil + SX, benomyl + SX, benthiavalicarb + SX, benthiavalicarb-isopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroinconazide + SX, chloroneb + SX, chlorothalonil + SX, chlozolinate + SX, colletochlorin B + SX, copper(II) acetate + SX, copper(II) hydroxide + SX, copper oxychloride + SX, copper(II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper(II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract of Melaleuca alternifolia + SX, extract of Reynoutria sachalinensis + SX, extract of the cotyledons of lupine plantlets ("BLAD") + SX, extract of Allium sativum + SX, extract of Equisetum arvense + SX, extract of Tropaeolum majus + SX, famoxadone + SX, fenamidone + SX, fenaminstrobin + SX, fenarimol + SX, fenbuconazole + SX, fenfuram + SX, fenhexamid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin + SX, fluoxastrobin + SX, fluoxytioconazole + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, guazatine + SX, hexaconazole + SX, hymexazole + SX, imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, Quillaja extract + SX, quinconazole + SX, quinofumelin + SX, quinoxyfen + SX, quintozene + SX, Saponins of Chenopodium quinoa + SX, seboctylamine + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole

+ SX, triforine + SX, triticonazole + SX, validamycin + SX, valifenalate + SX, vinclozolin + SX, yellow mustard powder + SX, zinc thiazole + SX, zineb + SX, ziram + SX, zoxamide + SX, N'-[4-({3-[(4-chlorophenyl)methyl]-1,2,4-thiadiazol-5-yl}oxy)-2,5-dimethylphenyl]-N-ethyl-N-methylmethanimidamide (1202781-91-6) + SX, N'-{4-[(4,5-dichlorothiazol-2-yl)oxy]-2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethanimidamide (2055589-28-9) + SX, N'-[2-chloro-4-(2-fluorophenoxy)-5-methylphenyl]-N-ethyl-N-methylmethanimidamide (2055756-21-1) + SX, N'-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine (1362477-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenylacrylate (39491-78-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R, 2S, 5S)-5-(4-chlorobenzyl)-2-(chloromethyl)-2-methyl-1-(1H-1,2,4-triazol-1-ylmethyl)cyclopentan-1-ol (1801930-06-2) + SX, (1S, 2R, 5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R, 2S, 5S)-2-(chloromethyl)-5-(4-fluorobenzyl)-2-methyl-1-(1H-1,2,4-triazol-1-ylmethyl)cyclopentan-1-ol (1801930-08-4) + SX, (1S, 2R, 5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl]ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-02-9) + SX, 2-(difluoromethyl)-N-[3-propyl-1,1-dimethyl-2,3-dihydro-1H-inden-4-yl]pyridine-3-carboxamide (1847460-05-2) + SX, (2E,3Z)-5-{[1-(4-chlorophenyl)-1H-pyrazol-3-yl]oxy}-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, N-(1-benzyl-1,3-dimethylbutyl)-8-fluoroquinoline-3-carboxamide (2132414-04-9) + SX, N-(1-benzyl-3,3,3-trifluoro-1-methylpropyl)-8-fluoroquinoline-3-carboxamide (2132414-00-5) + SX, 4,4-dimethyl-2-({4-[5-(trifluoromethyl)-1,2,4-oxadiazol-3-yl]phenyl}methyl)isoxazolidin-3-one (2098918-25-1) + SX, 5,5-dimethyl-2-({4-[5-(trifluoromethyl)-1,2,4-oxadiazol-3-yl]phenyl}methyl)isoxazolidin-3-one (2098918-26-2) + SX, N-ethyl-2-methyl-N-({4-[5-(trifluoromethyl)-1,2,4-oxadiazol-3-yl]phenyl}methyl)propanamide + SX, N,2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl)cyclopropanecarboxamide + SX, N-methoxy-N'-methyl-N-({4-[5-(trifluoromethyl)-1,2,4-oxadiazol-3-yl]phenyl}methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2,4-oxadiazol-3-yl]phenyl}methyl)urea + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, 3-(4-bromo-7-fluoroindol-1-yl)butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl}-L-alaninate + SX, (1S)-1-[1-(naphthalen-1-yl)cyclopropyl]ethyl N-[(3-hydroxy-4-methoxypyridin-2-yl)carbonyl]-L-alaninate + SX, (1S)-1-[1-(naphthalen-1-yl)cyclopropyl]ethyl N-[(3-acetoxy-4-methoxypyridin-2-yl)carbonyl]-L-alaninate + SX, (1S)-1-[1-(naphthalen-1-yl)cyclopropyl]ethyl N-{[3-(acetoxymethoxy)-4-methoxypyridin-2-yl]carbonyl}-L-alaninate + SX, N-({4-[5-(trifluoromethyl)-1,2,4-oxadiazol-3-yl]phenyl}methyl)cyclopropanecarboxamide + SX, N-allyl-N-({4-[5-(trifluoromethyl)-1,2,4-oxadiazol-3-yl]phenyl}methyl)acetamide + SX, N-allyl-N-({4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl}methyl)propanamide + SX, 3,3,3-trifluoro-N-({2-fluoro-4-[5-(trifluoromethyl)-1,2,4-oxadiazol-3-yl]phenyl}methyl)propanamide + SX, 3,3,3-trifluoro-N-({3-fluoro-4-[5-(trifluoromethyl)-1,2,4-oxadiazol-3-yl]phenyl}methyl)propanamide + SX, 3,3,3-trifluoro-N-({2,3-difluoro-4-[5-(trifluoromethyl)-1,2,4-oxadiazol-3-yl]phenyl}methyl)propanamide + SX, N-({2,3-difluoro-4-[5-(trifluoromethyl)-1,2,4-oxadiazol-3-yl]phenyl}methyl)butanamide + SX, N-methoxy-N-methyl-N'-({4-[5-(trifluoromethyl)-1,2,4-oxadiazol-3-yl]phenyl}methyl)urea + SX, N,N-diethyl-N'-({4-[5-(trifluoromethyl)-1,2,4-oxadiazol-3-yl]phenyl}methyl)urea + SX, N-methyl-N'-({4-[5-(trifluoromethyl)-1,2,4-oxadiazol-3-yl]phenyl}methyl)urea + SX, 1-({4-[5-(trifluoromethyl)-1,2,4-oxadiazol-3-yl]phenyl}methyl)pyrrolidin-2-one + SX, 1-({4-[5-(trifluoromethyl)-1,2,4-oxadiazol-3-yl]phenyl}methyl)piperidin-2-one + SX, 4-({4-[5-(trifluoromethyl)-1,2,4-oxadiazol-3-yl]phenyl}methyl)morpholin-3-one + SX, 2-({4-[5-(trifluoromethyl)-1,2,4-oxadiazol-3-yl]phenyl}methyl)isoxazolidin-3-one + SX, 3,3-dimethyl-1-({4-[5-(trifluoromethyl)-1,2,4-oxadiazol-3-yl]phenyl}methyl)piperidin-2-one + SX, 2-({4-[5-(trifluoromethyl)-1,2,4-oxadiazol-3-yl]phenyl}methyl)-1,2-oxazinan-3-one + SX, 1-({3-fluoro-4-[5-(trifluoromethyl)-1,2,4-oxadiazol-3-yl]phenyl}methyl)azepan-2-one + SX, 4,4-dimethyl-1-({4-[5-(trifluoromethyl)-1,2,4-oxadiazol-3-yl]phenyl}methyl)pyrrolidin-2-one + SX, 5-methyl-1-({4-[5-(trifluoromethyl)-1,2,4-oxadiazol-3-yl]phenyl}methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-({4-[5-(trifluoromethyl)-1,2,4-oxadiazol-3-yl]phenyl}methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-({4-[5-(trifluoromethyl)-1,2,4-oxadiazol-3-yl]phenyl}methyl)-1H-pyrazole-4-carboxamide + SX, N,N-dimethyl-1-({4-[5-(trifluoromethyl)-1,2,4-oxadiazol-3-yl]phenyl}methyl)-1H-1,2,4-triazol-3-amine + SX, N-methyl-4-[5-(trifluoromethyl)-1,2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl]-4,4-difluoro-3,3-dimethyl-3,4-dihydroisoquinoline + SX, 2,2-difluoro-N-[6-({[1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl)-2,2-difluoroethyl]-5-methylpyridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-5-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl)(2,6-difluoropyridin-4-yl)amino]-N-(2,2-dimethylcyclobutyl)-5-methylthiazole-4-carboxamide + SX, 2-[(2-methylpropanoyl)(2,6-difluoropyridin-4-yl)amino]-N-(2,2-dimethylcyclobutyl)-5-methylthiazole-4-carboxamide + SX, 5-[5-(difluoromethyl)-1,3,4-oxadiazol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl]pyrimidin-2-amine + SX, 5-[5-(difluoromethyl)-1,3,4-oxadiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oxadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-{[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (5S)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, Agrobacterium radiobactor strain K1026 + SX, Agrobacterium radiobactor strain K84 + SX, Bacillus amyloliquefaciens strain PTA-4838 (Aveo(registered trademark) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp. plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 + SX, Bacillus simplex strain CGF2856 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus

subtilis strain QST713 + SX, Bacillus subtilis strain QST714 + SX, Bacillus subtilis var. Amyloliquefaciens strain FZB24 + SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyxa strain AC-1 + SX, Paenibacillus polymyxa strain BS-0105 + SX, Pantoea agglomerans strain E325 + SX, Phlebiopsis gigantea strain VRA1992 + SX, Pseudomonas aureofaciens strain TX-1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX, Pseudomonas fluorescens strain A506 + SX, Pseudomonas fluorescens strain CL145A + SX, Pseudomonas fluorescens strain G7090 + SX, Pseudomonas sp. strain CAB-02 + SX, Pseudomonas syringae strain 742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 + SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G-41 + SX, Trichoderma virens strain GL-21 + SX, Trichoderma viride + SX, Variovorax paradoxus strain CGF4526 + SX, Harpin protein + SX.

Combination of the Present ingredient in the above Group (c) and the Present compound:
1-methylcyclopropene + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, paclobutrazol + SX, pendimethalin + SX, prohexadione-calcium + SX, prohydrojasmon + SX, pyraflufen-ethyl + SX, sintofen + SX, sodium 1-naphthaleneacetate + SX, sodium cyanate + SX, thidiazuron + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butylate + SX, methyl 5-(trifluoromethyl)benzo[b]thiophene-2-carboxylate + SX, 3-[(6-chloro-4-phenylquinazolin-2-yl)amino]propan-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus intraradices RTI-801 + SX, Rhizophagus irregularis DAOM 197198 + SX, Azorhizobium caulinodans + SX, Azospirillum amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMIA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY209 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, Zucchini Yellow Mosaik Virus weak strain + SX.

Combination of the Present ingredient in the above Group (d) and the Present compound:
anthraquinone + SX, deet + SX, icaridin + SX.

The ratio of the Present compound to the Present ingredient includes, but not limited thereto, as a ratio by weight (the Present compound : the Present ingredient) 1,000:1 to 1:1,000, 500:1 to 1:500, 100:1 to 1:100, 50:1, 20:1,

10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:20, and 1:50, and the others.

The Present compound has an efficacy against pests. Examples of the pests include plant phytopathogenic microorganism, harmful arthropods such as harmful insects and harmful mites, harmful nematicides, and harmful mollusks.

The Present compound can control plant diseases caused by plant pathogenic microorganisms such as fungi, Oomycete, Phytomyxea, and bacteria. Examples of the fungi include Ascomycota, Basidiomycota, Blastocladiomycota, Chytridiomycota, Mucoromycota, and Olpidiomycota. Specific examples thereof include the followings. The scientific name of plant pathogenic microorganism which causes each disease is shown in parentheses.

Rice diseases:
blast (Pyricularia oryzae), brown spot (Cochliobolus miyabeanus), sheath blight (Rhizoctonia solani), bakanae disease (Gibberella fujikuroi), downy mildew (Sclerophthora macrospora), false blast and head blight (Epicoccum nigrum), seedling blight(Trichoderma viride, Rhizopus oryzae), bordered sheath spot (Waitea circinata), brown sclerotium disease (Ceratobasidium setariae), and brown sheath blight (Thanatephorus cucumeris);
Wheat diseases:
powdery mildew (Blumeria graminis), fusarium blight (Fusarium graminearum, Fusarium avenaceum, Fusarium culmorum, Microdochium nivale), stripe rust (Puccinia striiformis), stem rust (Puccinia graminis), leaf rust (Puccinia recondita), snow mould (Microdochium nivale, Microdochium majus), typhula snow blight (Typhula incarnata, Typhula ishikariensis), loose smut (Ustilago tritici), stinking smut (Tilletia caries, Tilletia controversa), eyespot (Pseudocercosporella herpotrichoides ), leaf blotch (Septoria tritici), glume blotch (Stagonospora nodorum), tan spot (Pyrenophora tritici-repentis), rhizoctonia seeding blight (Rhizoctonia solani), take-all disease (Gaeumannomyces graminis), and blast (Pyricularia graminis-tritici);
Barley diseases:
powdery mildew (Blumeria graminis), fusarium head blight (Fusarium graminearum, Fusarium avenaceum, Fusarium culmorum, Microdochium nivale), stripe rust (Puccinia striiformis), stem rust (Puccinia graminis), dwarf leaf rust (Puccinia hordei), loose smut (Ustilago nuda), scald (Rhynchosporium secalis), net blotch (Pyrenophora teres), spot blotch (Cochliobolus sativus), stripe (Pyrenophora graminea), Ramularia disease (Ramularia collo-cygni), and rhizoctonia seeding blight (Rhizoctonia solani);
Corn diseases:
rust (Puccinia sorghi), southern rust (Puccinia polysora), northern leaf blight (Setosphaeria turcica), tropical rust (Physopella zeae), southern leaf blight (Cochliobolus heterostrophus), anthracnose (Colletotrichum graminicola), gray leaf spot (Cercospora zeae-maydis), eyespot (Kabatiella zeae), phaeosphaeria leaf spot (Phaeosphaeria maydis), diplodia rot (Stenocarpella maydis, Stenocarpella macrospora), stalk rot (Fusarium graminearum, Fusarium verticilioides, Colletotrichum graminicola), smut (Ustilago maydis), and Physoderma brown spot and Physoderma stalk rot (Physoderma maydis);
Cotton diseases:
anthracnose (Colletotrichum gossypii), grey mildew (Ramularia areola), alternaria leaf spot (Alternaria macrospora, Alternaria gossypii), and black root rot (Thielaviopsis basicola);
Coffee diseases:
rust (Hemileia vastatrix), and leaf spot (Cercospora coffeicola) ;
Rape seed diseases:
sclerotinia rot (Sclerotinia sclerotiorum), gray leaf spot (Alternaria brassicae), root rot (Phoma lingam), and light leaf spot (Pyrenopeziza brassicae);
Sugar cane diseases:
rust (Puccinia melanocephela, Puccinia kuehnii), and smut (Ustilago scitaminea);
Sunflower diseases:
rust (Puccinia helianthi), and downy mildew (Plasmopara halstedii) ;
Citrus diseases:
melanose (Diaporthe citri), scab (Elsinoe fawcetti), green mold (Penicillium digitatum), blue mold (Penicillium italicum), Phytophthora rot (Phytophthora parasitica, Phytophthora citrophthora), and aspergillus rot (Aspergillus niger);
Apple diseases:
blossom blight (Monilinia mali), valsa canker (Valsa ceratosperma), powdery mildew (Podosphaera leucotricha), alternaria leaf spot (Alternaria alternata apple pathotype), scab (Venturia inaequalis), bitter rot (Glomerella cingulata, Colletotrichum acutatum), blotch (Diplocarpon mali), ring rot (Botryosphaeria berengeriana), crown rot (Phytophtora cactorum), and rust (Gymnosporangium juniperi-virginianae, Gymnosporangium yamadae);
Pear diseases:
scab (Venturia nashicola, Venturia pirina), black spot (Alternaria alternata Japanese pear pathotype), and rust (Gymnosporangium haraeanum);

Peach diseases:
brown rot (Monilinia fructicola), scab (Cladosporium carpophilum), Phomopsis rot (Phomopsis sp.), and leaf curl (Taphrina deformans);
Grapes diseases:
anthracnose (Elsinoe ampelina), ripe rot (Glomerella cingulata, Colletotrichum acutatum), powdery mildew (Uncinula necator), rust (Phakopsora ampelopsidis), black rot (Guignardia bidwellii), and downy mildew (Plasmopara viticola) ;
Japanese persimmon diseases:
anthracnose (Gloeosporium kaki, Colletotrichum acutatum), and leaf spot (Cercospora kaki, Mycosphaerella nawae);
Diseases of gourd family:
anthracnose (Colletotrichum lagenarium), powdery mildew (Sphaerotheca fuliginea), gummy stem blight (Didymella bryoniae), Corynespora leaf spot (Corynespora cassiicola), fusarium wilt (Fusarium oxysporum), downy mildew (Pseudoperonospora cubensis), phytophthora rot (Phytophthora capsici), and damping-off (Pythium sp.);
Tomato diseases:
early blight (Alternaria solani), leaf mold (Cladosporium fulvum), Cercospora leaf mold (Pseudocercospora fuligena), late blight (Phytophthora infestans), and powdery mildew (Leveillula taurica); Eggplant diseases:
brown spot (Phomopsis vexans), and powdery mildew (Erysiphe cichoracearum);
Cruciferous vegetables diseases:
alternaria leaf spot (Alternaria japonica), white spot (Cercosporella brassicae), clubroot (Plasmodiophora brassicae), downy mildew (Peronospora parasitica), and white rust (Albugo candida);
Welsh onion disease:
rust (Puccinia allii);
Soybean diseases:
purple stain (Cercospora kikuchii), sphaceloma scab (Elsinoe glycines), pod and stem blight (Diaporthe phaseolorum var. sojae), rust (Phakopsora pachyrhizi), target spot (Corynespora cassiicola), anthracnose (Colletotrichum glycines, Colletotrichum truncatum), Rhizoctonia rot (Rhizoctonia solani), septoria brown spot (Septoria glycines), Cercospora leaf spot (Cercospora sojina), stem rot (Sclerotinia sclerotiorum), powdery mildew (Microsphaera diffusa), phytophthora stem and root rot (Phytophthora sojae), downy mildew (Peronospora manshurica), sudden death syndrome (Fusarium virguliforme), red crown rot (Calonectria ilicicola), and Diaporthe/Phomopsis complex (Diaporthe longicolla);
Kidney bean diseases:
stem rot (Sclerotinia sclerotiorum), rust (Uromyces appendiculatus), angular leaf spot (Phaeoisariopsis griseola), and anthracnose (Colletotrichum lindemuthianum), and Fusarium root-rot (Fusarium solani);
Peanut diseases:
leaf spot (Cercospora personata), brown leaf spot (Cercospora arachidicola), southern blight (Sclerotium rolfsii), and Cylindrocladium black rot (Calonectria ilicicola);
Garden pea disease:
powdery mildew (Erysiphe pisi), and root rot (Fusarium solani);
Potato diseases:
early blight (Alternaria solani), late blight (Phytophthora infestans), Pink rot (Phytophthora erythroseptica), powdery scab (Spongospora subterranea f. sp. subterranea), verticillium wilt (Verticillium albo-atrum, Verticillium dahliae, Verticillium nigrescens), dry rot (Fusarium solani), and potato wart (Synchytrium endobioticum);
Strawberry disease:
powdery mildew (Sphaerotheca humuli);
Tea diseases:
net blister blight (Exobasidium reticulatum), white scab (Elsinoe leucospila), gray blight (Pestalotiopsis sp.), and anthracnose (Colletotrichum theae-sinensis);
Tobacco diseases:
brown spot (Alternaria longipes), anthracnose (Colletotrichum tabacum), blue mold (Peronospora tabacina), and black shank (Phytophthora nicotianae);
Sugar beet diseases:
cercospora leaf spot (Cercospora beticola), leaf blight (Thanatephorus cucumeris), root rot (Thanatephorus cucumeris), and aphanomyces root rot (Aphanomyces cochlioides), and rust (Uromyces betae);
Rose diseases:
black spot (Diplocarpon rosae), and powdery mildew (Sphaerotheca pannosa);
Chrysanthemum diseases:
leaf blight (Septoria chrysanthemi-indici), and white rust (Puccinia horiana);
Onion diseases:
botrytis leaf blight (Botrytis cinerea, Botrytis byssoidea, Botrytis squamosa), gray-mold neck rot (Botrytis allii), and

small sclerotial neck rot (Botrytis squamosa);
Various crops diseases:
Botrytis rot (Botrytis cinerea), sclerotinia rot (Sclerotinia sclerotiorum), seedling blight (Pythium aphanidermatum, Pythium irregulare, Pythium ultimum);
Japanese radish disease:
alternaria leaf spot (Alternaria brassicicola);
Turfgrass diseases:
dollar spot (Sclerotinia homoeocarpa), brown patch and large patch (Rhizoctonia solani), and pythium bligt (Pythium aphanidermatum);
Banana disease:
Sigatoka disease (Mycosphaerella fijiensis, Mycosphaerella musicola);
Lentils disease:
ascochyta blight (Ascochyta lentis);
Chickpea disease:
ascochyta blight (Ascochyta rabiei);
Green pepper disease:
anthracnose (Colletotrichum scovillei);
Mango disease:
anthracnose (Colletotrichum acutatum);
Fruit trees diseases:
white root rot (Rosellinia necatrix), and violet root rot (Helicobasidium mompa);
Postharvest disease of fruits (for example, apple and pear):

Mucor rot diseases (Mucor piriformis);
Seed diseases or diseases in the early stages of the growth of various plants caused by Aspergillus spp., Penicillium spp., Fusarium spp., Gibberella spp., Tricoderma spp., Thielaviopsis spp., Rhizopus spp., Mucor spp., Corticium spp., Phoma spp., Rhizoctonia spp. or Diplodia spp.; and the like;

Viral diseases:
Lettuce big-vein disease transmitted by Olpidium brassicae, and viral diseases of several crops transmitted by Polymixa spp. (e.g. Polymyxa betae and Polymyxa graminis);
Diseases caused by bacteria:
bacterial seedling blight of rice (Burkholderia plantarii), bacterial palea browning of rice (Pantoea ananatis), bacterial leaf blight of rice (Xanthomonas oryzae pv. oryzae.), bacterial spot of cucumber (Pseudomonas syringae pv. lachrymans), bacterial wilt of eggplant (Ralstonia solanacearum), canker of citrus (Xanthomonas citri), bacterial soft rot of Chinese cabbage (Erwinia carotovora), scab of potato (Streptomyces scabiei), Goss's wilt of corn (Clavibacter michiganensis), Pierce's disease of grapes, olive and peach (Xylella fastidiosa), andcrown gall of Rosaceae plants such as apple, peach, cherries (Agrobacterium tumefaciens).

The Present compound has control effect on harmful arthropods such as harmful insects and harmful mites, harmful nematodes, and harmful mollusks. Examples of the harmful arthropods, harmful nematodes, and harmful mollusks include the followings.

Hemiptera:

from the family Delphacidae, for example, small brown planthopper (Laodelphax striatellus), brown planthopper (Nilaparvata lugens), white-backed planthopper (Sogatella furcifera), corn planthopper (Peregrinus maidis), cereal leafhopper(Javesella pellucida), sugarcane leafhopper (Perkinsiella saccharicida), and Tagosodes orizicolus;
from the family Cicadellidae, for example, green rice leafhopper (Nephotettix cincticeps), green paddy leafhopper (Nephotettix virescens), rice leafhopper (Nephotettix nigropictus), zigzag-striped leafhopper (Recilia dorsalis), tea green leafhopper (Empoasca onukii), potato leafhopper (Empoasca fabae), corn leafhopper (Dalbulus maidis), rice leafhopper (Cofana spectra), and Amrasca biguttula biguttula;
from the family Aphrophoridae, for example, European spittlebug (Philaenus spumarius);
from the family Cercopidae, for example, Mahanarva posticata and Mahanarva fimbriolata;
from the family Aphididae, for example, bean aphid (Aphis fabae), soybean aphid (Aphis glycines), cotton aphid (Aphis gossypii), green apple aphid (Aphis pomi), apple aphid (Aphis spiraecola), green peach aphid (Myzus persicae), leaf-curling plum aphid (Brachycaudus helichrysi), cabbage aphid (Brevicoryne brassicae), rosy apple

aphid(Dysaphis plantaginea), false cabbage aphid (Lipaphis erysimi), potato aphid (Macrosiphum euphorbiae), foxglove aphid (Aulacorthum solani), lettuce aphid (Nasonovia ribisnigri), grain aphid (Rhopalosiphum padi), corn aphid (Rhopalosiphum maidis), brown citrus aphid (Toxoptera citricida), mealy plum aphid (Hyalopterus pruni), cane aphid (Melanaphis sacchari), black rice root aphid (Tetraneura nigriabdominalis), sugarcane cottony aphid (Ceratovacuna lanigera), apple woolly aphid (Eriosoma lanigerum), and English grain aphid (Sitobion avenae);

from the family Phylloxeridae, for example, grapevine phylloxera (Daktulosphaira vitifoliae), Pecan phylloxera (Phylloxera devastatrix), Pecan leaf phylloxera (Phylloxera notabilis), and Southern pecan leaf phylloxera (Phylloxera russelae);

from the family Adelgidae, for example, hemlock woolly aphid (Adelges tsugae), balsam woolly aphid (Adelges piceae), and Aphrastasia pectinatae;

from the family Pentatomidae, for example, black rice bug (Scotinophara lurida), black paddy bug (Scotinophara coarctata), common green stink bug (Nezara antennata), white-spotted spined bug (Eysarcoris aeneus), lewis spined bug (Eysarcoris lewisi), white-spotted bug (Eysarcoris ventralis), Eysarcoris annamita, brown marmorated stink bug (Halyomorpha halys), green plant bug (Nezara viridula), brown stink bug(Euschistus heros), red banded stink bug(Piezodorus guildinii), Oebalus pugnax, and Dichelops melacanthus;

from the family Cydnidae, for example, Scaptocoris castanea;

from the family Alydidae, for example, bean bug (Riptortus clavatus), corbett rice bug (Leptocorisa chinensis), and rice bug (Leptocorisa acuta);

from the family Coreidae, for example, Cletus punctiger and Australian leaf-footed bug (Leptoglossus australis);

from the family Lygaeidae, for example, oriental chinch bug (Cavelerius saccharivorus), seed bug (Togo hemipterus), and chinch bug (Blissus leucopterus);

from the family Miridae, for example, rice leaf bug (Trigonotylus caelestialium), sorghum plant bug (Stenotus rubrovittatus), wheat leaf bug (Stenodema calcarata), and American tarnished plant bug (Lygus lineolaris);

from the family Aleyrodidae, for example, greenhouse whitefly (Trialeurodes vaporariorum), tobacco whitefly (Bemisia tabaci), citrus whitefly (Dialeurodes citri), citrus spiny whitefly (Aleurocanthus spiniferus), tea spiny whitefly (Aleurocanthus camelliae), and Pealius euryae;

from the family Diaspididae, for example, Abgrallaspis cyanophylli, red scale (Aonidiella aurantii), San José scale (Diaspidiotus perniciosus), white peach scale (Pseudaulacaspis pentagona), arrowhead scale (Unaspis yanonensis), and citrus snow scale (Unaspis citri);

from the family Coccidae, for example, pink wax scale (Ceroplastes rubens);

from the family Margarodidae, for example, fluted scale (Icerya purchasi) and seychelles fluted scale (Icerya seychellarum);

from the family Pseudococcidae, for example, solanum mealybug (Phenacoccus solani), cotton mealybug (Phenacoccus solenopsis), Japanese mealybug (Planococcus kraunhiae), white peach scale (Pseudococcus comstocki), citrus mealybug (Planococcus citri), currant mealybug (Pseudococcus calceolariae), long-tailed mealybug (Pseudococcus longispinus), and tuttle mealybug (Brevennia rehi);

from the family Psyllidae, for example, citrus psylla (Diaphorina citri), two-spotted citrus psyllid (Trioza erytreae), pear sucker (Cacopsylla pyrisuga), Cacopsylla chinensis, potato psyllid (Bactericera cockerelli), and Pear psylla (Cacopsylla pyricola);

from the family Tingidae, for example, sycamore lace bug (Corythucha ciliata), aster tingid (Corythucha marmorata), Japanese pear lace bug (Stephanitis nashi), and azalea lace bug (Stephanitis pyrioides);

from the family Cimicidae, for example, common bed bug (Cimex lectularius) and tropical bed bug (Cimex hemipterus); from the family Cicadidae, for example, Quesada gigas;

from the family Reduviidae, for example, Triatoma infestans, large kissing bug (Triatoma rubrofasciata), Triatoma dimidiata, and Rhodonius prolixus; and the others.

Lepidoptera:

from the family Crambidae, for example, rice stem borer (Chilo suppressalis), Dark-headed stem borer (Chilo polychrysus), white stem borer (Scirpophaga innotata), yellow paddy borer (Scirpophaga incertulas), Rupela albina, rice leaf roller (Cnaphalocrocis medinalis), Marasmia patnalis, rice leaf roller (Marasmia exigua), cotton leaf roller (Notarcha derogata), corn borer (Ostrinia furnacalis), European corn borer (Ostrinia nubilalis), cabbage webworm (Hellula undalis), grape leafroller (Herpetogramma luctuosale), bluegrass webworm (Parapediasia teterrellus), rice case-worm (Nymphula depunctalis), Sugarcane borer (Diatraea saccharalis), and eggplant fruit borer (Leucinodes orbonalis);

from the family Pyralidae, for example, lesser cornstalk borer (Elasmopalpus lignosellus), mealworm moth (Plodia interpunctella), persimmon bark borer (Euzophera batangensis), and fig moth (Cadra cautella);

from the family Noctuidae, for example, cotton worm (Spodoptera litura), beet armyworm (Spodoptera exigua), rice armyworm (Mythimna separata), cabbage moth (Mamestra brassicae), pink borer (Sesamia inferens), grass armyworm (Spodoptera mauritia), green rice caterpillar (Naranga aenescens), Spodoptera frugiperda, true armyworm (Spodoptera exempta), semitropical armyworm (Spodoptera eridania), black cutworm (Agrotis ipsilon), beet worm (Autographa nigrisigna), rice looper (Plusia festucae), Soybean looper (Chrysodeixis includens), Trichoplusia spp., Heliothis spp.(such as tobacco budworm (Heliothis virescens)), Helicoverpa spp. (such as tobacco budworm (Helicoverpa armigera), corn earworm (Helicoverpa zea)), velvet-bean caterpillar (Anticarsia gemmatalis), cotton leafworm (Alabama argillacea), and hop vine borer (Hydraecia immanis);
from the family Pieridae, for example, common cabbage worm (Pieris rapae);
from the family Tortricidae, for example, oriental fruit moth (Grapholita molesta), Grapholita dimorpha, soybean moth (Leguminivora glycinivorella), Matsumuraeses azukivora, summer fruit tortrix (Adoxophyes orana fasciata), smaller tea tortrix (Adoxophyes honmai), Japanese tea tortrix (Homona magnanima), apple tortrix (Archips fuscocupreanus), codling moth (Cydia pomonella), sugarcane shoot borer (Tetramoera schistaceana), bean shoot borer (Epinotia aporema), citrus fruit borer (Citripestis sagittiferella), and European grapevine moth (Lobesia botrana);
from the family Gracillariidae, for example, tea leaf roller (Caloptilia theivora) and Asiatic apple leaf miner (Phyllonorycter ringoniella);
from the family Carposinidae, for example, peach fruit moth (Carposina sasakii);
from the family Lyonetiidae, for example, coffee leaf miner (Leucoptera coffeella), peach leaf miner (Lyonetia clerkella), and Lyonetia prunifoliella;
from the family Lymantriidae, for example, Lymantria spp. (such as gypsy moth (Lymantria dispar)) and Euproctis spp. (such as tea lymantriid (Euproctis pseudoconspersa));
from the family Plutellidae, for example, diamondback moth (Plutella xylostella);
from the family Gelechiidae, for example, peach worm (Anarsia lineatella), sweetpotato leaf folder (Helcystogramma triannulella), pink bollworm (Pectinophora gossypiella), potato moth (Phthorimaea operculella), and Tuta absoluta;
from the family Arctiidae, for example, American white moth (Hyphantria cunea);
from the family Castniidae, for example, giant sugarcane borer (Telchin licus);
from the family Cossidae, for example, Cossus insularis;
from the family Geometridae, for example, Ascotis selenaria;
from the family Limacodidae, for example, blue-striped nettle grub (Parasa lepida);
from the family Stathmopodidae, for example, persimmon fruit moth (Stathmopoda masinissa);
from the family Sphingidae, for example, tobacco hornworm (Acherontia lachesis);
from the family Sesiidae, for example, Nokona feralis, cherry borer (Synanthedon hector), and Synanthedon tenuis:

from the family Hesperiidae, for example, rice skipper (Parnara guttata);
from the family Tineidae, for example, casemaking clothes moth (Tinea translucens), common clothes moth (Tineola bisselliella);

and the others.
Thysanoptera:

from the family Thripidae, for example, western flower thrips (Frankliniella occidentalis), oriental thrips (Thrips palmi), yellow tea thrips (Scirtothrips dorsalis), onion thrips (Thrips tabaci), eastern flower thrips (Frankliniella intonsa), rice thrips (Stenchaetothrips biformis), Echinothrips americanus, and avocado thrips (Scirtothrips perseae);
from the family Phlaeothripidae, for example, aculeated rice thrips (Haplothrips aculeatus);

and the others.
Diptera:

from the family Anthomyiidae, for example, seedcorn maggot (Delia platura), onion maggot (Delia antiqua), and beet leaf miner (Pegomya cunicularia);
from the family Ulidiidae, for example, sugarbeet root maggot (Tetanops myopaeformis);
from the family Agromyzidae, for example, rice leaf miner (Agromyza oryzae), tomato leaf miner (Liriomyza sativae), chrysanthemum leaf miner (Liriomyza trifolii), and pea leafminer (Chromatomyia horticola);
from the family Chloropidae, for example, rice stem maggot (Chlorops oryzae);

from the family Tephritidae, for example, melon fly (Bactrocera cucurbitae), oriental fruit fly (Bactrocera dorsalis), Malaysian fruit fly (Bactrocera latifrons), olive fruit fly (Bactrocera oleae), Queensland fruit fly (Bactrocera tryoni), Mediterranean fruit fly (Ceratitis capitata), apple maggot (Rhagoletis pomonella), and Japanese cherry fruit fly (Rhacochlaena japonica);
from the family Ephydridae, for example, smaller rice leaf miner (Hydrellia griseola), whorl maggot (Hydrellia philippina), and paddy stem maggot (Hydrellia sasakii);
from the family Drosophilidae, for example, cherry drosophila (Drosophila suzukii), and common fruit fly (Drosophila melanogaster);
from the family Phoridae, for example, Megaselia spiracularis;
from the family Psychodidae, for example, Clogmia albipunctata;
from the family Sciaridae, for example, Bradysia difformis;
from the family Cecidomyiidae, for example, hessian fly (Mayetiola destructor) and paddy gall fly (Orseolia oryzae);
from the family Diopsidae, for example, Diopsis macrophthalma;
from the family Glossinidae, for example, Glossina palpalis and Glossina morsitans;
from the family Simuliidae, for example, Simulium japonicum and Simulium damnosum;
from the subfamily Phlebotominae;
from the family Tipulidae, for example, rice crane fly (Tipula aino), common cranefly (Tipula oleracea), and European cranefly (Tipula paludosa);
from the family Culicidae, for example, southern house mosquito (Culex pipiens pallens), Culex tritaeniorhynchus ,Culex pipiens f. molestus, brown house mosquito (Culex quinquefasciatus), northern house mosquito (Culex pipiens pipiens), Culex vishnui, Asian tiger mosquito (Aedes albopictus), dengue mosquito (Aedes aegypti), Chinese malaria mosquito (Anopheles sinensis), Anopheles gambiae, Anopheles stephensi, Anopheles coluzzii, Anopheles albimanus, Anopheles sundaicus, Anopheles arabiensis, Anopheles funestus, Anopheles darlingi, Anopheles farauti, and Anopheles minimus;
from the family Simulidae, for example, Prosimulium yezoensis and Simulium ornatum;
from the family Tabanidae, for example, Tabanus trigonus;
from the family Muscidae, for example, house fly (Musca domestica), false stable fly (Muscina stabulans), biting house fly (Stomoxys calcitrans), and buffalo fly (Haematobia irritans);
from the family Calliphoridae;
from the family Sarcophagidae;
from the family Chironomidae, for example, Chironomus plumosus, Chironomus yoshimatsui, and Glyptotendipes tokunagai;
from the family Fannidae;

and the others.
Coleoptera:

from the family Chrysomelidae, for example, Diabrotica spp. (such as western corn rootworm (Diabrotica virgifera virgifera), southern corn rootworm (Diabrotica undecimpunctata howardi), northern corn rootworm (Diabrotica barberi), Mexican corn rootworm (Diabrotica virgifera zeae), banded cucumber beetle (Diabrotica balteata), cucurbit beetle(Diabrotica speciosa)), bean leaf beetle (Cerotoma trifurcata), barley leaf beetle (Oulema melanopus), cucurbit leaf beetle (Aulacophora femoralis), striped flea beetle (Phyllotreta striolata), cabbage flea beetle (Phyllotreta cruciferae), western black flea beetle (Phyllotreta pusilla), cabbage stem flea beetle (Psylliodes chrysocephala), hop flea beetle (Psylliodes punctulata), Colorado potato beetle (Leptinotarsa decemlineata), rice leaf beetle (Oulema oryzae), grape colaspis (Colaspis brunnea), corn flea beetle (Chaetocnema pulicaria), sweet-potato flea beetle (Chaetocnema confinis), potato flea beetle (Epitrix cucumeris), rice leaf beetle (Dicladispa armigera), southern corn leaf beetle (Myochrous denticollis), Laccoptera quadrimaculata, and tobacco flea beetle (Epitrix hirtipennis);
from the family Carabidae, for example, Seedcorn beetle (Stenolophus lecontei) and slender seed-corn ground beetle (Clivina impressifrons);
from the family Scarabaeidae, for example, cupreus chafer (Anomala cuprea), soybean beetle (Anomala rufocuprea), Anomala albopilosa, Japanese beetle (Popillia japonica), yellowish elongate chafer (Heptophylla picea), European Chafer(Rhizotrogus majalis), Tomarus gibbosus, Holotrichia spp., Phyllophaga spp. (such as June beetle (Phyllophaga crinita)), and Diloboderus spp. (such as Diloboderus abderus);
from the family Anthriibidae, for example, coffee bean weevil(Araecerus coffeae);
from the family Aponidae, for example, sweet-potato weevil (Cylas formicarius);
from the family Bruchidae, for example, Mexican bean weevil (Zabrotes subfasciatus);

from the family Scolytidae, for example, pine beetle (Tomicus piniperda) and coffee berry borer (Hypothenemus hampei);
from the family Curculionidae, for example, West Indian sweet-potato weevil (Euscepes postfasciatus), alfalfa weevil (Hypera postica), maize wevil (Sitophilus zeamais), rice weevil (Sitophilus oryzae), grain weevil (Sitophilus granarius), rice plant weevil (Echinocnemus squameus), rice water weevil (Lissorhoptrus oryzophilus), Rhabdoscelus lineaticollis, boll weevil (Anthonomus grandis), nunting billbug (Sphenophorus venatus), southern corn billbug (Sphenophorus callosus), soybean stalk weevil (Sternechus subsignatus), sugarcane weevil (Sphenophorus levis), rusty gourd-shaped weevil (Scepticus griseus), brown gourd-shaped weevil (Scepticus uniformis), Aracanthus spp. (such as Aracanthus mourei), and cotton root borer (Eutinobothrus brasiliensis);
from the family Tenebrionidae, for example, red meal beetle (Tribolium castaneum), mason beetle (Tribolium confusum), and lesser mealworm (Alphitobius diaperinus);
from the family Coccinellidae, for example, twenty-eight-spotted ladybird (Epilachna vigintioctopunctata);
from the family Bostrychidae, for example, common powder-post beetle (Lyctus brunneus), and lesser grain borer (Rhizopertha dominica);
from the family Ptinidae;
from the family Cerambycidae, for example, citrus long-horned beetle (Anoplophora malasiaca), Migdolus fryanus, and peach borer (Aromia bungii);
from the family Elateridae, for example, Melanotus okinawensis, barley wireworm (Agriotes fuscicollis), Melanotus legatus, Anchastus spp., Conoderus spp., Ctenicera spp., Limonius spp., and Aeolus spp.;
from the family Staphylinidae, for example, Paederus fuscipes;
from the family Dermestidae, for example, varied carpet beetle (Anthrenus verbasci), hide beetle (Dermestes maculates), and khapra beetle (Trogoderma granarium);
from the family Anobiidae, for example, tobacco beetle (Lasioderma serricorne), and biscuit beetle (Stegobium paniceum);
from the family Laemophloeidae, for example, flat grain beetle (Cryptolestes ferrugineus);
from the family Silvanidae, for example, saw-toothed grain beetle (Oryzaephilus surinamensis);
from the family Nitidulidae, for example, blossom beetle (Brassicogethes aeneus);

and the others.
Orthoptera:

from the family Acrididae, for example, oriental migratory locust (Locusta migratoria), Moroccan locust (Dociostaurus maroccanus), Australian plague locust (Chortoicetes terminifera), red locust (Nomadacris septemfasciata), brown locust(Locustana pardalina), tree locust (Anacridium melanorhodon), Italian locust (Calliptamus italicus), differential grasshopper (Melanoplus differentialis), two-striped grasshopper (Melanoplus bivittatus), migratory grasshopper (Melanoplus sanguinipes), red-legged grasshopper (Melanoplus femurrubrum), clear-winged grasshopper (Camnula pellucida), desert locust (Schistocerca gregaria), Yellow-winged locust(Gastrimargus musicus), spur-throated locust (Austracris guttulosa), Japanese grasshopper (Oxya yezoensis), rice grasshopper (Oxya japonica), and Bombay locust (Patanga succincta);
from the family Gryllotalpidae, for example, oriental mole cricket (Gryllotalpa orientalis);
from the family Gryllidae, for example, house cricket (Acheta domestica), and emma field cricket (Teleogryllus emma);
from the family Tettigoniidae, for example, mormon cricket (Anabrus simplex);

and the others.
Hymenoptera:

from the family Tenthredinidae, for example, beet sawfly (Athalia rosae) and nippon cabbage sawfly (Athalia japonica);
from the family Formicidae, for example, Solenopsis spp. (such as red imported fire ant (Solenopsis invicta), tropical fire ant (Solenopsis geminata)), Atta spp. (such as brown leaf-cutting ant (Atta capiguara), Acromyrmex spp., Paraponera clavata, black house ant (Ochetellus glaber), little red ant (Monomorium pharaonis), Argentine ant (Linepithema humile), Formica japonica, Pristomyrmex punctutus, Pheidole noda, big-headed ant (Pheidole megacephala), Camponotus spp. (such as Camponotus japonicus, Camponotus obscuripes), Pogonomyrmex spp. (such as western harvester ant (Pogonomyrmex occidentalis)), Wasmania spp. (such as Wasmania auropunctata), and long-legged ant (Anoplolepis gracilipes);
from the family Vespidae, for example, Asian giant hornet (Vespa mandarinia, Vespa simillima, Vespa analis, Asian hornet (Vespa velutina), and Polistes jokahamae;

from the family Siricidae, for example, pine wood wasp (Urocerus gigas);
from the family Bethylidae;

and the others.
Blattodea:

from the family Ectobiidae, for example, German cockroach (Blattella germanica);
from the family Blattidae, for example, smoky-brown cockroach (Periplaneta fuliginosa), American cockroach (Periplaneta americana), Australian cockroach (Periplaneta australasiae), brown cockroach (Periplaneta brunnea), and black cockroach (Blatta orientalis);
from the family Termitidae, for example, Japanese termite (Reticulitermes speratus), Formosan termite (Coptotermes formosanus), western drywood termite (Incisitermes minor), Cryptotermes domesticus, Odontotermes formosanus, Neotermes koshunensis, Glyptotermes satsumensis, Glyptotermes nakajimai, Glyptotermes fuscus, Hodotermopsis sjostedti, Coptotermes guangzhouensis, Reticulitermes amamianus, Reticulitermes miyatakei, Reticulitermes kanmonensis, Nasutitermes takasagoensis, Pericapritermes nitobei, Sinocapritermes mushae, and Cornitermes cumulans;

and the others.
Siphonaptera:

from the family Pulicidae, for example, human flea (Pulex irritans), cat flea (Ctenocephalides felis), dog flea (Ctenocephalides canis), oriental rat flea (Xenopsylla cheopis), and chicken flea (Echidnophaga gallinacea);
from the family Pulicidae, for example, chigoe flea (Tunga penetrans);
from the family Ceratophyllidae, for example, European rat flea (Nosopsyllus fasciatus); and the others.

Psocodae:

from the family Pediculidae, for example, head louse (Pediculus humanus capitis);
from the family Pthiridae, for example, crab louse (Pthirus pubis);
from the family Haematopinidae, for example, short-nosed cattle louse (Haematopinus eurysternus) and pig louse (Haematopinus suis);
from the family Linognathidae, for example, blue cattle louse (Linognathus vituli), sheep face louse (Linognathus ovillus), and capillate louse (Solenopotes capillatus);
from the family Bovicoliidae, for example, cattle biting louse (Bovicola bovis), sheep biting louse (Bovicola ovis), Bovicola breviceps, Damalinia forficula, and Werneckiella spp.;
from the family Trichodectidae, for example, dog biting louse (Trichodectes canis) and cat louse (Felicola subrostratus);
from the family Menoponidae, for example, common chicken louse (Menopon gallinae), chicken body louse (Menacanthus stramineus), and Trinoton spp;
from the family Trimenoponidae, for example, Cummingsia spp. ;
from the family Trogiidae, for example, death watch (Trogium pulsatorium);
from the family Liposcelidae or Liposcelididae, for example, book louse (Liposcelis corrodens), Liposcelis bostrychophila, Liposcelis pearmani and Liposcelis entomophila;

and the other.
Thysanura:
from the family Lepismatidae, for example, oriental silverfish (Ctenolepisma villosa) and moth fish (Lepisma saccharina); and the others.
Acari:

from the family Tetranychidae, for example, common red spider mite (Tetranychus urticae), kanzawa spider mite (Tetranychus kanzawai), red spider mite (Tetranychus evansi), citrus red mite (Panonychus citri), fruit-tree red spider mite (Panonychus ulmi), and Oligonychus spp.;
from the family Eriophyidae, for example, Japanese citrus rust mite (Aculops pelekassi), Phyllocoptruta citri, tomato mite (Aculops lycopersici), purple mite (Calacarus carinatus), tea rust mite (Acaphylla theavagrans), Eriophyes chibaensis, apple bud mite (Aculus schlechtendali), Aceria diospyri, Aceria tosichella, and Shevtchenkella sp.;
from the family Tarsonemidae, for example, broad mite (Polyphagotarsonemus latus);

from the family Tenuipalpidae, for example, Brevipalpus phoenicis;
from the family Tuckerellidae;
from the family Ixodidae, for example, Haemaphysalis longicornis, Haemaphysalis flava, Haemaphysalis japonica, Haemaphysalis campanulata, American dog tick (Dermacentor variabilis), Dermacentor taiwanensis, Rocky Mountain wood tick (Dermacentor andersoni), netted tick (Dermacentor reticulatus), Ixodes ovatus, Ixodes persulcatus, black-legged tick (Ixodes scapularis), Ixodes pacificus, Ixodes holocyclus, Ixodes ricinus, lone star tick (Amblyomma americanum), gulf coast tick (Amblyomma maculatum), Rhipicephalus microplus, cattle tick (Rhipicephalus annulatus), brown dog tick (Rhipicephalus sanguineus), Rhipicephalus appendiculatus, and Rhipicephalus decoloratus;
from the family Argasidae, for example, fowl tick (Argas persicus), Ornithodoros hermsi, and Ornithodoros turicata;
from the family Acaridae, for example, cereal mite (Tyrophagus putrescentiae) and grassland mite (Tyrophagus similis);
from the family Pyroglyphidae, for example, American house dust mite (Dermatophagoides farinae) and European house dust mite (Dermatophagoides pteronyssinus);
from the family Cheyletidae, for example, Cheyletus eruditus, Cheyletus malaccensis, Chelacaropsis moorei, and Cheyletiella yasguri;
from the family Psoroptidae, for example, sheep scab mite (Psoroptes ovis), horse psoroptic mange mite (Psoroptes equi), Knemidocoptes mutans, ear mange mite (Otodectes cynotis), and Chorioptes spp.;
from the family Sarcoptidae, for example, Notoedres cati, Notoedres muris, and itch mite (Sarcoptes scabiei);
from the family Listrophoridae, for example, Listrophorus gibbus;
from the family Dermanyssidae, for example, bird mite (Dermanyssus gallinae);
from the family Macronyssidae, for example, feather mite (Ornithonyssus sylviarum) and tropical rat mite (Ornithonyssus bacoti);
from the family Varroidae, for example, Varroa jacobsoni;
from the family Demodicidae, for example, dog follicle mite (Demodex canis) and cat follicle mite (Demodex cati);
from the family Trombiculidae, for example, Leptotrombidium akamushi, Leptotrombidium pallidum, and Leptotrombidium scutellare;

and the others.
Araneae:

from the family Eutichuridae, for example, Cheiracanthium japonicum;
from the family Theridiidae, for example, red-back spider (Latrodectus hasseltii);

and the others.
Polydesmida:
from the family Paradoxosomatidae, for example, flat-backed millipede (Oxidus gracilis) and Nedyopus tambanus;
and the others.
Isopoda:
from the family Armadillidiidae, common pill bug (Armadillidium vulgare);
and the others.
Chilopoda:

from the family Scutigeridae, for example, Thereuonema hilgendorfi;
from the family Scolopendridae, giant tropical centipede (Scolopendra subspinipes);
from the family Ethopolyidae, Bothropolys rugosus; and the others.

Gastropoda:

from the family Limacidae, for example, tree slug (Limax marginatus) and garden tawny slug (Limax flavus);
from the family Philomycidae, for example, Meghimatium bilineatum;
from the family Ampullariidae, for example, golden apple snail (Pomacea canaliculata);
from the family Lymnaeidae, for example, Austropeplea ollula;

and the others.
Nematoda:

from the family Aphelenchoididae, for example, rice white-tip nematode (Aphelenchoides besseyi);
from the family Pratylenchidae, for example, root lesion nematode (Pratylenchus coffeae), Pratylenchus brachyurus, California meadow nematode (Pratylenchus neglectus), and Radopholus similis;
from the family Heteroderidae, for example, javanese root-knot nematode (Meloidogyne javanica), southern root-knot nematode (Meloidogyne incognita), guava root-knot nematodes (Meloidogyne enterolobii), northern root-knot nematode (Meloidogyne hapla), soybean cyst nematode (Heterodera glycines), potato cyst nematode (Globodera rostochiensis), and white potato cyst nematode (Globodera pallida);
from the family Hoplolaimidae, for example, Rotylenchulus reniformis;
from the family Anguinidae, for example, strawberry bud nematode (Nothotylenchus acris), and stem nematode (Ditylenchus dipsaci);
from the family Tylenchulidae, for example, citrus nematode (Tylenchulus semipenetrans);
from the family Longidoridae, for example, dagger nematode (Xiphinema index);
from the family Trichodoridae;
from the family Parasitaphelenchidae, for example, pine wilt disease (Bursaphelenchus xylophilus);

and the others.

The harmful arthropods such as harmful insects and harmful mites, harmful mollusks and harmful nematodes may be those having a reduced susceptibility to or a developed resistance to an insecticide, a mitecide, a molluscicide or a nematicide.

The method for controlling pests of the present invention is carried out by applying an effective amount of the Present compound or the Composition A to a harmful pest directly and/or a habitat where the harmful pest lives (for example, plant, soil, an interior of a house, and animal). Examples of a method for controlling pests of the present invention include foliar application, soil application, root application, shower application, smoking application, water-surface application, and seed application.

The Present compound or the Composition A is usually used by mixing it with inert carrier (s) such as solid carrier(s), liquid carrier(s), and gaseous carrier(s), surfactant(s), and the like, and as needed, adding thereto auxiliary agent(s) for formulation such as binder(s), dispersant(s), and stabilizer(s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, a tablet, an aerosol formulation, a resin formulation, or the like. In addition to these formulations, the Present compound or the Composition A may be used by formulating it into a dosage form described in Manual on development and use of FAO and WHO Specifications for pesticides, FAO Plant Production and Protection Papers-271-276, prepared by the FAO/WHO Joint Meeting on Pesticide Specifications, 2016, ISSN:0259-2517.

These formulations usually comprise 0.0001 to 99% by weight ratio of the Present compound or the Composition A.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the gaseous carrier include fluorocarbon, butane gas, LPG (liquefied petroleum gas), dimethyl ether, nitrogen, and carbon dioxide.

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

Moreover, some adjuvants can be employed to improve or help the efficacy of the Present compound. The

specific examples thereof include Nimbus (registered trademark), Assist (registered trademark), Aureo (registered trademark), Iharol (registered trademark), Silwet L-77 (registered trademark), BreakThru (registered trademark), SundanceII (registered trademark), Induce (registered trademark), Penetrator (registered trademark), AgriDex (registered trademark), Lutensol A8 (registered trademark), NP-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray N (registered trademark), and BANGLE (registered trademark).

As used herein, examples of the plant include whole plant, stem and leaf, flower, ear, fruit, tree stem, branch, crown, seed, vegetative reproductive organ, and seedling.

A vegetative reproduction organ means a part of plant such as root, stem, and leaf which has a growth capability even when said part is separated from the plant body and placed into soil. Examples of the vegetative reproduction organ include tuberous root, creeping root, bulb, corm or solid bulb, tuber, rhizome, stolon, rhizophore, cane cuttings, propagule, and vine cutting. Stolon is also referred to as "runner", and propagule is also referred to as "propagulum" and categorized into broad bud and bulbil. Vine cutting means a shoot (collective term of leaf and stem) of sweet potato, glutinous yam, or the like. Bulb, corm or solid bulb, tuber, rhizome, cane cuttings, rhizophore, and tuberous root are also collectively referred to as "bulb". For example, cultivation of potato starts with planting a tuber into soil, and the tuber to be used is generally referred to as "seed potato".

Examples of a method for controlling pests by applying an effective amount of the Present compound or the Composition A to soils include a method of applying an effective amount of the Present compound or the Composition A to soils before planting plants or after planting plants, a method of applying an effective amount of the Present compound or the Composition A to a root part of a crop to be protected from damage such as ingestion by harmful arthropods, and a method of controlling harmful arthropods that ingest a plant by permeating and transferring an effective amount of the Present compound or the Composition A from a root into the interior of the plant body. Specifically, examples of the application method include planting hole treatment (spraying into planting holes, soil mixing after planting hole treatment), plant foot treatment (plant foot spraying, soil mixing after plant foot treatment, irrigation at plant foot, plant foot treatment at a later seeding raising stage), planting furrow treatment (planting furrow spraying, soil mixing after planting furrow treatment), planting row treatment (planting row spraying, soil mixing after planting row treatment, planting row spraying at a growing stage), planting row treatment at the time of sowing (planting row spraying at the time of sowing, soil mixing after planting row treatment at the time of sowing), broadcast treatment (overall soil surface spraying, soil mixing after broadcast treatment), side-article treatment, treatment of water surface (application to water surface, application to water surface after flooding), other soil spraying treatment (spraying of a granular formulation on leaves at a growing stage, spraying under a canopy or around a tree stem, spraying on the soil surface, mixing with surface soil, spraying into seed holes, spraying on the ground surfaces of furrows, spraying between plants), other irrigation treatment (soil irrigation, irrigation at a seedling raising stage, chemical solution injection treatment, irrigation of a plant part just above the ground, chemical solution drip irrigation, chemigation), seedling raising box treatment (spraying into a seedling raising box, irrigation of a seedling raising box, flooding into a seedling raising box with chemical solution), seedling raising tray treatment (spraying on a seedling raising tray, irrigation of a seedling raising tray, flooding into a seedling raising tray with chemical solution), seedbed treatment (spraying on a seedbed, irrigation of a seedbed, spraying on a lowland rice nursery, immersion of seedlings), seedbed soil incorporation treatment (mixing with seedbed soil, mixing with seedbed soil before sowing, spraying at sowing before covering with soils, spraying at sowing after covering with soils, mixing with covering with soils), and other treatment (mixing with culture soil, plowing under, mixing with surface soil, mixing with soil at the place where raindrops fall from a canopy, treatment at a planting position, spraying of a granule formulation on flower clusters, mixing with a paste fertilizer).

Examples of the application to seeds (or seed treatments) include an application of the Present compound or the Composition A to seeds or vegetative reproductive organs, and specific examples thereof include spraying treatment in which a suspension of the Present compound or the Composition A is sprayed onto seed surface or the vegetative reproductive organ surface in the form of mist; smearing treatment in which the Present compound or the Composition A is coated a surface of seeds or the vegetative reproductive organ; a soaking treatment in which the seeds are soaked into the solution of the Present compound or the Composition A for a certain time; and a method for coating the seeds or the vegetative reproductive organ with a carrier containing the Present compound or the Composition A (film coating treatment, pellet coating treatment). Examples of the above-described vegetative reproductive organ include particularly seed potato.

When the Composition A is applied to seeds or vegetative reproductive organs, the Composition A may be also applied to seeds or vegetative reproductive organs as a single formulation, or the Composition A may be applied to seeds or vegetative reproductive organs as multiple different formulations by multiple times. Examples of the method in which the Composition A is applied as multiple different formulations by multiple times include, for example, a method in which the formulations comprising as an active component the Present compound only are applied, and seeds or vegetative reproductive organs are air dried, followed by applying the formulations comprising the Present ingredient:

and a method in which the formulations comprising as an active component the present compound and the Present ingredients are applied, and seeds or vegetative reproductive organs are air dried, followed by applying the formulations comprising the Present ingredients other than the already-applied Present ingredients, are included.

As used herein, seeds or vegetative reproductive organs carrying the present compound or the Composition A means seeds or vegetative reproductive organs in the state where the present compound or the Composition A is adhered to a surface of the seeds or the vegetative reproductive organ. The above-described seeds or vegetative reproductive organs carrying the present compound or the Composition A may be adhered by any other materials that are different from the present compound or the Composition A before or after being adhered the present compound or the Composition A to the seeds or vegetative reproductive organs.

Also, when the Composition A is adhered in a form of layer(s) to a surface of seeds or vegetative reproductive organ, the layer(s) is/are composed of one layer or a multiple layers. Also, when multiple layers are formed, each of the layer may be composed of a layer comprising one or more active ingredients, or a combination of a layer comprising one or more active ingredients and a layer not comprising an active ingredient.

Seeds or vegetative reproductive organs carrying the present compound or the Composition A can be obtained, for example, by applying the formulations comprising the present compound or the Composition A by the above-described application method to seeds to seeds or vegetative reproductive organs.

When the Present compound or the Composition A is applied for controlling pests in agricultural fields, the application dose thereof is usually within a range of 1 to 10,000 g of the Present compound per 10,000 m2. In the case of being applied to seeds or vegetative reproductive organs, the dose of application dose thereof is usually within a range of 0.001 to 100 g of the Present compound per 1 Kg of seeds. When the Present compound or the Composition A is formulated into an emulsifiable concentrate, a wettable powder or a flowable etc., they are usually applied by diluting them with water so as to make an effective concentration of the active ingredients 0.01 to 10,000 ppm, and the dust formulation or the granular formulation, etc., is usually applied as itself without diluting them.

Also, the resin preparation which is processed into a sheet or a string may be applied by winding a plant with a sheet or a string of the resin preparation, putting a string of the resin preparation around a crop so that the plant is surrounded by the string, or laying a sheet of the resin preparation on the soil surface near the root of a plant.

When the Present compound or the Composition A is used to control harmful arthropods that live inside a house, the application dose as an amount of the Present compound is usually within a range from 0.01 to 1,000 mg per 1 m2 of an area to be treated, in the case of using it on a planar area. In the case of using it spatially, the application dose as an amount of the Present compound is usually within a range from 0.01 to 500 mg per 1 m3 of the space to be treated. When the Present compound or the Composition A is formulated into emulsifiable concentrates, wettable powders, flowables or the others, such formulations are usually applied after diluting it with water in such a way that a concentration of the active ingredient is within a range from 0.1 to 10,000 ppm. In the case of being formulated into oil solutions, aerosols, smoking agents, poison baits and the others, such formulations are used as itself without diluting it.

When the Present compound or the composition A is used for controlling external parasites of livestock such as cows, horses, pigs, sheep, goats and chickens and small animals such as dogs, cats, rats and mice, the composition of the present invention may be applied to the animals by a known method in the veterinary field. Specifically, when systemic control is intended, the composition of the present invention is administered to the animals as a tablet, a mixture with feed or a suppository, or by injection (including intramuscular, subcutaneous, intravenous and intraperitoneal injections). On the other hand, when non-systemic control is intended, the composition of the present invention is applied to the animals by means of spraying of the oil solution or aqueous solution, pour-on or spot-on treatment, or washing of the animal with a shampoo formulation, or by putting a collar or ear tag made of the resin formulations to the animal. In the case of being administered to an animal body, the dose of the compound of the Present compound is usually within a range from 0.1 to 1,000 mg per 1 kg of an animal body weight.

Also, the composition of the Present compound or the Composition A may be used as an agent for controlling pests in agricultural lands such as paddy fields, fields, turfs, and orchards. Examples of the plants include the followings.

corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous vegetables (for example, Japanese radish, white turnip, horseradish, kohlrabi, Chinese cabbage, cabbage, leaf mustard, broccoli, and cauliflower), asteraceous vegetables (for example, burdock, crown daisy, artichoke, and lettuce), liliaceous vegetables (for example, welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus unshiu, orange, lemon, lime, and grapefruit), nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus spp. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

EXAMPLES

Hereinafter, the present invention is explained in more detail by Preparation Examples, Reference Preparation Examples, Formulation Examples, and Test Examples, however, the present invention should not be limited to the these Examples.

As used herein, Me represents methyl group, Et represents ethyl group, Pr represents propyl group, i-Pr represents isopropyl group, Bu represents butyl group, *i-Bu* represents isobutyl group, t-Bu represents tert-butyl group, Pen represents pentyl group, c-Pr represents cyclopropyl group, c-Bu represents cyclobutyl group, c-Pen represents cyclopentyl group, c-Hex represents cyclohexyl group, and Ph represents phenyl group.

Firstly, Preparation examples of the compound of the present invention are described.

When a physical property of a compound is measured by a liquid chromatography / mass spectrometry analysis (hereinafter, referred to as LCMS), a measured molecular ion value $[M+H]^+$ or $[M-H]^-$ and a retention time (hereinafter, referred to as RT) is described. The measured condition for liquid chromatography (hereinafter, referred to as LC) and mass spectrometry (hereinafter, referred to as MS) is described below.

[LC condition]

Column: L-column2 CDS, inner diameter 4.6 mm, length 30 m m, particle size 3 μm
(Chemicals Evaluation and Research Institute, Japan) UV measurement wavelength: 254 nm
Mobile phase: A solution: 0.1 % aqueous formic acid solution, B solution: 0.1 % formic acid in acetonitrile
Flow Rate: 2.0 mL/min
Pump: LC-20AD (manufacturer SHIMAZU) 2 umps (high pressure gradient)
Gradient condition: The following concentration gradient shown in [Table LC1] is liquid-transferred.

[Table LC1]

| Time (min) | A solution (%) | B solution (%) |
|---|---|---|
| 0.01 | 90 | 10 |
| 2.00 | 0 | 100 |
| 4.00 | 0 | 100 |
| 4.01 | 90 | 10 |

[MS condition]

Detector: LCMS-2020 (manufacturer SHIMADZU)
Ionization method: DUIS

Reference Prepration Example 1-1

Under nitrogen atmosphere, a mixture of 1,5-dibromo-2.4-dimethylbenzene 1.06 g, methyl acetoacetate 1.03 g, palladium (II) acetate 0.09 g, 2-di-tert-buthylphosphino-2',4',6'-triisopropylbiphenyl 0.17 g, tripotassium phosphate 2.55 g, and toluene 20 mL was stirred under reflux for 2 hours. Water was added to the resulting mixture, and the mixture was extracted with ethyl acetate. The resulting organic layer was washed with saturated brine, dried over anhydrous sodium sulfate, and concentrated under reduced pressure. The resulting residue was subjected to a silica gel column chromatography to obtain the intermediate compound 1-1 represented by the following formula 0.71 g.

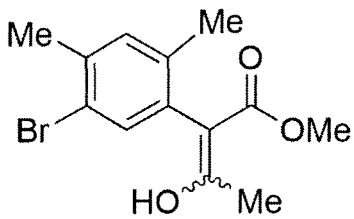

Intermediate compound 1-1: $^1$H-NMR ($CDCl_3$) $\delta$: 12.95 (1H, s), 7.23 (1H, s), 7.10 (1H, s), 3.68 (3H, s), 2.37 (3H, s), 2.07 (3H, s), 1.77 (3H, s).

Reference Prepration Example 1-2

To a mixture of the intermediate compound 1-1 0.30 g, THF 2 mL, and methanol 2 mL was added 28 % solution of sodium methoxide in methanol 0.3 mL, and the mixture was stirred at 60°C for 3 hours. To the resulting mixture was added saturated aqueous ammonium chloride solution, and the mixture was extracted with ethyl acetate. The resulting organic layer was washed with saturated brine, dried over anhydrous sodium sulfate, and concentrated under reduced pressure, to obtain the intermediate compound 1-2 represented by the following formula 0.22 g.

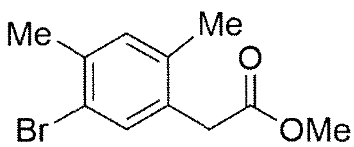

Intermediate compound 1-2: $^1$H-NMR ($CDCl_3$) $\delta$: 7.35 (1H, s), 7.05 (1H, s), 3.69 (3H, s), 3.57 (2H, s), 2.33 (3H, s), 2.22 (3H, s).

Reference Prepration Example 1-3

A mixture of the intermediate compound 1-2 3.72 g, N,N-dimethylformamide dimethylacetal 3.45 g, and DMF 15 mL was stirred under reflux for one and a half days. Water was added to the resulting mixture, and the mixture was extracted with MTBE. The resulting organic layer was washed with saturated brine, dried over anhydrous sodium sulfate, and concentrated under reduced pressure to obtain the intermediate compound 1-3 represented by the following formula 3.10 g.

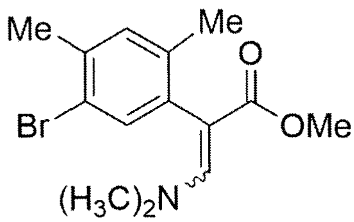

Intermediate compound 1-3: $^1$H-NMR ($CDCl_3$) δ: 7.56 (1H, s), 7.28 (1H, s), 7.03 (1H, s), 3.61 (3H, s), 2.67 (6H, s), 2.35 (3H, s), 2.10 (3H, s).

Reference Prepration Example 1-4

To a mixture of the intermediate compound 1-3 0.27 g, and methanol 10 mL was added 2N hydrochloric acid 5 mL under ice-cooling, and the mixture was stirred at room temperature for 3 hours. To the resulting mixture was added saturated brine, and the mixture was extracted with ethyl acetate. The resulting organic layer was dried over anhydrous sodium sulfate, and concentrated under reduced pressure to obtain the intermediate compound 1-4 represented by the following formula 0.23 g.

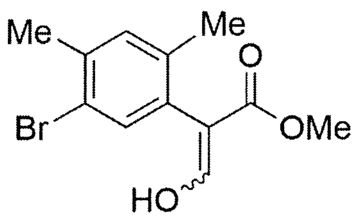

Reference Prepration Example 1-5

To a mixture of the intermediate compound 1-4 0.23 g, cesium carbonate 0.34 g, and NMP 4 mL was added dimethyl sulfate 0.15 g, and the mixture was stirred at room temperature for 3 hours. To the resulting mixture was added saturated brine, and the mixture was extracted with ethyl acetate. The resulting organic layer was dried over anhydrous sodium sulfate, and concentrated under reduced pressure. The resulting residue was subjected to a silica gel column chromatography to obtain the intermediate compound 1-5 represented by the following formula 0.19 g.

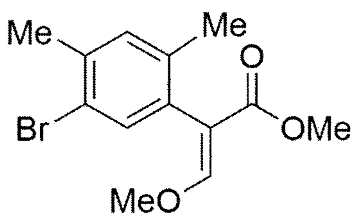

Intermediate compound 1-5: $^1$H-NMR ($CDCl_3$) δ: 7.55 (1H, s), 7.27 (1H, s), 7.10 (1H, s), 3.84 (3H, s), 3.70 (3H, s), 2.35 (3H, s), 2.09 (3H, s).

Reference Prepration Example 2

Under nitrogen atmosphere, mixture of the intermediate compound 1-5 1.0 g, bis(pinacolato)diboron 1.1 g, $PdCl_2$(dppf) 0.24 g, tripotassium phosphate 0.66 g, and dimethoxyethane 30 mL were stirred at 80°C for 6 hours. Water was added to the resulting mixture, and the mixture was extracted with ethyl acetate. The resulting organic layer was washed with saturated brine, dried over anhydrous sodium sulfate, and concentrated under reduced pressure. The resulting residue was subjected to a silica gel column chromatography to obtain the intermediate compound 2-1 represented by the following formula 1.0 g.

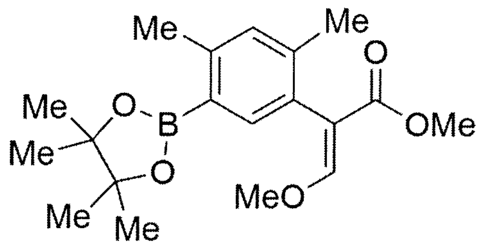

Intermediate compound 2-1: $^1$H-NMR ($CDCl_3$) δ: 7.54 (1H, s), 7.50 (1H, s), 7.04 (1H, s), 3.80 (3H, s), 3.68 (3H, s), 2.50 (3H, s), 2.14 (3H, s), 1.31 (12H, s).

Reference Prepration Example 3

Under nitrogen atmosphere, mixtures of the intermediate compound 1-5 2.0 g, bis(triphenylphosphine)palladium (II) dichloride 0.47 g, tributyl (1-ethoxyvinyl)stannate 4.4 mL and 1,4-dioxane 20 mL were stirred at 110°C for 4 hours. To the resulting mixture was added 1N hydrochloric acid, and the mixture was stirred at room temperature for 1 hour. Water was added to the resulting mixture, and the mixture was extracted with ethyl acetate. The resulting organic layer was washed with saturated brine, dried over anhydrous sodium sulfate, and concentrated under reduced pressure. The resulting residue was subjected to a silica gel column chromatography to obtain the intermediate compound 3-1 represented by the following formula 1.4 g.

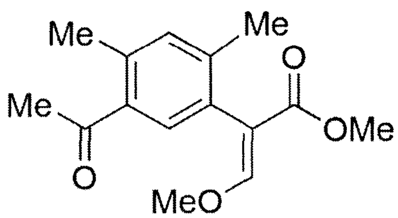

Intermediate compound 3-1: $^1$H-NMR ($CDCl_3$) δ: 7.60 (1H, s), 7.50 (1H, s), 7.11 (1H, s), 3.85 (3H, s), 3.72 (3H, s), 2.55 (3H, s), 2.52 (3H, s), 2.18 (3H, s).

Reference Prepration Example 4

A mixture of the intermediate compound 2-1 0.54 g, m-chloroperoxybenzoic acid 0.50 g, acetonitrile 5 mL, ethanol 5 mL, and water 5 mL was stirred at room temperature for 2.5 hours. To the resulting mixture were added aqueous sodium thiosulfate solution and aqueous sodium bicarbonate solution, and the mixtures were stirred at room temperature for 2 hours. The resulting mixture was extracted with ethyl acetate. The resulting organic layer was washed with saturated brine, dried over anhydrous sodium sulfate, and concentrated under reduced pressure. The resulting residue was subjected to a silica gel column chromatography to obtain the intermediate compound 4-1 represented by the following formula 0.37 g.

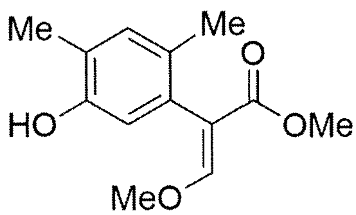

Intermediate compound 4-1: $^1$H-NMR ($CDCl_3$) δ: 7.54 (1H, s), 6.98 (1H, s), 6.55 (1H, s), 4.51 (1H, s), 3.83 (3H, s), 3.70 (3H, s), 2.21 (3H, s), 2.07 (3H, s).

Reference Prepration Example 5

Under nitrogen atmosphere, a mixture of 3-bromo-4,5-dimethylphenol 2.0 g, bis(pinacolato)diboron 3.8 g,

$PdCl_2$(dppf) 0.36 g, potassium acetate 2.0 g, and DMSO 25 mL were stirred at 80°C for 7 hours. Water was added to the resulting mixture, and the mixture was extracted with ethyl acetate. The resulting organic layer was washed with saturated brine, dried over anhydrous sodium sulfate, and concentrated under reduced pressure. The resulting residue was subjected to a silica gel column chromatography to obtain the intermediate compound 5-1 represented by the following formula 2.6 g.

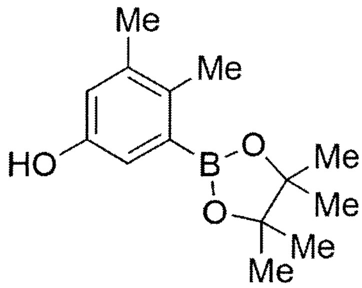

Intermediate compound 5-1: $^1$H-NMR ($CDCl_3$) δ: 7.05 (1H, d), 6.75 (1H, d), 4.62 (1H, br s), 2.38 (3H, s), 2.22 (3H, s), 1.34 (12H, s).

Reference Prepration Example 5-1

The compounds which were prepared according to the Reference Preparation Example 5 and their physical property value are shown below.

A compound represented by formula (C5-a):

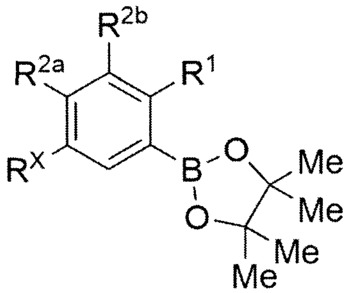

(C5-a)

wherein $R^1$, $R^{2a}$, $R^{2b}$, and $R^x$ represents any combination described in [Table A1].

[Table A1]

| Intermediate compound | $R^1$ | $R^{2a}$ | $R^{2b}$ | $R^X$ |
|---|---|---|---|---|
| 5-2 | Me | F | H | OH |
| 5-3 | Me | OMe | H | OH |
| Intermediate compound 5-2: $^1$H-NMR ($CDCl_3$) δ: 7.38 (1H, d), 6.87 (1H, d), 4.83 (1H, d), 2.45 (3H, s), 1.33 (12H, s). Intermediate compound 5-3: $^1$H-NMR ($CDCl_3$) δ: 7.31 (1H, s), 6.66 (1H, s), 5.29 (1H, s), 3.88 (3H, s), 2.48 (3H, s), 1.32 (12H, s). | | | | |

Reference Prepration Example 6

Under nitrogen atmosphere, a mixture of the intermediate compound 5-1 2.5 g, methyl (Z)-2-iodo-3-methoxy acrylate 2.9 g, 2-(dicyclohexylphoshino)-2',6'-dimethoxybiphenyl 0.21 g, $Pd_2(dba)_3$ 0.23 g, tripotassium phosphate 4.22 g, toluene 25 mL and water 5 mL were stirred at 110°C for 4 hours. Water was added to the resulting mixture, and the mixture was extracted with ethyl acetate. The resulting organic layer was washed with saturated brine, dried over anhydrous sodium sulfate, and concentrated under reduced pressure. The resulting residue was subjected to a silica gel column chromatography to obtain the intermediate compound 6-1 represented by the following formula 1.6 g.

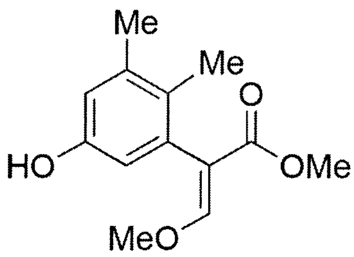

Intermediate compound 6-1: $^1$H-NMR ($CDCl_3$) $\delta$: 7.54 (1H, s), 6.64 (1H, d), 6.46 (1H, d), 4.82-4.78 (1H, br m), 3.82 (3H, s), 3.70 (3H, s), 2.24 (3H, s), 1.99 (3H, s).

Reference Prepration Example 6-1

The compounds which were prepared according to the Reference Preparation Example 6 and their physical property value are shown below.

A compound represented by formula (C6-a):

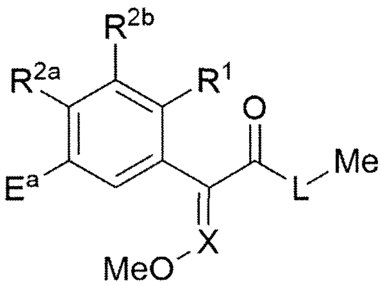

(C6-a)

wherein $R^1$, $R^{2a}$, $R^{2b}$, $E^a$, X and L represent any combination described in [Table A2].

[Table A2]

| Intermediate compound | $R^1$ | $R^{2a}$ | $R^{2b}$ | $E^a$ | X | L |
|---|---|---|---|---|---|---|
| 6-2 | Me | F | H | OH | CH | O |
| 6-3 | Me | OMe | H | OH | CH | O |
| Intermediate compound 6-2: $^1$H-NMR ($CDCl_3$) $\delta$: 7.55 (1H, s), 6.93 (1H, d), 6.76 (1H, d), 4.96 (1H, d), 3.84 (3H, s), 3.71 (3H, s), 2.08 (3H, s).<br>Intermediate compound 6-3: $^1$H-NMR ($CDCl_3$) $\delta$: 7.54 (1H, s), 6.72 (1H, s), 6.70 (1H, s), 5.40 (1H, s), 3.87 (3H, s), 3.83 (3H, s), 3.70 (3H, s), 2.11 (3H, s). | | | | | | |

Reference Prepration Example 7

Under nitrogen atmosphere, to a mixture of the intermediate compound 6-1 0.6 g and chloroform 6 mL were added triethylamine 0.46 mL and trifluoroacetic anhydride 0.50 mL successively at 0°C and the mixture was stirred for 30 minutes. To the resulting mixture was added aqueous sodium bicarbonate solution, and the mixture was extracted with ethyl acetate. The resulting organic layer was washed with saturated brine, dried over anhydrous sodium sulfate, and concentrated under reduced pressure to obtain the intermediate compound 7-1 represented by the following formula 0.70 g.

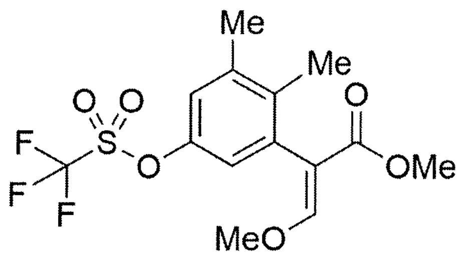

Intermediate compound 7-1: $^1$H-NMR ($CDCl_3$) δ: 7.58 (1H, s), 7.02 (1H, d), 6.89 (1H, d), 3.85 (3H, s), 3.71 (3H, s), 2.32 (3H, s), 2.07 (3H, s).

Reference Prepration Example 7-1

The compounds which were prepared according to the Reference Preparation Example 7 and their physical property value are shown below.

A compound represented by formula (C7-a):

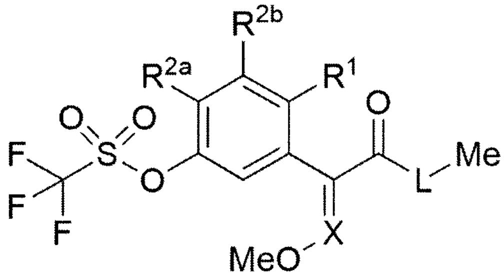

(C7-a)

wherein $R^1$, $R^{2a}$, $R^{2b}$, X and L represent any combination described in [Table A3].

[Table A3]

| Intermediate compound | $R^1$ | $R^{2a}$ | $R^{2b}$ | X | L |
|---|---|---|---|---|---|
| 7-2 | Me | F | H | CH | O |
| 7-3 | Me | OMe | H | CH | O |
| Intermediate compound 7-2: $^1$H-NMR ($CDCl_3$) δ: 7.59 (1H, s), 7.12-7.05 (2H, m), 3.86 (3H, s), 3.71 (3H, s), 2.18 (3H, s). intermediate compound 7 - 3 : $^1$H-NMR ($CDCl_3$) 5: 7.57 (1H, s), 6.97 (1H, s), 6.87 (1H, s), 3.90 (3H, s), 3.85 (3H, s), 3.71 (3H, s), 2.19 (3H, s). | | | | | |

Reference Prepration Example 8

Under nitrogen atmosphere, to a mixture of 1,5-dibromo-2.4-dimethylbenzene 5 g and THF 30 mL was added butyl lithium (1.6 M hexane solution) 12 mL dropwise at -78°C, and the mixture was stirred at -78°C for 1 hour. To the resulting mixture was added dimethyl oxalate 2.5 g and the mixture was stirred at 0°C, for 3 hours. To the resulting mixture was added saturated aqueous ammonium chloride solution, and the mixture was extracted with ethyl acetate. The resulting mixture was washed with saturated brine, dried over anhydrous sodium sulfate, and concentrated under reduced pressure. The resulting residue was subjected to a silica gel column chromatography to obtain the intermediate compound 8-1 represented by the following formula 3.9 g.

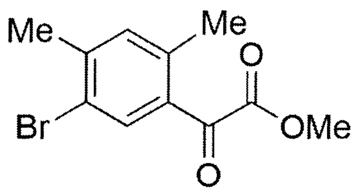

Intermediate compound 8-1: $^1$H-NMR ($CDCl_3$) δ: 7.83 (1H, s), 7.18 (1H, s), 3.97 (3H, s), 2.51 (3H, s), 2.42 (3H, s).

Reference Prepration Example 8-1

The compounds which were prepared according to the Reference Preparation Example 8 and their physical property value are shown below.

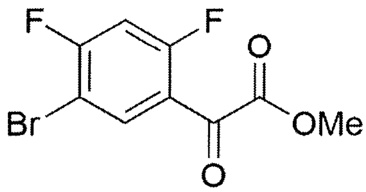

Intermediate compound 8-2: $^1$H-NMR ($CDCl_3$) δ: 7.79-7.72 (1H, m), 6.96 (1H, m), 3.97 (3H, s).

Reference Prepration Example 9

A mixture of the intermediate compound 8-1 3.5 g, O-methyl hydroxylamine hydrochloride salt 2.3 g, and ethanol 80 mL was stirred at 55°C for 6 hours. Water was added to the resulting mixture, and the mixture was extracted with ethyl acetate. The resulting organic layer was washed with saturated brine, dried over anhydrous sodium sulfate, and concentrated under reduced pressure. The resulting residue was subjected to a silica gel column chromatography to obtain the intermediate compound 9-1 represented by the following formula 1.3 g.

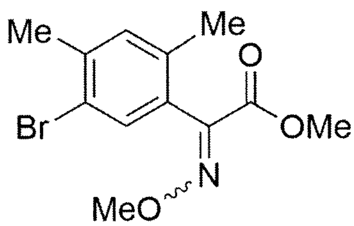

Intermediate compound 9-1: $^1$H-NMR ($CDCl_3$) δ: 7.26 (1H, s), 7.13 (1H, s), 4.06 (3H, s), 3.88 (3H, s), 2.37 (3H, s), 2.10 (3H, s).

Reference Prepration Example 9-1

The compounds which were prepared according to the Reference Preparation Example 9 and their physical property value are shown below.

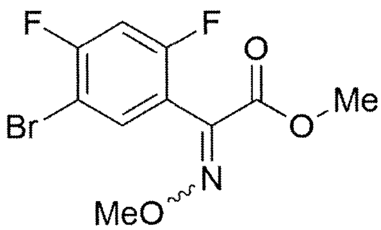

Intermediate compound 9-2: $^1$H-NMR ($CDCl_3$) δ: 7.64-7.57 (1H, m), 6.92-6.86 (1H, m), 4.14 (3H, s), 3.91 (3H, s).

Preparation Example 1

To a mixture of the intermediate compound 3-1 0.20 g, o-methyl hydroxylamine hydrochloride salt 0.13 g, and ethanol 5 mL was added pyridine 0.10 mL at room temperature, and the mixture was stirred at room temperature overnight. The resulting mixture was diluted with ethyl acetate, and concentrated under reduced pressure. The resulting residue was subjected to a silica gel column chromatography (ethyl acetate : hexane = 3:7) to obtain the present compound 1-1 represented by the following formula 0.22 g.

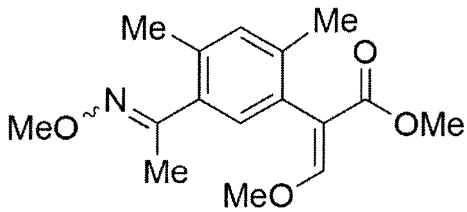

Present compound 1-1: $^1$H-NMR ($CDCl_3$) δ: 7.55 (1H, s), 7.06 (1H, s), 6.98 (1H, s), 3.95 (3H, s), 3.81 (3H, s), 3.68 (3H, s), 2.33 (3H, s), 2.15 (3H, s), 2.14 (3H, s).

Preparation Example 1-1

The compounds which were prepared according to the Preparation Example 1 and their physical property value are shown below.

A compound represented by formula (1-1):

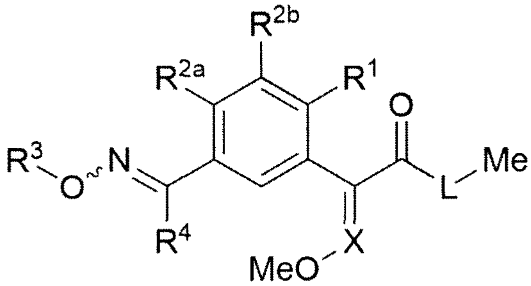

(1–1)

wherein $R^1$, $R^{2a}$, $R^{2b}$, $R^3$, $R^4$, X and L represent any combination described in [Table A4].

[Table A4]

| Present compound | $R^1$ | $R^{2a}$ | $R^{2b}$ | $R^3$ | $R^4$ | X | L |
|---|---|---|---|---|---|---|---|
| 1-2 | Me | Me | H | Et | Me | CH | O |
| 1-3 | Me | Me | H | $CH_2$c-Pr | Me | CH | O |
| 1-4 | Me | Me | H | Ph | Me | CH | O |
| 1-5 | Me | Me | H | $CH_2Ph$ | Me | CH | O |
| 1-6 | Me | Me | H | $CH_2CH_2F$ | Me | CH | O |
| 1-7 | Me | Me | H | Et | Me | CH | O |
| 1-8 | Me | Me | H | $CH_2Ph$ | Me | CH | O |

(continued)

| Present compound | $R^1$ | $R^{2a}$ | $R^{2b}$ | $R^3$ | $R^4$ | X | L |
|---|---|---|---|---|---|---|---|
| 1-9 | Me | Me | H | $CH_2$ (4-Cl-Ph) | Me | CH | O |

Present compound 1-2: $^1$H-NMR ($CDCl_3$) δ: 7.55 (1H, s), 7.06 (1H, s), 6.98 (1H, s), 4.19 (2H, q), 3.80 (3H, s), 3.68 (3H, s), 2.34 (3H, s), 2.16 (3H, s), 2.14 (3H, s), 1.31 (3H, t).
Present compound 1-3: $^1$H-NMR ($CDCl_3$) δ: 7.55 (1H, s), 7.06 (1H, s), 6.99 (1H, s), 3.96 (2H, d), 3.81 (3H, s), 3.68 (3H, s), 2.35 (3H, s), 2.19 (3H, s), 2.14 (3H, s), 1.25-1.15 (1H, m), 0.58-0.52 (2H, m), 0.33-0.28 (2H, m).
Present compound 1-4: $^1$H-NMR ($CDCl_3$) δ: 7.58 (1H, s), 7.33-7.27 (2H, m), 7.25-7.21 (2H, m), 7.13 (1H, s), 7.09 (1H, s), 7.02-6.98 (1H, m), 3.83 (3H, s), 3.71 (3H, s), 2.43 (3H, s), 2.39 (3H, s), 2.18 (3H, s).
Present compound 1-5: $^1$H-NMR ($CDCl_3$) δ: 7.55 (1H, s), 7.43-7.32 (5H, m), 7.04 (1H, s), 6.97 (1H, s), 5.19 (2H, s), 3.80 (3H, s), 3.68 (3H, s), 2.25 (3H, s), 2.20 (3H, s), 2.13 (3H, s).
Present compound 1-6: $^1$H-NMR ($CDCl_3$) δ: 7.56 (1H, s), 7.07 (1H, s), 6.98 (1H, s), 4.74 (1H, t), 4.62 (1H, t), 4.41 (1H, t), 4.34 (1H, t), 3.82 (3H, s), 3.69 (3H, s), 2.34 (3H, s), 2.20 (3H, s), 2.14 (3H, s).
Present compound 1-7: $^1$H-NMR ($CDCl_3$) δ: 8.29 (1H, s), 7.56 (1H, s), 7.44 (1H, s), 7.05 (1H, s), 4.21 (2H, q), 3.82 (3H, s), 3.69 (3H, s), 2.38 (3H, s), 2.15 (3H, s), 1.32 (3H, t).
Present compound 1-8: $^1$H-NMR ($CDCl_3$) δ: 8.35 (1H, s), 7.56 (1H, s), 7.43-7.40 (2H, m), 7.38-7.30 (4H, m), 7.05 (1H, s), 5.19 (2H, s), 3.82 (3H, s), 3.69 (3H, s), 2.36 (3H, s), 2.15 (3H, s).
Present compound 1-9: $^1$H-NMR ($CDCl_3$) δ: 8.33 (1H, s), 7.56 (1H, s), 7.40 (1H, s), 7.37-7.30 (4H, m), 7.05 (1H, s), 5.14 (2H, s), 3.82 (3H, s), 3.69 (3H, s), 2.36 (3H, s), 2.15 (3H, s).

Preparation Example 2

Under nitrogen atmosphere, a mixture of the intermediate compound 1-5 0.10 g, $PdCl_2(PPh_3)_4$ 0.02 g, cyclopropyl acetylene 0.15 mL, tetrabutylammonium fluoride (1 mol/L THF solution) 1.0 mL, and THF 3 mL was stirred at 80°C for 6 hours. Aqueous sodium bicarbonate solution was added to the resulting mixture, and the mixture was extracted with ethyl acetate. The resulting organic layer was dried over anhydrous sodium sulfate, and concentrated under reduced pressure. The resulting residue was subjected to a silica gel column chromatography (ethyl acetate : hexane = 1:3) to obtain the present compound 2-1 represented by the following formula 0.09 g.

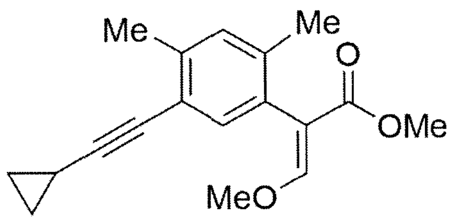

Present compound 2-1: $^1$H-NMR ($CDCl_3$) δ: 7.53 (1H, s), 7.10 (1H, s), 7.02 (1H, s), 3.81 (3H, s), 3.68 (3H, s), 2.34 (3H, s), 2.11 (3H, s), 1.49-1.42 (1H, m), 0.88-0.82 (2H, m), 0.79-0.74 (2H, m).

Preparation Example 2-1

The compounds which were prepared according to the Preparation Example 2 and their physical property value are shown below.

A compound represented by formula (2-1):

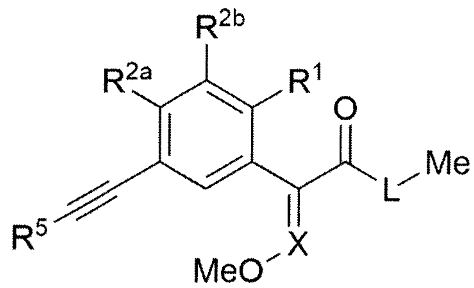

(2–1)

wherein $R^1$, $R^{2a}$, $R^{2b}$, $R^5$, X and L represent any combination described in [Table B1].

[Table B1]

| Present compound | $R^1$ | $R^{2a}$ | $R^{2b}$ | $R^5$ | X | L |
|---|---|---|---|---|---|---|
| 2-2 | Me | Me | H | t-Bu | N | O |
| 2-3 | Me | Me | H | t-Bu | CH | O |
| 2-4 | F | F | H | Ph | N | O |

Present compound 2-2: $^1$H-NMR ($CDCl_3$) $\delta$: 7.09 (1H, s), 7.06 (1H, s), 4.04 (3H, s), 3.86 (3H, s), 2.37 (3H, s), 2.12 (3H, s), 1.31 (9H, s).
Present compound 2-3: LCMS: 301 $[M+H]^+$, RT = 2.33 min.
Present compound 2-4: $^1$H-NMR ($CDCl_3$) $\delta$: 7.62-7.51 (3H, m), 7.38-7.34 (3H, m), 6.98-6.92 (1H, m), 4.14 (3H, s), 3.91 (3H, s).

Preparation Example 3

A Mixture of the intermediate compound 4-1 0.18 g, butanol 0.06 mL, bis(2-methoxyethyl) azodicarboxylate 0.13 g, triphenylphosphine 0.13 g, and chloroform 4 mL was stirred a room temperature for 4 hours. To the resulting mixture was added hydrobicarbonate water, and the mixture was extracted with ethyl acetate. The resulting organic layer was washed with saturated brine, dried over anhydrous sodium sulfate, and concentrated under reduced pressure. The resulting residue was subjected to a silica gel column chromatography (ethyl acetate : hexane = 1:3) to obtain the present compound 3-1 represented by the following formula 0.13 g.

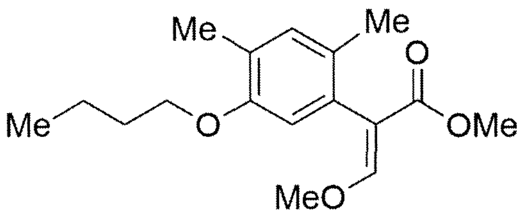

Present compound 3-1: $^1$H-NMR ($CDCl_3$) $\delta$: 7.54 (1H, s), 6.98 (1H, s), 6.56 (1H, s), 3.92 (2H, t), 3.82 (3H, s), 3.70 (3H, s), 2.18 (3H, s), 2.07 (3H, s), 1.79-1.70 (2H, m), 1.54-1.44 (2H, m), 0.96 (3H, t).

Preparation Example 3-1

The compounds which were prepared according to the Preparation Example 3 and their physical property value are shown below.

A compound represented by formula (3-1):

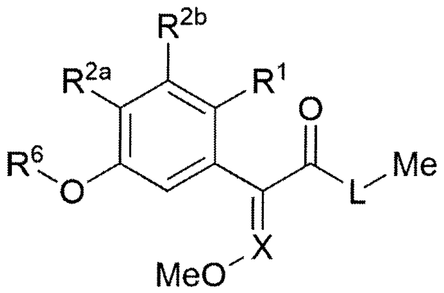

(3–1)

wherein $R^1$, $R^{2a}$, $R^{2b}$, $R^6$, X and L represent any combination described in [Table A5].

[Table A5]

| Present compound | $R^1$ | $R^{2a}$ | $R^{2b}$ | $R^6$ | X | L |
|---|---|---|---|---|---|---|
| 3-2 | Me | H | Me | Bu | CH | O |
| 3-3 | Me | F | H | Bu | CH | O |
| 3-4 | Me | OMe | H | Bu | CH | O |
| Present compound 3-2: $^1$H-NMR ($CDCl_3$) δ: 7.54 (1H, s), 6.70 (1H, d), 6.52 (1H, d), 3.91 (2H, t), 3.82 (3H, s), 3.70 (3H, s), 2.26 (3H, s), 2.00 (3H, s), 1.77-1.69 (2H, m), 1.52-1.41 (2H, m), 0.96 (3H, t).<br>Present compound 3-3: $^1$H-NMR ($CDCl_3$) δ: 7.55 (1H, s), 6.93 (1H, d), 6.70 (1H, d), 3.99 (2H, t), 3.84 (3H, s), 3.71 (3H, s), 2.08 (3H, s), 1.82-1.73 (2H, m), 1.53-1.43 (2H, m), 0.96 (3H, t).<br>Present compound 3-4: $^1$H-NMR ($CDCl_3$) δ: 7.55 (1H, s), 6.73 (1H, s), 6.63 (1H, s), 3.97 (2H, t), 3.85 (3H, s), 3.84 (3H, s), 3.71 (3H, s), 2.12 (3H, s), 1.84-1.74 (2H, m), 1.52-1.41 (2H, m), 0.95 (3H, t). | | | | | | |

Preparation Example 4

Under nitrogen atmosphere, a mixture of the intermediate compound 7-2 0.20 g, 3-methoxyphenylboronic acid 0.11 g, $PdCl_2$(dppf) 0.02 g, tripotassium phosphate 0.23 g, dimethoxyethane 4 mL, and water 0.4 mL was stirred at 80°C for 3 hours. Water was added to the resulting mixture, and the mixture was extracted with ethyl acetate. The resulting organic layer was washed with saturated brine, dried over anhydrous sodium sulfate, and concentrated under reduced pressure. The resulting residue was subjected to a silica gel column chromatography (ethyl acetate : hexane = 1:3) to obtain the present compound 4-1 represented by the following formula 0.16 g.

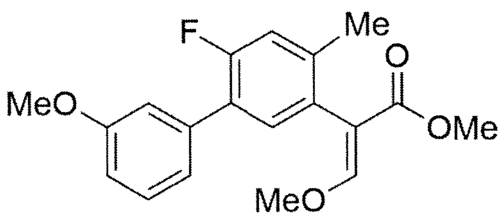

Present compound 4-1: $^1$H-NMR ($CDCl_3$) δ: 7.59 (1H, s), 7.33 (1H, t), 7.19 (1H, d), 7.16-7.09 (2H, m), 7.02 (1H, d), 6.91-6.87 (1H, m), 3.84 (3H, s), 3.84 (3H, s), 3.72 (3H, s), 2.20 (3H, s).

Preparation Example 4-1

The compounds which were prepared according to the Preparation Example 4 and their physical property value are shown below.

A compound represented by formula (4-1):

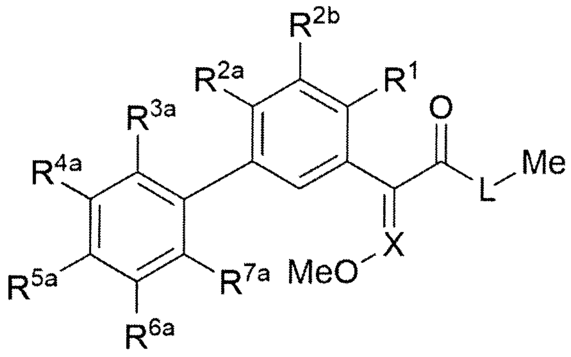

(4-1)

wherein $R^1$, $R^{2a}$, $R^{2b}$, $R^{3a}$, $R^{4a}$, $R^{5a}$, $R^{6a}$, $R^{7a}$, X and L represent any combination described in [Table A6].

[Table A6]

| Present compound | $R^1$ | $R^{2a}$ | $R^{2b}$ | $R^{3a}$ | $R^{4a}$ | $R^{5a}$ | $R^{6a}$ | $R^{7a}$ | X | L |
|---|---|---|---|---|---|---|---|---|---|---|
| 4-2 | Me | Me | H | H | H | H | H | H | CH | O |
| 4-3 | Me | Me | H | H | OMe | H | H | H | CH | O |
| 4-4 | Me | H | Me | Me | H | H | H | H | CH | O |
| 4-5 | Me | H | Me | H | OMe | H | H | H | CH | O |
| 4-6 | Me | F | H | H | CN | H | H | H | CH | O |
| 4-7 | Me | Me | H | H | H | H | H | H | N | O |
| 4-8 | Me | Me | H | H | Cl | H | H | H | N | O |
| 4-9 | Me | Me | H | H | OMe | H | H | H | N | O |
| 4-10 | Me | Me | H | H | $OCF_3$ | H | H | H | N | O |
| 4-11 | Me | Me | H | H | $CF_3$ | H | H | H | N | O |
| 4-12 | Me | Me | H | H | H | H | H | H | N | NH |
| 4-13 | Me | Me | H | H | Cl | H | H | H | N | NH |
| 4-14 | Me | Me | H | Me | H | H | H | H | CH | O |
| 4-15 | Me | Me | H | H | Me | H | H | H | CH | O |
| 4-16 | Me | Me | H | H | H | Me | H | H | CH | O |
| 4-17 | Me | Me | H | F | H | H | H | H | CH | O |
| 4-18 | Me | Me | H | H | F | H | H | H | CH | O |
| 4-19 | Me | Me | H | H | H | F | H | H | CH | O |
| 4-20 | Me | Me | H | OMe | H | H | H | H | CH | O |
| 4-21 | Me | Me | H | H | H | OMe | H | H | CH | O |
| 4-22 | Me | Me | H | H | Cl | H | H | H | CH | O |
| 4-23 | Me | Me | H | H | SMe | H | H | H | CH | O |
| 4-24 | Me | Me | H | H | OPh | H | H | H | CH | O |
| 4-25 | Me | Me | H | H | $OCH_2Ph$ | H | H | H | CH | O |
| 4-26 | Me | Me | H | H | $NO_2$ | H | H | H | CH | O |
| 4-27 | Me | Me | H | H | H | c-Pr | H | H | CH | O |
| 4-28 | Me | Me | H | F | OMe | H | H | H | CH | O |
| 4-29 | Me | Me | H | H | F | H | F | H | CH | O |

(continued)

| Present compound | $R^1$ | $R^{2a}$ | $R^{2b}$ | $R^{3a}$ | $R^{4a}$ | $R^{5a}$ | $R^{6a}$ | $R^{7a}$ | X | L |
|---|---|---|---|---|---|---|---|---|---|---|
| 4-30 | F | F | H | F | H | H | H | H | N | O |

Present compound 4-2: $^1$H-NMR ($CDCl_3$) δ: 7.57 (1H, s), 7.40-7.30 (5H, m), 7.15 (1H, s), 7.01 (1H, s), 3.83 (3H, s), 3.71 (3H, s), 2.26 (3H, s), 2.20 (3H, s).
Present compound 4-3: $^1$H-NMR ($CDCl_3$) δ: 7.57 (1H, s), 7.30 (1H, t), 7.14 (1H, s), 7.01 (1H, s), 6.95-6.92 (1H, m), 6.90-6.84 (2H, m), 3.83 (3H, s), 3.82 (3H, s), 3.71 (3H, s), 2.26 (3H, s), 2.20 (3H, s).
Present compound 4-4: $^1$H-NMR ($CDCl_3$) δ: 7.58 (1H, s), 7.28-7.20 (4H, m), 7.10 (1H, s), 6.97 (1H, s), 3.82 (3H, s), 3.71 (3H, s), 2.34 (3H, s), 2.31 (3H, s), 2.13 (3H, s).
Present compound 4-5: $^1$H-NMR ($CDCl_3$) δ: 7.60 (1H, s), 7.35 (1H, d), 7.32 (1H, t), 7.20 (1H, d), 7.19-7.15 (1H, m), 7.11 (1H, t), 6.87-6.83 (1H, m), 3.85 (3H, s), 3.83 (3H, s), 3.71 (3H, s), 2.36 (3H, s), 2.12 (3H, s).
Present compound 4-6: $^1$H-NMR ($CDCl_3$) δ: 7.83 (1H, s), 7.81-7.77 (1H, m), 7.64-7.59 (2H, m), 7.52 (1H, t), 7.16 (1H, d), 7.06 (1H, d), 3.86 (3H, s), 3.73 (3H, s), 2.22 (3H, s).
Present compound 4-7: $^1$H-NMR ($CDCl_3$) δ: 7.42-7.31 (5H, m), 7.17 (1H, s), 7.00 (1H, s), 4.07 (3H, s), 3.88 (3H, s), 2.27 (3H, s), 2.20 (3H, s).
Present compound 4-8: $^1$H-NMR ($CDCl_3$) δ: 7.35-7.29 (3H, m), 7.23-7.19 (1H, m), 7.17 (1H, s), 6.96 (1H, s), 4.07 (3H, s), 3.88 (3H, s), 2.26 (3H, s), 2.20 (3H, s).
Present compound 4-9: $^1$H-NMR ($CDCl_3$) δ: 7.34-7.28 (1H, m), 7.17 (1H, s), 7.00 (1H, s), 6.93-6.86 (3H, m), 4.07 (3H, s), 3.87 (3H, s), 3.82 (3H, s), 2.28 (3H, s), 2.20 (3H, s).
Present compound 4-10: $^1$H-NMR ($CDCl_3$) δ: 7.45-7.39 (1H, m), 7.28-7.25 (1H, m), 7.21-7.17 (3H, m), 6.98 (1H, s), 4.07 (3H, s), 3.88 (3H, s), 2.27 (3H, s), 2.21 (3H, s).
Present compound 4-11: $^1$H-NMR ($CDCl_3$) δ: 7.61-7.57 (2H, m), 7.53-7.50 (2H, m), 7.21-7.19 (1H, m), 6.98 (1H, s), 4.07 (3H, s), 3.88 (3H, s), 2.26 (3H, s), 2.21 (3H, s).
Present compound 4-12: $^1$H-NMR ($CDCl_3$) δ: 7.41-7.28 (5H, m), 7.16 (1H, s), 6.99 (1H, s), 6.78 (1H, s), 3.97 (3H, s), 2.93 (3H, d), 2.26 (3H, s), 2.20 (3H, s).
Present compound 4-13: $^1$H-NMR ($CDCl_3$) δ: 7.34-7.32 (1H, m), 7.31-7.27 (2H, m), 7.23-7.20 (1H, m), 7.15 (1H, s), 6.95 (1H, s), 6.79 (1H, br s), 3.98 (3H, s), 2.93 (3H, d), 2.25 (3H, s), 2.19 (3H, s).
Present compound 4-14: LCMS: 311 $[M+H]^+$, RT = 2.19 min.
Present compound 4-15: $^1$H-NMR ($CDCl_3$) δ: 7.57 (1H, s), 7.31-7.25 (1H, m), 7.17-7.10 (4H, m), 7.00 (1H, s), 3.82 (3H, s), 3.71 (3H, s), 2.38 (3H, s), 2.26 (3H, s), 2.19 (3H, s).
Present compound 4-16: LCMS: 311 $[M+H]^+$, RT = 2.20 min.
Present compound 4-17: LCMS: 315 $[M+H]^+$, RT = 2.09 min.
Present compound 4-18: $^1$H-NMR ($CDCl_3$) δ: 7.58 (1H, s), 7.37-7.31 (1H, m), 7.16-7.10 (2H, m), 7.08-6.97 (3H, m), 3.84 (3H, s), 3.71 (3H, s), 2.25 (3H, s), 2.20 (3H, s).
Present compound 4-19: LCMS: 315 $[M+H]^+$, RT = 2.12 min.
Present compound 4-20: LCMS: 327 $[M+H]^+$, RT = 2.07 min.
Present compound 4-21: LCMS: 327 $[M+H]^+$, RT = 2.08 min.
Present compound 4-22: LCMS: 331 $[M+H]^+$, RT = 2.22 min.
Present compound 4-23: LCMS: 343 $[M+H]^+$, RT = 2.18 min.
Present compound 4-24: $^1$H-NMR ($CDCl_3$) δ: 7.56 (1H, s), 7.37-7.31 (3H, m), 7.13-7.03 (5H, m), 7.00-6.94 (3H, m), 3.82 (3H, s), 3.70 (3H, s), 2.24 (3H, s), 2.18 (3H, s).
Present compound 4-25: $^1$H-NMR ($CDCl_3$) δ: 7.57 (1H, s), 7.47-7.27 (6H, m), 7.13 (1H, s), 7.00 (1H, s), 6.97-6.92 (3H, m), 5.08 (2H, s), 3.82 (3H, s), 3.71 (3H, s), 2.21 (3H, s), 2.19 (3H, s).
Present compound 4-26: LCMS: 342 $[M+H]^+$, RT = 2.11 min.
Present compound 4-27: LCMS: 337 $[M+H]^+$, RT = 2.31 min.
Present compound 4-28: LCMS: 345 $[M+H]^+$, RT = 2.04 min.
Present compound 4-29: LCMS: 333 $[M+H]^+$, RT = 2.16 min.
Present compound 4-30: LCMS: 324 $[M+H]^+$, RT = 2.04 min.

Preparation Example 5

Under nitrogen atmosphere, a mixture of the intermediate compound 2-1 0.18 g, 2-bromo-3-fluoropyridine 0.09 mL, $PdCl_2$(dppf) 0.02 g, tripotassium phosphate 0.24 g, dimethoxyethane 4 mL, and water 1 mL was stirred at 80°C for 8 hours. Water was added to the resulting mixture, and the mixture was extracted with ethyl acetate. The resulting organic layer was washed with saturated brine, dried over anhydrous sodium sulfate, and concentrated under reduced

pressure. The resulting residue was subjected to a silica gel column chromatography (ethyl acetate : hexane= 2 : 3) to obtain the present compound 5-1 represented by the following formula 0.10 g.

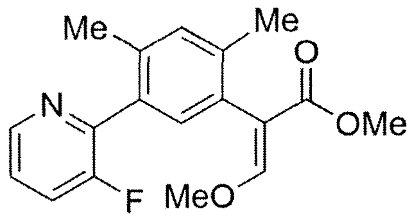

Present compound 5-1: $^{1}$H-NMR ($CDCl_3$) $\delta$: 8.51-8.49 (1H, m), 7.57 (1H, s), 7.49-7.43 (1H, m), 7.31-7.25 (1H, m), 7.17 (1H, d), 7.14 (1H, s), 3.81 (3H, s), 3.68 (3H, s), 2.24 (3H, s), 2.20 (3H, s).

Preparation Example 5-1

The compounds which were prepared according to the Preparation Example 5 and their physical property value are shown below.
A compound represented by formula (5-1):

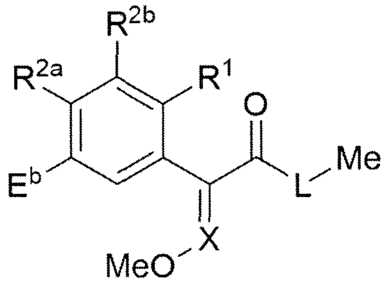

(5-1)

wherein $R^1$, $R^{2a}$, $R^{2b}$, $E^b$, X and L represent any combination described in [Table B2] (where • represents a binding site to a benzene ring).

[Table B2]

| Present compound | $R^1$ | $R^{2a}$ | $R^{2b}$ | $E^b$ | X | L |
|---|---|---|---|---|---|---|
| 5-2 | Me | Me | H | 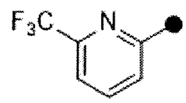 | CH | O |
| 5-3 | Me | Me | H | 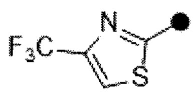 | CH | O |
| 5-4 | Me | Me | H | 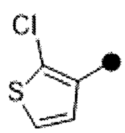 | N | O |

(continued)

| Present compound | $R^1$ | $R^{2a}$ | $R^{2b}$ | $E^b$ | X | L |
|---|---|---|---|---|---|---|
| 5-5 | Me | Me | H | 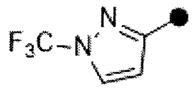 | CH | O |
| Present compound 5-2: $^1$H-NMR ($CDCl_3$) δ: 7.89 (1H, t), 7.63-7.57 (3H, m), 7.19 (1H, s), 7.17 (1H, s), 3.83 (3H, s), 3.70 (3H, s), 2.40 (3H, s), 2.20 (3H, s).<br>Present compound 5-3: $^1$H-NMR ($CDCl_3$) δ: 7.18 (1H, s), 7.13 (1H, d), 7.01 (1H, s), 6.90 (1H, d), 4.07 (3H, s), 3.87 (3H, s), 2.22 (3H, s), 2.20 (3H, s).<br>Present compound 5-4: $^1$H-NMR ($CDCl_3$) δ: 7.18 (1H, s), 7.13 (1H, d), 7.01 (1H, s), 6.90 (1H, d), 4.07 (3H, s), 3.87 (3H, s), 2.22 (3H, s), 2.20 (3H, s).<br>Present compound 5-5: $^1$H-NMR ($CDCl_3$) δ: 7.87 (1H, s), 7.84 (1H, s), 7.58 (1H, s), 7.16 (1H, s), 7.07 (1H, s), 3.84 (3H, s), 3.72 (3H, s), 2.35 (3H, s), 2.18 (3H, s). | | | | | | |

Preparation Example 6

Under nitrogen atmosphere, a mixture of the intermediate compound 1-5 0.10 g, 1-cyclopentenyl boronic acid 0.08 g, $PdCl_2$(dppf) 0.02 g, tripotassium phosphate 0.21 g, dimethoxyethane 4 mL, and water 1 mL was stirred at 80°C for 6 hours. Water was added to the resulting mixture, and the mixture was extracted with ethyl acetate. The resulting organic layer was washed with saturated brine, dried over anhydrous sodium sulfate, and concentrated under reduced pressure. The resulting residue was subjected to a silica gel column chromatography (ethyl acetate : hexane= 1 : 3) to obtain the present compound 6- 1 represented by the following formula 0.08 g.

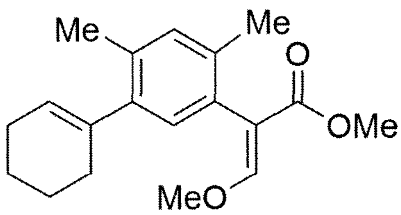

Present compound 6-1: $^1$H-NMR ($CDCl_3$) δ: 7.55 (1H, s), 7.02 (1H, s), 6.82 (1H, s), 5.58-5.54 (1H, m), 3.81 (3H, s), 3.70 (3H, s), 2.24 (3H, s), 2.21-2.16 (2H, m), 2.16-2.11 (5H, m), 1.76-1.69 (2H, m), 1.69-1.62 (2H, m).

Preparation Example 6-1

The compounds which were prepared according to the Preparation Example 6 and their physical property value are shown below.

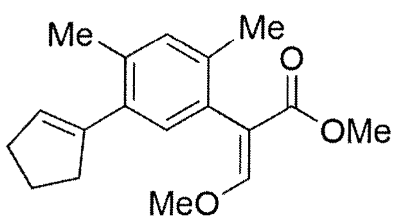

Present compound 6-2: LCMS: 287 $[M+H]^+$, RT = 2.25 min.

Next, examples of the present compounds and intermediate compounds which are prepared according to either the Preparation Examples described in the Working Examples or the processes described in the Description of the present application are indicated below.

A compound represented by formula (1A):

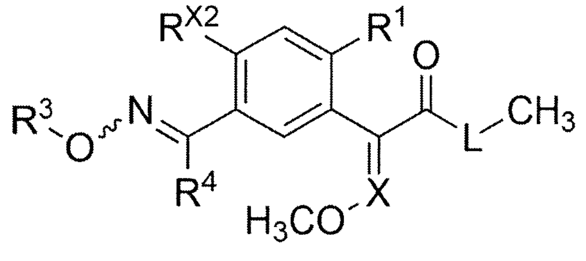

(1A)

(hereinafter, referred to as Compound (1A)) wherein X represents CH, L represents an oxygen atom, $R^1$ represents a methyl group, $R^{X2}$ represents a methyl group, and $R^3$ and $R^4$ represent any combination described in Combination A (hereinafter, referred to as Compound class SX1).

Combination A consists of substituent numbers ZA1 to ZA364. Substituent numbers ZA1 to ZA364 represent any combination of $R^3$ and $R^4$ in the compound (1A), which is hereinafter referred to as [Substituent No.; $R^3$, $R^4$]. For example, Substituent No. ZA2 represents a combination wherein $R^3$ represents an ethyl group, and $R^4$ represents a hydrogen atom.

Combination A

[ZA1;Me,H], [ZA2;Et,H], [ZA3;Pr,H], [ZA4;Bu,H], [ZA5;Pen,H], [ZA 6;Hex,H],[ZA7;i-Pr,H],[ZA8;i-Bu,H],[ZA9;t-Bu,H],[ZA10;c-Pr, H],[ZA11;c-Bu,H],[ZA12;c-Pen,H],[ZA13;c-Hex,H],[ZA14;$CH_2$c-P r,H],[ZA15;$CH_2$c-Pen,H],[ZA16;$CH_2$c-Hex,H],[ZA17;$CH_2CH=CH_2$,H], [ZA18;$CH_2CH=CHMe$,H],[ZA19;$CH_2CH=CMe_2$,H],[ZA20;$CH_2C≡CH$,H],[Z A21;$CH_2C≡CMe$,H],[ZA22;$CH_2Ph$,H],[ZA23;$CH_2$(2-F-Ph),H],[ZA24;C $H_2$(3-F-Ph),H],[ZA25;$CH_2$(4-F-Ph),H],[ZA26;$CH_2$(2-Cl-Ph),H],[Z A27;$CH_2$(3-Cl-Ph),H],[ZA28;$CH_2$(4-Cl-Ph),H],[ZA29;$CH_2$(2-Br-P h),H],[ZA30;$CH_2$ (3-Br-Ph),H],[ZA31;$CH_2$(4-Br-Ph),H],[ZA32;$CH_2$ (2-Me-Ph),H],[ZA33;$CH_2$(3-Me-Ph),H],[ZA34;$CH_2$(4-Me-Ph),H],[Z A35;$CH_2$(2-Et-Ph),H],[ZA36;$CH_2$(3-Et-Ph),H],[ZA37;$CH_2$(4-Et-P h),H],[ZA38;$CH_2$(2-t-Bu-Ph),H],[ZA39;$CH_2$(3-t-Bu-Ph),H],[ZA4 0;$CH_2$(4-t-Bu-Ph),H],[ZA41;$CH_2$(2-$CF_3$-Ph),H],[ZA42;$CH_2$(3-$CF_3$-Ph),H],[ZA43;$CH_2$(4-$CF_3$-Ph),H],[ZA44;$CH_2$(2-OMe-Ph),H],[ZA45; $CH_2$(3-OMe-Ph),H],[ZA46;$CH_2$(4-OMe-Ph),H],[ZA47;$CH_2$(2-SMe-Ph), H],[ZA48;$CH_2$(3-SMe-Ph),H],[ZA49;$CH_2$(4-SMe-Ph),H],[ZA50;$CH_2$ (2-CN-Ph),H],[ZA51;$CH_2$(3-CN-Ph),H],[ZA52;$CH_2$(4-CN-Ph),H],[Z A53;$CH_2$(2,4-$F_2$-Ph),H],[ZA54;$CH_2$(2,5-$F_2$-Ph),H],[ZA55;$CH_2$(3,4 -$F_2$-Ph),H],[ZA56;$CH_2$(3,5-$F_2$-Ph),H],[ZA57;$CH_2$(2,4-$Cl_2$-Ph),H], [ZA58;$CH_2$(2,5-$Cl_2$-Ph),H],[ZA59;$CH_2$(3,4-$Cl_2$-Ph),H],[ZA60;$CH_2$ (3,5-$Cl_2$-Ph),H],[ZA61;$CH_2$(2,4-$Me_2$-Ph),H],[ZA62;$CH_2$(2,5-$Me_2$-Ph),H],[ZA63;$CH_2$(3,4-$Me_2$-Ph),H],[ZA64;$CH_2$(3,5-$Me_2$-Ph),H],[Z A65;$CH_2$(2-F-4-Cl-Ph),H],[ZA66;$CH_2$(2-Cl-4-F-Ph),H],[ZA67;$CH_2$ (2-F-4-Me-Ph),H],[ZA68;$CH_2$(2-Me-4-F-Ph),H],[ZA69;$CH_2$(2-F-4-OMe-Ph),H],[ZA70;$CH_2$(2-OMe-4-F-Ph),H],[ZA71;$CH_2$(2-Me-4-Cl-P h),H],[ZA72;$CH_2$(2-Cl-4-Me-Ph),H],[ZA73;$CH_2$(2-Me-4-OMe-Ph), H],[ZA74;$CH_2$(2-OMe-4-Me-Ph),H],[ZA75;$CH_2$(2-Cl-4-OMe-Ph),H], [ZA76;$CH_2$(2-OMe-4-Cl-Ph),H],[ZA77;$CH_2$(2,4,6-$F_3$-Ph),H],[ZA7 8;$CH_2$(3,4,5-$F_3$-Ph),H],[ZA79;$CH_2$(2-pyridyl),H],[ZA80;$CH_2$(3-p yridyl),H],[ZA81;$CH_2$(4-pyridyl),H],[ZA82;$CH_2$(3-F-pyridin-2-yl),H],[ZA83;$CH_2$(4-F-pyridin-2-yl),H],[ZA84;$CH_2$(5-F-pyridin -2-yl),H],[ZA85;$CH_2$(6-F-pyridin-2-yl),H],[ZA86;$CH_2$(3-Cl-pyr idin-2-yl),H],[ZA87;$CH_2$(4-Cl-pyridin-2-yl),H],[ZA88;$CH_2$(5-C 1-pyridin-2-yl),H],[ZA89;$CH_2$(6-Cl-pyridin-2-yl),H],[ZA90;CH $_2$(3-Me-pyridin-2-yl),H],[ZA91;$CH_2$(4-Me-pyridin-2-yl),H],[ZA 92;$CH_2$(5-Me-pyridin-2-yl),H],[ZA93;$CH_2$(6-Me-pyridin-2-yl), H],[ZA94;$CH_2$(3-OMe-pyridin-2-yl),H],[ZA95;$CH_2$(4-OMe-pyridin -2-yl),H],[ZA96;CH2(5-OMe-pyridin-2-yl),H],[ZA97;CH2(6-OMe-pyridin-2-yl),H],[ZA98;$CH_2$(3-$CF_3$-pyridin-2-yl),H],[ZA99;$CH_2$ (4-$CF_3$-pyridin-2-yl),H],[ZA100;$CH_2$(5-$CF_3$-pyridin-2-yl),H], [ZA101;$CH_2$(6-$CF_3$-pyridin-2-yl),H],[ZA102;$CH_2$(3-CN-pyridin-2 -yl),H],[ZA103;$CH_2$(4-CN-pyridin-2-yl),H],[ZA104;$CH_2$(5-CN-py ridin-2-yl),H],[ZA105;$CH_2$(6-CN-pyridin-2-yl),H],[ZA106;$CH_2$ (2-F-pyridin-3-yl),H],[ZA107;$CH_2$(4-F-pyridin-3-yl),H],[ZA10 8;$CH_2$(5-F-pyridin-3-yl),H],[ZA109;$CH_2$(6-F-pyridin-3-yl),H], [ZA110;$CH_2$(2-Cl-pyridin-3-yl),H],[ZA111;$CH_2$(4-Cl-pyridin-3-yl),H],[ZA112;$CH_2$(5-Cl-pyridin-3-yl),H],[ZA113;$CH_2$(6-Cl-pyr idin-3-yl),H],[ZA114;$CH_2$(2-Me-pyridin-3-yl),H],[ZA115;$CH_2$(4 -Me-pyridin-3-yl),H],[ZA116;$CH_2$(5-Me-pyridin-3-yl),H],[ZAll 7;$CH_2$(6-Me-pyridin-3-yl),H],[ZA118;$CH_2$(2-OMe-pyridin-3-yl), H],[ZA119;$CH_2$ (4-OMe-pyridin-3-yl),H],[ZA120;$CH_2$(5-OMe-pyrid in-3-yl),H],[ZA121;$CH_2$(6-OMe-pyridin-3-yl),H],[ZA122;$CH_2$(2-$CF_3$-pyridin-3-yl),H],[ZA123;$CH_2$(4-$CF_3$-pyridin-3-yl),H],[ZA1 24;$CH_2$(5-$CF_3$-pyridin-3-yl),H],[ZA125;$CH_2$(6-$CF_3$-pyridin-3-y 1),H],[ZA126;$CH_2$(2-CN-pyridin-3-yl),H],[ZA127;$CH_2$(4-CN-pyri din-3-yl),H],[ZA128;$CH_2$(5-CN-pyridin-3-yl),H],[ZA129;$CH_2$(6-CN-pyridin-3-yl),H],[ZA130;$CH_2$(2-F-pyridin-4-yl),H],[ZA131; $CH_2$(3-F-pyridin-4-yl),H],[ZA132;$CH_2$(2-Cl-pyridin-4-yl),H], [ZA133;$CH_2$(3-Cl-pyridin-4-yl),H],[ZA134;$CH_2$(2-Me-pyridin-4-yl),H],[ZA135;$CH_2$(3-Me-pyridin-4-yl),H],[ZA136;$CH_2$(2-OMe-py ridin-4-yl),H],[ZA137;$CH_2$(3-OMe-pyridin-4-yl),H],[ZA138;$CH_2$ (2-$CF_3$-pyridin-4-yl),H],[ZA139;$CH_2$(3-$CF_3$-pyridin-4-yl),H], [ZA140;$CH_2$(2-CN-pyridin-4-yl),H],[ZA141;$CH_2$(3-CN-pyridin-4-yl),H],[ZA142;$CH_2$(2-Thienyl),H],[ZA143;$CH_2$(3-Thienyl),H],[Z A144;$CH_2$(2-pyrimidinyl),H],[ZA145;$CH_2$(4-pyrimidinyl),H],[ZA 146;$CH_2$(5-pyrimidinyl),H],[ZA147;$CH_2$(3-

pyridazinyl),H],[ZA1 48;$CH_2$(4-pyridazinyl),H],[ZA149;$CH_2$(4-Me-thiazol-2-yl),H], [ZA150;$CH_2$(4-Cl-thiazol-2-yl),H],[ZA151;$CH_2$(5-Me-thiazol-2-yl),H],[ZA152;$CH_2$(5-Cl-thiazol-2-yl),H],[ZA153;$(CH_{22}$Ph,H], [ZA154;$(CH_{23}$Ph,H],[ZA155;$CH_2$OMe,H],[ZA156;$CH_2$OEt,H],[ZA15 7;$CH_2$OPr,H],[ZA158;$CH_2$OPh,H],[ZA159;$CH_2$CN,H],[ZA160;Ph,H], [ZA161;2-F-Ph,H],[ZA162;3-F-Ph,H],[ZA163;4-F-Ph,H],[ZA164;2 -Cl-Ph,H],[ZA165;3-Cl-Ph,H],[ZA166;4-Cl-Ph,H],[ZA167;2-Me-P h,H],[ZA168;3-Me-Ph,H],[ZA169;4-Me-Ph,H],[ZA170;2-OMe-Ph,H], [ZA171;3-OMe-Ph,H],[ZA172;4-OMe-Ph,H],[ZA173;2-Pyridyl,H], [ZA174;3-Pyridyl,H],[ZA175;4-Pyridyl,H],[ZA176;2-Thienyl,H], [ZA177;3-Thienyl,H],[ZA178;2-pyrimidinyl,H],[ZA179;4-pyrimi dinyl,H],[ZA180;5-pyrimidinyl,H],[ZA181;3-pyridazinyl,H],[Z A182;4-pyridazinyl,H],[ZA183;Me,Me],[ZA184;Et,Me],[ZA185;P r,Me],[ZA186;Bu,Me],[ZA187;Pen,Me],[ZA188;Hex,Me],[ZA189;i-Pr,Me],[ZA190;i-Bu,Me],[ZA191;t-Bu,Me],[ZA192;c-Pr,Me],[ZA1 93;c-Bu,Me],[ZA194;c-Pen,Me],[ZA195;c-Hex,Me],[ZA196;$CH_2$c-P r,Me],[ZA197;$CH_2$c-Pen,Me],[ZA198;$CH_2$c-Hex,Me],[ZA199;$CH_2$CH=$CH_2$,Me],[ZA200;$CH_2$CH=CHMe,Me], [ZA201;$CH_2$CH=$CMe_2$,Me],[ZA202;$CH_2$C=CH,Me],[ZA203;$CH_2$C=CMe,Me],[ZA204;$CH_2$Ph,Me],[ZA205;$CH_2$(2-F-Ph),Me],[ZA206;$CH_2$(3-F-P h),Me],[ZA207;$CH_2$(4-F-Ph),Me],[ZA208;$CH_2$(2-Cl-Ph),Me],[ZA20 9;$CH_2$(3-Cl-Ph),Me],[ZA210;$CH_2$(4-Cl-Ph),Me],[ZA211;$CH_2$(2-Br-Ph),Me],[ZA212;$CH_2$(3-Br-Ph),Me],[ZA213;$CH_2$(4-Br-Ph),Me],[ZA 214;$CH_2$(2-Me-Ph)Me],[ZA215;$CH_2$(3-Me-Ph),Me],[ZA216;$CH_2$(4-M e-Ph),Me],[ZA217;$CH_2$(2-Et-Ph),Me],[ZA218;$CH_2$(3-Et-Ph),Me], [ZA219;$CH_2$(4-Et-Ph),Me],[ZA220;$CH_2$(2-t-Bu-Ph),Me],[ZA221;CH $_2$(3-t-Bu-Ph),Me],[ZA222;$CH_2$(4-t-Bu-Ph),Me],[ZA223;$CH_2$(2-$CF_3$ -Ph),Me],[ZA224;$CH_2$(3-$CF_3$-Ph),Me],[ZA225;$CH_2$(4-$CF_3$-Ph),Me], [ZA226;$CH_2$(2-OMe-Ph),Me],[ZA227;$CH_2$(3-OMe-Ph),Me],[ZA228;CH $_2$(4-OMe-Ph),Me],[ZA229;$CH_2$(2-SMe-Ph),Me],[ZA230;$CH_2$(3-SMe-P h),Me],[ZA231;$CH_2$(4-SMe-Ph),Me],[ZA232;$CH_2$(2-CN-Ph),Me],[ZA 233;$CH_2$(3-CN-Ph),Me],[ZA234;$CH_2$(4-CN-Ph),Me],[ZA235;$CH_2$(2,4 -$F_2$-Ph),Me],[ZA236;$CH_2$(2,5-$F_2$-Ph),Me],[ZA237;$CH_2$(3,4-$F_2$-Ph), Me],[ZA238;$CH_2$(3,5-$F_2$-Ph),Me],[ZA239;$CH_2$(2,4-$Cl_2$-Ph),Me],[Z A240;$CH_2$(2,5-$Cl_2$-Ph),Me],[ZA241;$CH_2$(3,4-$Cl_2$-Ph),Me],[ZA242; $CH_2$(3,5-$Cl_2$-Ph),Me],[ZA243;$CH_2$(2,4-$Me_2$-Ph),Me],[ZA244;$CH_2$(2,5-$Me_2$-Ph),Me],[ZA245;$CH_2$(3,4-$Me_2$-Ph),Me],[ZA246;$CH_2$(3,5-$Me_2$ -Ph),Me],[ZA247;$CH_2$(2-F-4-Cl-Ph),Me],[ZA248;$CH_2$(2-Cl-4-F-P h),Me],[ZA249;$CH_2$(2-F-4-Me-Ph),Me],[ZA250;$CH_2$(2-Me-4-F-Ph), Me],[ZA251;$CH_2$(2-F-4-OMe-Ph),Me],[ZA252;$CH_2$(2-OMe-4-F-Ph),M e],[ZA253;$CH_2$(2-Me-4-Cl-Ph),Me],[ZA254;$CH_2$(2-Cl-4-Me-Ph),Me],[ZA255;$CH_2$(2-Me-4-OMe-Ph),Me],[ZA256;$CH_2$(2-OMe-4-Me-Ph), Me],[ZA257;$CH_2$(2-Cl-4-OMe-Ph),Me],[ZA258;$CH_2$(2-OMe-4-Cl-Ph), Me],[ZA259;$CH_2$(2,4,6-$F_3$-Ph),Me],[ZA260;$CH_2$(3,4,5-$F_3$-Ph),Me], [ZA261;$CH_2$(2-pyridyl),Me],[ZA262;$CH_2$(3-pyridyl),Me],[ZA263; $CH_2$(4-pyridyl),Me],[ZA264;$CH_2$(3-F-pyridin-2-yl),Me],[ZA265; $CH_2$(4-F-pyridin-2-yl),Me],[ZA266;$CH_2$(5-F-pyridin-2-yl),Me], [ZA267;$CH_2$(6-F-pyridin-2-yl),Me],[ZA268;$CH_2$(3-Cl-pyridin-2-yl),Me],[ZA269;$CH_2$(4-Cl-pyridin-2-yl),Me],[ZA270;$CH_2$(5-Cl-p yridin-2-yl),Me],[ZA271;$CH_2$(6-Cl-pyridin-2-yl),Me],[ZA272;C $H_2$(3-Me-pyridin-2-yl),Me],[ZA273;$CH_2$(4-Me-pyridin-2-yl),Me], [ZA274;$CH_2$(5-Me-pyridin-2-yl),Me],[ZA275;$CH_2$(6-Me-pyridin-2 -yl),Me],[ZA276;$CH_2$(3-OMe-pyridin-2-yl),Me],[ZA277;$CH_2$(4-OM e-pyridin-2-yl),Me],[ZA278;$CH_2$(5-OMe-pyridin-2-yl),Me],[ZA2 79;$CH_2$(6-OMe-pyridin-2-yl),Me],[ZA280;$CH_2$(3-$CF_3$-pyridin-2-y 1),Me],[ZA281;$CH_2$(4-$CF_3$-pyridin-2-yl),Me],[ZA282;$CH_2$(5-$CF_3$-pyridin-2-yl),Me],[ZA283;$CH_2$(6-$CF_3$-pyridin-2-yl),Me],[ZA28 4;$CH_2$(3-CN-pyridin-2-yl),Me],[ZA285;$CH_2$(4-CN-pyridin-2-yl), Me],[ZA286;$CH_2$(5-CN-pyridin-2-yl),Me],[ZA287;$CH_2$(6-CN-pyrid in-2-yl),Me],[ZA288;$CH_2$(2-F-pyridin-3-yl),Me],[ZA289;$CH_2$(4-F-pyridin-3-yl),Me],[ZA290;$CH_2$(5-F-pyridin-3-yl),Me],[ZA29 1;$CH_2$(6-F-pyridin-3-yl),Me],[ZA292;$CH_2$(2-Cl-pyridin-3-yl),M e],[ZA293;$CH_2$(4-Cl-pyridin-3-yl),Me],[ZA294;$CH_2$(5-Cl-pyridi n-3-yl),Me],[ZA295;$CH_2$(6-Cl-pyridin-3-yl),Me],[ZA296;$CH_2$(2-Me-pyridin-3-yl),Me],[ZA297;$CH_2$(4-Me-pyridin-3-yl),Me],[ZA2 98;$CH_2$(5-Me-pyridin-3-yl),Me],[ZA299;$CH_2$(6-Me-pyridin-3-yl), Me],[ZA300;$CH_2$(2-OMe-pyridin-3-yl),Me], [ZA301;$CH_2$(4-OMe-pyridin-3-yl),Me],[ZA302;$CH_2$(5-OMe-pyridin -3-yl),Me],[ZA303;$CH_2$(6-OMe-pyridin-3-yl),Me],[ZA304;$CH_2$(2-$CF_3$-pyridin-3-yl),Me],[ZA305;$CH_2$(4-$CF_3$-pyridin-3-yl),Me],[Z A306;$CH_2$(5-$CF_3$-pyridin-3-yl),Me],[ZA307;$CH_2$(6-$CF_3$-pyridin-3 -yl),Me],[ZA308;$CH_2$(2-CN-pyridin-3-yl),Me],[ZA309;$CH_2$(4-CN-pyridin-3-yl),Me],[ZA310;$CH_2$(5-CN-pyridin-3-yl),Me],[ZA311; $CH_2$(6-CN-pyridin-3-yl),Me],[ZA312;$CH_2$(2-F-pyridin-4-yl),Me], [ZA313;$CH_2$(3-F-pyridin-4-yl),Me],[ZA314;$CH_2$(2-Cl-pyridin-4-yl),Me],[ZA315;$CH_2$(3-Cl-pyridin-4-yl),Me],[ZA316;$CH_2$(2-Me-p yridin-4-yl),Me],[ZA317;$CH_2$(3-Me-pyridin-4-yl),Me],[ZA318;C $H_2$(2-OMe-pyridin-4-yl),Me],[ZA319;$CH_2$(3-OMe-pyridin-4-yl),M e],[ZA320;$CH_2$(2-$CF_3$-pyridin-4-yl),Me],[ZA321;$CH_2$(3-$CF_3$-pyri din-4-yl),Me],[ZA322;$CH_2$(2-CN-pyridin-4-yl),Me],[ZA323;$CH_2$ (3-CN-pyridin-4-yl),Me],[ZA324;$CH_2$(2-Thienyl),Me],[ZA325;CH $_2$(3-Thienyl),Me],[ZA326;$CH_2$(2-pyrimidinyl),Me],[ZA327;$CH_2$(4 -pyrimidinyl),Me],[ZA328;$CH_2$(5-pyrimidinyl),Me],[ZA329;$CH_2$ (3-pyridazinyl),Me],[ZA330;$CH_2$(4-pyridazinyl),Me],[ZA331;CH $_2$(4-Me-thiazol-2-yl),Me],[ZA332;$CH_2$(4-Cl-thiazol-2-yl),Me], [ZA333;$CH_2$(5-Me-thiazol-2-yl),Me],[ZA334;$CH_2$(5-Cl-thiazol-2 -yl),Me],[ZA335;$(CH_{22}$Ph,Me],[ZA336;$(CH_{23}$Ph,Me],[ZA337;$CH_2$OM e,Me],[ZA338;$CH_2$OEt,Me],[ZA339;$C_2$OPr,Me],[ZA340;$CH_2$OPh,Me], [ZA341;$CH_2$CN,Me],[ZA342;Ph,Me],[ZA343;2-F-Ph,Me],[ZA344;3-F -Ph,Me],[ZA345;4-F-Ph,Me],[ZA346;2-Cl-Ph,Me],[ZA347;3-Cl-P h,Me],[ZA348;4-Cl-Ph,Me],[ZA349;2-Me-Ph,Me],[ZA350;3-Me-Ph, Me], [ZA351;4-Me-Ph,Me],[ZA352;2-OMe-Ph,Me],[ZA353;3-OMe-Ph, Me],[ZA354;4-OMe-Ph,Me],[ZA355;2-Pyridyl,Me],[ZA356;3-Pyrid yl,Me],[ZA357;4-Pyridyl,Me],[ZA358;2-Thienyl,Me],[ZA359;3-T hienyl,Me],[ZA360;2-pyrimidinyl,Me],[ZA361;4-pyrimidinyl,M e],[ZA362;5-pyrimidinyl,Me],[ZA363;3-pyridazinyl,Me],[ZA36 4;4-pyridazinyl,Me]

The Compound (1A) wherein X represents CH, L represents an oxygen atom, $R^1$ represents a methyl group, $R^{x2}$ represents an ethyl group, and $R^3$ and $R^4$ represent any combination described in Combination A (hereinafter, referred to as Compound Class SX2).

The Compound (1A) wherein X represents CH, L represents an oxygen atom, $R^1$ represents a methyl group, $R^{x2}$ represents a fluorine atom, and $R^3$ and $R^4$ represent any combination described in Combination A (hereinafter, referred to as Compound Class SX3).

The Compound (1A) wherein X represents CH, L represents an oxygen atom, $R^1$ represents a methyl group, $R^{x2}$ represents a chlorine atom, and $R^3$ and $R^4$ represent any combination described in Combination A (hereinafter, referred to as Compound Class SX4).

The Compound (1A) wherein X represents CH, L represents an oxygen atom, $R^1$ represents a methyl group, $R^{x2}$ represents a methoxy group, and $R^3$ and $R^4$ represent any combination described in Combination A (hereinafter, referred to as Compound Class SX5).

The Compound (1A) wherein X represents CH, L represents an oxygen atom, $R^1$ represents a methyl group, $R^{x2}$ represents a cyclopropyl group, and $R^3$ and $R^4$ represent any combination described in Combination A (hereinafter, referred to as Compound Class SX6).

The Compound (1A) wherein X represents CH, L represents an oxygen atom, $R^1$ represents a fluorine atom, $R^{x2}$ represents a methyl group, and $R^3$ and $R^4$ represent any combination described in Combination A (hereinafter, referred to as Compound Class SX7).

The Compound (1A) wherein X represents CH, L represents an oxygen atom, $R^1$ represents a fluorine atom, $R^{x2}$ represents a fluorine atom, and $R^3$ and $R^4$ represent any combination described in Combination A (hereinafter, referred to as Compound Class SX8).

The Compound (1A) wherein X represents CH, L represents an oxygen atom, $R^1$ represents a fluorine atom, $R^{x2}$ represents a chlorine atom, and $R^3$ and $R^4$ represent any combination described in Combination A (hereinafter, referred to as Compound Class SX9).

The Compound (1A) wherein X represents CH, L represents an oxygen atom, $R^1$ represents a chlorine atom, $R^{x2}$ represents a methyl group, and $R^3$ and $R^4$ represent any combination described in Combination A (hereinafter, referred to as Compound Class SX10).

The Compound (1A) wherein X represents CH, L represents an oxygen atom, $R^1$ represents a chlorine atom, $R^{x2}$ represents a fluorine atom, and $R^3$ and $R^4$ represent any combination described in Combination A (hereinafter, referred to as Compound Class SX11).

The Compound (1A) wherein X represents CH, L represents an oxygen atom, $R^1$ represents a chlorine atom, $R^{x2}$ represents a chlorine atom, and $R^3$ and $R^4$ represent any combination described in Combination A (hereinafter, referred to as Compound Class SX12).

The Compound (1A) wherein X represents a nitrogen atom, L represents an oxygen atom, $R^1$ represents a methyl group, $R^{x2}$ represents a methyl group, and $R^3$ and $R^4$ represent any combination described in Combination A (hereinafter, referred to as Compound Class SX13).

The Compound (1A) wherein X represents a nitrogen atom, L represents an oxygen atom, $R^1$ represents a methyl group, $R^{x2}$ represents an ethyl group, and $R^3$ and $R^4$ represent any combination described in Combination A (hereinafter, referred to as Compound Class SX14).

The Compound (1A) wherein X represents a nitrogen atom, L represents an oxygen atom, $R^1$ represents a methyl group, $R^{x2}$ represents a fluorine atom, and $R^3$ and $R^4$ represent any combination described in Combination A (hereinafter, referred to as Compound Class SX15).

The Compound (1A) wherein X represents a nitrogen atom, L represents an oxygen atom, $R^1$ represents a methyl group, $R^{x2}$ represents a chlorine atom, and $R^3$ and $R^4$ represent any combination described in Combination A (hereinafter, referred to as Compound Class SX16).

The Compound (1A) wherein X represents a nitrogen atom, L represents an oxygen atom, $R^1$ represents a methyl group, $R^{x2}$ represents a methoxy group, and $R^3$ and $R^4$ represent any combination described in Combination A (hereinafter, referred to as Compound Class SX17).

The Compound (1A) wherein X represents a nitrogen atom, L represents an oxygen atom, $R^1$ represents a methyl group, $R^{x2}$ represents a cyclopropyl group, and $R^3$ and $R^4$ represent any combination described in Combination A (hereinafter, referred to as Compound Class SX18).

The Compound (1A) wherein X represents a nitrogen atom, L represents an oxygen atom, $R^1$ represents a fluorine atom, $R^{x2}$ represents a methyl group, and $R^3$ and $R^4$ represent any combination described in Combination A (hereinafter, referred to as Compound Class SX19).

The Compound (1A) wherein X represents a nitrogen atom, L represents an oxygen atom, $R^1$ represents a fluorine atom, $R^{x2}$ represents a fluorine atom, and $R^3$ and $R^4$ represent any combination described in Combination A (hereinafter, referred to as Compound Class SX20).

The Compound (1A) wherein X represents a nitrogen atom, L represents an oxygen atom, $R^1$ represents a

fluorine atom, $R^{x2}$ represents a chlorine atom, and $R^3$ and $R^4$ represent any combination described in Combination A (hereinafter, referred to as Compound Class SX21).

The Compound (1A) wherein X represents a nitrogen atom, L represents an oxygen atom, $R^1$ represents a chlorine atom, $R^{x2}$ represents a methyl group, and $R^3$ and $R^4$ represent any combination described in Combination A (hereinafter, referred to as Compound Class SX22).

The Compound (1A) wherein X represents a nitrogen atom, L represents an oxygen atom, $R^1$ represents a chlorine atom, $R^{x2}$ represents a fluorine atom, and $R^3$ and $R^4$ represent any combination described in Combination A (hereinafter, referred to as Compound Class SX23).

The Compound (1A) wherein X represents a nitrogen atom, L represents an oxygen atom, $R^1$ represents a chlorine atom, $R^{x2}$ represents a chlorine atom, and $R^3$ and $R^4$ represent any combination described in Combination A (hereinafter, referred to as Compound Class SX24).

The Compound (1A) wherein X represents a nitrogen atom, L represents NH, $R^1$ represents a methyl group, $R^{x2}$ represents a methyl group, and $R^3$ and $R^4$ represent any combination described in Combination A (hereinafter, referred to as Compound Class SX25).

The Compound (1A) wherein X represents a nitrogen atom, L represents NH, $R^1$ represents a methyl group, $R^{x2}$ represents an ethyl group, and $R^3$ and $R^4$ represent any combination described in Combination A (hereinafter, referred to as Compound Class SX26).

The Compound (1A) wherein X represents a nitrogen atom, L represents NH, $R^1$ represents a methyl group, $R^{x2}$ represents a fluorine atom, and $R^3$ and $R^4$ represent any combination described in Combination A (hereinafter, referred to as Compound Class SX27).

The Compound (1A) wherein X represents a nitrogen atom, L represents NH, $R^1$ represents a methyl group, $R^{x2}$ represents a chlorine atom, and $R^3$ and $R^4$ represent any combination described in Combination A (hereinafter, referred to as Compound Class SX28).

The Compound (1A) wherein X represents a nitrogen atom, L represents NH, $R^1$ represents a methyl group, $R^{x2}$ represents a methoxy group, and $R^3$ and $R^4$ represent any combination described in Combination A (hereinafter, referred to as Compound Class SX29).

The Compound (1A) wherein X represents a nitrogen atom, L represents NH, $R^1$ represents a methyl group, $R^{x2}$ represents a cyclopropyl group, and $R^3$ and $R^4$ represent any combination described in Combination A (hereinafter, referred to as Compound Class SX30).

The Compound (1A) wherein X represents a nitrogen atom, L represents NH, $R^1$ represents a fluorine atom, $R^{x2}$ represents a methyl group, and $R^3$ and $R^4$ represent any combination described in Combination A (hereinafter, referred to as Compound Class SX31).

The Compound (1A) wherein X represents a nitrogen atom, L represents NH, $R^1$ represents a fluorine atom, $R^{x2}$ represents a fluorine atom, and $R^3$ and $R^4$ represent any combination described in Combination A (hereinafter, referred to as Compound Class SX32).

The Compound (1A) wherein X represents a nitrogen atom, L represents NH, $R^1$ represents a fluorine atom, $R^{x2}$ represents a chlorine atom, and $R^3$ and $R^4$ represent any combination described in Combination A (hereinafter, referred to as Compound Class SX33).

The Compound (1A) wherein X represents a nitrogen atom, L represents NH, $R^1$ represents a chlorine atom, $R^{x2}$ represents a methyl group, and $R^3$ and $R^4$ represent any combination described in Combination A (hereinafter, referred to as Compound Class SX34).

The Compound (1A) wherein X represents a nitrogen atom, L represents NH, $R^1$ represents a chlorine atom, $R^{x2}$ represents a fluorine atom, and $R^3$ and $R^4$ represent any combination described in Combination A (hereinafter, referred to as Compound Class SX35).

The Compound (1A) wherein X represents a nitrogen atom, L represents NH, $R^1$ represents a chlorine atom, $R^{x2}$ represents a chlorine atom, and $R^3$ and $R^4$ represent any combination described in Combination A (hereinafter, referred to as Compound Class SX36).

A compound represented by formula (1B):

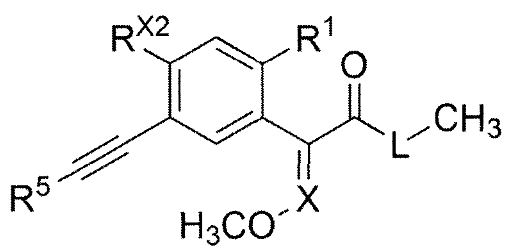

(1B)

(hereinafter, referred to as Compound (1B)) wherein X represents CH, L represents an oxygen atom, $R^1$ represents a methyl group, $R^{x2}$ represents a methyl group, and $R^5$ represents any substituent selected from Group V (hereinafter, referred to as Compound Class SX37).

Group V is a group consisting of Me,Et,Pr,i-Pr,c-Pr,c-Bu,c-Pen,c-Hex,$(CH_2)_3CH_3$,$CH_2CH(CH_3)_2$,$CH(CH_3)CH_2CH_3$,t-Bu,$CH_2$c-Pr,$(CH_2)_4CH_3$,$(CH_2)_2CH(CH_3)_2$,$CH(CH_3)(CH_2)_2CH_3$,$CH(CH_2CH_3)CH_2CH_3$,$CH_2CH(CH_3)CH_2CH_3$,$C(CH_3)_2CH_2CH_3$,$CH_2$t-Bu,$CH_2$c-Bu,$CH_2$c-P en, $(CH_2)_2$c-Pr, (1-methylcyclopropyl)methyl, (2-methylcyclopro pyl)methyl, 1-cyclopropylethyl, $(CH_2)_3$c-Pr, $(CH_2)_5CH_3$, $(CH_2)_3CH(CH_3)_2$, $(CH_2)_2CH(CH_3)CH_2CH_3$, $CH_2CH(CH_3)(CH_2)_2CH_3$, $CH(CH_3)(CH_2)_3CH_3$,$CH_2CH(CH_2CH_3)_2$, heptyl, $CH_2CH=CH_2$, $CH_2CH=CHCH_3$, $CH_2CH=C(CH_3)_2$,$CH_2CH=CF_2$,$CH_2CH=CCl_2$,$CH_2CH=CHCH_2CH_3$,$CH_2CH=CH(CH_2)_2CH_3$,$CH_2C(CH_3)=CH_2$,$CH_2C(CH_3)=CHCH_3$,$CH_2C(CH_3)=C(CH_3)_2$,$CH_2C(CH_3)=CH CH_2CH_3$, $CH_2CF=CH_2$, $CH_2CF=CHCH_3$, $CH_2CF=C(CH_3)_2$, $CH_2CF=CF_2$, $CH_2CF=CHCH_2CH_3$,$CH_2CF=CH(CH_2)_2CH_3$,$CH_2CCl=CH_2$,$CH_2CCl=CHCH_3$,$CH_2CCl=C(CH_3)_2$,$CH_2CCl=CCl_2$,$CH_2CCl=CHCH_2CH_3$,$CH_2CCl=CH(CH_2)_2CH_3$,$(CH_2)_2CH=CH_2$,$(CH_2)_2CH=CHCH_3$,$(CH_2)_2CH=CHCH_2CH_3$,$(CH_2)_2CH=C(CH_3)_2$,$(CH_2)_2C(CH_3)=CH_2$,$(CH_2)_2C(CH_3)=CHCH_3$,$(CH_2)_3CH=CH_2$,$(CH_2)_3C(CH_3)=CH_2$,$(CH_2)_4CH=CH_2$,$CH_2C\equiv CH$,$CH_2C\equiv CCH_3$,$CH_2C\equiv CCH_2CH_3$,$CH_2C\equiv C$c-Pr,$(CH_2)_2C\equiv CH$,$(CH_2)_2C\equiv CCH_3$,$(CH_2)_2C\equiv CCH_2CH_3$,$(CH_2)_3C\equiv CH$,$(CH_2)_3C\equiv CCH_3$,$CH_2Cl$,$CH_2Br$,$CH_2OCH_3$,$CH_2OCH_2CH_3$,$CH_2O(CH_2)_2CH_3$,$CHF_2$,$CF_3$,$CH_2CF_3$,$CH_2CHF_2$,$CH_2CH_2CHF_2$,$CH_2CH_2CF_3$,$CH_2CF_2CF_3$,$(CH_2)_2CF_2CF_3$,$CH_2(CF_2)_2CF_3$,$(CH_2)_2CF(CF_3)_2$,$(CH_2)_2(CF_2)_5CF_3$,$CF_3$,$CF_2CHF(CF_3)$,$CF_2CHF(OCF_3)$,$CH_2CF_2CF_2H$,Ph,2-F-Ph,3-F-Ph,4-F-Ph,2-Cl-Ph, 3-Cl-Ph,4-Cl-Ph,2-Me-Ph,3-Me-Ph,4-Me-Ph,2-OMe-Ph,3-OMe-Ph,4 -OMe-Ph,2-Pyridyl,3-Pyridyl,4-Pyridyl,2-Thienyl,3-Thienyl,2 -pyrimidinyl,4-pyrimidinyl,5-pyrimidinyl,3-pyridazinyl,and 4-pyridazinyl.

A compound (1B) wherein X represents CH, L represents an oxygen atom, $R^1$ represents methyl group, $R^{x2}$ represents an ethyl group, and $R^5$ represents any substituent selected from Group V (hereinafter, referred to as Compound Class SX38).

A compound (1B) wherein X represents CH, L represents an oxygen atom, $R^1$ represents a methyl group, $R^{x2}$ represents a fluorine atom, and $R^5$ represents any substituent selected from Group V (hereinafter, referred to as Compound Class SX39).

A compound (1B) wherein X represents CH, L represents an oxygen atom, $R^1$ represents a methyl group, $R^{x2}$ represents a chlorine atom, and $R^5$ represents any substituent selected from Group V (hereinafter, referred to as Compound Class SX40) .

A compound (1B) wherein X represents CH, L represents an oxygen atom, $R^1$ represents a methyl group, $R^{x2}$ represents a methoxy group, and $R^5$ represents any substituent selected from Group V (hereinafter, referred to as Compound Class SX41).

A compound (1B) wherein X represents CH, L represents an oxygen atom, $R^1$ represents a methyl group, $R^{x2}$ represents a cyclopropyl group, and $R^5$ represents any substituent selected from Group V (hereinafter, referred to as Compound Class SX42).

A compound (1B) wherein X represents CH, L represents an oxygen atom, $R^1$ represents a fluorine atom, $R^{x2}$ represents a methyl group, and $R^5$ represents any substituent selected from Group V (hereinafter, referred to as Compound Class SX43).

A compound (1B) wherein X represents CH, L represents an oxygen atom, $R^1$ represents a fluorine atom, $R^{x2}$ represents a fluorine atom, and $R^5$ represents any substituent selected from Group V (hereinafter, referred to as Compound Class SX44).

A compound (1B) wherein X represents CH, L represents an oxygen atom, $R^1$ represents a fluorine atom, $R^{x2}$ represents a chlorine atom, and $R^5$ represents any substituent selected from Group V (hereinafter, referred to as Compound Class SX45) .

A compound (1B) wherein X represents CH, L represents an oxygen atom, $R^1$ represents a chlorine atom, $R^{x2}$ represents a methyl group, and $R^5$ represents any substituent selected from Group V (hereinafter, referred to as Compound Class SX46) .

A compound (1B) wherein X represents CH, L represents an oxygen atom, $R^1$ represents a chlorine atom, $R^{x2}$ represents a fluorine atom, and $R^5$ represents any substituent selected from Group V (hereinafter, referred to as Compound Class SX47) .

A compound (1B) wherein X represents CH, L represents an oxygen atom, $R^1$ represents a chlorine atom, $R^{x2}$ represents a chlorine atom, and $R^5$ represents any substituent selected from Group V (hereinafter, referred to as Compound Class SX48).

A compound (1B) wherein X represents CH, L represents an oxygen atom, $R^1$ represents a methyl group, $R^{x2}$ represents a methyl group, and $R^5$ represents any substituent selected from Group V (hereinafter, referred to as Compound Class SX49) .

A compound (1B) wherein X represents a nitrogen atom, L represents an oxygen atom, $R^1$ represents a methyl group, $R^{x2}$ represents an ethyl group, and $R^5$ represents any substituent selected from Group V (hereinafter, referred to as Compound Class SX50).

A compound (1B) wherein X represents a nitrogen atom, L represents an oxygen atom, $R^1$ represents a methyl group, $R^{x2}$ represents a fluorine atom, and $R^5$ represents any substituent selected from Group V (hereinafter, referred to as Compound Class SX51).

A compound (1B) wherein X represents a nitrogen atom, L represents an oxygen atom, $R^1$ represents a methyl group, $R^{x2}$ represents a chlorine atom, and $R^5$ represents any substituent selected from Group V (hereinafter, referred to as Compound Class SX52).

A compound (1B) wherein X represents a nitrogen atom, L represents an oxygen atom, $R^1$ represents a methyl group, $R^{x2}$ represents a methoxy group, and $R^5$ represents any substituent selected from Group V (hereinafter, referred to as Compound Class SX53).

A compound (1B) wherein X represents a nitrogen atom, L represents an oxygen atom, $R^1$ represents a methyl group, $R^{x2}$ represents a cyclopropyl group, and $R^5$ represents any substituent selected from Group V (hereinafter, referred to as Compound Class SX54).

A compound (1B) wherein X represents a nitrogen atom, L represents an oxygen atom, $R^1$ represents a fluorine atom, $R^{x2}$ represents a methyl group, and $R^5$ represents any substituent selected from Group V (hereinafter, referred to as Compound Class SX55).

A compound (1B) wherein X represents a nitrogen atom, L represents an oxygen atom, $R^1$ represents a fluorine atom, $R^{x2}$ represents a fluorine atom, and $R^5$ represents any substituent selected from Group V (hereinafter, referred to as Compound Class SX56).

A compound (1B) wherein X represents a nitrogen atom, L represents an oxygen atom, $R^1$ represents a fluorine atom, $R^{x2}$ represents a chlorine atom, and $R^5$ represents any substituent selected from Group V (hereinafter, referred to as Compound Class SX57).

A compound (1B) wherein X represents a nitrogen atom, L represents an oxygen atom, $R^1$ represents a chlorine atom, $R^{x2}$ represents a methyl group, and $R^5$ represents any substituent selected from Group V (hereinafter, referred to as Compound Class SX58).

A compound (1B) wherein X represents a nitrogen atom, L represents an oxygen atom, $R^1$ represents a chlorine atom, $R^{x2}$ represents a fluorine atom, and $R^5$ represents any substituent selected from Group V (hereinafter, referred to as Compound Class SX59).

A compound (1B) wherein X represents a nitrogen atom, L represents an oxygen atom, $R^1$ represents a chlorine atom, $R^{x2}$ represents a chlorine atom, and $R^5$ represents any substituent selected from Group V (hereinafter, referred to as Compound Class SX60).

A compound (1B) wherein X represents a nitrogen atom, L represents NH, $R^1$ represents a methyl group, $R^{x2}$ represents a methyl group, and $R^5$ represents any substituent selected from Group V (hereinafter, referred to as Compound Class SX61).

A compound (1B) wherein X represents a nitrogen atom, L represents NH, $R^1$ represents a methyl group, $R^{x2}$ represents an ethyl group, and $R^5$ represents any substituent selected from Group V (hereinafter, referred to as Compound Class SX62).

A compound (1B) wherein X represents a nitrogen atom, L represents NH, $R^1$ represents a methyl group, $R^{x2}$ represents a fluorine atom, and $R^5$ represents any substituent selected from Group V (hereinafter, referred to as Compound Class SX63).

A compound (1B) wherein X represents a nitrogen atom, L represents NH, $R^1$ represents a methyl group, $R^{x2}$ represents a chlorine atom, and $R^5$ represents any substituent selected from Group V (hereinafter, referred to as Compound Class SX64).

A compound (1B) wherein X represents a nitrogen atom, L represents NH, $R^1$ represents a methyl group, $R^{x2}$ represents a methoxy group, and $R^5$ represents any substituent selected from Group V (hereinafter, referred to as Compound Class SX65).

A compound (1B) wherein X represents a nitrogen atom, L represents NH, $R^1$ represents a methyl group, $R^{x2}$ represents a cyclopropyl group, and $R^5$ represents any substituent selected from Group V (hereinafter, referred to as Compound Class SX66).

A compound (1B) wherein X represents a nitrogen atom, L represents NH, $R^1$ represents a fluorine atom, $R^{x2}$ represents a methyl group, and $R^5$ represents any substituent selected from Group V (hereinafter, referred to as Compound Class SX67).

A compound (1B) wherein X represents a nitrogen atom, L represents NH, $R^1$ represents a fluorine atom, $R^{x2}$ represents a fluorine atom, and $R^5$ represents any substituent selected from Group V (hereinafter, referred to as Compound Class SX68).

A compound (1B) wherein X represents a nitrogen atom, L represents NH, $R^1$ represents a fluorine atom, $R^{x2}$ represents a chlorine atom, and $R^5$ represents any substituent selected from Group V (hereinafter, referred to as Compound Class SX69).

A compound (1B) wherein X represents a nitrogen atom, L represents NH, $R^1$ represents a chlorine atom, $R^{x2}$ represents a methyl group, and $R^5$ represents any substituent selected from Group V (hereinafter, referred to as Compound Class SX70).

A compound (1B) wherein X represents a nitrogen atom, L represents NH, $R^1$ represents a chlorine atom, $R^{x2}$ represents a fluorine atom, and $R^5$ represents any substituent selected from Group V (hereinafter, referred to as Compound Class SX71).

A compound (1B) wherein X represents a nitrogen atom, L represents NH, $R^1$ represents a chlorine atom, $R^{x2}$ represents a chlorine atom, and $R^5$ represents any substituent selected from Group V (hereinafter, referred to as Compound Class SX72).

A compound represented by formula (2B):

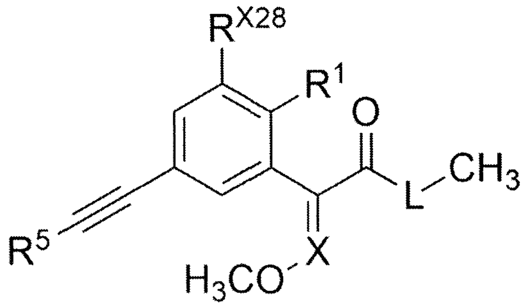

(2B)

(heeinafter, referred to as Compound (2B)) wherein X represents CH, L represents an oxygen atom, $R^1$ represents a methyl group, $R^{X28}$ represents a methyl group, and $R^5$ represents any substituent selected from Group V (hereinafter, referred to as Compound Class SX73).

A compound (2B) wherein X represents CH, L represents an oxygen atom, $R^1$ represents a methyl group, $R^{x28}$ represents a fluorine atom, and $R^5$ represents any substituent selected from Group V (hereinafter, referred to as Compound Class SX74).

A compound (2B) wherein X represents CH, L represents an oxygen atom, $R^1$ represents a methyl group, $R^{x28}$ represents a chlorine atom, and $R^5$ represents any substituent selected from Group V (hereinafter, referred to as Compound Class SX75).

A compound (2B) wherein X represents CH, L represents an oxygen atom, $R^1$ represents a methyl group, $R^{x28}$ represents a methoxy group, and $R^5$ represents any substituent selected from Group V (hereinafter, referred to as Compound Class SX76) .

A compound (2B) wherein X represents a nitrogen atom, L represents an oxygen atom, $R^1$ represents a methyl group, $R^{x28}$ represents a methyl group, and $R^5$ represents any substituent selected from Group V (hereinafter, referred to as Compound Class SX77).

A compound (2B) wherein X represents a nitrogen atom, L represents an oxygen atom, $R^1$ represents a methyl group, $R^{x28}$ represents a fluorine atom, and $R^5$ represents any substituent selected from Group V (hereinafter, referred to as Compound Class SX78).

A compound (2B) wherein X represents a nitrogen atom, L represents an oxygen atom, $R^1$ represents a methyl group, $R^{x28}$ represents a chlorine atom, and $R^5$ represents any substituent selected from Group V (hereinafter, referred to as Compound Class SX79).

A compound (2B) wherein X represents a nitrogen atom, L represents an oxygen atom, $R^1$ represents a methyl group, $R^{x28}$ represents a methyl group, and $R^5$ represents any substituent selected from Group V (hereinafter, referred to as Compound Class SX80).

A compound (2B) wherein X represents a nitrogen atom, L represents NH, $R^1$ represents a methyl group, $R^{x28}$ represents a fluorine atom, and $R^5$ represents any substituent selected from Group V (hereinafter, referred to as Compound Class SX81) .

A compound (2B) wherein X represents a nitrogen atom, L represents NH, $R^1$ represents a methyl group, $R^{x28}$ represents a chlorine atom, and $R^5$ represents any substituent selected from Group V (hereinafter, referred to as Compound Class SX82) .

A compound represented by formula (1C):

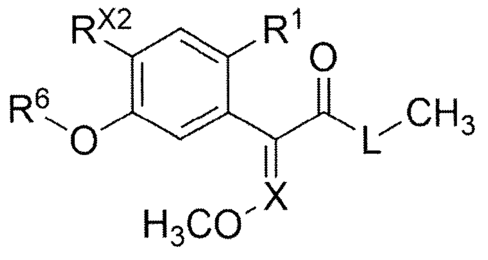

(1C)

(hereinafter, referred to as Compound (1C)) wherein X represents CH, L represents an oxygen atom, $R^1$ represents a methyl group, $R^{X2}$ represents a methyl group, and $R^6$ represents any substituent selected from Group Y (hereinafter, referred to as Compound Class SX83).

Group Y is a group consisting of Et,Pr,i-Pr,$(CH_2)_3CH_3$, $CH_2CH(CH_3)_2$,$CH(CH_3)CH_2CH_3$,$C(CH_3)_3$,$CH_2$c-Pr,$(CH_2)_4CH_3$,$(CH_2)_2CH(CH_3)_2$,$CH(CH_3)(CH_2)_2CH_3$,$CH(CH_2CH_3)CH_2CH_3$,$CH_2CH(CH_3)CH_2CH_3$,$C(CH_3)_2CH_2CH_3$,$CH_2C(CH_3)_3$,$CH_2$c-Bu,$CH_2$c-Pen,$CH_2$c-Hex,$(CH_2)_2$c-Pr, (1-methylcyclopropyl)methyl, (2-methylcyclopropyl)methyl, 1-cyclopropylethyl,$(CH_2)_3$c-Pr,$(CH_2)_5CH_3$,$(CH_2)_3CH(CH_3)_2$,$(CH_2)_2CH(CH_3)CH_2CH_3$,$CH_2CH(CH_3)(CH_2)_2CH_3$,$CH(CH_3)(CH_2)_3CH_3$,$CH_2CH(CH_2CH_3)_2$,heptyl,octyl,nonyl,decyl,$CH_2CH=CH_2$,$CH_2CH=CHCH_3$,$CH_2CH=C(CH_3)_2$,$CH_2CH=CF2$,$CH_2CH=CCl2$,$CH_2CH=CHCH_2CH_3$,$CH_2CH=CH(CH_2)_2CH_3$,$CH_2C(CH_3)=CH_2$,$CH_2C(CH_3)=CHCH_3$,$CH_2C(CH_3)=C(CH_3)_2$,$CH_2C(CH_3)=CHCH_2CH_3$,$CH_2C(CH_3)=CH(CH_2)_2CH_3$,$CH_2CF=CH_2$,$CH_2CF=CHCH_3$, $CH_2CF=C(CH_3)_2$,$CH_2CF=CF2$,$CH_2CF=CHCH_2CH_3$,$CH_2CF=CH(CH_2)_2CH_3$,$CH_2CCl=CH_2$,$CH_2CCl=CHCH_3$,$CH_2CCl=C(CH_3)_2$,$CH_Z2Cl=CCl2$,$CH_2CCl=CHCH_2CH_3$,$CH_2CCl=CH(CH_2)_2CH_3$,$(CH_2)_2CH=CH_2$,$(CH_2)_2CH=CHCH_3$,$(CH_2)_2CH=CHCH_2CH_3$,$(CH_2)_2CH=C(CH_3)_2$,$(CH_2)_2C(CH_3)=CH_2$,$(CH_2)_2C(CH_3)=CHCH_3$,$(CH_2)_2C(CH_3)=CHCH_2CH_3$,$(CH_2)_2C(CH_3)=C(CH_3)_2$,$(CH_2)_3CH=CH_2$,$(CH_2)_3C(CH_3)=CH_2$,$(CH_2)_4CH=CH_2$,$(CH_2)_4C(CH_3)=CH_2$,$CH_2C\equiv CH$,$CH_2C\equiv CCH_3$,$CH_2C\equiv CCH_2CH_3$,$CH_2C\equiv C$c-Pr,$CH_2C\equiv CPh$,$(CH_2)_2C\equiv CH$,$(CH_2)_2C\equiv CCH_3$,$(CH_2)_2C\equiv CCH_2CH_3$,$(CH_2)_2C\equiv C$c-Pr,$(CH_2)_2C\equiv CPh$,$(CH_2)_3C\equiv CH$,$(CH_2)_3C\equiv CCH_3$,$(CH_2)_3C\equiv CCH_2CH_3$,$(CH_2)_3C\equiv C$c-Pr,$(CH_2)_3C\equiv CPh$,$CH_2Cl$,$CH_2Br$,$CH_2CN$,$CH_2OCH_3$,$CH_2OCH_2CH_3$,$CH_2O(CH_2)_2CH_3$,$CH_2SCH_3$,$CH_2SCH_2CH_3$,$CH_2S(CH_2)_2CH_3$,$CH_2C(O)CH_3$,$CH_2C(O)CH_2CH_3$,$CH_2C(O)Ph$,$CH_2C(O)NH_2$,$CH_2C(O)NHCH_3$,$CH_2C(O)N(CH_3)_2$,$CH_2C(O)NHPh$,$CH_2C(O)N(CH_3)Ph$,$CH_2C(O)OCH_3$,$CH_2C(O)OCH_2CH_3$,$CH_2OC(O)Ph$,$CH_2OC(O)OCH_3$,$CH_2OC(O)OCH_2CH_3$,$CH_2OC(O)OPh$,$CH_2OC(O)NHCH_3$,$CH_2OC(O)NHCH_2CH_3$,$CH_2OC(O)NHPh$,$CH_2OC(O)N(CH_3)_2$,$CH_2OC(O)N(CH_3)CH_2CH_3$,$CH_2OC(O)N(CH_3)Ph$,$CH_2OC(O)N(CH_2CH_3)_2$,$CH_2$(2-oxiranyl),$CH_2$(2-tetrahydrofuranyl),$CH_2$(2-tetrahydropyranyl), $CH_2CH=NOCH_3$,$CH_2CH=NOCH_2CH_3$,$CH_2CH=NOCH_2Ph$,$CH_2C(CH_3)=NOCH_3$,$CH_2C(CH_3)=NOCH_2CH_3$, $CH_2C(CH_3)=NOCH_2Ph$,$(CH_2)_2F$,$CH_2CF_3$,$(CH_2)_2Cl$, $CH_2CCl_3$,$(CH_2)_2Br$,$(CH_2)_2I$,$(CH_2)_2CF_3$,$(CH_2)_2CN$,$(CH_2)_2NO2$,$(CH_2)_2OCH_3$,$(CH_2)_2OCH_2CH_3$,$(CH_2)_2SCH_3$,$(CH_2)_2SCH_2CH_3$,$(CH_2)_2SPh$,$(CH_2)_2NHCH_3$,$(CH_2)_2N(CH_3)_2$,$(CH_2)_2NHPh$,$(CH_2)_2NHCH_2Ph$,$(CH_2)_2N(CH_3)CH_2Ph$,$(CH_2)_2C(O)CH_3$,$(CH_2)_2C(O)CH_2CH_3$,$(CH_2)_2C(O)Ph$,$(CH_2)_2C(O)NH_2$,$(CH_2)_2C(O)NHCH_3$,$(CH_2)_2C(O)N(CH_3)_2$, $(CH_2)_2C(O)NHPh$,$(CH_2)_2C(O)N(CH_3)Ph$,$(CH_2)_2C(O)OCH_3$,$(CH_2)_2C(O)OCH_2CH_3$,$(CH_2)_2NHC(O)CH_3$,$(CH_2)_2NHC(O)CH_2CH_3$,$(CH_2)_2NHC(O)Ph$,$(CH_2)_2NCH_3C(O)CH_3$,$(CH_2)_2NCH_3C(O)CH_2CH_3$,$(CH_2)_2NCH_3C(O)Ph$,$(CH_2)_2NHC(O)OCH3$,$(CH_2)_2NHC(O)OCH_2CH_3$,$(CH_2)_2NHC(O)OPh$,$(CH_2)_2NCH_3C(O)OCH_3$,$(CH_2)_2NCH_3C(O)OCH_2CH_3$,$(CH_2)_2NCH_3C(O)OPh$,$(CH_2)_2NHC(O)NHCH_3$,$(CH_2)_2NHC(O)NHCH_2CH_3$, $(CH_2)_2NHC(O)NHPh$,$(CH_2)_2NHC(O)N(CH_3)_2$,$(CH_2)_2NHC(O)N(CH_3)CH_2CH_3$,$(CH_2)_2NHC(O)N(CH_3)Ph$,$(CH_2)_2NHC(O)N(CH_2CH_3)_2$,$(CH_2)_2NCH_3C(O)NHCH_3$,$(CH_2)_2NCH_3C(O)NHCH_2CH_3$, $(CH_2)_2NCH_3C(O)NHPh$, $(CH_2)_2NCH_3C(O)N(CH_3)_2$,$(CH_2)_2NCH_3C(O)N(CH_3)CH_2CH_3$,$(CH_2)_2NCH_3C(O)N(CH_3)Ph$,$(CH_2)_2NCH_3C(O)N(CH_2CH_3)_2$,$(CH_2)_2OC(O)CH_3$,$(CH_2)_2OC(O)CH_2CH_3$,$(CH_2)_2OC(O)Ph$,$(CH_2)_2OC(O)OCH_3$,$(CH_2)_2OC(O)OCH_2CH_3$,$(CH_2)_2OC(O)OPh$,$(CH_2)_2OC(O)NHCH_3$,$(CH_2)_2OC(O)NHCH_2CH_3$, $(CH_2)_2OC(O)NHPh$,$(CH_2)_2OC(O)N(CH_3)_2$,$(CH_2)_2OC(O)N(CH_3)CH_2CH_3$,$(CH_2)_2OC(O)N(CH_3)Ph$,$(CH_2)_2OC(O)N(CH_2CH_3)_2$,$(CH_2)_3F$,$(CH_2)_3Cl$,$(CH_2)_3Br$,$(CH_2)_3I$,$(CH_2)_3CF_3$,$(CH_2)_3CN$,$(CH_2)_3NO2$,$(CH_2)_3OCH_3$,$(CH_2)_3OCH_2CH_3$,$(CH_2)_3SCH_3$,$(CH_2)_3SCH_2CH_3$,$(CH_2)_3NHCH_3$,$(CH_2)_3N(CH_3)_2$,$(CH_2)_4F$,$(CH_2)_9Cl$,$(CH_2)_4CF_3$,$(CH_2)_4CN$,$(CH_2)_4$

$NO2,(CH_2)_4Ph,(CH_2)_4OCH_3,(CH_2)_4SCH_3,(CH_2)_4NHCH_3,(CH_2)_4N(CH_3)_2,(CH_2)_5F,(CH_2)_5Cl,(CH_2)_5CF_3,(CH_2)_5CN,(CH_2)_5NO2,(CH_2)_5Ph,(CH_2)_sOCH_3,(CH_2)_5SCH_3,(CH_2)_5NHCH_3,(CH_2)_5N(CH_3)_2,(CH_2)_6F,(CH_2)_6Cl,(CH_2)_6CF_3,(CH_2)_6CN,(CH_2)_6NO2,(CH_2)_6Ph,(CH_2)_6OCH_3,(CH_2)_6SCH_3,(CH_2)_6NHCH_3,(CH_2)_6N(CH_3)_2,CH_2CF2CF_3,(CH_2)_2CF2CF_3,CH_2(CF2)_2CF_3,(CH_2)_2CF(CF_3)_2,(CH_2)_2(CF_2)_5CF_3,CF_3,CHF_2$,and $CH_2F$.

A compound (1C) wherein X represents CH, L represents an oxygen atom, $R^1$ represents a methyl group, $R^{x2}$ represents an ethyl group, and $R^6$ represents any substituent selected from Group Y (hereinafter, referred to as Compound Class SX84) .

A compound (1C) wherein X represents CH, L represents an oxygen atom, $R^1$ represents a methyl group, $R^{x2}$ represents a fluorine atom, and $R^6$ represents any substituent selected from Group Y (hereinafter, referred to as Compound Class SX85) .

A compound (1C) wherein X represents CH, L represents an oxygen atom, $R^1$ represents a methyl group, $R^{x2}$ represents a chlorine atom, and $R^6$ represents any substituent selected from Group Y (hereinafter, referred to as Compound Class SX86) .

A compound (1C) wherein X represents CH, L represents an oxygen atom, $R^1$ represents a methyl group, $R^{x2}$ represents a methoxy group, and $R^6$ represents any substituent selected from Group Y (hereinafter, referred to as Compound Class SX87) .

A compound (1C) wherein X represents CH, L represents an oxygen atom, $R^1$ represents a methyl group, $R^{x2}$ represents a cyclopropyl group, and $R^6$ represents any substituent selected from Group Y (hereinafter, referred to as Compound Class SX88).

A compound (1C) wherein X represents CH, L represents an oxygen atom, $R^1$ represents a fluorine atom, $R^{x2}$ represents a methyl group, and $R^6$ represents any substituent selected from Group Y (hereinafter, referred to as Compound Class SX89).

A compound (1C) wherein X represents CH, L represents an oxygen atom, $R^1$ represents a fluorine atom, $R^{x2}$ represents a fluorine atom, and $R^6$ represents any substituent selected from Group Y (hereinafter, referred to as Compound Class SX90) .

A compound (1C) wherein X represents CH, L represents an oxygen atom, $R^1$ represents a fluorine atom, $R^{x2}$ represents a chlorine atom, and $R^6$ represents any substituent selected from Group Y (hereinafter, referred to as Compound Class SX91).

A compound (1C) wherein X represents CH, L represents an oxygen atom, $R^1$ represents a chlorine atom, $R^{x2}$ represents a methyl group, and $R^6$ represents any substituent selected from Group Y (hereinafter, referred to as Compound Class SX92).

A compound (1C) wherein X represents CH, L represents an oxygen atom, $R^1$ represents a chlorine atom, $R^{x2}$ represents a fluorine atom, and $R^6$ represents any substituent selected from Group Y (hereinafter, referred to as Compound Class SX93) .

A compound (1C) wherein X represents CH, L represents an oxygen atom, $R^1$ represents a chlorine atom, $R^{x2}$ represents a chlorine atom, and $R^6$ represents any substituent selected from Group Y (hereinafter, referred to as Compound Class SX94).

A compound (1C) wherein X represents a nitrogen atom, L represents an oxygen atom, $R^1$ represents a methyl group, $R^{x2}$ represents a methyl group, and $R^6$ represents any substituent selected from Group Y (hereinafter, referred to as Compound Class SX95).

A compound (1C) wherein X represents a nitrogen atom, L represents an oxygen atom, $R^1$ represents a methyl group, $R^{x2}$ represents an ethyl group, and $R^6$ represents any substituent selected from Group Y (hereinafter, referred to as Compound Class SX96).

A compound (1C) wherein X represents a nitrogen atom, L represents an oxygen atom, $R^1$ represents a methyl group, $R^{x2}$ represents a fluorine atom, and $R^6$ represents any substituent selected from Group Y (hereinafter, referred to as Compound Class SX97).

A compound (1C) wherein X represents a nitrogen atom, L represents an oxygen atom, $R^1$ represents a methyl group, $R^{x2}$ represents a chlorine atom, and $R^6$ represents any substituent selected from Group Y (hereinafter, referred to as Compound Class SX98).

A compound (1C) wherein X represents a nitrogen atom, L represents an oxygen atom, $R^1$ represents a methyl group, $R^{x2}$ represents a methoxy group, and $R^6$ represents any substituent selected from Group Y (hereinafter, referred to as Compound Class SX99).

A compound (1C) wherein X represents a nitrogen atom, L represents an oxygen atom, $R^1$ represents a methyl group, $R^{x2}$ represents a cyclopropyl group, and $R^6$ represents any substituent selected from Group Y (hereinafter, referred to as Compound Class SX100).

A compound (1C) wherein X represents a nitrogen atom, L represents an oxygen atom, $R^1$ represents a fluorine atom, $R^{x2}$ represents a methyl group, and $R^6$ represents any substituent selected from Group Y (hereinafter, referred

to as Compound Class SX101).

A compound (1C) wherein X represents a nitrogen atom, L represents an oxygen atom, $R^1$ represents a fluorine atom, $R^{x2}$ represents a fluorine atom, and $R^6$ represents any substituent selected from Group Y (hereinafter, referred to as Compound Class SX102).

A compound (1C) wherein X represents a nitrogen atom, L represents an oxygen atom, $R^1$ represents a fluorine atom, $R^{x2}$ represents a chlorine atom, and $R^6$ represents any substituent selected from Group Y (hereinafter, referred to as Compound Class SX103).

A compound (1C) wherein X represents a nitrogen atom, L represents an oxygen atom, $R^1$ represents a chlorine atom, $R^{x2}$ represents a methyl group, and $R^6$ represents any substituent selected from Group Y (hereinafter, referred to as Compound Class SX104).

A compound (1C) wherein X represents a nitrogen atom, L represents an oxygen atom, $R^1$ represents a chlorine atom, $R^{x2}$ represents a fluorine atom, and $R^6$ represents any substituent selected from Group Y (hereinafter, referred to as Compound Class SX105).

A compound (1C) wherein X represents a nitrogen atom, L represents an oxygen atom, $R^1$ represents a chlorine atom, $R^{x2}$ represents a chlorine atom, and $R^6$ represents any substituent selected from Group Y (hereinafter, referred to as Compound Class SX106).

A compound (1C) wherein X represents a nitrogen atom, L represents NH, $R^1$ represents a methyl group, $R^{x2}$ represents a methyl group, and $R^6$ represents any substituent selected from Group Y (hereinafter, referred to as Compound Class SX107).

A compound (1C) wherein X represents a nitrogen atom, L represents NH, $R^1$ represents a methyl group, $R^{x2}$ represents an ethyl group, and $R^6$ represents any substituent selected from Group Y (hereinafter, referred to as Compound Class SX108).

A compound (1C) wherein X represents a nitrogen atom, L represents NH, $R^1$ represents a methyl group, $R^{x2}$ represents a fluorine atom, and $R^6$ represents any substituent selected from Group Y (hereinafter, referred to as Compound Class SX109).

A compound (1C) wherein X represents a nitrogen atom, L represents NH, $R^1$ represents a methyl group, $R^{x2}$ represents a chlorine atom, and $R^6$ represents any substituent selected from Group Y (hereinafter, referred to as Compound Class SX110).

A compound (1C) wherein X represents a nitrogen atom, L represents NH, $R^1$ represents a methyl group, $R^{x2}$ represents a methoxy group, and $R^6$ represents any substituent selected from Group Y (hereinafter, referred to as Compound Class SX111).

A compound (1C) wherein X represents a nitrogen atom, L represents NH, $R^1$ represents a methyl group, $R^{x2}$ represents a cyclopropyl group, and $R^6$ represents any substituent selected from Group Y (hereinafter, referred to as Compound Class SX112).

A compound (1C) wherein X represents a nitrogen atom, L represents NH, $R^1$ represents a fluorine atom, $R^{x2}$ represents a methyl group, and $R^6$ represents any substituent selected from Group Y (hereinafter, referred to as Compound Class SX113).

A compound (1C) wherein X represents a nitrogen atom, L represents NH, $R^1$ represents a fluorine atom, $R^{x2}$ represents a fluorine atom, and $R^6$ represents any substituent selected from Group Y (hereinafter, referred to as Compound Class SX114).

A compound (1C) wherein X represents a nitrogen atom, L represents NH, $R^1$ represents a fluorine atom, $R^{x2}$ represents a chlorine atom, and $R^6$ represents any substituent selected from Group Y (hereinafter, referred to as Compound Class SX115).

A compound (1C) wherein X represents a nitrogen atom, L represents NH, $R^1$ represents a chlorine atom, $R^{x2}$ represents a methyl group, and $R^6$ represents any substituent selected from Group Y (hereinafter, referred to as Compound Class SX116).

A compound (1C) wherein X represents a nitrogen atom, L represents NH, $R^1$ represents a chlorine atom, $R^{x2}$ represents a fluorine atom, and $R^6$ represents any substituent selected from Group Y (hereinafter, referred to as Compound Class SX117).

A compound (1C) wherein X represents a nitrogen atom, L represents NH, $R^1$ represents a chlorine atom, $R^{x2}$ represents a chlorine atom, and $R^6$ represents any substituent selected from Group Y (hereinafter, referred to as Compound Class SX118).

A compound represented by formula (2C):

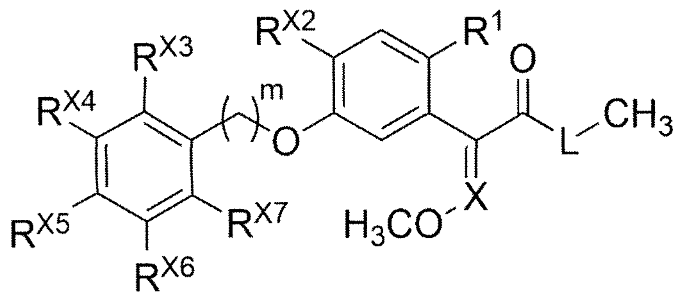

(2C)

(hereinafter, referred to as Compound (2C)) wherein X represents CH, L represents an oxygen atom, $R^1$ represents a methyl group, $R^{X2}$ represents a methyl group, m is 2, and $R^{X3}$, $R^{X4}$, $R^{X5}$, $R^{X6}$, and $R^{X7}$ represent any combination selected from Combination B (hereinafter, referred to as Compound Class SX119).

A combination B consists of Substituent No. ZB1 to ZB306. The substituent No. ZB1 to ZB306 represent a combination of $R^{X3}$, $R^{X4}$, $R^{X5}$, $R^{X6}$, and $R^{X7}$ in the compound (2C), a compound represented by formula (1D) and a compound represented by formula (2D), which is hereinafter as described as [Substituent No.; $R^{X3}$, $R^{X4}$, $R^{X5}$, $R^{X6}$, $R^{X7}$] . For example, the Substituent No. ZB2 represents a combination wherein $R^{3X}$ represents a methyl group, and $R^{X4}$, $R^{X5}$, $R^{X6}$, and $R^{X7}$ represent a hydrogen atom.

Combination B

[ZB1;H,H,H,H,H],[ZB2;Me,H,H,H,H],[ZB3;F,H,H,H,H],[ZB4;Cl,H, H,H,H],[ZB5;OMe,H,H,H,H],[ZB6;$CF_3$,H,H,H,H],[ZB7;H,Me,H,H,H], [ZB8;H,Et,H,H,H],[ZB9;H,Pr,H,H,H],[ZB10;H,i-Pr,H,H,H],[ZB1 1;H,t-Bu,H,H,H],[ZB12;H,OMe,H,H,H],[ZB13;H,OEt,H,H,H],[ZB1 4;H,OPr,H,H,H],[ZB15;H,Oi-Pr,H,H,H],[ZB16;H,$CF_3$,H,H,H],[ZB1 7;H,$CF_2$H,H,H,H],[ZB18;H,$CFH_2$,H,H,H],[ZB19;H,F,H,H,H],[ZB20; H,Cl,H,H,H],[ZB21;H,Br,H,H,H],[ZB22;H,CN,H,H,H],[ZB23;H,Ph, H,H,H],[ZB24;H,OPh,H,H,H],[ZB25;H,c-Pr,H,H,H],[ZB26;H,c-Pe n,H,H,H],[ZB27;H,c-Hex,H,H,H],[ZB28;H,H,Me,H,H],[ZB29;H,H,E t,H,H],[ZB30;H,H,Pr,H,H],[ZB31;H,H,i-Pr,H,H],[ZB32;H,H,t-B u,H,H],[ZB33;H,H,OMe,H,H],[ZB34;H,H,OEt,H,H],[ZB35;H,H,OPr, H,H],[ZB36;H,H,Oi-Pr,H,H],[ZB37;H,H,$CF_3$,H,H],[ZB38;H,H,$CF_2$ H,H,H],[ZB39;H,H,$CFH_2$,H,H],[ZB40;H,H,F,H,H],[ZB41;H,H,Cl,H, H],[ZB42;H,H,Br,H,H],[ZB43;H,H,CN,H,H],[ZB44;H,H,Ph,H,H],[Z B45;H,H,OPh,H,H],[ZB46;H,H,c-Pr,H,H],[ZB47;H,H,c-Pen,H,H], [ZB48;H,H,c-Hex,H,H],[ZB49;Me,H,H,H,F],[ZB50;F,H,H,H,F],[ZB 51;Cl,H,H,H,F],[ZB52;H,Me,H,H,F],[ZB53;H,Et,H,H,F],[ZB54;H, Pr,H,H,F],[ZB55;H,i-Pr,H,H,F],[ZB56;H,t-Bu,H,H,F],[ZB57;H,O Me,H,H,F],[ZB58;H,OEt,H,H,F],[ZB59;H,OPr,H,H,F],[ZB60;H,Oi-Pr,H,H,F],[ZB61;H,$CF_3$,H,H,F],[ZB62;H,$CF_Z$H,H,H,F],[ZB63;H,CF $H_2$,H,H,F],[ZB64;H,F,H,H,F],[ZB65;H,C1,H,H,F],[ZB66;H,Br,H, H,F],[ZB67;H,CN,H,H,F],[ZB68;H,Ph,H,H,F],[ZB69;H,OPh,H,H,F], [ZB70;H,c-Pr,H,H,F],[ZB71;H,c-Pen,H,H,F],[ZB72;H,c-Hex,H,H, F],[ZB73;H,H,Me,H,F],[ZB74;H,H,Et,H,F],[ZB75;H,H,Pr,H,F],[Z B76;H,H,i-Pr,H,F],[ZB77;H,H,t-Bu,H,F],[ZB78;H,H,OMe,H,F],[Z B79;H,H,OEt,H,F],[ZB80;H,H,OPr,H,F],[ZB81;H,H,Oi-Pr,H,F],[Z B82;H,H,$CF_3$,H,F],[ZB83;H,H,$CF_2$H,H,F],[ZB84;H,H,$CFH_2$,H,F],[Z B85;H,H,F,H,F],[ZB86;H,H,Cl,H,F],[ZB87;H,H,Br,H,F],[ZB88;H, H,CN,H,F],[ZB89;H,H,Ph,H,F],[ZB90;H,H,OPh,H,F],[ZB91;H,H,c-Pr,H,F],[ZB92;H,H,c-Pen,H,F],[ZB93;H,H,c-Hex,H,F],[ZB94;Me, H,H,H,Cl],[ZB95;F,H,H,H,Cl],[ZB96;Cl,H,H,H,Cl],[ZB97;H,Me, H,H,Cl],[ZB98;H,Et,H,H,Cl],[ZB99;H,Pr,H,H,Cl],[ZB100;H,i-P r,H,H,Cl],[ZB101;H,t-Bu,H,H,Cl],[ZB102;H,OMe,H,H,Cl],[ZB10 3;H,OEt,H,H,Cl],[ZB104;H,OPr,H,H,Cl],[ZB105;H,Oi-Pr,H,H,Cl], [ZB106;H,$CF_3$,H,H,Cl],[ZB107;H,$CF_2$H,H,H,Cl],[ZB108;H,$CFH_2$,H, H,Cl],[ZB109;H,F,H,H,Cl],[ZB110;H,Cl,H,H,Cl],[ZB111;H,Br,H, H,Cl],[ZB112;H,CN,H,H,Cl],[ZB113;H,Ph,H,H,Cl],[ZB114;H,OPh, H,H,Cl],[ZB115;H,c-Pr,H,H,Cl],[ZB116;H,c-Pen,H,H,Cl],[ZB11 7;H,c-Hex,H,H,Cl],[ZB118;H,H,Me,H,Cl],[ZB119;H,H,Et,H,Cl], [ZB120;H,H,Pr,H,Cl],[ZB121;H,H,i-Pr,H,Cl],[ZB122;H,H,t-Bu, H,Cl],[ZB123;H,H,OMe,H,Cl],[ZB124;H,H,OEt,H,Cl],[ZB125;H,H, OPr,H,Cl],[ZB126;H,H,Oi-Pr,H,C1],[ZB127;H,H,$CF_3$,H,C1],[ZB12 8;H,H,$CF_2$H,H,Cl],[ZB129;H,H,$CFH_2$,H,Cl],[ZB130;H,H,F,H,Cl], [ZB131;H,H,Cl,H,Cl],[ZB132;H,H,Br,H,Cl],[ZB133;H,H,CN,H,Cl], [ZB134;H,H,Ph,H,Cl],[ZB135;H,H,OPh,H,Cl],[ZB136;H,H,c-Pr,H, Cl],[ZB137;H,H,c-Pen,H,Cl],[ZB138;H,H,c-Hex,H,Cl],[ZB139;M e,H,H,H,Me],[ZB140;F,H,H,H,Me],[ZB141;Cl,H,H,H,Me],[ZB142; H,Me,H,H,Me],[ZB143;H,Et,H,H,Me],[ZB144;H,Pr,H,H,Me],[ZB14 5;H,i-Pr,H,H,Me],[ZB146;H,t-Bu,H,H,Me],[ZB147;H,OMe,H,H,Me], [ZB148;H,OEt,H,H,Me],[ZB149;H,OPr,H,H,Me],[ZB150;H,Oi-Pr,H, H,Me],[ZB151;H,$CF_3$,H,H,Me],[ZB152;H,$CF_2$H,H,H,Me],[ZB153;H,C

$FH_2$,H,H,Me],[ZB154;H,F,H,H,Me],[ZB155;H,Cl,H,H,Me],[ZB156; H,Br,H,H,Me],[ZB157;H,CN,H,H,Me],[ZB158;H,Ph,H,H,Me],[ZB15 9;H,OPh,H,H,Me],[ZB160;H,c-Pr,H,H,Me],[ZB161;H,c-Pen,H,H,M e],[ZB162;H,c-Hex,H,H,Me],[ZB163;H,H,Me,H,Me],[ZB164;H,H,E t,H,Me],[ZB165;H,H,Pr,H,Me],[ZB166;H,H,i-Pr,H,Me],[ZB167;H, H,t-Bu,H,Me], [ZB168;H,H,OMe,H,Me], [ZB169;H,H,OEt,H,Me], [ZB1 70;H,H,OPr,H,Me],[ZB171;H,H,Oi-Pr,H,Me],[ZB172;H,H,$CF_3$,H,M e],[ZB173;H,H,$CF_2H$,H,Me],[ZB174;H,H,$CFH_2$,H,Me],[ZB175;H,H, F,H,Me], [ZB176;H,H,Cl,H,Me], [ZB177;H,H,Br,H,Me], [ZB178;H,H, CN,H,Me],[ZB179;H,H,Ph,H,Me],[ZB180;H,H,OPh,H,Me],[ZB181;H, H,c-Pr,H,Me],[ZB182;H,H,c-Pen,H,Me],[ZB183;H,H,c-Hex,H,Me], [ZB184;Me,H,H,H,OMe],[ZB185;F,H,H,H,OMe],[ZB186; Cl,H,H,H,OM e],[ZB187;H,Me,H,H,OMe],[ZB188;H,Et,H,H,OMe],[ZB189;H,Pr,H, H,OMe],[ZB190;H,i-Pr,H,H,OMe],[ZB191;H,t-Bu,H,H,OMe],[ZB19 2;H,OMe,H,H,OMe],[ZB193;H,OEt,H,H,OMe],[ZB194;H,OPr,H,H,OM e],[ZB195;H,Oi-Pr,H,H,OMe],[ZB196;H,$CF_3$,H,H,OMe],[ZB197;H,C $F_2$H,H,H,OMe],[ZB198;H,$CFH_2$,H,H,OMe],[ZB199;H,F,H,H,OMe],[ZB 200;H,Cl,H,H,OMe],[ZB201;H,Br,H,H,OMe],[ZB202;H,CN,H,H,OMe], [ZB203;H,Ph,H,H,OMe],[ZB204;H,OPh,H,H,OMe],[ZB205;H,c-Pr,H, H,OMe],[ZB206;H,c-Pen,H,H,OMe],[ZB207;H,c-Hex,H,H,OMe],[ZB2 08;H,H,Me,H,OMe],[ZB209;H,H,Et,H,OMe],[ZB210;H,H,Pr,H,OMe], [ZB211;H,H,i-Pr,H,OMe],[ZB212;H,H,t-Bu,H,OMe],[ZB213;H,H,OM e,H,OMe],[ZB214;H,H,OEt,H,OMe],[ZB215;H,H,OPr,H,OMe],[ZB21 6;H,H,Oi-Pr,H,OMe],[ZB217;H,H,$CF_3$,H,OMe],[ZB218;H,H,$CF_2$H,H, OMe],[ZB219;H,H,$CFH_2$,H,OMe],[ZB220;H,H,F,H,OMe],[ZB221;H,H, Cl,H,OMe],[ZB222;H,H,Br,H,OMe],[ZB223;H,H,CN,H,OMe],[ZB224; H,H,Ph,H,OMe],[ZB225;H,H,OPh,H,OMe],[ZB226;H,H,c-Pr,H,OMe], [ZB227;H,H,c-Pen,H,OMe],[ZB228;H,H,c-Hex,H,OMe],[ZB229;Me, H,H,H,$CF_3$],[ZB230;F,H,H,H,$CF_3$],[ZB231;Cl,H,H,H,$CF_3$],[ZB232; H,Me,H,H,$CF_3$],[ZB233;H,Et,H,H,$CF_3$],[ZB234;H,Pr,H,H,$CF_3$],[ZB 235;H,i-Pr,H,H,$CF_3$],[ZB236;H,t-Bu,H,H,$CF_3$],[ZB237;H,OMe,H, H,$CF_3$],[ZB238;H,OEt,H,H,$CF_3$],[ZB239;H,OPr,H,H,$CF_3$],[ZB240; H,Oi-Pr,H,H,$CF_3$],[ZB241;H,$CF_3$,H,H,$CF_3$],[ZB242;H,$CF_2$H,H,H,CF $_3$],[ZB243;H,$CFH_2$,H,H,$CF_3$],[ZB244;H,F,H,H,$CF_3$],[ZB245;H,Cl, H,H,$CF_3$],[ZB246;H,Br,H,H,$CF_3$],[ZB247;H,CN,H,H,$CF_3$],[ZB248; H,Ph,H,H,$CF_3$],[ZB249;H,OPh,H,H,$CF_3$],[ZB250;H,c-Pr,H,H,$CF_3$], [ZB251;H,c-Pen,H,H,$CF_3$],[ZB252;H,c-Hex,H,H,$CF_3$],[ZB253;H,H, Me,H,$CF_3$],[ZB254;H,H,Et,H,$CF_3$],[ZB255;H,H,Pr,H,$CF_3$],[ZB256; H,H,i-Pr,H,$CF_3$],[ZB257;H,H,t-Bu,H,$CF_3$],[ZB258;H,H,OMe,H,CF $_3$],[ZB259;H,H,OEt,H,$CF_3$],[ZB260;H,H,OPr,H,$CF_3$],[ZB261;H,H,O i-Pr,H,$CF_3$],[ZB262;H,H,$CF_3$,H,$CF_3$],[ZB263;H,H,$CF_2$H,H,$CF_3$],[Z B264;H,H,$CFH_2$,H,$CF_3$],[ZB265;H,H,F,H,$CF_3$],[ZB266;H,H,Cl,H,CF $_3$],[ZB267;H,H,Br,H,$CF_3$],[ZB268;H,H,CN,H,$CF_3$],[ZB269;H,H,Ph, H,$CF_3$],[ZB270;H,H,OPh,H,$CF_3$],[ZB271;H,H,c-Pr,H,$CF_3$],[ZB272; H,H,c-Pen,H,$CF_3$],[2B273;H,H,c-Hex,H,$CF_3$],[ZB274;H,F,F,H,H], [ZB275;H,F,H,F,H],[ZB276;H,F,F,F,H],[ZB277;F,F,F,H,H],[ZB27 8;F,F,H,F,H],[ZB279;F,H,F,H,F],[ZB280;F,F,F,F,F],[ZB281;H,C l,H,Cl,H],[ZB282;H,OMe,H,OMe,H],[ZB283;H,F,Cl,H,H],[ZB284; H,F,Me,H,H],[ZB285;H,F,OMe,H,H],[ZB286;H,F,$CF_3$,H,H],[ZB287; H,Cl,F,H,H], [ZB288;H,Cl,Cl,H,H], [ZB289;H,Cl,Me,H,H], [ZB290; H,Cl,OMe,H,H],[ZB291;H,Cl,$CF_3$,H,H],[ZB292;H,Me,F,H,H],[ZB29 3;H,Me,Cl,H,H],[ZB294;H,Me,Me,H,H],[ZB295;H,Me,OMe,H,H],[ZB 296;H,Me,$CF_3$,H,H],[ZB297;H,OMe,F,H,H],[ZB298;H,OMe,Cl,H,H], [ZB299;H,OMe,Me,H,H],[ZB300;H,OMe,OMe,H,H],[ZB301;H,OMe,CF $_3$,H,H],[ZB302;H,$CF_3$,F,H,H],[ZB303;H,$CF_3$,Cl,H,H],[ZB304;H,CF $_3$,F,H,H],[ZB305;H,$CF_3$,Cl,H,H],[ZB306;H,$CF_3$,F,H,H]

A compound (2C) wherein X represents CH, L represents an oxygen atom, $R^1$ represents a fluorine atom, $R^{X2}$ represents a fluorine atom, m is 2, and $R^{X3}$, $R^{X4}$, $R^{X5}$, $R^{X6}$, and $R^{X7}$ represent any combination described in Combination B (hereinafter, referred to as Compound Class SX120).

A compound (2C) wherein X represents CH, L represents an oxygen atom, $R^1$ represents a fluorine atom, $R^{X2}$ represents a chlorine atom, m is 2, and $R^{X3}$, $R^{X4}$, $R^{X5}$, $R^{X6}$, and $R^{X7}$ represent any combination described in Combination B (hereinafter, referred to as Compound Class SX121).

A compound (2C) wherein X represents CH, L represents an oxygen atom, $R^1$ represents a chlorine atom, $R^{X2}$ represents a fluorine atom, m is 2, and $R^{X3}$, $R^{X4}$, $R^{X5}$, $R^{X6}$, and $R^{X7}$ represent any combination described in Combination B (hereinafter, referred to as Compound Class SX122).

A compound (2C) wherein X represents CH, L represents an oxygen atom, $R^1$ represents a chlorine atom, $R^{X2}$ represents a chlorine atom, m is 2, and $R^{X3}$, $R^{X4}$, $R^{X5}$, $R^{X6}$, and $R^{X7}$ represent any combination described in Combination B (hereinafter, referred to as Compound Class SX123).

A compound (2C) wherein X represents CH, L represents an oxygen atom, $R^1$ represents a methyl group, $R^{X2}$ represents a methyl group, m is 3, and $R^{X3}$, $R^{X4}$, $R^{X5}$, $R^{X6}$, and $R^{X7}$ represent any combination described in Combination

B (hereinafter, referred to as Compound Class SX124).

A compound (2C) wherein X represents CH, L represents an oxygen atom, $R^1$ represents a fluorine atom, $R^{X2}$ represents a fluorine atom, m is 3, and $R^{X3}$, $R^{X4}$, $R^{X5}$, $R^{X6}$, and $R^{X7}$ represent any combination described in Combination B (hereinafter, referred to as Compound Class SX125).

A compound (2C) wherein X represents CH, L represents an oxygen atom, $R^1$ represents a fluorine atom, $R^{X2}$ represents a chlorine atom, m is 3, and $R^{X3}$, $R^{X4}$, $R^{X5}$, $R^{X6}$, and $R^{X7}$ represent any combination described in Combination B (hereinafter, referred to as Compound Class SX126).

A compound (2C) wherein X represents CH, L represents an oxygen atom, $R^1$ represents a chlorine atom, $R^{X2}$ represents a fluorine atom, m is 3, and $R^{X3}$, $R^{X4}$, $R^{X5}$, $R^{X6}$, and $R^{x7}$ represent any combination described in Combination B (hereinafter, referred to as Compound Class SX127).

A compound (2C) wherein X represents CH, L represents an oxygen atom, $R^1$ represents a chlorine atom, $R^{X2}$ represents a chlorine atom, m is 3, and $R^{X3}$, $R^{X4}$, $R^{X5}$, $R^{X6}$, and $R^{X7}$ represent any combination described in Combination B (hereinafter, referred to as Compound Class SX128).

A compound (2C) wherein X represents CH, L represents an oxygen atom, $R^1$ represents a methyl group, $R^{X2}$ represents a methyl group, m is 4, and $R^{X3}$, $R^{X4}$, $R^{X5}$, $R^{X6}$, and $R^{X7}$ represent any combination described in Combination B (hereinafter, referred to as Compound Class SX129).

an oxygen atom, $R^1$ represents a methyl group, $R^{X2}$ represents

A compound (2C) wherein X represents CH, L represents an oxygen atom, $R^1$ represents a fluorine atom, $R^{X2}$ represents a fluorine atom, m is 4, and $R^{X3}$, $R^{X4}$, $R^{X5}$, $R^{X6}$, and $R^{X7}$ represent any combination described in Combination B (hereinafter, referred to as Compound Class SX130).

A compound (2C) wherein X represents CH, L represents an oxygen atom, $R^1$ represents a fluorine atom, $R^{X2}$ represents a chlorine atom, m is 4, and $R^{X3}$, $R^{X4}$, $R^{X5}$, $R^{X6}$, and $R^{X7}$ represent any combination described in Combination B (hereinafter, referred to as Compound Class SX131).

A compound (2C) wherein X represents CH, L represents an oxygen atom, $R^1$ represents a chlorine atom, $R^{X2}$ represents a fluorine atom, m is 4, and $R^{X3}$, $R^{X4}$, $R^{X5}$, $R^{X6}$, and $R^{X7}$ represent any combination described in Combination B (hereinafter, referred to as Compound Class SX132).

A compound (2C) wherein X represents CH, L represents an oxygen atom, $R^1$ represents a chlorine atom, $R^{X2}$ represents a chlorine atom, m is 4, and $R^{X3}$, $R^{X4}$, $R^{X5}$, $R^{X6}$, and $R^{X7}$ represent any combination described in Combination B (hereinafter, referred to as Compound Class SX133).

A compound represented by formula (3C):

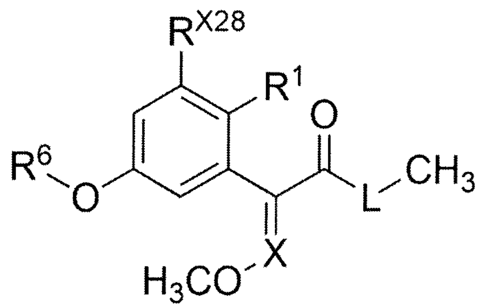

(3C)

(hereinafter, referred to as Compound (3C)) wherein X represents CH, L represents an oxygen atom, $R^1$ represents a methyl group, $R^{X28}$ represents a methyl group, and $R^6$ represents any substituent selected from Group Y (hereinafter, referred to as Compound Class SX134).

A compound (3C) wherein X represents CH, L represents an oxygen atom, $R^1$ represents a methyl group, $R^{X28}$ represents a fluorine atom, and $R^6$ represents any substituent selected from Group Y (hereinafter, referred to as Compound Class SX135).

A compound (3C) wherein X represents CH, L represents an oxygen atom, $R^1$ represents a methyl group, $R^{X28}$ represents a chlorine atom, and $R^6$ represents any substituent selected from Group Y (hereinafter, referred to as Compound Class SX136) .

A compound (3C) wherein X represents CH, L represents an oxygen atom, $R^1$ represents a methyl group, $R^{X28}$ represents a methoxy group, and $R^6$ represents any substituent selected from Group Y (hereinafter, referred to as Compound Class SX137) .

A compound (3C) wherein X represents a nitrogen atom, L represents an oxygen atom, $R^1$ represents a methyl group, $R^{X28}$ represents a methyl group, and $R^6$ represents any substituent selected from Group Y (hereinafter, referred to as Compound Class SX138).

A compound (3C) wherein X represents a nitrogen atom, L represents an oxygen atom, $R^1$ represents a methyl

group, $R^{X28}$ represents a fluorine atom, and $R^6$ represents any substituent selected from Group Y (hereinafter, referred to as Compound Class SX139).

A compound (3C) wherein X represents a nitrogen atom, L represents an oxygen atom, $R^1$ represents a methyl group, $R^{X28}$ represents a chlorine atom, and $R^6$ represents any substituent selected from Group Y (hereinafter, referred to as Compound Class SX140).

A compound (3C) wherein X represents a nitrogen atom, L represents NH, $R^1$ represents a methyl group, $R^{X28}$ represents a methyl group, and $R^6$ represents any substituent selected from Group Y (hereinafter, referred to as Compound Class SX141).

A compound (3C) wherein X represents a nitrogen atom, L represents NH, $R^1$ represents a methyl group, $R^{X28}$ represents a fluorine atom, and $R^6$ represents any substituent selected from Group Y (hereinafter, referred to as Compound Class SX142).

A compound (3C) wherein X represents a nitrogen atom, L represents NH, $R^1$ represents a methyl group, $R^{X28}$ represents a chlorine atom, and $R^6$ represents any substituent selected from Group Y (hereinafter, referred to as Compound Class SX143).

A compound represented by formula (1D):

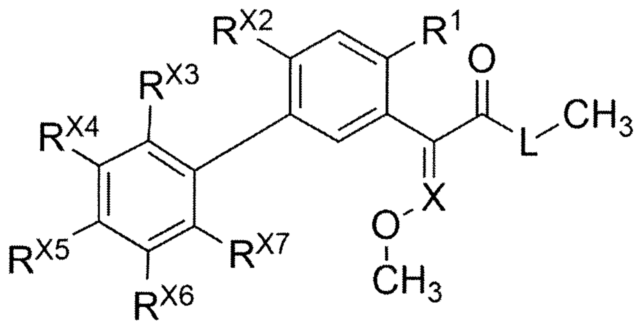

(1D)

(hereinafter, referred to as Compound (1D)) wherein X represents CH, L represents an oxygen atom, $R^1$ represents a methyl group, $R^{X2}$ represents a methyl group, and $R^{X3}$, $R^{X4}$, $R^{X5}$, $R^{X6}$, and $R^{X7}$ represent any combination described in Combination B (hereinafter, referred to as Compound Class SX144).

A compound (1D) wherein X represents CH, L represents an oxygen atom, $R^1$ represents a methyl group, $R^{X2}$ represents an ethyl group, and $R^{X3}$, $R^{X4}$, $R^{X5}$, $R^{X6}$, and $R^{X7}$ represent any combination described in Combination B (hereinafter, referred to as Compound Class SX145).

A compound (1D) wherein X represents CH, L represents an oxygen atom, $R^1$ represents a methyl group, $R^{X2}$ represents a fluorine atom, and $R^{X3}$, $R^{X4}$, $R^{X5}$, $R^{X6}$, and $R^{X7}$ represent any combination described in Combination B (hereinafter, referred to as Compound Class SX146).

A compound (10) wherein X represents CH, L represents an oxygen atom, $R^1$ represents a methyl group, $R^{X2}$ represents a chlorine atom, and $R^{X3}$, $R^{X4}$, $R^{X5}$, $R^{X6}$, and $R^{X7}$ represent any combination described in Combination B (hereinafter, referred to as Compound Class SX147).

A compound (1D) wherein X represents CH, L represents an oxygen atom, $R^1$ represents a methyl group, $R^{X2}$ represents a methoxy group, and $R^{X3}$, $R^{X4}$, $R^{X5}$, $R^{X6}$, and $R^{X7}$ represent any combination described in Combination B (hereinafter, referred to as Compound Class SX148).

A compound (10) wherein X represents CH, L represents an oxygen atom, $R^1$ represents a methyl group, $R^{X2}$ represents a cyclopropyl group, and $R^{X3}$, $R^{X4}$, $R^{X5}$, $R^{X6}$, and $R^{X7}$ represent any combination described in Combination B (hereinafter, referred to as Compound Class SX149).

A compound (1D) wherein X represents CH, L represents an oxygen atom, $R^1$ represents a fluorine atom, $R^{X2}$ represents a methyl group, and $R^{X3}$, $R^{X4}$, $R^{X5}$, $R^{X6}$, and $R^{X7}$ represent any combination described in Combination B (hereinafter, referred to as Compound Class SX150).

A compound (10) wherein X represents CH, L represents an oxygen atom, $R^1$ represents a fluorine atom, $R^{X2}$ represents a fluorine atom, and $R^{X3}$, $R^{X4}$, $R^{X5}$, $R^{X6}$, and $R^{X7}$ represent any combination described in Combination B (hereinafter, referred to as Compound Class SX151).

A compound (1D) wherein X represents CH, L represents an oxygen atom, $R^1$ represents a fluorine atom, $R^{X2}$ represents a chlorine atom, and $R^{X3}$, $R^{X4}$, $R^{X5}$, $R^{X6}$, and $R^{X7}$ represent any combination described in Combination B (hereinafter, referred to as Compound Class SX152).

A compound (1D) wherein X represents CH, L represents an oxygen atom, $R^1$ represents a chlorine atom, $R^{X2}$ represents a methyl group, and $R^{X3}$, $R^{X4}$, $R^{X5}$, $R^{X6}$, and $R^{X7}$ represent any combination described in Combination B (hereinafter, referred to as Compound Class SX153).

A compound (1D) wherein X represents CH, L represents an oxygen atom, $R^1$ represents a chlorine atom, $R^{X2}$ represents a fluorine atom, and $R^{X3}$, $R^{X4}$, $R^{X5}$, $R^{X6}$, and $R^{X7}$ represent any combination described in Combination B (hereinafter, referred to as Compound Class SX154).

A compound (1D) wherein X represents CH, L represents an oxygen atom, $R^1$ represents a chlorine atom, $R^{X2}$ represents a chlorine atom, and $R^{X3}$, $R^{X4}$, $R^{X5}$, $R^{X6}$, and $R^{X7}$ represent any combination described in Combination B (hereinafter, referred to as Compound Class SX155).

A compound (1D) wherein X represents a nitrogen atom, L represents an oxygen atom, $R^1$ represents a methyl group, $R^{X2}$ represents a methyl group, and $R^{X3}$, $R^{X4}$, $R^{X5}$, $R^{X6}$, and $R^{X7}$ represent any combination described in Combination B (hereinafter, referred to as Compound Class SX156).

A compound (1D) wherein X represents a nitrogen atom, L represents an oxygen atom, $R^1$ represents a methyl group, $R^{X2}$ represents an ethyl group, and $R^{X3}$, $R^{X4}$, $R^{X5}$, $R^{X6}$, and $R^{X7}$ represent any combination described in Combination B (hereinafter, referred to as Compound Class SX157).

A compound (1D) wherein X represents a nitrogen atom, L represents an oxygen atom, $R^1$ represents a methyl group, $R^{X2}$ represents a fluorine atom, and $R^{X3}$, $R^{X4}$, $R^{X5}$, $R^{X6}$, and $R^{X7}$ represent any combination described in Combination B (hereinafter, referred to as Compound Class SX158).

A compound (1D) wherein X represents a nitrogen atom, L represents an oxygen atom, $R^1$ represents a methyl group, $R^{X2}$ represents a chlorine atom, and $R^{X3}$, $R^{X4}$, $R^{X3}$, $R^{X6}$, and $R^{X7}$ represent any combination described in Combination B (hereinafter, referred to as Compound Class SX159).

A compound (10) wherein X represents a nitrogen atom, L represents an oxygen atom, $R^1$ represents a methyl group, $R^{X2}$ represents a methoxy group, and $R^{X3}$, $R^{X4}$, $R^{X5}$, $R^{X6}$, and $R^{X7}$ represent any combination described in Combination B (hereinafter, referred to as Compound Class SX160).

A compound (1D) wherein X represents a nitrogen atom, L represents an oxygen atom, $R^1$ represents a methyl group, $R^{X2}$ represents a cyclopropyl group, and $R^{X3}$, $R^{X4}$, $R^{X5}$, $R^{X6}$, and $R^{X7}$ represent any combination described in Combination B (hereinafter, referred to as Compound Class SX161).

A compound (1D) wherein X represents a nitrogen atom, L represents an oxygen atom, $R^1$ represents a fluorine atom, $R^{X2}$ represents a methyl group, and $R^{X3}$, $R^{X4}$, $R^{X5}$, $R^{X6}$, and $R^{X7}$ represent any combination described in Combination B (hereinafter, referred to as Compound Class SX162).

A compound (1D) wherein X represents a nitrogen atom, L represents an oxygen atom, $R^1$ represents a fluorine atom, $R^{X2}$ represents a fluorine atom, and $R^{X3}$, $R^{X4}$, $R^{X5}$, $R^{X6}$, and $R^{X7}$ represent any combination described in Combination B (hereinafter, referred to as Compound Class SX163).

A compound (1D) wherein X represents a nitrogen atom, L represents an oxygen atom, $R^1$ represents a fluorine atom, $R^{X2}$ represents a chlorine atom, and $R^{X3}$, $R^{X4}$, $R^{X5}$, $R^{X6}$, and $R^{X7}$ represent any combination described in Combination B (hereinafter, referred to as Compound Class SX164).

A compound (1D) wherein X represents a nitrogen atom, L represents an oxygen atom, $R^1$ represents a chlorine atom, $R^{X2}$ represents a methyl group, and $R^{X3}$, $R^{X4}$, $R^{X5}$, $R^{X6}$, and $R^{X7}$ represent any combination described in Combination B (hereinafter, referred to as Compound Class SX165).

A compound (1D) wherein X represents a nitrogen atom, L represents an oxygen atom, $R^1$ represents a chlorine atom, $R^{X2}$ represents a fluorine atom, and $R^{X3}$, $R^{X4}$, $R^{X5}$, $R^{X6}$, and $R^{X7}$ represent any combination described in Combination B (hereinafter, referred to as Compound Class SX166).

A compound (1D) wherein X represents a nitrogen atom, L represents an oxygen atom, $R^1$ represents a chlorine atom, $R^{X2}$ represents a chlorine atom, and $R^{X3}$, $R^{X4}$, $R^{X5}$, $R^{X6}$, and $R^{X7}$ represent any combination described in Combination B (hereinafter, referred to as Compound Class SX167).

A compound (1D) wherein X represents a nitrogen atom, L represents NH, $R^1$ represents a methyl group, $R^{X2}$ represents a methyl group, and $R^{X3}$, $R^{X4}$, $R^{X5}$, $R^{X6}$, and $R^{X7}$ represent any combination described in Combination B (hereinafter, referred to as Compound Class SX168).

A compound (1D) wherein X represents a nitrogen atom, L represents NH, $R^1$ represents a methyl group, $R^{X2}$ represents an ethyl group, and $R^{X3}$, $R^{X4}$, $R^{X5}$, $R^{X6}$, and $R^{X7}$ represent any combination described in Combination B (hereinafter, referred to as Compound Class SX169).

A compound (1D) wherein X represents a nitrogen atom, L represents NH, $R^1$ represents a methyl group, $R^{X2}$ represents a fluorine atom, and $R^{X3}$, $R^{X4}$, $R^{X5}$, $R^{X6}$, and $R^{X7}$ represent any combination described in Combination B (hereinafter, referred to as Compound Class SX170).

A compound (1D) wherein X represents a nitrogen atom, L represents NH, $R^1$ represents a methyl group, $R^{X2}$ represents a chlorine atom, and $R^{X3}$, $R^{X4}$, $R^{X5}$, $R^{X6}$, and $R^{X7}$ represent any combination described in Combination B (hereinafter, referred to as Compound Class SX171).

A compound (1D) wherein X represents a nitrogen atom, L represents NH, $R^1$ represents a methyl group, $R^{X2}$ represents a methoxy group, and $R^{X3}$, $R^{X4}$, $R^{X5}$, $R^{X6}$, and $R^{X7}$ represent any combination described in Combination B (hereinafter, referred to as Compound Class SX172).

A compound (1D) wherein X represents a nitrogen atom, L represents NH, $R^1$ represents a methyl group, $R^{X2}$

represents a cyclopropyl group, and $R^{X3}$, $R^{X4}$, $R^{X5}$, $R^{X6}$, and $R^{X7}$ represent any combination described in Combination B (hereinafter, referred to as Compound Class SX173).

A compound (1D) wherein X represents a nitrogen atom, L represents NH, $R^1$ represents a fluorine atom, $R^{X2}$ represents a methyl group, and $R^{X3}$, $R^{X4}$, $R^{X5}$, $R^{X6}$, and $R^{X7}$ represent any combination described in Combination B (hereinafter, referred to as Compound Class SX174).

A compound (1D) wherein X represents a nitrogen atom, L represents NH, $R^1$ represents a fluorine atom, $R^{X2}$ represents a fluorine group, and $R^{X3}$, $R^{X4}$, $R^{X5}$, $R^{X6}$, and $R^{X7}$ represent any combination described in Combination B (hereinafter, referred to as Compound Class SX175).

A compound (1D) wherein X represents a nitrogen atom, L represents NH, $R^1$ represents a fluorine atom, $R^{X2}$ represents a chlorine group, and $R^{X3}$, $R^{X4}$, $R^{X5}$, $R^{X6}$, and $R^{X7}$ represent any combination described in Combination B (hereinafter, referred to as Compound Class SX176).

A compound (1D) wherein X represents a nitrogen atom, L represents NH, $R^1$ represents a chlorine atom, $R^{X2}$ represents a methyl group, and $R^{X3}$, $R^{X4}$, $R^{X5}$, $R^{X6}$, and $R^{X7}$ represent any combination described in Combination B (hereinafter, referred to as Compound Class SX177).

A compound (1D) wherein X represents a nitrogen atom, L represents NH, $R^1$ represents a chlorine atom, $R^{X2}$ represents a fluorine atom, and $R^{X3}$, $R^{X4}$, $R^{X5}$, $R^{X6}$, and $R^{X7}$ represent any combination described in Combination B (hereinafter, referred to as Compound Class SX178).

A compound (1D) wherein X represents a nitrogen atom, L represents NH, $R^1$ represents a chlorine atom, $R^{X2}$ represents a chlorine atom, and $R^{X3}$, $R^{X4}$, $R^{X5}$, $R^{X6}$, and $R^{X7}$ represent any combination described in Combination B (hereinafter, referred to as Compound Class SX179).

A compound represented by formula (2D):

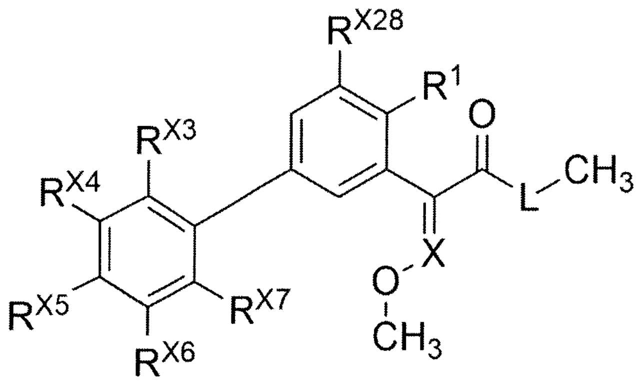

(2D)

(hereinafter, referred to as Compound (2D)) wherein X represents CH, L represents an oxygen atom, $R^1$ represents a methyl group, $R^{X28}$ represents a methyl group, and $R^{X3}$, $R^{X4}$, $R^{X5}$, $R^{X6}$, and $R^{X7}$ represent any combination described in Combination B (hereinafter, referred to as Compound Class SX180).

A compound (2D) wherein X represents CH, L represents an oxygen atom, $R^1$ represents a methyl group, $R^{X28}$ represents a fluorine atom, and $R^{X3}$, $R^{X4}$, $R^{X5}$, $R^{X6}$, and $R^{X7}$ represent any combination described in Combination B (hereinafter, referred to as Compound Class SX181).

A compound (2D) wherein X represents CH, L represents an oxygen atom, $R^1$ represents a methyl group, $R^{X28}$ represents a chlorine atom, and $R^{X3}$, $R^{X4}$, $R^{X5}$, $R^{X6}$, and $R^{X7}$ represent any combination described in Combination B (hereinafter, referred to as Compound Class SX182).

A compound (2D) wherein X represents CH, L represents an oxygen atom, $R^1$ represents a methyl group, $R^{X28}$ represents a methoxy group, and $R^{X3}$, $R^{X4}$, $R^{X5}$, $R^{X6}$, and $R^{X7}$ represent any combination described in Combination B (hereinafter, referred to as Compound Class SX183).

A compound (2D) wherein X represents a nitrogen atom, L represents an oxygen atom, $R^1$ represents a methyl group, $R^{X28}$ represents a methyl group, and $R^{X3}$, $R^{X4}$, $R^{X5}$, $R^{X6}$, and $R^{X7}$ represent any combination described in Combination B (hereinafter, referred to as Compound Class SX184).

A compound (2D) wherein X represents a nitrogen atom, L represents an oxygen atom, $R^1$ represents a methyl group, $R^{X28}$ represents a fluorine atom, and $R^{X3}$, $R^{X4}$, $R^{X5}$, $R^{X6}$, and $R^{X7}$ represent any combination described in Combination B (hereinafter, referred to as Compound Class SX185).

A compound (2D) wherein X represents a nitrogen atom, L represents an oxygen atom, $R^1$ represents a methyl group, $R^{X28}$ represents a chlorine atom, and $R^{X3}$, $R^{X4}$, $R^{X5}$, $R^{X6}$, and $R^{X7}$ represent any combination described in Combination B (hereinafter, referred to as Compound Class SX186).

A compound (2D) wherein X represents a nitrogen atom, L represents NH, $R^1$ represents a methyl group, $R^{X28}$

represents a methyl group, and $R^{X3}$, $R^{X4}$, $R^{X5}$, $R^{X6}$, and $R^{X7}$ represent any combination described in Combination B (hereinafter, referred to as Compound Class SX187).

A compound (2D) wherein X represents a nitrogen atom, L represents NH, $R^1$ represents a methyl group, $R^{X28}$ represents a fluorine atom, and $R^{X3}$, $R^{X4}$, $R^{X5}$, $R^{X6}$, and $R^{X7}$ represent any combination described in Combination B (hereinafter, referred to as Compound Class SX188).

A compound (2D) wherein X represents a nitrogen atom, L represents NH, $R^1$ represents a methyl group, $R^{X28}$ represents a chlorine atom, and $R^{X3}$, $R^{X4}$, $R^{X5}$, $R^{X6}$, and $R^{X7}$ represent any combination described in Combination B (hereinafter, referred to as Compound Class SX189).

A compound represented by formula (1E):

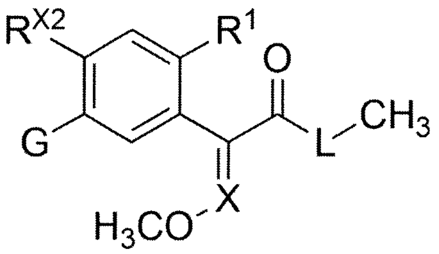

(1E)

wherein G represents any one of formulae G1 to G36.

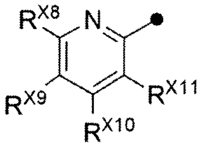
G1

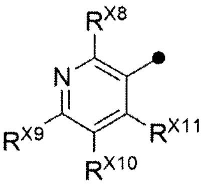
G2

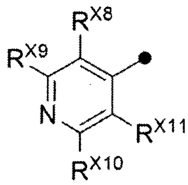
G3

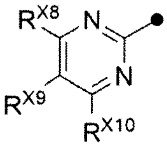
G4

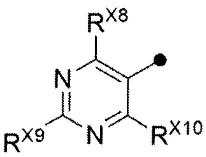
G5

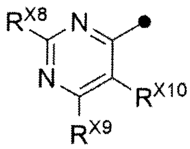
G6

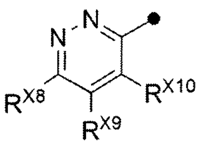
G7

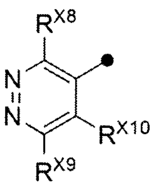
G8

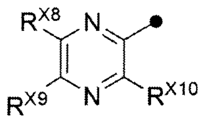
G9

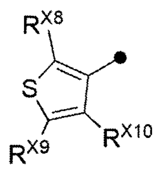
G10

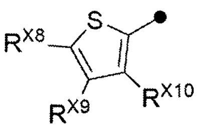
G11

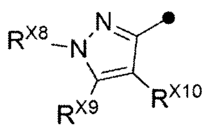
G12

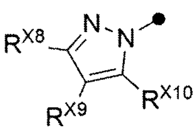
G13

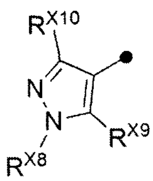
G14

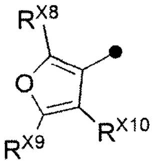
G15

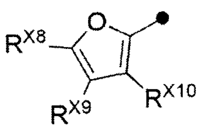
G16

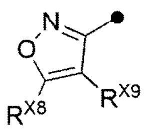
G17

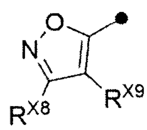
G18

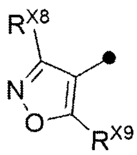
G19

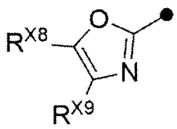
G20

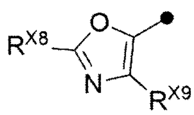
G21

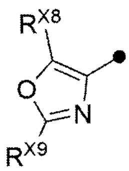
G22

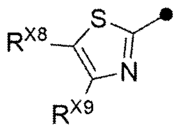
G23

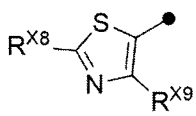
G24

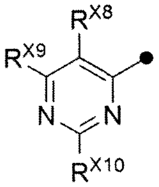 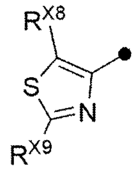 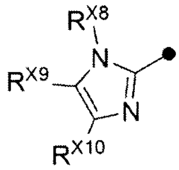 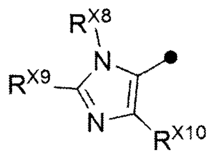 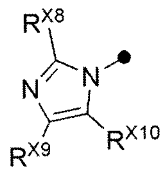 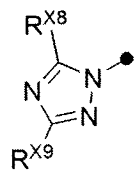

G25 G26 G27 G28 G29 G30

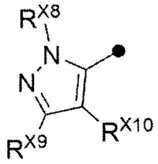 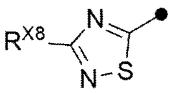 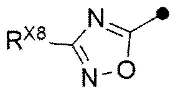 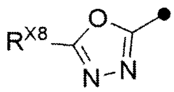 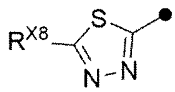 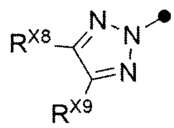

G31 G32 G33 G34 G35 G36

]

(hereinafter, referred to as Compound (1E)) wherein X represents CH, L represents an oxygen atom, $R^1$ represents a methyl group, $R^{X2}$ represents a methyl group, and a combination of structure of G and substituent of $R^{X8}$, $R^{X9}$, $R^{X10}$ and $R^{X11}$ applied to the structure of G represents any combination described in Combination C (hereinafter, referred to as Compound Class SX190).

Combination C consists of the Substituent No. ZC1 to ZC1459. The Substituent No. ZC1 to ZC1459 represent a combination of the structure of G and the substituents of $R^{X8}$, $R^{X9}$, $R^{X10}$ and $R^{X11}$ applied to the structure of G in the Compound (1E) and the compounds represented by formula (2E), which hereinafter, is described as [Substituent No.; G, $R^{X8}$, $R^{X9}$, $R^{X10}$, $R^{X11}$]. For example, the Substituent No. ZC2 represents a combination wherein G represents G1, $R^{X8}$ represents a methyl group, and $R^{X9}$, $R^{X10}$, and $R^{X11}$ represent a hydrogen atom.

Combination C:

[ZC1;G1,H,H,H,H],[ZC2;G1,Me,H,H,H],[ZC3;G1,Et,H,H,H],[ZC4;G 1,Pr,H,H,H],[ZC5;G1,i-Pr,H,H,H],[ZC6;G1,t-Bu,H,H,H],[ZC7;G 1,OMe,H,H,H], [ZC8;G1,OEt,H,H,H], [ZC9;G1,OPr,H,H,H], [ZC10;G 1,Oi-Pr,H,H,H],[ZC11;G1,$CF_3$,H,H,H],[ZC12;G1,$CF_2H$,H,H,H],[ZC 13;G1,$CFH_2$,H,H,H],[ZC14;G1,F,H,H,H],[ZC15;G1,Cl,H,H,H],[ZC1 6;G1,Br,H,H,H],[ZC17;G1,CN,H,H,H],[ZC18;G1,Ph,H,H,H],[ZC19; G1,OPh,H,H,H], [ZC20;G1,c-Pr,H,H,H], [ZC21;G1,H,Me,H,H], [ZC2 2;G1,H,Et,H,H],[ZC23;G1,H,Pr,H,H],[ZC24;G1,H,i-Pr,H,H],[ZC2 5;G1,H,t-Bu,H,H],[ZC26;G1,H,OMe,H,H],[ZC27;G1,H,OEt,H,H],[Z C28;G1,H,OPr,H,H],[ZC29;G1,H,Oi-Pr,H,H],[ZC30;G1,H,$CF_3$,H,H], [ZC31;G1,H,$CF_2H$,H,H],[ZC32;G1,H,$CFH_2$,H,H],[ZC33;G1,H,F,H,H], [ZC34;G1,H,Cl,H,H],[ZC35;G1,H,Br,H,H],[ZC36;G1,H,CN,H,H],[Z C37;G1,H,Ph,H,H], [ZC38;G1,H,OPh,H,H], [ZC39;G1,H,c-Pr,H,H], [ZC40;G1,H,H,Me,H],[ZC41;G1,H,H,Et,H],[ZC42;G1,H,H,Pr,H],[Z C43;G1,H,H,i-Pr,H],[ZC44;G1,H,H,t-Bu,H],[ZC45;G1,H,H,OMe,H], [ZC46;G1,H,H,OEt,H],[ZC47;G1,H,H,OPr,H],[ZC48;G1,H,H,Oi-Pr, H],[ZC49;G1,H,H,$CF_3$,H],[ZC50;G1,H,H,$CF_2H$,H],[ZC51;G1,H,H,CF $H_2$,H],[ZC52;G1,H,H,F,H],[ZC53;G1,H,H,Cl,H],[ZC54;G1,H,H,Br, H],[ZC55;G1,H,H,CN,H],[ZC56;G1,H,H,Ph,H],[ZC57;G1,H,H,OPh, H],[ZC58;G1,H,H,c-Pr,H],[ZC59;G1,H,H,H,Me],[ZC60;G1,H,H,H,E t],[ZC61;G1,H,H,H,Pr],[ZC62;G1,H,H,H,i-Pr],[ZC63;G1,H,H,H,t -Bu],[ZC64;G1,H,H,H,OMe],[ZC65;G1,H,H,H,OEt],[ZC66;G1,H,H, H,OPr],[ZC67;G1,H,H,H,Oi-Pr],[ZC68;G1,H,H,H,$CF_3$],[ZC69;G1, H,H,H,$CF_2H$],[ZC70;G1,H,H,H,$CFH_2$],[ZC71;G1,H,H,H,F],[ZC72;G 1,H,H,H,Cl],[ZC73;G1,H,H,H,Br],[ZC74;G1,H,H,H,CN],[ZC75;G1, H,H,H,Ph],[ZC76;G1,H,H,H,OPh],[ZC77;G1,H,H,H,c-Pr],[ZC78;G 1,F,F,H,H],[ZC79;G1,F,H,F,H],[ZC80;G1,F,H,H,F],[ZC81;G1,H, F,F,H],[ZC82;G1,H,F,H,F],[ZC83;G1,H,H,F,F],[ZC84;G1,Cl,Cl, H,H],[ZC85;G1,Cl,H,Cl,H],[ZC86;G1,Cl,H,H,Cl],[ZC87;G1,H,Cl, Cl,H],[ZC88;G1,H,Cl,H,Cl],[ZC89;G1,H,H,Cl,Cl],[ZC90;G2,H,H, H,H],[ZC91;G2,Me,H,H,H],[ZC92;G2,Et,H,H,H],[ZC93;G2,Pr,H,H, H],[ZC94;G2,i-Pr,H,H,H],[ZC95;G2,t-Bu,H,H,H],[ZC96;G2,OMe, H,H,H],[ZC97;G2,OEt,H,H,H],[ZC98;G2,OPr,H,H,H],[ZC99;G2,Oi-Pr,H,H,H],[ZC100;G2,$CF_3$,H,H,H],[ZC101;G2,$CF_2H$,H,H,H],[ZC10

2;G2,$CFH_2$,H,H,H],[ZC103;G2,F,H,H,H],[ZC104;G2,C1,H,H,H],[ZC105;G2,Br,H,H,H],[ZC106;G2,CN,H,H,H],[ZC107;G2,Ph,H,H,H],[Z C108;G2,OPh,H,H,H],[ZC109;G2,c-Pr,H,H,H],[ZC110;G2,H,Me,H, H],[ZC111;G2,H,Et,H,H],[ZC112;G2,H,Pr,H,H],[ZC113;G2,H,i-P r,H,H],[ZC114;G2,H,t-Bu,H,H],[ZC115;G2,H,OMe,H,H],[ZC116;G 2,H,OEt,H,H],[ZC117;G2,H,OPr,H,H],[ZC118;G2,H,Oi-Pr,H,H],[ZC119;G2,H,$CF_3$,H,H],[ZC120;G2,H,$CF_2$H,H,H],[ZC121;G2,H,$CFH_2$,H,H],[ZC122;G2,H,F,H,H],[ZC123;G2,H,Cl,H,H],[ZC124;G2,H,Br,H,H],[ZC125;G2,H,CN,H,H],[ZC126;G2,H,Ph,H,H],[ZC127;G2,H,OP h,H,H],[ZC128;G2,H,c-Pr,H,H],[ZC129;G2,H,H,Me,H],[ZC130;G2, H,H,Et,H],[ZC131;G2,H,H,Pr,H],[ZC132;G2,H,H,i-Pr,H],[ZC133; G2,H,H,t-Bu,H],[ZC134;G2,H,H,OMe,H],[ZC135;G2,H,H,OEt,H],[Z C136;G2,H,H,OPr,H], [ZC137 ; G2,H,H,Oi-Pr,H], [ZC138;G2,H,H,$CF_3$,H],[ZC139;G2,H,H,$CF_2$H,H],[ZC140;G2,H,H,$CFH_2$,H],[ZC141;G2,H,H,F,H],[ZC142;G2,H,H,Cl,H],[ZC143;G2,H,H,Br,H],[ZC144;G2,H,H,CN,H],[ZC145;G2,H,H,Ph,H],[ZC146;G2,H,H,OPh,H],[ZC147;G 2,H,H,c-Pr,H],[ZC148;G2,H,H,H,Me],[ZC149;G2,H,H,H,Et],[ZC15 0;G2,H,H,H,Pr],[ZC151;G2,H,H,H,i-Pr],[ZC152;G2,H,H,H,t-Bu], [ZC153;G2,H,H,H,OMe],[ZC154;G2,H,H,H,OEt],[ZC155;G2,H,H,H,0 Pr],[ZC156;G2,H,H,H,Oi-Pr],[ZC157;G2,H,H,H,$CF_3$],[ZC158;G2, H,H,H,$CF_2$H],[ZC159;G2,H,H,H,$CFH_2$],[ZC160;G2,H,H,H,F],[ZC161;G2,H,H,H,Cl],[ZC162;G2,H,H,H,Br],[ZC163;G2,H,H,H,CN],[ZC164;G2,H,H,H,Ph],[ZC165;G2,H,H,H,OPh],[ZC166;G2,H,H,H,c-Pr],[ZC167;G2,F,F,H,H],[ZC168;G2,F,H,F,H],[ZC169;G2,F,H,H,F],[ZC170;G2,H,F,F,H],[ZC171;G2,H,F,H,F],[ZC172;G2,H,H,F,F],[ZC173;G2,Cl,Cl,H,H],[ZC174;G2,Cl,H,Cl,H],[ZC175;G2,Cl,H,H,Cl],[ZC176;G2,H,Cl,Cl,H],[ZC177;G2,H,Cl,H,Cl],[ZC178;G2,H,H,Cl,Cl],[ZC179;G3,H,H,H,H],[ZC180;G3,Me,H,H,H],[ZC181;G3,Et,H, H,H],[ZC182;G3,Pr,H,H,H],[ZC183;G3,i-Pr,H,H,H],[ZC184;G3,t-Bu,H,H,H],[ZC185;G3,OMe,H,H,H],[ZC186;G3,OEt,H,H,H],[ZC187;G3,OPr,H,H,H],[ZC188;G3,Oi-Pr,H,H,H],[ZC189;G3,$CF_3$,H,H,H],[ZC190;G3,$CF_2$H,H,H,H],[ZC191;G3,$CFH_2$,H,H,H],[ZC192;G3,F,H,H,H],[ZC193;G3,Cl,H,H,H],[ZC194;G3,Br,H,H,H],[ZC195;G3,CN,H,H,H],[ZC196;G3,Ph,H,H,H],[ZC197;G3,OPh,H,H,H],[ZC198;G3,c-Pr,H,H,H],[ZC199;G3,H,Me,H,H],[ZC200;G3,H,Et,H,H],[ZC201;G 3,H,Pr,H,H],[ZC202;G3,H,i-Pr,H,H],[ZC203;G3,H,t-Bu,H,H],[ZC 204;G3,H,OMe,H,H],[ZC205;G3,H,OEt,H,H],[ZC206;G3,H,OPr,H,H], [ZC207;G3,H,Oi-Pr,H,H],[ZC208;G3,H,$CF_3$,H,H],[ZC209;G3,H,$CF_2$ H,H,H],[ZC210;G3,H,$CFH_2$,H,H],[ZC211;G3,H,F,H,H],[ZC212;G3,H,Cl,H,H], [ZC213;G3,H,Br,H,H], [ZC214;G3,H,CN,H,H], [ZC215;G3,H,Ph,H,H],[ZC216;G3,H,OPh,H,H],[ZC217;G3,H,c-Pr,H,H],[ZC218;G3,H,H,Me,H],[ZC219;G3,H,H,Et,H],[ZC220;G3,H,H,Pr,H],[ZC 221;G3,H,H,i-Pr,H],[ZC222;G3,H,H,t-Bu,H],[ZC223;G3,H,H,OMe, H],[ZC224;G3,H,H,OEt,H],[ZC225;G3,H,H,OPr,H],[ZC226;G3,H,H, Oi-Pr,H],[ZC227;G3,H,H,$CF_3$,H],[ZC228;G3,H,H,$CF_2$H,H],[ZC229;G3,H,H,$CFH_2$,H],[ZC230;G3,H,H,F,H],[ZC231;G3,H,H,Cl,H],[ZC232;G3,H,H,Br,H],[ZC233;G3,H,H,CN,H],[ZC234;G3,H,H,Ph,H],[ZC2 35;G3,H,H,OPh,H],[ZC236;G3,H,H,c-Pr,H],[ZC237;G3,H,H,H,Me], [ZC238;G3,H,H,H,Et],[ZC239;G3,H,H,H,Pr],[ZC240;G3,H,H,H,i-P r],[ZC241;G3,H,H,H,t-Bu],[ZC242;G3,H,H,H,OMe],[ZC243;G3,H, H,H,OEt],[ZC244;G3,H,H,H,OPr],[ZC245;G3,H,H,H,Oi-Pr],[ZC246;G3,H,H,H,$CF_3$],[ZC247;G3,H,H,H,$CF_2$H],[ZC248;G3,H,H,H,$CFH_2$],[ZC249;G3,H,H,H,F],[ZC250;G3,H,H,H,Cl],[ZC251;G3,H,H,H,Br],[ZC252;G3,H,H,H,CN],[ZC253;G3,H,H,H,Ph],[ZC254;G3,H,H,H,OP h],[ZC255;G3,H,H,H,c-Pr],[ZC256;G3,F,F,H,H],[ZC257;G3,F,H, F,H],[ZC258;G3,F,H,H,F],[ZC259;G3,H,F,F,H],[ZC260;G3,H,F,H, F],[ZC261;G3,H,H,F,F], [ZC262;G3,Cl,Cl,H,H], [ZC263;G3,Cl,H,C1,H],[ZC264;G3,Cl,H,H,Cl],[ZC265;G3,H,Cl,Cl,H],[ZC266;G3,H,Cl,H,Cl],[ZC267;G3,H,H,Cl,Cl],[ZC268;G4,H,H,H,-],[ZC269;G4,Me,H,H,-],[ZC270;G4,Et,H,H,-],[ZC271;G4,Pr,H,H,-],[ZC272;G 4,i-Pr,H,H,-],[ZC273;G4,OMe,H,H,-],[ZC274;G4,$CF_3$,H,H,-],[ZC275;G4,F,H,H,-],[ZC276;G4,Cl,H,H,-],[ZC277;G4,Br,H,H,-],[ZC278;G4,CN,H,H,-],[ZC279;G4,Ph,H,H,-],[ZC280;G4,OPh,H,H,-], [ZC281;G4,c-Pr,H,H,-],[ZC282;G4,H,Me,H,-],[ZC283;G4,H,Et,H, -],[ZC284;G4,H,Pr,H,-],[ZC285;G4,H,i-Pr,H,-],[ZC286;G4,H,OMe,H,-],[ZC287;G4,H,$CF_3$,H,-],[ZC288;G4,H,F,H,-],[ZC289;G4,H,Cl,H,-],[ZC290;G4,H,Br,H,-],[ZC291;G4,H,CN,H,-],[ZC292;G4, H,Ph,H,-],[ZC293;G4,H,OPh,H,-],[ZC294;G4,H,c-Pr,H,-],[ZC29 5;G4,H,H,Me,-],[ZC296;G4,H,H,Et,-],[ZC297;G4,H,H,Pr,-],[ZC2 98;G4,H,H,i-Pr,-],[ZC299;G4,H,H,OMe,-],[ZC300;G4,H,H,$CF_3$,-], [ZC301;G4,H,H,F,-],[ZC302;G4,H,H,Cl,-],[ZC303;G4,H,H,Br,-],[ZC304;G4,H,H,CN,-],[ZC305;G4,H,H,Ph,-],[ZC306;G4,H,H,OPh, -],[ZC307;G4,H,H,c-Pr,-],[ZC308;G5,H,H,H,-],[ZC309;G5,Me,H, H,-],[ZC310;G5,Et,H,H,-],[ZC311;G5,Pr,H,H,-],[ZC312;G5,i-P r,H,H,-],[ZC313;G5,OMe,H,H,-],[ZC314;G5,$CF_3$,H,H,-],[ZC315;G

5,F,H,H,-],[ZC316;G5,Cl,H,H,-],[ZC317;G5,Br,H,H,-],[ZC318;G5,CN,H,H,-],[ZC319;G5,c-Pr,H,H,-],[ZC320;G5,H,Me,H,-],[ZC321;G5,H,Et,H,-],[ZC322;G5,H,Pr,H,-],[ZC323;G5,H,i-Pr,H,-],[ZC324;G5,H,OMe,H,-],[ZC325;G5,H,$CF_3$,H,-],[ZC326;G5,H,F,H,-],[ZC327;G5,H,Cl,H,-],[ZC328;G5,H,Br,H,-],[ZC329;G5,H,CN,H,-],[ZC330;G5,H,Ph,H,-],[ZC331;G5,H,OPh,H,-],[ZC332;G5,H,c-Pr, H,-], [ZC333;G5,H,H,Me,-], [ZC334;G5,H,H,Et,-],[ZC335;G5,H,H,Pr,-],[ZC336;G5,H,H,i-Pr,-],[ZC337;G5,H,H,OMe,-],[ZC338;G5,H,H,$CF_3$,-],[ZC339;G5,H,H,F,-],[ZC340;G5,H,H,Cl,-],[ZC341;G5,H,H,Br,-],[ZC342;G5,H,H,CN,-],[ZC343;G5,H,H,c-Pr,-],[ZC344;G6,H,H,H,-],[ZC345;G6,Me,H,H,-],[ZC346;G6,Et,H,H,-],[ZC347;G6,Pr,H,H,-],[ZC348;G6,i-Pr,H,H,-],[ZC349;G6,OMe,H,H,-],[ZC350;G6,$CF_3$,H,H,-],[ZC351;G6,F,H,H,-],[ZC352;G6,Cl,H,H,-],[ZC353;G6,Br,H,H,-],[ZC354;G6,CN,H,H,-],[ZC355;G6,Ph,H,H,-],[ZC356;G6,OPh,H,H,-],[ZC357;G6,c-Pr,H,H,-],[ZC358;G6,H,Me,H,-],[ZC359;G6,H,Et,H,-],[ZC360;G6,H,Pr,H,-],[ZC361;G6,H,i-Pr,H,-],[ZC362;G6,H,OMe,H,-],[ZC363;G6,H,$CF_3$,H,-],[ZC364;G6,H,F,H,-],[ZC365;G6,H,Cl,H,-],[ZC366;G6,H,Br,H,-],[ZC367;G6,H,CN,H,-],[ZC368;G6,H,Ph,H,-],[ZC369;G6,H,OPh,H,-],[ZC370;G6,H,c-Pr,H,-],[ZC371;G6,H,H,Me,-],[ZC372;G6,H,H,Et,-],[ZC373;G6,H,H,Pr,-],[ZC374;G6,H,H,i-Pr,-],[ZC375;G6,H,H,OMe,-],[ZC376;G6,H,H,$CF_3$,-],[ZC377;G6,H,H,F,-],[ZC378;G6,H,H,C1,-],[ZC379;G6,H,H,Br,-],[ZC380;G6,H,H,CN,-],[ZC381;G6,H,H,c-Pr,-],[ZC382;G7,H,H,H,-],[ZC383;G7,Me,H,H,-],[ZC384;G7,Et,H,H,-],[ZC385;G7,Pr,H,H,-],[ZC386;G7,i-Pr,H,H,-],[ZC387;G7,OMe,H,H,-],[ZC388;G7,$CF_3$,H,H,-],[ZC389;G7,F,H,H,-],[ZC390;G7,Cl,H,H,-],[ZC391;G7,Br,H,H,-],[ZC392;G7,CN,H,H,-],[ZC393;G7,Ph,H,H,-],[ZC394;G7,OPh,H,H,-],[ZC395;G7,c-Pr,H,H,-], [ZC396;G7,H,Me,H,-],[ZC397;G7,H,Et,H,-],[ZC398;G7,H,Pr,H,-], [ZC399;G7,H,i-Pr,H,-],[ZC400;G7,H,OMe,H,-],[ZC401;G7,H,$CF_3$,H,-],[ZC402;G7,H,F,H,-],[ZC403;G7,H,Cl,H,-],[ZC404;G7,H,Br,H,-],[ZC405;G7,H,CN,H,-],[ZC406;G7,H,Ph,H,-],[ZC407;G7,H,OPh,H,-],[ZC408;G7,H,c-Pr,H,-],[ZC409;G7,H,H,Me,-],[ZC410;G7,H,H,Et,-], [ZC411;G7,H,H,Pr,-], [ZC412;G7,H,H,i-Pr,-], [ZC413;G7,H,H,OMe,-],[ZC414;G7,H,H,$CF_3$,-],[ZC415;G7,H,H,F,-],[ZC416;G7,H,H,Cl,-],[ZC417;G7,H,H,Br,-],[ZC418;G7,H,H,CN,-],[ZC419;G7,H,H,c-Pr,-],[ZC420;G8,H,H,H,-],[ZC421;G8,Me,H,H,-],[ZC422;G8,Et,H,H,-], [ZC423;G8,Pr,H,H,-], [ZC424;G8,i-Pr,H,H,-],[ZC425;G8,OMe,H,H,-],[ZC426;G8,$CF_3$,H,H,-],[ZC427;G8,F,H,H,-],[ZC428;G8,Cl,H,H,-],[ZC429;G8,Br,H,H,-],[ZC430;G8,CN,H,H,-], [ZC431;G8,c-Pr,H,H,-], [ZC432;G8,H,Me,H,-], [ZC433;G8,H,Et,H,-],[ZC434;G8,H,Pr,H,-],[ZC435;G8,H,i-Pr,H,-],[ZC436;G8,H,OMe,H,-],[ZC437;G8,H,$CF_3$,H,-],[ZC438;G8,H,F,H,-],[ZC439;G8,H,C1,H,-],[ZC440;G8,H,Br,H,-],[ZC441;G8,H,CN,H,-],[ZC442;G8,H,Ph,H,-],[ZC443;G8,H,OPh,H,-],[ZC444;G8,H,c-Pr,H,-],[ZC445;G8,H,H,Me,-],[ZC446;G8,H,H,Et,-],[ZC447;G8,H,H,Pr,-], [ZC448;G8,H,H,i-Pr,-],[ZC449;G8,H,H,OMe,-],[ZC450;G8,H,H,$CF_3$,-],[ZC451;G8,H,H,F,-],[ZC452;G8,H,H,Cl,-],[ZC453;G8,H,H,Br,-],[ZC454;G8,H,H,CN,-],[ZC455;G8,H,H,c-Pr,-],[ZC456;G9,H,H,H,-], [ZC457;G9,Me,H,H,-], [ZC458;G9,Et,H,H,-],[ZC459;G9,Pr,H,H,-],[ZC460;G9,i-Pr,H,H,-],[ZC461;G9,OMe,H,H,-],[ZC462;G9,$CF_3$,H,H,-],[ZC463;G9,F,H,H,-],[ZC464;G9,Cl,H,H,-],[ZC465;G9,Br,H,H,-],[ZC466;G9,CN,H,H,-],[ZC467;G9,Ph,H,H,-],[ZC468;G9,OPh,H,H,-],[ZC469;G9,c-Pr,H,H,-],[ZC470;G9,H,Me,H,-], [ZC471;G9,H,Et,H,-],[ZC472;G9,H,Pr,H,-],[ZC473;G9,H,i-Pr,H,-],[ZC474;G9,H,OMe,H,-],[ZC475;G9,H,$CF_3$,H,-],[ZC476;G9,H,F,H,-],[ZC477;G9,H,Cl,H,-],[ZC478;G9,H,Br,H,-],[ZC479;G9,H,CN,H,-],[ZC480;G9,H,Ph,H,-],[ZC481;G9,H,OPh,H,-],[ZC482;G9,H,c-Pr,H,-],[ZC483;G9,H,H,Me,-],[ZC484;G9,H,H,Et,-],[ZC485;G9,H,H,Pr,-],[ZC486;G9,H,H,i-Pr,-],[ZC487;G9,H,H,OMe,-],[ZC488;G9,H,H,$CF_3$,-],[ZC489;G9,H,H,F,-],[ZC490;G9,H,H,Cl,-],[ZC491;G9,H,H,Br,-],[ZC492;G9,H,H,CN,-],[ZC493;G9,H,H,c-Pr,-],[ZC494;G10,H,H,H,-],[ZC495;G10,Me,H,H,-],[ZC496;G10,Et,H,H,-],[ZC497;G10,Pr,H,H,-],[ZC498;G10,i-Pr,H,H,-],[ZC499;G10,OMe,H,H,-],[ZC500;G10,$CF_3$,H,H,-],[ZC501;G10,F,H,H,-],[ZC502;G10,Cl,H,H,-],[ZC503;G10,Br,H,H,-], [ZC504;G10,CN,H,H,-], [ZC505;G10,Ph,H,H,-],[ZC506;G10,OPh,H,H,-],[ZC507;G10,c-Pr,H,H,-],[ZC508;G10,H,Me,H,-],[ZC509;G10,H,Et,H,-],[ZC510;G10,H,Pr,H,-],[ZC511;G10,H,i-Pr,H,-],[ZC512;G10,H,OMe,H,-],[ZC513;G10,H,$CF_3$,H,-],[ZC514;G10,H,F,H,-],[ZC515;G10,H,C1,H,-],[ZC516;G10,H,Br,H,-],[ZC517;G10,H,CN,H,-],[ZC518;G10,H,Ph,H,-],[ZC519;G10,H,OPh,H,-],[ZC520;G10,H,c-Pr,H,-],[ZC521;G10,H,H,Me,-],[ZC522;G10,H,H,Et,-],[ZC523;G10,H,H,Pr,-],[ZC524;G10,H,H,i-Pr,-],[ZC525;G10,H,H,OMe,-], [ZC526;G10,H,H,$CF_3$,-],[ZC527;G10,H,H,F,-],[ZC528;G10,H,H,Cl,-],[ZC529;G10,H,H,Br,-],[ZC530;G10,H,H,CN,-],[ZC531;G10,H,H,Ph,-],[ZC532;G10,H,H,OPh,-],[ZC533;G10,H,H,c-Pr,-],[ZC534;G11,H,H,H,-],[ZC535;G11,Me,H,H,-],[ZC536;G11,Et,H,H,-],[ZC537;G11,Pr,H,H,-], [ZC538;G11,i-Pr,H,H,-],[ZC539;G11,OMe,H,H,-],[ZC540;G11,$CF_3$,H,H,-],[ZC541;G11,F,H,H,-],[ZC542;G11,Cl,H,H,-],[ZC543;G11,Br,H,H,-],[ZC544;G11,CN,H,H,-],[ZC545;G11,Ph,H,H,-],[ZC546;G11,OPh,H,H,-],[ZC547;G11,c-Pr,H,H,-],[ZC548;G11,H,Me,H, -],[ZC549;G11,H,Et,H,-],[ZC550;G11,H,Pr,H,-],[ZC551;G11,H,i-Pr,H,-],[ZC552;G11,H,OMe,H,-],[ZC553;G11,H,$CF_3$,H,-],[ZC554;G11,H,F,H,-],[ZC555;G11,H,Cl,H,-],[ZC556;G11,H,Br,H,-],[ZC557;G11,H,CN,H,-],[ZC558;G11,H,Ph,H,-],[ZC559;G11,H,OPh,H, -],[ZC560;G11,H,c-Pr,H,-],[ZC561;G11,H,H,Me,-],[ZC562;G11, H,H,Et,-],[ZC563;G11,H,H,Pr,-],[ZC564;G11,H,H,i-Pr,-],[ZC565;G11,H,H,OMe,-],[ZC566;G11,H,H,$CF_3$,-],[ZC567;G11,H,H,F,-],[ZC568;G11,H,H,Cl,-],[ZC569;G11,H,H,Br,-],[ZC570;G11,H,H,CN,-],[ZC571;G11,H,H,Ph,-],[ZC572;G11,H,H,OPh,-],[ZC573;G11, H,H,c-Pr,-],[ZC574;G12,H,H,H,-],[ZC575;G12,Me,H,H,-],[ZC576;G12,Et,H,H,-],[ZC577;G12,Pr,H,H,-],[ZC578;G12,i-Pr,H,H,-], [ZC579;G12,t-Bu, H, H,-],[ZC580;G12,$CF_3$,H,H,-],[ZC581;G12,$CHF_2$,H,H,-],[ZC582;G12,$CH_2F$,H,H,-],[ZC583;G12,Ph,H,H,-],[ZC584;G12,c-Pr,H,H,-],[ZC585;G12,c-Bu,H,H,-],[ZC586;G12,c-Pen, ,[ZC587;G12,C-Hex,H,H,-],[ZC588;G12,Me,Me,H,-],[ZC589;G12,Me,Et,H,-],[ZC590;G12,Me,Pr,H,-],[ZC591;G12,Me,i-Pr, H,-],[ZC592;G12,Me,OMe,H,-],[ZC593;G12,Me,$CF_3$,H,-],[ZC594;G12,Me,F,H,-],[ZC595;G12,Me,Cl,H,-],[ZC596;G12,Me,Br,H,-],[ZC597;G12,Me,CN,H,-],[ZC598;G12,Me,c-Pr,H,-],[ZC599;G12,Me, H,Me,-],[ZC600;G12,Me,H,Et,-],[ZC601;G12,Me,H,Pr,-],[ZC602;G12,Me,H,i-Pr,-],[ZC603;G12,Me,H,OMe,-],[ZC604;G12,Me,H,$CF_3$,-],[ZC605;G12,Me,H,F,-],[ZC606;G12,Me,H,Cl,-],[ZC607;G12,Me,H,Br,-],[ZC608;G12,Me,H,CN, -],[ZC609;G12,Me,H,c-Pr,-],[ZC610;G13,H,H,H,-],[ZC611;G13,Me,H,H,-],[ZC612;G13,Et,H,H,-],[ZC613;G13,Pr,H,H,-],[ZC614;G13,i-Pr,H,H,-],[ZC615;G13,t-Bu,H,H,-],[ZC616;G13,OMe,H,H,-],[ZC617;G13,OEt,H,H,-],[ZC618;G13,OPr,H,H,-],[ZC619;G13,Oi-Pr,H,H,-],[ZC620;G13,$CF_3$,H,H,-],[ZC621;G13,$CF_2H$,H,H,-],[ZC622;G13,$CFH_2$,H,H,-],[ZC623;G13,F,H,H,-],[ZC624;G13,Cl,H,H,-], [ZC625; G13, Br, H, H, -], [ZC626; G13, CN, H, H, -], [ZC627; G13, Ph, H, H,-],[ZC628;G13,OPh,H,H,-],[ZC629;G13,c-Pr,H,H,-],[ZC630;G13,H,Me,H,-],[ZC631;G13,H,Et,H,-],[ZC632;G13,H,Pr,H,-],[ZC633;G13,H,i-Pr,H,-],[ZC634;G13,H,t-Bu,H,-],[ZC635;G13,H,OMe, H,-],[ZC636;G13,H,OEt,H,-],[ZC637;G13,H,OPr,H,-],[ZC638;G13,H,Oi-Pr,H,-],[ZC639;G13,H,$CF_3$,H,-],[ZC640;G13,H,$CF_2H$,H,-],[ZC641;G13,H,$CFH_2$,H,-],[ZC642;G13,H,F,H,-],[ZC643;G13,H,Cl, H,-],[ZC644;G13,H,Br,H,-],[ZC645;G13,H,CN,H,-],[ZC646;G13, H,Ph,H,-],[ZC647;G13,H,OPh,H,-],[ZC648;G13,H,c-Pr,H,-],[ZC649;G13,H,H,Me,-],[ZC650;G13,H,H,Et,-],[ZC651;G13,H,H,Pr,-], [ZC652;G13,H,H,i-Pr,-],[ZC653;G13,H,H,t-Bu,-],[ZC654;G13,H, H,OMe,-],[ZC655;G13,H,H,OEt,-],[ZC656;G13,H,H,OPr,-],[ZC657;G13,H,H,Oi-Pr,-],[ZC658;G13,H,H,$CF_3$,-],[ZC659;G13,H,H,$CF_2H$,-],[ZC660;G13,H,H,$CFH_2$,-],[ZC661;G13,H,H,F,-],[ZC662;G13, H,H,Cl,-], [ZC663;G13,H,H,Br,-], [ZC664;G13,H,H,CN,-], [ZC665; G13,H,H,Ph,-],[ZC666;G13,H,H,OPh,-],[ZC667;G13,H,H,c-Pr,-],[ZC668;G13,CH(Me)Et,H,H,-],[ZC669;G13,CH(Et)2,H,H,-],[ZC670;G13,$CH_2$(i-Pr),H,H,-],[ZC671;G13,C(Me)=CH(Me),H,H,-],[ZC672;G13,C(Me)=$CH_2$,H,H,-],[ZC673;G13,CH=CH(Me),H,H,-],[ZC674;G13,CH=$CH_2$,H,H,-],[ZC675;G13,CC(c-Pr),H,H,-],[ZC676;G13,CC(Me),H,H,-],[ZC677;G13,$CHF_2$,H,H,-],[ZC678;G13,$CF_2Me$,H,H,-],[ZC679;G13,$CF_2CF_3$,H,H,-],[ZC680;G13,C($CF_3$)$CH_2$,H,H,-],[ZC681;G13,$CF_2Cl$,H,H,-],[ZC682;G13,CH(Me)$CF_3$,H,H,-],[ZC683;G13,c-Bu,H,H,-],[ZC684;G13,c-Pen,H,H,-],[ZC685;G13,c-Hex,H,H,-],[ZC686;G13,1-Me-(c-Pr),H,H,-],[ZC687;G13,1-F-(c-Pr),H,H,-],[ZC688;G13,2,2-$F_2$-(c-Pr),H,H,-],[ZC689;G13,I,H,H,-],[ZC690;G13,$CH_2CN$,H,H,-],[ZC691;G13,$CH_2OMe$,H,H,-],[ZC692;G13,$CH_2SMe$,H,H,-],[ZC693;G13,c-hexen-1-yl,H,H,-],[ZC694;G13,$OCHF_2$,H,H,-],[ZC695;G13,$OCF_3$,H,H,-],[ZC696;G13,$SCF_3$,H,H,-],[ZC697;G13,C(Me)=NOMe,H,H,-],[ZC698;G13,H,c-Bu,H,-],[ZC699;G13,H,c-Pen,H,-],[ZC700;G13,H,c-Hex,H,-],[ZC701;G13,H,CCH,H,-],[ZC702;G13,H,$CH_2OMe$,H,-],[ZC703;G13,H,I,H,-],[ZC704;G13,H,S(i-Pr),H,-],[ZC705;G13,Me,Me,H,-],[ZC706;G13,Pr,Me,H,-],[ZC707; G13,i-Pr,Me,H,-],[ZC708;G13,$CHF_2$,Me,H,-],[ZC709;G13,$CF_3$,Me, H,-],[ZC710;G13,c-Pr,Me,H,-],[ZC711;G13,c-Hex,Me,H,-],[ZC712;G13,OMe,Me,H,-],[ZC713;G13,0(i-Pr),Me,H,-],[ZC714;G13,F,Me,H,-],[ZC715;G13,Cl,Me,H,-],[ZC716;G13,Br,Me,H,-],[ZC717;G13,i-Pr,F,H,-],[ZC718;G13,Me,F,H,-],[ZC719;G13,Pr,F,H,-],[ZC720;G13,$CHF_2$,F,H,-],[ZC721;G13,$CF_3$,F,H,-],[ZC722;G13,c-Pr, F,H,-],[ZC723;G13,c-Hex,F,H,-],[ZC724;G13,OMe,F,H,-],[ZC725;G13,Oi-Pr,F,H,-],[ZC726;G13,F,F,H,-],[ZC727;G13,Cl,F,H,-],[ZC728;G13,Br,F,H,-],[ZC729¡G13,Pr,Cl,H,-],[ZC730;G13,i-Pr, Cl,H,-],[ZC731;G13,c-Hex,Cl,H,-],[ZC732;G13,Cl,Cl,H,-],[ZC733;G13,Oi-Pr,Cl,H,-],[ZC734;G13,Me,Cl,H,-],[ZC735;G13,$CHF_2$,C1,H,-],[ZC736;G13,$CF_3$,Cl,H,-],[ZC737;G13,cPr,Cl,H,-],[ZC738;G13,OMe,Cl,H,-],[ZC739;G13,F,Cl,H,-],[ZC740;G13,Br,Cl,H, -],[ZC741;G13,Me,Br,H,-],[ZC742;G13,c-Hex,Br,H,-],[ZC743;G13,Pr,Br,H,-],[ZC744;G13,i-Pr,Br,H,-],[ZC745;G13,CHF$_2$,Br,H,-],[ZC746;G13,CF$_3$,Br,H,-],[ZC747;G13,c-Pr,Br,H,-],[ZC748;G13,OMe,Br,H,-],[ZC749;G13,Oi-Pr,Br,H,-],[ZC750;G13,F,Br,H,-],[ZC751;G13,Cl,Br,H,-],[ZC752;G13,Br,Br,H,-],[ZC753;G14,H,H,H,-],[ZC754;G14,Me,H,H,-],[ZC755;G14,Et,H,H,-],[ZC756;G14,Pr,H,H,-],[ZC757;G14,i-Pr,H,H,-],[ZC758;G14,t-Bu,H,H,-],[ZC759;G14,CF$_3$,H,H,-],[ZC760;G14,CHF$_2$,H,H,-],[ZC761;G14,CH$_2$F,H,H,-],[ZC762;G14,Ph,H,H,-],[ZC763;G14,c-Pr,H,H,-],[ZC764;G14,c-Bu,H,H,-],[ZC765;G14,c-Pen,H,H,-],[ZC766;G14,C-Hex,H,H,-],[ZC767;G14,Me,Me,H,-],[ZC768;G14,Me,Et,H,-],[ZC769;G14,Me,Pr,H,-],[ZC770;G14,Me,i-Pr,H,-],[ZC771;G14,Me,OMe,H,-],[ZC772;G14,Me,CF$_3$,H,-],[ZC773;G14,Me,F,H,-],[ZC774;G14,Me,Cl,H,-],[ZC775;G14,Me,Br,H,-],[ZC776;G14,Me,CN,H,-],[ZC777;G14,Me,c-Pr,H,-],[ZC778;G14,Me,H,Me,-],[ZC779;G14,Me,H,Et,-],[ZC780;G14,Me,H,Pr,-],[ZC781;G14,Me,H,i-Pr,-],[ZC782;G14,Me,H,OMe,-],[ZC783;G14,Me,H,CF$_3$,-],[ZC784;G14,Me,H,F,-],[ZC785;G14,Me,H,Cl,-],[ZC786;G14,Me,H,Br,-],[ZC787;G14,Me,H,CN,-],[ZC788;G14,Me,H,c-Pr,-],[ZC789;G15,H,H,H,-],[ZC790;G15,Me,H,H,-],[ZC791;G15,Et,H,H,-],[ZC792;G15,Pr,H,H,-],[ZC793;G15,i-Pr,H,H,-],[ZC794;G15,OMe,H,H,-],[ZC795;G15,CF$_3$,H,H,-],[ZC796;G15,F,H,H,-],[ZC797;G15,Cl,H,H,-],[ZC798;G15,Br,H,H,-],[ZC799;G15,CN,H,H,-],[ZC800;G15,Ph,H,H,-],[ZC801;G15,OPh,H,H,-],[ZC802;G15,c-Pr,H,H,-],[ZC803;G15,H,Me,H,-],[ZC804;G15,H,Et,H,-],[ZC805;G15,H,Pr,H,-],[ZC806;G15,H,i-Pr,H,-],[ZC807;G15,H,OMe,H,-],[ZC808;G15,H,CF$_3$,H,-],[ZC809;G15,H,F,H,-],[ZC810;G15,H,Cl,H,-],[ZC811;G15,H,Br,H,-],[ZC812;G15,H,CN,H,-],[ZC813;G15,H,Ph,H,-],[ZC814;G15,H,OPh,H,-],[ZC815;G15,H,c-Pr,H,-],[ZC816;G15,H,H,Me,-],[ZC817;G15,H,H,Et,-],[ZC818;G15,H,H,Pr,-],[ZC819;G15,H,H,i-Pr,-],[ZC820;G15,H,H,OMe,-],[ZC821;G15,H,H,CF$_3$,-],[ZC822;G15,H,H,F,-],[ZC823;G15,H,H,Cl,-],[ZC824;G15,H,H,Br,-],[ZC825;G15,H,H,CN,-],[ZC826;G15,H,H,Ph,-],[ZC827;G15,H,H,OPh,-],[ZC828;G15,H,H,c-Pr,-],[ZC829;G16,H,H,H,-],[ZC830;G16,Me,H,H,-],[ZC831;G16,Et,H,H,-],[ZC832;G16,Pr,H,H,-],[ZC833;G16,i-Pr,H,H,-],[ZC834;G16,OMe,H,H,-],[ZC835;G16,CF$_3$,H,H,-],[ZC836;G16,F,H,H,-],[ZC837;G16,Cl,H,H,-],[ZC838;G16,Br,H,H,-],[ZC839;G16,CN,H,H,-],[ZC840;G16,Ph,H,H,-],[ZC841;G16,OPh,H,H,-],[ZC842;G16,c-Pr,H,H,-],[ZC843;G16,H,Me,H,-],[ZC844;G16,H,Et,H,-],[ZC845;G16,H,Pr,H,-],[ZC846;G16,H,i-Pr,H,-],[ZC847;G16,H,OMe,H,-],[ZC848;G16,H,CF$_3$,H,-],[ZC849;G16,H,F,H,-],[ZC850;G16,H,Cl,H,-],[ZC851;G16,H,Br,H,-],[ZC852;G16,H,CN,H,-],[ZC853;G16,H,c-Pr,H,-],[ZC854;G16,H,H,Me,-],[ZC855;G16,H,H,Et,-],[ZC856;G16,H,H,Pr,-],[ZC857;G16,H,H,i-Pr,-],[ZC858;G16,H,H,OMe,-],[ZC859;G16,H,H,CF$_3$,-],[ZC860;G16,H,H,F,-],[ZC861;G16,H,H,C1,-],[ZC862;G16,H,H,Br,-],[ZC863;G16,H,H,CN,-],[ZC864;G16,H,H,c-Pr,-],[ZC865;G17,H,H,-,-],[ZC866;G17,Me,H,-,-],[ZC867;G17,Et,H,-,-],[ZC868;G17,Pr,H,-,-],[ZC869;G17,i-Pr,H,-,-],[ZC870;G17,OMe,H,-,-],[ZC871;G17,CF$_3$,H,-,-],[ZC872;G17,F,H,-,-],[ZC873;G17,Cl,H,-,-],[ZC874;G17,Br,H,-,-],[ZC875;G17,CN,H,-,-],[ZC876;G17,Ph,H,-,-],[ZC877;G17,CH$_2$Ph,H,-,-],[ZC878;G17,c-Pr,H,-,-],[ZC879;G17,H,Me,-,-],[ZC880;G17,H,Et,-,-],[ZC881;G17,H,Pr,-,-],[ZC882;G17,H,i-Pr,-,-],[ZC883;G17,H,OMe,-,-],[ZC884;G17,H,CF$_3$,-,-],[ZC885;G17,H,F,-,-],[ZC886;G17,H,Cl,-,-],[ZC887;G17,H,Br,-,-],[ZC888;G17,H,CN,-,-],[ZC889;G17,H,c-Pr,-,-],[ZC890;G18,H,H,-,-],[ZC891;G18,Me,H,-,-],[ZC892;G18,Et,H,-,-],[ZC893;G18,Pr,H,-,-],[ZC894;G18,i-Pr,H,-,-],[ZC895;G18,OMe,H,-,-],[ZC896;G18,CF$_3$,H,-,-],[ZC897;G18,F,H,-,-],[ZC898;G18,Cl,H,-,-],[ZC899;G18,Br,H,-,-],[ZC90 0;G18,CN,H,-,-],[ZC901;G18,Ph,H,-,-],[ZC902;G18,CH$_2$Ph,H,-,-],[ZC903;G18,c-Pr,H,-,-],[ZC904;G18,H,Me,-,-],[ZC905;G18,H,Et,-,-],[ZC906;G18,H,Pr,-,-],[ZC907;G18,H,i-Pr,-,-],[ZC908;G18,H,OMe,-,-],[ZC909;G18,H,CF$_3$,-,-],[ZC910;G18,H,F,-,-],[ZC911;G18,H,Cl,-,-],[ZC912;G18,H,Br,-,-],[ZC913;G18,H,CN,-,-],[ZC914;G18,H,c-Pr,-,-],[ZC915;G19,H,H,-,-],[ZC916;G19,Me,H,-,-],[ZC917;G19,Et,H,-,-],[ZC918;G19,Pr,H,-,[ZC919;G19,i-Pr,H,-,-],[ZC920;G19,OMe,H,-,-],[ZC921;G19,CF$_3$,H,-,-],[ZC922;G19,F,H,-,-],[ZC923;G19,Cl,H,-,-],[ZC924;G19,Br,H,-,-],[ZC925;G19,CN,H,-,-],[ZC926;G19,c-Pr,H,-,-],[ZC927;G19,H,Me,-,-],[ZC928;G19,H,Et,-,-],[ZC929;G19,H,Pr,-,-],[ZC930;G19,H,i-Pr,-,-],[ZC931;G19,H,OMe,-,-],[ZC932;G19,H,CF$_3$,-,-],[ZC933;G19,H,F,-,-],[ZC934;G19,H,Cl,-,-],[ZC935;G19,H,Br,-,-],[ZC936;G19,H,CN,-,-],[ZC937;G19,H,c-Pr,-,-],[ZC938;G20,H,H,-,-],[ZC939;G20,Me,H,-,-],[ZC940;G20,Et,H,-,-],[ZC941;G20,Pr,H,-,-],[ZC942;G20,i-Pr,H,-,-],[ZC943;G20,OMe,H,-,-],[ZC944;G20,CF$_3$,H,-,-],[ZC945;G20,F,H,-,-],[ZC946;G20,Cl,H,-,-],[ZC947;G20,Br,H,-,-],[ZC948;G20,CN,H,-,-],[ZC949;G20,Ph,H,-,-],[ZC950;G20,OPh,H,-,-],[ZC951;G20,c-Pr,H,-,-],[ZC952;G20,H,Me,-, -],[ZC953;G20,H,Et,-,-],[ZC954;G20,H,Pr,-,-],[ZC955;G20,H,i -Pr,-,-],[ZC956;G20,H,OMe,-,-],[ZC957;G20,H,$CF_3$,-,-],[ZC95 8;G20,H,F,-,-],[ZC959;G20,H,Cl,-,-],[ZC960;G20,H,Br,-,-],[Z C961;G20,H,CN,-,-],[ZC962;G20,H,Ph,-,-],[ZC963;G20,H,OPh,-, -],[ZC964;G20,H,c-Pr,-,-],[ZC965;G21,H,H,-,-],[ZC966;G21,M e,H,-,-],[ZC967;G21,Et,H,-,-],[ZC968;G21,Pr,H,-,-],[ZC969;G 21,i-Pr,H,-,-],[ZC970;G21,OMe,H,-,-],[ZC971;G21,$CF_3$,H,-,-], [ZC972;G21,F,H,-,-],[ZC973;G21,Cl,H,-,-],[ZC974;G21,Br,H,-, -],[ZC975;G21,CN,H,-,-],[ZC976;G21,Ph,H,-,-],[ZC977;G21,OP h,H,-,-],[ZC978;G21,c-Pr,H,-,-],[ZC979;G21,H,Me,-,-],[ZC98 0;G21,H,Et,-,-],[ZC981;G21,H,Pr,-,-],[ZC982;G21,H,i-Pr,-,-], [ZC983;G21,H,OMe,-,-],[ZC984;G21,H,$CF_3$,-,-],[ZC985;G21,H,F, ,[ZC986;G21,H,Cl,-,-],[ZC987;G21,H,Br,-,-],[ZC988; G21, H,CN,-,-],[ZC989;G21,H,c-Pr,-,-],[ZC990;G22,H,H,-,-],[ZC99 1;G22,Me,H,-,-],[ZC992;G22,Et,H,-,-],[ZC993;G22,Pr,H,-,-], [ZC994;G22,i-Pr,H,-,-],[ZC995;G22,OMe,H,-,-],[ZC996;G22,CF $_3$,H,-,-],[ZC997;G22,F,H,-,-],[ZC998;G22,Cl,H,-,-],[ZC999;G2 2,Br,H,-,-],[ZC1000;G22,CN,H,-,-],[ZC1001;G22,c-Pr,H,-,-], [ZC1002;G22,H,Me,-,-],[ZC1003;G22,H,Et,-,-],[ZC1004;G22,H,P r,-,-],[ZC1005;G22,H,i-Pr,-,-],[ZC1006;G22,H,OMe,-,-],[ZC10 07;G22,H,$CF_3$,-,-],[ZC1008;G22,H,F,-,-],[ZC1009;G22,H,Cl,-, -],[ZC1010;G22,H,Br,-,-],[ZC1011;G22,H,CN,-,-],[ZC1012;G22, H,Ph,-,-],[ZC1013;G22,H,OPh,-,-],[ZC1014;G22,H,c-Pr,-,-],[Z C1015;G23,H,H,-,-],[ZC1016;G23,Me,H,-,-],[ZC1017;G23,Et,H, -,-],[ZC1018;G23,Pr,H,-,-],[ZC1019;G23,i-Pr,H,-,-],[ZC1020; G23,Bu,H,-,-],[ZC1021;G23,t-Bu,H,-,-],[ZC1022;G23,$CH_2$(i-Pr), H,-,-],[ZC1023;G23,OMe,H,-,-],[ZC1024;G23,$CF_3$,H,-,-],[ZC102 5;G23,F,H,-,-],[ZC1026;G23,Cl,H,-,-],[ZC1027;G23,Br,H,-,-], [ZC1028;G23,CN,H,-,-],[ZC1029;G23,Ph,H,-,-],[ZC1030;G2 3,OP h,H,-,-J , [ZC1031;G2 3,c-Pr,H,-,-], [ZC1032;G23,c-Bu,H,-,-], [Z C1033;G23,c-Pen,H,-,-],[ZC1034;G23,c-Hex,H,-,-],[ZC1035;G2 3,c-hexen-1-yl,H,-,-],[ZC1036;G23,$CHF_2$,H,-,-],[ZC1037;G23,C $F_2CF_3$,H,-,-],[ZC1038;G23,CH=$CH_2$,H,-,-],[ZC1039;G23,C(Me)=CH $_2$,H,-,-],[ZC1040;G23,CCH,H,-,-],[ZC1041;G23,CC(c-Pr),H,-,-], [ZC1042;G23,CC(i-Pr),H,-,-],[ZC1043;G23,CC(Me),H,-,-],[ZC10 44;G23,CH=NOMe,H,-,-],[ZC1045;G23,C(Me)=NOMe,H,-,-],[ZC104 6;G23,$CH_2$O(i-Pr),H,-,-],[ZC1047;G23,$CH_2$OEt,H,-,-],[ZC1048;G 23,$CH_2$OPh,H,-,-],[ZC1049;G23,H,Me,-,-],[ZC1050;G23,H,Et,-, -],[ZC1051;G23,H,Pr,-,-],[ZC1052;G23,H,i-Pr,-,-],[ZC1053;G2 3,H,Bu,-,-],[ZC1054;G23,H,t-Bu,-,-],[ZC1055;G23,H,$CH_2$(i-Pr), -,-],[ZC1056;G23,H,OMe,-,-],[ZC1057;G23,H,$CF_3$,-,-],[ZC1058; G23,H,F,-,-],[ZC1059;G23,H,Cl,-,-],[ZC1060;G23,H,Br,-,-],[Z C1061;G23,H,CN,-,-],[ZC1062;G23,H,Ph,-,-],[ZC1063;G23,H,OP h,-,-],[ZC1064;G23,H,c-Pr,-,-],[ZC1065;G23,H,c-Bu,-,-],[ZC1 066;G23,H,c-Pen,-,-],[ZC1067;G23,H,c-Hex,-,-],[ZC1068;G23, H,c-hexen-1-yl,-,-],[ZC1069;G23,H,$CHF_2$,-,-],[ZC1070;G23,H,C $F_2CF_3$,-,-],[ZC1071;G23,H,CH=$CH_2$,-,-],[ZC1072;G23,H,C(Me)=CH $_2$,-,-],[ZC1073;G23,H,CCH,-,-],[ZC1074;G23,H,CC(c-Pr, [ZC1075;G23,H,CC(i-Pr),-,-],[ZC1076;G23,H,CC(Me),-,-],[ZC10 77;G23,H,CH=NOMe,-,-],[ZC1078;G23,H,C(Me)=NOMe,-,-],[ZC107 9;G23,H,$CH_2$O(i-Pr),-,-],[ZC1080;G23,H,$CH_2$OEt,-,-],[ZC1081;G 23,H,$CH_2$OPh,-,-],[ZC1082;G23,Me,Me,-,-],[ZC1083;G23,Me,Et, -,-],[ZC1084;G23,Me,i-Pr,-,-],[ZC1085;G23,Me,c-Pr,-,-],[ZC1 086;G23,Me,c-Hex,-,-],[ZC1087;G23,Me,$CHF_2$,-,-],[ZC1088;G23, Me,$CF_3$,-,-],[ZC1089;G23,Me,F,-,-],[ZC1090;G23,Me,Cl,-,-],[Z C1091;G23,Me,Br,-,-],[ZC1092;G23,Cl,Me,-,-],[ZC1093;G23,Cl, Et,-,-],[ZC1094;G23,Cl,i-Pr,-,-],[ZC1095;G23,Cl,c-Pr,-,-], [ZC1096;G23,Cl,c-Hex,-,-],[ZC1097;G23,Cl,$CHF_2$,-,-],[ZC1098; G23,Cl,$CF_3$,-,-],[ZC1099;G23,Cl,F,-,-],[ZC1100;G23,Cl,Cl,-, -],[ZC1101;G23,Cl,Br,-,-],[ZC1102;G23,F,Me,-,-],[ZC1103;G2 3,F,Et,-,-],[ZC1104;G23,F,i-Pr,-,-],[ZC1105;G23,F,c-Pr,-,-], [ZC1106;G23,F,c-Hex,-,-],[ZC1107;G23,F,$CHF_2$,-,-],[ZC1108;G2 3,F,$CF_3$,-,-],[ZC1109;G23,F,F,-,-],[ZC1110;G23,F,Cl,-,-],[ZC 1111;G23,F,Br,-,-],[ZC1112;G24,H,H,-,-],[ZC1113;G24,Me,H,-, -],[ZC1114;G24,Et,H,-,-],[ZC1115;G24,Pr,H,-,-],[ZC1116;G24, i-Pr,H,-,-],[ZC1117;G24,OMe,H,-,-],[ZC1118;G24,$CF_3$,H,-,-], [ZC1119;G24,F,H,-,-],[ZC1120;G24,Cl,H,-,-],[ZC1121;G24,Br, H,-,-],[ZC1122;G24,CN,H,-,-],[ZC1123;G24,Ph,H,-,-],[ZC1124; G24 , OPh,H,-,-], [ZC112 5;G24,c-Pr,H, -, -], [ZC1126;G24,H,Me,-, -],[ZC1127;G24,H,Et,-,-],[ZC1128;G24,H,Pr,-,-],[ZC1129;G24, H,i-Pr,-,-],[ZC1130;G24,H,OMe,-,-],[ZC1131;G24,H,$CF_3$,-,-], [ZC1132;G24,H,F,-,-],[ZC1133;G24,H,Cl,-,-],[ZC1134;G24,H,B r,-,-],[ZC1135;G24,H,CN,-,-],[ZC1136;G24,H,c-Pr,-,-],[ZC113 7;G25,H,H,H,-],[ZC1138;G25,Me,H,H,-],[ZC1139;G25,Et,H,H,-], [ZC1140;G25,Pr,H,H,-],[ZC1141;G25,i-Pr,H,H,-],[ZC1142;G25,0 Me,H,H,-],[ZC1143;G25,$CF_3$,H,H,-],[ZC1144;G25,F,H,H,-],[ZC11 45;G25,Cl,H,H,-],[ZC1146;G25,Br,H,H,-],[ZC1147;G25,CN,H,H, -],[ZC1148;G25,Ph,H,H,-],[ZC1149;G25,OPh,H,H,-],[ZC1150;G2 5,c-Pr,H,H,-],[ZC1151;G25,H,Me,H,-],[ZC1152;G25,H,Et,H,-], [ZC1153;G25,H,Pr,H,-],[ZC1154;G25,H,i-Pr,H,-],[ZC1155;G25, H,OMe,H,-],[ZC1156;G25,H,$CF_3$,H,-],[ZC1157;G25,H,F,H,-],[ZC1158;G25,H,C1,H,-],[ZC1159;G25,H,Br,H,-],[ZC1160;G25,H,CN,H, -],[ZC1161;G25,H,Ph,H,-],[ZC1162;G25,H,OPh,H,-],[ZC1163;G25,H,c-Pr,H,-],[ZC1164;G25,H,H,Me,-],[ZC1165;G25,H,H,Et,-], [ZC1166;G25,H,H,Pr,-],[ZC1167;G25,H,H,i-Pr,-],[ZC1168;G25, H,H,OMe,-],[ZC1169;G25,H,H,$CF_3$,-],[ZC1170;G25,H,H,F,-],[ZC1171;G25,H,H,Cl,-],[ZC1172;G25,H,H,Br,-],[ZC1173;G25,H,H,CN, -],[ZC1174;G25,H,H,c-Pr,-],[ZC1175;G26,H,H,-,-],[ZC1176;G26,Me,H,-,-],[ZC1177;G26,Et,H,-,-],[ZC1178;G26,Pr,H,-,-],[ZC 1179;G26,i-Pr,H,-,-],[ZC1180;G26,OMe,H,-,-],[ZC1181;G26,$CF_3$,H,-,-],[ZC1182;G26,F,H,-,-], [ZC1183;G26,C1,H,-,-],[ZC1184;G26,Br,H,-,-],[ZC1185;G26,CN,H,-,-],[ZC1186;G26,c-Pr,H,-, -],[ZC1187;G26,H,Me,-,-],[ZC1188;G26,H,Et,-,-],[ZC1189;G26, H,Pr,-,-],[ZC1190;G26,H,i-Pr,-,-],[ZC1191;G26,H,OMe,-,-],[Z C1192;G26,H,$CF_3$,-,-],[ZC1193;G26,H,F,-,-],[ZC1194;G26,H,Cl, -,-],[ZC1195;G26,H,Br,-,-],[ZC1196;G26,H,CN,-,-],[ZC1197;G2 6,H,Ph,-,-],[ZC1198;G26,H,OPh,-,-],[ZC1199;G26,H,c-Pr,-,-], [ZC1200;G27,H,H,H,-],[ZC1201;G27,Me,H,H,-],[ZC1202;G27,Et, H,H,-],[ZC1203;G27,Pr,H,H,-],[ZC1204;G27,i-Pr,H,H,-],[ZC1205;G27,$CF_3$,H,H,-],[ZC1206;G27,c-Pr,H,H,-],[ZC1207;G27,Me,Me, H,-],[ZC1208;G27,Me,Et,H,-],[ZC1209;G27,Me,Pr,H,-],[ZC1210; G27, Me, i-Pr, H,-],[ZC1211;G27,Me,OMe,H,-],[ZC1212;G27,Me,$CF_3$,H,-],[ZC1213;G27,Me,F,H,-],[ZC1214;G27,Me,C1,H,-],[ZC1215;G27,Me,CN,H,-],[ZC1216;G27,Me,Ph,H,-],[ZC1217;G27,Me,c-Pr,H,-],[ZC1218;G27,Me,H,Me,-],[ZC1219;G27,Me,H,Et,-],[ZC1220;G27,Me,H,Pr,-],[ZC1221;G27,Me,H,i-Pr,-],[ZC1222;G27,Me,H, OMe,-],[ZC1223;G27,Me,H,$CF_3$,-],[ZC1224;G27,Me,H,F,-],[ZC1225;G27,Me,H,Cl,-],[ZC1226;G27,Me,H,CN,-],[ZC1227;G27,Me,H,Ph,-],[ZC1228;G27,Me,H,c-Pr,-],[ZC1229;G28,H,H,H,-],[ZC1230; G28,Me,H,H,-],[ZC1231;G28,Et,H,H,-],[ZC1232;G28,Pr,H,H,-], [ZC1233;G28,i-Pr,H,H,-],[ZC1234;G28,$CF_3$,H,H,-],[ZC1235;G28, c-Pr,H,H,-],[ZC1236;G28,Me,Me,H,-],[ZC1237;G28,Me,Et,H,-], [ZC1238;G28,Me,Pr,H,-],[ZC1239;G28,Me,i-Pr,H,-],[ZC1240;G28,Me,OMe,H,-],[ZC1241;G28,Me,$CF_3$,H,-],[ZC1242;G28,Me,F,H,-], [ZC1243;G28,Me,Cl,H,-],[ZC1244;G28,Me,CN,H,-],[ZC1245;G28,Me,Ph,H,-],[ZC1246;G28,Me,c-Pr,H,-],[ZC1247;G28,Me,H,Me,-], [ZC1248;G28,Me,H,Et,-],[ZC1249;G28,Me,H,Pr,-],[ZC1250;G28,Me,H,i-Pr,-],[ZC1251;G28,Me,H,OMe,-],[ZC1252;G28,Me,H,$CF_3$,-], [ZC1253;G28,Me,H,F,-], [ZC1254;G28,Me,H,Cl,-], [ZC1255;G28,Me,H,CN,-], [ZC1256;G28,Me,H,c-Pr,-], [ZC1257;G29,H,H,H,-], [ZC 1258;G29,Me,H,H,-],[ZC1259;G29,Et,H,H,-],[ZC1260;G29,Pr,H, H,-],[ZC1261;G29,i-Pr,H,H,-], [ZC1262;G29,OMe,H,H,-], [ZC126 3;G29,$CF_3$,H,H,-], [ZC1264;G29,F,H,H,-], [ZC1265;G29,Cl,H,H,-], [ZC1266;G29,CN,H,H,-], [ZC1267;G29,c-Pr,H,H,-], [ZC1268;G29, H,Me,H,-], [ZC1269;G29,H,Et,H,-], [ZC1270;G29,H,Pr,H,-], [ZC12 71;G29,H,i-Pr,H,-], [ZC1272;G29,H,OMe,H,-], [ZC1273;G29,H,$CF_3$,H,-], [ZC1274;G29,H,F,H,-], [ZC1275;G29,H,Cl,H,-], [ZC1276;G 29,H,CN,H,-], [ZC1277;G29,H,Ph,H,-], [ZC1278;G29,H,c-Pr,H,-], [ZC1279;G29,H,H,Me,-], [ZC1280;G29,H,H,Et,-], [ZC1281;G29,H, H,Pr,-J , [ZC1282;G2 9,H,H,i-Pr,-], [ZC1283;G2 9,H,H,OMe,-], [ZC1 284;G29,H,H,$CF_3$,-], [ZC1285;G29,H,H,F,-], [ZC1286;G29,H,H,Cl, -], [ZC1287;G29,H,H,CN,-], [ZC1288;G29,H,H,Ph,-], [ZC1289;G29, H,H,c-Pr,-], [ZC1290;G30,H,H,-,-], [ZC1291;G30,Me,H,-,-], [ZC1292;G30,Et,H,-,-],[ZC1293;G30,Pr,H,-,-],[ZC1294;G30,i-Pr,H, -,-], [ZC1295;G30,OMe,H,-,-], [ZC1296;G30,$CF_3$,H,-,-], [ZC1297; G30,F,H,-,-], [ZC1298;G30,Cl,H,-,-], [ZC1299;G30,CN,H,-,-], [Z C1300;G30,c-Pr,H,-,-], [ZC1301;G30,H,Me,-,-], [ZC1302;G30,H,E t,-,-], [ZC1303;G30,H,Pr,-,-], [ZC1304;G30,H,i-Pr,-,-], [ZC1305;G30,H,OMe,-,-], [ZC1306;G30,H,$CF_3$,-,-], [ZC1307;G30,H,F,-, -], [ZC1308;G30,H,Cl,-,-], [ZC1309;G30,H,CN,-,-], [ZC1310;G30, H,Ph,-,-], [ZC1311;G30,H,c-Pr,-,-], [ZC1312;G31,H,H,H,-], [ZCl 313;G31,Me,H,H,-],[ZC1314;G31,Et,H,H,-],[ZC1315;G31,Pr,H,H, -], [ZC1316;G31,i-Pr,H,H,-], [ZC1317;G31,$CF_3$,H,H,-], [ZC1318;G 31,c-Pr,H,H,-], [ZC1319;G31,Me,Me,H,-], [ZC1320;G31,Me,Et,H, -], [ZC1321;G31,Me,Pr,H,-], [ZC1322;G31,Me,i-Pr,H,-], [ZC1323; G31,Me,OMe,H,-], [ZC1324;G31,Me,$CF_3$,H,-], [ZC1325;G31,Me,F,H, -], [ZC1326;G31,Me,Cl,H,-], [ZC1327;G31,Me,CN,H,-], [ZC1328;G3 1,Me,Ph,H,-], [ZC1329;G31,Me,c-Pr,H,-], [ZC1330;G31,Me,H,Me, -], [ZC1331;G31,Me,H,Et,-], [ZC1332;G31,Me,H,Pr,-], [ZC1333;G31,Me,H,i-Pr,-], [ZC1334;G31,Me,H,OMe,-], [ZC1335;G31,Me,H,$CF_3$,-], [ZC1336;G31,Me,H,F,-], [ZC1337;G31,Me,H,Cl,-], [ZC1338;G 31,Me,H,CN,-], [ZC1339;G31,Me,H,c-Pr,-], [ZC1340;G32,H,-,-,-], [ZC1341;G32,Me,-,-,-],[ZC1342;G32,Et,-,-,-],[ZC1343;G32,Pr, -,-,-],[ZC1344;G32,i-Pr,-,-,-],[ZC1345;G32,OMe,-,-,-],[ZC13 46;G32,$CF_3$,-,-,-], [ZC1347;G32,F,-,-,-], [ZC1348;G32,C1,-,-, -], [ZC1349;G32,Br,-,-,-], [ZC1350;G32,CN,-,-,-], [ZC1351;G32, Ph,-,-,-], [ZC1352;G32,OPh,-,-,-], [ZC1353;G32,c-Pr,-,-,-], [Z C1354;G33,H,-,-,-], [ZC1355;G33,Me,-,-,-], [ZC1356;G33,Et,-, -,-], [ZC1357;G33,Pr,-,-,-], [ZC1358;G33,i-Pr,-,-,-], [ZC1359; G33,OMe,-,-,-], [ZC1360;G33,$CF_3$,-,-,-], [ZC1361;G33,F,-,-,-], [ZC1362;G33,Cl,-,-,-], [ZC1363;G33,Br,-,-,-], [ZC1364;G33,CN, -,-,-], [ZC1365;G33,Ph,-,-,-], [ZC1366;G33,OPh,-,-,-], [ZC136 7;G33,c-Pr,-,-,-], [ZC1368;G34,H,-,-,-], [ZC1369;G34,Me,-,-, -], [ZC1370;G34,Et,-,-,-], [ZC1371;G34,Pr,-,-,-], [ZC1372;G34, i-Pr,-,-,-], [ZC1373;G34,OMe,-,-,-], [ZC1374;G34,$CF_3$,-,-,-], [ZC1375;G34,F,-,-,-],[ZC1376;G34,C1,-,-,-],[ZC1377;G34,Br, -,-,-],[ZC1378;G34,CN,-,-,-],[ZC1379;G34,Ph,-,-,-],[ZC1380; G34,OPh,-,-,-], [ZC1381;G34,c-Pr,-,-,-], [ZC1382;G35,H,-,-,-], [ZC1383;G35,Me,-,-,-], [ZC1384;G35,Et,-,-,-], [ZC1385;G35,Pr, -,-,-], [ZC1386;G35,i-Pr,-,-,-],

[ZC1387;G35,OMe,-,-,-], [ZC13 88;G35,$CF_3$,-,-,-], [ZC1389;G35,F,-,-,-], [ZC1390;G35,Cl,-,-,-],[ZC1391;G35,Br,-,-,-],[ZC1392;G35,CN,-,-,-],[ZC1393;G35, Ph,-,-,-],[ZC1394;G35,OPh,-,-,-],[ZC1395;G35,c-Pr,-,-,-],[Z C1396;G36,Me,H,-,-], [ZC1397;G36,Et,H,-,-], [ZC1398;G36,Pr,H, -,-], [ZC1399;G36,Bu,H,-,-], [ZC1400;G36,i-Pr,H,-,-], [ZC1401; G36,C(Me)=$CH_2$,H,-,-], [ZC1402;G36,$CHF_2$,H,-,-], [ZC1403;G36,CF $_3$,H,-,-], [ZC1404;G36,c-Pr,H,-,-], [ZC1405;G36,c-Pen,H,-,-], [ZC1406;G36,c-Hex,H,-,-], [ZC1407;G36,C(Me)=NOH,H,-,-], [ZC14 08;G36,C(Me)=NOEt,H,-,-], [ZC1409;G36,F,H,-,-], [ZC1410;G36,C 1,H,-,-], [ZC1411;G36,Br,H,-,-], [ZC1412;G36,Me,Cl,-,-], [ZC14 13;G36,Et,Cl,-,-],[ZC1414;G36,Pr,Cl,-,-],[ZC1415;G36,Bu,Cl, -,-], [ZC1416;G36,i-Pr,Cl,-,-], [ZC1417;G36,C(Me)=$CH_2$,Cl,-,-], [ZC1418;G36,$CHF_2$,Cl,-,-], [ZC1419;G36,$CF_3$,Cl,-,-], [ZC1420;G3 6,c-Pr,Cl,-,-], [ZC1421;G36,c-Pen,Cl,-,-], [ZC1422;G36,c-Hex, Cl,-,-], [ZC1423;G36,C(Me)=NOH,Cl,-,-], [ZC1424;G36,C(Me)=NOE t,Cl,-,-], [ZC1425;G36,F,Cl,-,-], [ZC1426;G36,Cl,Cl,-,-],[ZC1 427;G36,Br,Cl,-,-], [ZC1428;G36,Me,Br,-,-], [ZC1429;G36,Et,B r,-,-], [ZC14 30;G3 6,Pr,Br,-,-], [ZC14 31;G3 6,Bu,Br,-,-], [ZC14 3 2;G36,i-Pr,Br,-,-], [ZC1433;G36,C(Me)=$CH_2$,Br,-,-], [ZC1434;G3 6,$CHF_2$,Br,-,-], [ZC1435;G36,$CF_3$,Br,-,-], [ZC1436;G36,c-Pr,Br,-,-], [ZC1437;G36,c-Pen,Br,-,-], [ZC1438;G36,c-Hex,Br,-,-], [Z C1439;G36,C(Me)=NOH,Br,-,-], [ZC1440;G36,C(Me)=NOEt,Br,-,-], [ZC1441;G36,F,Br,-,-], [ZC1442;G36,Cl,Br,-,-], [ZC1443;G36,B r,Br,-,-], [ZC1444;G36,Me,Me,-,-], [ZC1445;G36,Et,Me,-,-], [ZC 1446;G36,Pr,Me,-,-], [ZC1447;G36,Bu,Me,-,-], [ZC1448;G36,i-P r,Me,-,-], [ZC1449;G36,C(Me)=$CH_2$,Me,-,-], [ZC1450;G36,$CHF_2$,M e,-,-], [ZC1451;G36,$CF_3$,Me,-,-], [ZC1452;G36,c-Pr,Me,-,-], [ZC 1453;G36,c-Pen,Me,-,-], [ZC1454;G36,c-Hex,Me,-,-], [ZC1455;G3 6,C(Me)=NOH,Me,-,-], [ZC1456;G36,C(Me)=NOEt,Me,-,-], [ZC1457; G36,F,Me,-,-], [ZC1458;G36,Cl,Me,-,-], [ZC1459;G36,Br,Me,-,-]

A compound (1E) wherein X represents CH, L represents an oxygen atom, $R^1$ represents a methyl group, $R^{x2}$ represents an ethyl group, and a combination of structure of G and substituent of $R^{X8}$, $R^{X9}$, $R^{X10}$ and $R^{X11}$ applied to the structure of G represents any combination described in Combination C (hereinafter, referred to as Compound Class SX191).

A compound (1E) wherein X represents CH, L represents an oxygen atom, $R^1$ represents a methyl group, $R^{x2}$ represents a fluorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX192).

A compound (1E) wherein X represents CH, L represents an oxygen atom, $R^1$ represents a methyl group, $R^{x2}$ represents a chlorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX193).

A compound (1E) wherein X represents CH, L represents an oxygen atom, $R^1$ represents a methyl group, $R^{x2}$ represents a methoxy group, and a combination of structure of G and substituent of $R^{x8}$, $R^{X9}$, $R^{X10}$ and $R^{X11}$ applied to the structure of G represents any combination described in Combination C (hereinafter, referred to as Compound Class SX194).

A compound (1E) wherein X represents CH, L represents an oxygen atom, $R^1$ represents a methyl group, $R^{x2}$ represents a cyclopropyl group, and a combination of structure of G and substituent of $R^{x8}$, $R^{X9}$, $R^{X10}$ and $R^{X11}$ applied to the structure of G represents any combination described in Combination C (hereinafter, referred to as Compound Class SX195).

A compound (1E) wherein X represents CH, L represents an oxygen atom, $R^1$ represents a fluorine atom, $R^{x2}$ represents a methyl group, and a combination of structure of G and substituent of $R^{x8}$, $R^{X9}$, $R^{X10}$ and $R^{X11}$ applied to the structure of G represents any combination described in Combination C (hereinafter, referred to as Compound Class SX196).

A compound (1E) wherein X represents CH, L represents an oxygen atom, $R^1$ represents a fluorine atom, $R^{x2}$ represents a fluorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX197).

A compound (1E) wherein X represents CH, L represents an oxygen atom, $R^1$ represents a fluorine atom, $R^{x2}$ represents a chlorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX198).

A compound (1E) wherein X represents CH, L represents an oxygen atom, $R^1$ represents a chlorine atom, $R^{x2}$ represents a methyl group, and a combination of structure of G and substituent of $R^{X8}$, $R^{X9}$, $R^{X10}$ and $R^{X11}$ applied to the structure of G represents any combination described in Combination C (hereinafter, referred to as Compound Class SX199).

A compound (1E) wherein X represents CH, L represents an oxygen atom, $R^1$ represents a chlorine atom, $R^{x2}$ represents a fluorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX200).

A compound (1E) wherein X represents CH, L represents an oxygen atom, $R^1$ represents a chlorine atom, $R^{x2}$ represents a chlorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX201).

A compound (1E) wherein X represents a nitrogen atom, L represents an oxygen atom, $R^1$ represents a methyl group, $R^{x2}$ represents a methyl group, and a combination of structure of G and substituent of $R^{x8}$, $R^{X9}$, $R^{X10}$ and $R^{X11}$ applied to the structure of G represents any combination described in Combination C (hereinafter, referred to as Compound Class SX202).

A compound (1E) wherein X represents a nitrogen atom, L represents an oxygen atom, $R^1$ represents a methyl group, $R^{x2}$ represents an ethyl group, and a combination of structure of G and substituent of $R^{x8}$, $R^{X9}$, $R^{X10}$ and $R^{X11}$ applied to the structure of G represents any combination described in Combination C (hereinafter, referred to as Compound Class SX203).

A compound (1E) wherein X represents a nitrogen atom, L represents an oxygen atom, $R^1$ represents a methyl group, $R^{x2}$ represents a fluorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX204).

A compound (1E) wherein X represents a nitrogen atom, L represents an oxygen atom, $R^1$ represents a methyl group, $R^{x2}$ represents a chlorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX205).

A compound (1E) wherein X represents a nitrogen atom, L represents an oxygen atom, $R^1$ represents a methyl group, $R^{x2}$ represents a methoxy group, and a combination of structure of G and substituent of $R^{x8}$, $R^{X9}$, $R^{X10}$ and $R^{X11}$ applied to the structure of G represents any combination described in Combination C (hereinafter, referred to as Compound Class SX206).

A compound (1E) wherein X represents a nitrogen atom, L represents an oxygen atom, $R^1$ represents a methyl group, $R^{x2}$ represents a cyclopropyl group, and a combination of structure of G and substituent of $R^{x8}$, $R^{X9}$, $R^{X10}$ and $R^{X11}$ applied to the structure of G represents any combination described in Combination C (hereinafter, referred to as Compound Class SX207).

A compound (1E) wherein X represents a nitrogen atom, L represents an oxygen atom, $R^1$ represents a fluorine atom, $R^{x2}$ represents a methyl group, and a combination of structure of G and substituent of $R^{x8}$, $R^{X9}$, $R^{X10}$ and $R^{X11}$ applied to the structure of G represents any combination described in Combination C (hereinafter, referred to as Compound Class SX208).

A compound (1E) wherein X represents a nitrogen atom, L represents an oxygen atom, $R^1$ represents a fluorine atom, $R^{x2}$ represents a fluorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX209).

A compound (1E) wherein X represents a nitrogen atom, L represents an oxygen atom, $R^1$ represents a fluorine atom, $R^{x2}$ represents a chlorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX210).

A compound (1E) wherein X represents a nitrogen atom, L represents an oxygen atom, $R^1$ represents a chlorine atom, $R^{x2}$ represents a methyl group, and a combination of structure of G and substituent of $R^{x8}$, $R^{X9}$, $R^{X10}$ and $R^{X11}$ applied to the structure of G represents any combination described in Combination C (hereinafter, referred to as Compound Class SX211).

A compound (1E) wherein X represents a nitrogen atom, L represents an oxygen atom, $R^1$ represents a chlorine atom, $R^{x2}$ represents a fluorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX212).

A compound (1E) wherein X represents a nitrogen atom, L represents an oxygen atom, $R^1$ represents a chlorine atom, $R^{x2}$ represents a chlorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX213).

A compound (1E) wherein X represents a nitrogen atom, L represents NH, $R^1$ represents a methyl group, $R^{x2}$ represents a methyl group, and a combination of structure of G and substituent of $R^{x8}$, $R^{X9}$, $R^{X10}$ and $R^{X11}$ applied to the structure of G represents any combination described in Combination C (hereinafter, referred to as Compound Class SX214).

A compound (1E) wherein X represents a nitrogen atom, L represents NH, $R^1$ represents a methyl group, $R^{x2}$ represents an ethyl group, and a combination of structure of G and substituent of $R^{x8}$, $R^{X9}$, $R^{X10}$ and $R^{X11}$ applied to

the structure of G represents any combination described in Combination C (hereinafter, referred to as Compound Class SX215).

A compound (1E) wherein X represents a nitrogen atom, L represents NH, $R^1$ represents a methyl group, $R^{x2}$ represents a fluorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX216).

A compound (1E) wherein X represents a nitrogen atom, L represents NH, $R^1$ represents a methyl group, $R^{x2}$ represents a chlorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX217).

A compound (1E) wherein X represents a nitrogen atom, L represents NH, $R^1$ represents a methyl group, $R^{x2}$ represents a methoxy group, and a combination of structure of G and substituent of $R^{x8}$, $R^{X9}$, $R^{X10}$ and $R^{X11}$ applied to the structure of G represents any combination described in Combination C (hereinafter, referred to as Compound Class SX218).

A compound (1E) wherein X represents a nitrogen atom, L represents NH, $R^1$ represents a methyl group, $R^{x2}$ represents a cyclopropyl group, and a combination of structure of G and substituent of $R^{x8}$, $R^{X9}$, $R^{X10}$ and $R^{X11}$ applied to the structure of G represents any combination described in Combination C (hereinafter, referred to as Compound Class SX219).

A compound (1E) wherein X represents a nitrogen atom, L represents NH, $R^1$ represents a fluorine atom, $R^{x2}$ represents a methyl group, and a combination of structure of G and substituent of $R^{x8}$, $R^{X9}$, $R^{X10}$ and $R^{X11}$ applied to the structure of G represents any combination described in Combination C (hereinafter, referred to as Compound Class SX220).

A compound (1E) wherein X represents a nitrogen atom, L represents NH, $R^1$ represents a fluorine atom, $R^{x2}$ represents a fluorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX221).

A compound (1E) wherein X represents a nitrogen atom, L represents NH, $R^1$ represents a fluorine atom, $R^{x2}$ represents a chlorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX222).

A compound (1E) wherein X represents a nitrogen atom, L represents NH, $R^1$ represents a chlorine atom, $R^{x2}$ represents a methyl group, and a combination of structure of G and substituent of $R^{x8}$, $R^{X9}$, $R^{X10}$ and $R^{X11}$ applied to the structure of G represents any combination described in Combination C (hereinafter, referred to as Compound Class SX223).

A compound (1E) wherein X represents a nitrogen atom, L represents NH, $R^1$ represents a chlorine atom, $R^{x2}$ represents a fluorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX224).

A compound (1E) wherein X represents a nitrogen atom, L represents NH, $R^1$ represents a chlorine atom, $R^{x2}$ represents a chlorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX225).

A compound represented by formula (2E):

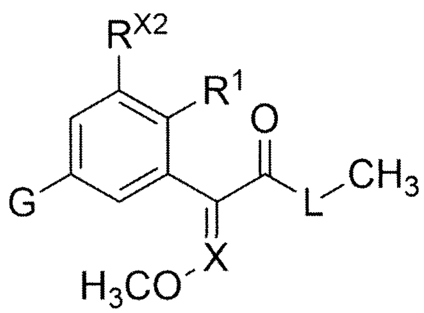

(2E)

wherein G represents any one of formulae G1 to G36. (hereinafter, referred to as Compound (2E)) wherein X represents CH, L represents an oxygen atom, $R^1$ represents a methyl group, $R^{X2}$ represents a methyl group, and a combination of structure of G and substituent of $R^{x8}$, $R^{X9}$, $R^{X10}$ and $R^{X11}$ applied to the structure of G represents any combination

described in Combination C (hereinafter, referred to as Compound Class SX226).

A compound (2E) wherein X represents CH, L represents an oxygen atom, $R^1$ represents a methyl group, $R^{X2}$ represents a fluorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX227).

A compound (2E) wherein X represents CH, L represents an oxygen atom, $R^1$ represents a methyl group, $R^{X2}$ represents a chlorine atom, and a combination of structure of G and substituent of $R^{X8}$, $R^{X9}$, $R^{X10}$ and $R^{X11}$ applied to the structure of G represents any combination described in Combination C (hereinafter, referred to as Compound Class SX228).

A compound (2E) wherein X represents CH, L represents an oxygen atom, $R^1$ represents a methyl group, $R^{X2}$ represents a methoxy group, and a combination of structure of G and substituent of $R^{x8}$, $R^{X9}$, $R^{X10}$ and $R^{X11}$ applied to the structure of G represents any combination described in Combination C (hereinafter, referred to as Compound Class SX229).

A compound (2E) wherein X represents a nitrogen atom, L represents an oxygen atom, $R^1$ represents a methyl group, $R^{X2}$ represents a methyl group, and a combination of structure of G and substituent of $R^{x8}$, $R^{X9}$, $R^{X10}$ and $R^{X11}$ applied to the structure of G represents any combination described in Combination C (hereinafter, referred to as Compound Class SX230).

A compound (2E) wherein X represents a nitrogen atom, L represents an oxygen atom, $R^1$ represents a methyl group, $R^{X2}$ represents a fluorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX231) .

A compound (2E) wherein X represents a nitrogen atom, L represents an oxygen atom, $R^1$ represents a methyl group, $R^{X2}$ represents a chlorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX232) .

A compound (2E) wherein X represents a nitrogen atom, L represents NH, $R^1$ represents a methyl group, $R^{X2}$ represents a methyl group, and a combination of structure of G and substituent of $R^{x8}$, $R^{X9}$, $R^{X10}$ and $R^{X11}$ applied to the structure of G represents any combination described in Combination C (hereinafter, referred to as Compound Class SX233).

A compound (2E) wherein X represents a nitrogen atom, L represents NH, $R^1$ represents a methyl group, $R^{X2}$ represents a fluorine atom, and a combination of structure of G and substituent of $R^{X8}$, $R^{X9}$, $R^{X10}$ and $R^{X11}$ applied to the structure of G represents any combination described in Combination C (hereinafter, referred to as Compound Class SX234).

A compound (2E) wherein X represents a nitrogen atom, L represents NH, $R^1$ represents a methyl group, $R^{X2}$ represents a chlorine atom, and a combination of structure of G and substituent of $R^{x8}$, $R^{X9}$, $R^{X10}$ and $R^{X11}$ applied to the structure of G represents any combination described in Combination C (hereinafter, referred to as Compound Class SX235).

A compound represented by formula (1F):

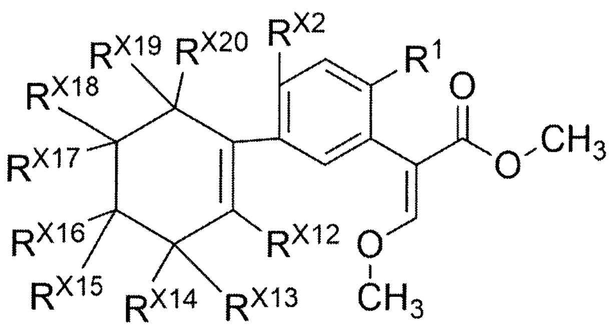

(1F)

(hereinafter, referred to as Compound (1F)) wherein $R^1$ represents a methyl group, $R^{X2}$ represents a methyl group, and a combination of $R^{X12}$, $R^{X13}$, $R^{X14}$, $R^{X15}$, $R^{X16}$, $R^{X17}$, $R^{X18}$, $R^{X19}$, and $R^{X20}$ represents any combinations described in Combination D (hereinafter, referred to as Compound Class SX236) .

The combination D consists of the Substituents No. ZD1 to ZD30. The Substituent No. ZD1 to ZD30 represent the combination of $R^{X12}$, $R^{X13}$, $R^{X14}$, $R^{X15}$, $R^{X16}$, $R^{X17}$, $R^{X18}$, $R^{X19}$, and $R^{X20}$ in the compound (1F), which hereinafter is described as [Substituent No.; $R^{X12}$, $R^{X13}$, $R^{X14}$, $R^{X15}$, $R^{X16}$, $R^{X17}$, $R^{X18}$, $R^{X19}$, $R^{X20}$]. For example, the Substituent No. ZD2 represents a combination wherein $R^{X12}$ represents a methyl group, and $R^{X13}$, $R^{X14}$, $R^{X15}$, $R^{X16}$, $R^{X17}$, $R^{X18}$,

$R^{X19}$, and $R^{X20}$ represent a hydrogen].

Combination D

[ZD1;H,H,H,H,H,H,H,H,H], [ZD2;Me,H,H,H,H,H,H,H,H], [ZD3;F,H, H,H,H,H,H,H,H], [ZD4;Cl,H,H,H,H,H,H,H,H], [ZD5;H,F,F,H,H,H,H, H,H], [ZD6;Me,F,F,H,H,H,H,H,H], [ZD7;F,F,F,H,H,H,H,H,H], [ZD8; Cl,F,F,H,H,H,H,H,H], [ZD9;H,H,H,H,H,H,H,F,F], [ZD10;Me,H,H,H, H,H,H,F,F], [ZD11;F,H,H,H,H,H,H,F,F], [ZD12;Cl,H,H,H,H,H,H,F, F], [ZD13;H,Cl,Cl,H,H,H,H,H,H], [ZD14;Me,Cl,Cl,H,H,H,H,H,H], [ZD15;F,Cl,Cl,H,H,H,H,H,H], [ZD16;Cl,Cl,Cl,H,H,H,H,H,H], [ZD1 7;H,H,H,H,H,H,H,Cl,Cl], [ZD18;Me,H,H,H,H,H,H,Cl,Cl], [ZD19;F, H,H,H,H,H,H,Cl,Cl], [ZD20;Cl,H,H,H,H,H,H,Cl,Cl], [ZD21;H,H,H, F,F,H,H,H,H], [ZD22;H,H,H,Me,Me,H,H,H,H], [ZD23;H,H,H,Cl,Cl, H,H,H,H], [ZD24;H,H,H,i-Pr,H,H,H,H,H], [ZD25;H,H,H,$CHF_2$,H,H, H,H,H], [ZD26;H,H,H,$CF_3$,H,H,H,H,H], [ZD27;H,H,H,CN,H,H,H,H,H], [ZD28;H,H,H,OH,H,H,H,H,H], [ZD29;H,H,H,OMe,H,H,H,H,H], [ZD30; H,H,H,$OCHF_2$,H,H,H,H,H]

A compound (1F) wherein $R^1$ represents a methyl group, $R^{X2}$ represents an ethyl group, and a combination of $R^{X12}$, $R^{X13}$, $R^{X14}$, $R^{X15}$, $R^{X16}$, $R^{X17}$, $R^{X18}$, $R^{X19}$, and $R^{X20}$ represents any combination described in Combination D (hereinafter, referred to as Compound Class SX237).

A compound (1F) wherein $R^1$ represents a methyl group, $R^{X2}$ represents a fluorine atom, and a combination of $R^{X12}$, $R^{X13}$, $R^{X14}$, $R^{X15}$, $R^{X16}$, $R^{X17}$, $R^{X18}$, $R^{X19}$, and $R^{X20}$ represents any combination described in Combination D (hereinafter, referred to as Compound Class SX238).

A compound (1F) wherein $R^1$ represents a methyl group, $R^{X2}$ represents a chlorine atom, and a combination of $R^{X12}$, $R^{X13}$, $R^{X14}$, $R^{X15}$, $R^{X16}$, $R^{X17}$, $R^{X18}$, $R^{X19}$, and $R^{X20}$ represents any combination described in Combination D (hereinafter, referred to as Compound Class SX239).

A compound (1F) wherein $R^1$ represents a methyl group, $R^{X2}$ represents a methoxy group, and a combination of $R^{X12}$, $R^{X13}$, $R^{X14}$, $R^{X15}$, $R^{X16}$, $R^{X17}$, $R^{X18}$, $R^{X19}$, and $R^{X20}$ represents any combination described in Combination D (hereinafter, referred to as Compound Class SX240).

A compound (1F) wherein $R^1$ represents a methyl group, $R^{X2}$ represents a cyclopropyl group, and a combination of $R^{X12}$, $R^{X13}$, $R^{X14}$, $R^{X15}$, $R^{X16}$, $R^{X17}$, $R^{X18}$, $R^{X19}$, and $R^{X20}$ represents any combination described in Combination D (hereinafter, referred to as Compound Class SX241).

A compound (1F) wherein $R^1$ represents a fluorine atom, $R^{X2}$ represents a methyl group, and a combination of $R^{X12}$, $R^{X13}$, $R^{X14}$, $R^{X15}$, $R^{X16}$, $R^{X17}$, $R^{X18}$, $R^{X19}$, and $R^{X20}$ represents any combination described in Combination D (hereinafter, referred to as Compound Class SX242).

A compound (1F) wherein $R^1$ represents a fluorine atom, $R^{X2}$ represents a fluorine atom, and a combination of $R^{X12}$, $R^{X13}$, $R^{X14}$, $R^{X15}$, $R^{X16}$, $R^{X17}$, $R^{X18}$, $R^{X19}$, and $R^{X20}$ represents any combination described in Combination D (hereinafter, referred to as Compound Class SX243).

A compound (1F) wherein $R^1$ represents a fluorine atom, $R^{X2}$ represents a chlorine atom, and a combination of $R^{X12}$, $R^{X13}$, $R^{X14}$, $R^{X15}$, $R^{X16}$, $R^{X17}$, $R^{X18}$, $R^{X19}$, and $R^{X20}$ represents any combination described in Combination D (hereinafter, referred to as Compound Class SX244).

A compound (1F) wherein $R^1$ represents a chlorine atom, $R^{X2}$ represents a methyl group, and a combination of $R^{X12}$, $R^{X13}$, $R^{X14}$, $R^{X15}$, $R^{X16}$, $R^{X17}$, $R^{X18}$, $R^{X19}$, and $R^{X20}$ represents any combination described in Combination D (hereinafter, referred to as Compound Class SX245).

A compound (1F) wherein $R^1$ represents a chlorine atom, $R^{X2}$ represents a fluorine atom, and a combination of $R^{X12}$, $R^{X13}$, $R^{X14}$, $R^{X15}$, $R^{X16}$, $R^{X17}$, $R^{X18}$, $R^{X19}$, and $R^{X20}$ represents any combination described in Combination D (hereinafter, referred to as Compound Class SX246).

A compound (1F) wherein $R^1$ represents a chlorine atom, $R^{X2}$ represents a chlorine atom, and a combination of $R^{X12}$, $R^{X13}$, $R^{X14}$, $R^{X15}$, $R^{X16}$, $R^{X17}$, $R^{X18}$, $R^{X19}$, and $R^{X20}$ represents any combination described in Combination D (hereinafter, referred to as Compound Class SX247).

A compound represented by formula (2F):

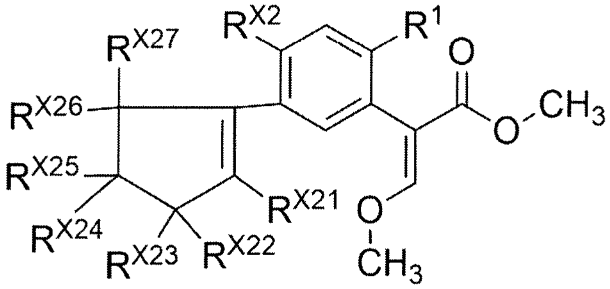

(2F)

(hereinafter, referred to as Compound (2F)) wherein $R^1$ represents a methyl group, $R^{x2}$ represents a methyl group, and a combination of $R^{x21}$, $R^{x22}$, $R^{x23}$, $R^{x24}$, $R^{x25}$, $R^{x26}$ and $R^{x27}$ represents any combinations described in Combination E (hereinafter, referred to as Compound Class SX248).

The Combination E consists of the Substituent No. ZE1 to ZE20. The Substituent No. ZE1 to ZE20 represent a combination of $R^{x21}$, $R^{x22}$, $R^{x23}$, $R^{x24}$, $R^{x25}$, $R^{x26}$ and $R^{x27}$ in the Compound (2F), which is hereinafter described as [Substituent No.; $R^{x21}$, $R^{x22}$, $R^{x23}$, $R^{x24}$, $R^{x25}$, $R^{x26}$, $R^{x27}$]. For example, the Substituent No. ZE2 represents a combination wherein $R^{X21}$ represents a methyl group, and $R^{x22}$, $R^{x23}$, $R^{x24}$, $R^{x25}$, $R^{x26}$ and $R^{x27}$ represent a hydrogen atom.

Combination E

[ZE1;H,H,H,H,H,H,H], [ZE2;Me,H,H,H,H,H,H], [ZE3;F,H,H,H,H,H,H ], [ZE4;Cl,H,H,H,H,H,H], [ZE5;H,F,F,H,H,H,H], [ZE6;Me,F,F,H,H, H,H], [ZE7;F,F,F,H,H,H,H], [ZE8;Cl,F,F,H,H,H,H], [ZE9;H,H,H,H, H,F,F], [ZE10;Me,H,H,H,H,F,F], [ZE11;F,H,H,H,H,F,F], [ZE12;Cl, H,H,H,H,F,F], [ZE13;H,Cl,Cl,H,H,H,H], [ZE14;Me,Cl,Cl,H,H,H,H] , [ZE15;F,Cl,Cl,H,H,H,H], [ZE16;Cl,Cl,Cl,H,H,H,H], [ZE17;H,H,H ,H,H,Cl,Cl], [ZE18;Me,H,H,H,H,Cl,Cl], [ZE19;F,H,H,H,H,Cl,Cl], [ZE20;Cl,H,H,H,H,Cl,Cl],

A compound (2F) wherein $R^1$ represents a methyl group, $R^{X2}$ represents an ethyl group, and a combination of $R^{x21}$, $R^{x22}$, $R^{x23}$, $R^{X24}$, $R^{X25}$, $R^{X26}$, and $R^{X27}$ represents any combination described in Combination E (hereinafter, referred to as Compound Class SX249).

A compound (2F) wherein $R^1$ represents a methyl group, $R^{X2}$ represents a fluorine atom, and a combination of $R^{x21}$, $R^{X22}$, $R^{X23}$, $R^{X24}$, $R^{X25}$, $R^{X26}$, and $R^{X27}$ represents any combination described in Combination E (hereinafter, referred to as Compound Class SX250).

A compound (2F) wherein $R^1$ represents a methyl group, $R^{X2}$ represents a chlorine atom, and a combination of $R^{x21}$, $R^{x22}$, $R^{x23}$, $R^{x24}$, $R^{X25}$, $R^{X26}$, and $R^{X27}$ represents any combination described in Combination E (hereinafter, referred to as Compound Class SX251).

A compound (2F) wherein $R^1$ represents a methyl group, $R^{X2}$ represents a methoxy group, and a combination of $R^{x21}$, $R^{X22}$, $R^{X23}$, $R^{X24}$, $R^{X25}$, $R^{X26}$, and $R^{X27}$ represents any combination described in Combination E (hereinafter, referred to as Compound Class SX252).

A compound (2F) wherein $R^1$ represents a methyl group, $R^{X2}$ represents a cyclopropyl group, and a combination of $R^{x21}$, $R^{X22}$, $R^{X23}$, $R^{X24}$, $R^{X25}$, $R^{X26}$, and $R^{X27}$ represents any combination described in Combination E (hereinafter, referred to as Compound Class SX253).

A compound (2F) wherein $R^1$ represents a fluorine atom, $R^{X2}$ represents a methyl group, and a combination of $R^{x21}$, $R^{X22}$, $R^{X23}$, $R^{X24}$, $R^{x25}$, $R^{X26}$, and $R^{X27}$ represents any combination described in Combination E (hereinafter, referred to as Compound Class SX254).

A compound (2F) wherein $R^1$ represents a fluorine atom, $R^{X2}$ represents a fluorine atom, and a combination of $R^{x21}$, $R^{X22}$, $R^{X23}$, $R^{X24}$, $R^{X25}$, $R^{X26}$, and $R^{X27}$ represents any combination described in Combination E (hereinafter, referred to as Compound Class SX255).

A compound (2F) wherein $R^1$ represents a fluorine atom, $R^{X2}$ represents a chlorine atom, and a combination of $R^{x21}$, $R^{X22}$, $R^{X23}$, $R^{X24}$, $R^{X25}$, $R^{X26}$, and $R^{X27}$ represents any combination described in Combination E (hereinafter, referred to as Compound Class SX256).

A compound (2F) wherein $R^1$ represents a chlorine atom, $R^{X2}$ represents a methyl group, and a combination of $R^{x21}$, $R^{x22}$, $R^{x23}$, $R^{x24}$, $R^{x25}$, $R^{X26}$, and $R^{X27}$ represents any combination described in Combination E (hereinafter, referred to as Compound Class SX257).

A compound (2F) wherein $R^1$ represents a chlorine atom, $R^{X2}$ represents a fluorine atom, and a combination of $R^{x21}$, $R^{X22}$, $R^{X23}$, $R^{X24}$, $R^{X25}$, $R^{X26}$, and $R^{X27}$ represents any combination described in Combination E (hereinafter, referred to as Compound Class SX258).

A compound (2F) wherein $R^1$ represents a chlorine atom, $R^{X2}$ represents a chlorine atom, and a combination of $R^{x21}$, $R^{X22}$, $R^{X23}$, $R^{X24}$, $R^{X25}$, $R^{X26}$, and $R^{X27}$ represents any combination described in Combination E (hereinafter, referred to as Compound Class SX259).

Next, the formulation examples of the compounds of the present invention are shown below. In the formulation examples, the "parts" represents "part by weight" unless otherwise specified. The present compound S represents the compounds described in the Compound Classes SX1 to SX259.

Formulation Example 1

Thirty five (35) parts of a mixture of ammonium polyoxyethylene alkyl ether sulfate and wet silica (weight ratio: 1:1), 10 parts of any one of the present compound S, and 55 parts of water are mixed, and the mixture is then finely-ground by a wet grinding method to obtain a formulation.

Formulation Example 2

Fifty (50) parts of any one of the present compound S, 3 parts of calcium lignin sulfonate, 2 parts of sodium lauryl sulfate, and 45 parts of silica are well mixed-grinding to obtain a formulation.

Formulation Example 3

Five (5) parts of any one of the present compound S, 9 parts of polyoxyethylene styryl phenyl ether, 5 parts of polyoxyethylene decyl ether (Number of ethylene oxide additions: 5), 6 parts of calcium dodecylbenzene sulfonate and 75 parts of xylene are well mixed to obtain a formulation.

Formulation Example 4

Two (2) parts of any one of the present compound S, 1 part of silica, 2 parts of calcium lignin sulfonate, 30 parts of bentonite and 65 parts of kaolin clay are mixed-grinding, and thereto is added an appropriate amount of water, and the mixture is well kneaded and is then granulated with a granulator and dried to obtain a formulation.

Formulation Example 5

Ten (10) parts of any one of the present compound S is mixed with a mixture of 18 parts of benzyl alcohol and 9 parts of DMSO, and thereto are added 6.3 parts of GERONOL (registered trademark) TE250, 2.7 parts of Ethylan (registered trademark) NS-500LQ, and 54 parts of Solvent naphtha, and the mixture is mixed to obtain a formulation.

Formulation Example 6

Zero point one (0.1) parts of any one of the present compound S is dissolved by mixing with 39.9 parts of kerosene, and the mixture is placed in an aerosol container, and 60 parts of liquefied petroleum gas (mixture of propane, butane and isobutane; saturated vapor pressure: 0.47 MPa (25°C)) is filled in the container to obtain a formulation.

Formulation Example 7

Zero point two (0.2) parts of any one of the present compound S, 50 parts of pyrethrum extract dreg powder, 30 parts of Machilus thunbergii powder, and 19.8 parts of wood powder are mixed, and an appropriate amount of water is added thereto, and the mixture is well kneaded, and is extructed with an extruder into a plate-like sheet, and the resulting sheet is made a spiral-like form thereof with a punching machine to obtain a formulation.

Next, Test Examples are described.

The untreated groups in Test Example 1 to Test Example 8 represent tested groups in which the same conditions as those of each of the Test Examples were conducted except that DMSO was dispensed in the place of a DMSO diluted solution comprising the present compound. Also the untreated groups in Test Example 9 to Test Example 19 represent tested groups in which an aqueous diluted solution of a formulation comprising the present compound is not applied. Further the untreated groups in Test Example 20 and Test Example 23 represent tested groups in which an aqueous diluted solution of a formulation comprising the present compound is not applied.

Test Example 1: Control test against wheat septoria leaf blotch fungus (Septoria tritici)

The present compound 1-1, 1-2, 1-3, 1-4, 1-5, 1-6, 2-1, 3-1, 3-2, 4-1, 4-2, 4-3, 4-4, 4-5, 4-6, 5-1, 6-1, 1-7, 1-8, 1-9, 2-2, 2-3, 3-3, 3-4, 4-7, 4-8, 4-9, 4-10, 4-11, 4-12, 4-13, 4-14, 4-15, 4-16, 4-17, 4-18, 4-19, 4-20, 4-21, 4-22, 4-23, 4-24, 4-25, 4-26, 4-27, 4-28, 4-29, 4-30, 5-2, 5-3, 5-4, 5-5, or 6-2 was diluted with DMSO so as to contain 150 ppm, and 1 μL of the dilution solutions were dispensed into titer plate (96 well), and thereafter, thereto was then dispensed 150 μL of YBG medium to which conidia of Septoria tritici were inoculated in advance. This plate was cultured at 18°C for 3 days, thereby allowing Septoria tritici to undergo proliferation, and the absorbance at 550 nm of each well of the titer plate was then measured to determine a degree of growth of Septoria tritici. As a result, every of the growth in the well in treated groups treated with each of the present compounds showed 50% or less compared to the growth in an untreated well.

Test Example 2: Control test against Cucurbitaceae phytophthora rot fungus (Phytophthora capsici)

The present compound 1-2, 1-7, 1-8, 1-9, 2-3, 4-7, 4-9, 4-13, 4-14, 4-15, 4-16, 4-18, 4-19, or 4-20 was diluted

with DMSO so as to contain 150 ppm, and 1 μL of the dilution solutions were dispensed into titer plate (96 well), and thereafter, thereto was then dispensed 150 μL of a potato dextrose broth (PDB broth) to which spores of Phytophthora capsici were inoculated in advance. This plate was cultured at 27°C for 3 days, thereby allowing Phytophthora capsici to undergo proliferation, and the absorbance at 550 nm of each well of the titer plate was then measured to determine a degree of growth of the Phytophthora capsici. As a result, every of the growth in the well in treated groups treated with each of the present compounds showed 50% or less compared to the growth in an untreated well.

Test Example 3: Control test against seedling blight fungus (Pythium ultimum)

The present compound 1-2, 2-1, 4-2, 4-3, 4-4, 4-5, 6-1, 1-3, 1-5, 1-6, 1-7, 1-8, 1-9, 2-3, 3-1, 3-2, 3-4, 4-7, 4-8, 4-9, 4-12, 4-13, 4-14, 4-15, 4-16, 4-17, 4-18, 4-19, 4-22, 4-23, 4-24, 4-25, 4-26, 4-27, 4-28, 4-29, 5-1, or 5-2 was diluted with DMSO so as to contain 150 ppm, and 1 μL of the dilution solutions were dispensed into titer plate (96 well), and thereafter, thereto was then dispensed 150 μL of Czapek medium to which spores of Pythium ultimum were inoculated in advance. This plate was cultured at 23°C for 5 days, thereby allowing Pythium ultimum to undergo proliferation, and the absorbance at 550 nm of each well of the titer plate was then measured to determine a degree of growth of the Pythium ultimum. As a result, every of the growth in the well in treated groups treated with each of the present compounds showed 50% or less compared to the growth in an untreated well.

Test Example 4: Control test against Corn smut fungus (Ustilago maydis)

The present compound 2-1, 4-2, 4-3, 4-4, 4-5, 6-1, 1-5, 1-7, 1-8, 1-9, 2-3, 4-1, 4-8, 4-9, 4-10, 4-11, 4-12, 4-13, 4-14, 4-15, 4-16, 4-17, 4-19, 4-21, 4-22, 4-23, 4-24, 4-25, 4-26, 4-27, 4-28, 4-29, 5-4, or 6-2 was diluted with DMSO so as to contain 150 ppm, and 1 μL of the dilution solutions were dispensed into titer plate (96 well), and thereafter, thereto was then dispensed 150 μL of a potato dextrose broth (PDB broth) to which conidia of Ustilago maydis were inoculated in advance. This plate was cultured at 18°C for 4 days, thereby allowing Ustilago maydis to undergo proliferation, and the absorbance at 550 nm of each well of the titer plate was then measured to determine a degree of growth of the Ustilago maydis. As a result, every of the growth in the well in treated groups treated with each of the present compounds showed 50% or less compared to the growth in an untreated well.

Test Example 5: Control test against Barley scald fungus (Rhynchosporium secalis)

The present compound 1-2, 2-1, 4-2, 4-3, 4-4, 4-5, 6-1, 1-1, 1-3, 1-4, 1-5, 1-6, 1-7, 1-8, 1-9, 2-2, 2-3, 3-1, 3-2, 3-3, 3-4, 4-1, 4-6, 4-7, 4-8, 4-9, 4-10, 4-11, 4-12, 4-13, 4-14, 4-15, 4-16, 4-17, 4-18, 4-19, 4-20, 4-21, 4-22, 4-23, 4-24, 4-25, 4-26, 4-27, 4-28, 4-29, 4-30, 5-1, 5-2, 5-3, 5-4, 5-5, or 6-2 was diluted with DMSO so as to contain 150 ppm, and 1 μL of the dilution solutions were dispensed into titer plate (96 well), and thereafter, thereto was then dispensed 150 μL of a potato dextrose broth (PDB broth) to which conidia of Rhynchosporium secalis were inoculated in advance. This plate was cultured at 18°C for 7 days, thereby allowing Rhynchosporium secalis to undergo proliferation, and the absorbance at 550 nm of each well of the titer plate was then measured to determine a degree of growth of the Rhynchosporium secalis. As a result, every of the growth in the well in treated groups treated with each of the present compounds showed 50% or less compared to the growth in an untreated well.

Test Example 6: Control test against Cucumber botrytis rot fungus (Botrytis cinerea)

The present compound 1-2, 2-1, 4-2, 4-3, 4-4, 4-5, 6-1, 1-1, 1-3, 1-4, 1-5, 1-6, 1-7, 1-8, 1-9, 2-2, 2-3, 3-1, 3-2, 3-3, 3-4, 4-1, 4-6, 4-7, 4-8, 4-9, 4-10, 4-11, 4-12, 4-13, 4-14, 4-15, 4-16, 4-17, 4-18, 4-19, 4-20, 4-21, 4-22, 4-23, 4-24, 4-25, 4-26, 4-27, 4-28, 4-29, 4-30, 5-1, 5-2, 5-3, 5-4, 5-5, or 6-2 was diluted with DMSO so as to contain 150 ppm, and 1 μL of the dilution solutions were dispensed into titer plate (96 well), and thereafter, thereto was then dispensed 150 μL of complete medium to which conidia of Botrytis cinerea were inoculated in advance. This plate was cultured at 18°C for 4 days, thereby allowing Botrytis cinerea to undergo proliferation, and the absorbance at 550 nm of each well of the titer plate was then measured to determine a degree of growth of the Botrytis cinerea. As a result, every of the growth in the well in treated groups treated with each of the present compounds showed 50% or less compared to the growth in an untreated well.

Test Example 7: Control test against Peach scab fungus (Cladosporium carpophilum)

The present compound 1-2, 2-1, 4-2, 4-3, 4-4, 4-5, 6-1, 1-1, 1-3, 1-4, 1-5, 1-6, 1-7, 1-8, 1-9, 2-2, 2-3, 3-1, 3-2, 3-3, 3-4, 4-1, 4-6, 4-7, 4-8, 4-9, 4-10, 4-11, 4-12, 4-13, 4-14, 4-15, 4-16, 4-17, 4-18, 4-19, 4-20, 4-21, 4-22, 4-23, 4-24, 4-25, 4-26, 4-27, 4-28, 4-29, 4-30, 5-1, 5-2, 5-3, 5-4, 5-5, or 6-2 was diluted with DMSO so as to contain 150 ppm, and

1 μL of the dilution solutions were dispensed into titer plate (96 well), and thereafter, thereto was then dispensed 150 μL of a potato dextrose broth (PDB broth) to which conidia of Cladosporium carpophilum were inoculated in advance. This plate was cultured at 18°C for 5 days, thereby allowing Cladosporium carpophilum to undergo proliferation, and the absorbance at 550 nm of each well of the titer plate was then measured to determine a degree of growth of the Cladosporium carpophilum. As a result, every of the growth in the well in treated groups treated with each of the present compounds showed 50% or less compared to the growth in an untreated well.

Test Example 8: Control test against rice brown spot fungus (Cochliobolus miyabeanus)

The present compound 1-2, 2-1, 4-2, 4-3, 4-4, 4-5, 6-1, 1-1, 1-3, 1-4, 1-5, 1-6, 1-7, 1-8, 1-9, 2-2, 2-3, 3-1, 3-2, 3-3, 3-4, 4-1, 4-6, 4-7, 4-8, 4-9, 4-10, 4-11, 4-12, 4-13, 4-14, 4-15, 4-16, 4-17, 4-18, 4-19, 4-20, 4-21, 4-22, 4-24, 4-25, 4-26, 4-27, 4-29, 4-30, 5-1, 5-2, 5-3, 5-4, or 5-5 was diluted with DMSO so as to contain 150 ppm, and 1 μL of the dilution solutions were dispensed into titer plate (96 well), and thereafter, thereto was then dispensed 150 μL of YB liquid medium to which conidia of Cochliobolus miyabeanus were inoculated in advance. This plate was cultured at 23°C for 3 days, thereby allowing Cochliobolus miyabeanus to undergo proliferation, and the absorbance at 550 nm of each well of the titer plate was then measured to determine a degree of growth of the Cochliobolus miyabeanus. As a result, every of the growth in the well in treated groups treated with each of the present compounds showed 50% or less compared to the growth in an untreated well.

Test Example 9: Control test against soybean rust (Phakopsora pachyrhizi)

Soybean leaf (cv; Kurosengoku) was punched out to 1 cm diameter to prepare a leaf disk. Each 1 mL of an agar medium (agar concentration 1.2 %) was dispensed in each well of 24 well microplate. A piece of the leaf disk was placed on agar medium on each well. To a mixture of 0.5 μL of Sorpol (registered trademark) 1200KX, 4.5 μL of DMSO, and 5 μL of xylene was added 20 μL of a solution containing 10000 ppm of the test compound in DMSO. The resulting mixture was diluted with ion exchange water to prepare a mixture containing a predetermined concentration of the test compound. The resulting mixture was sprayed in 10 μL per one leaf disk. After 1 day, an aqueous suspension of conidia of *Phakopsora pachyrhizi* having an amino acid substitution of F129L on mitochondrial cytochrome b protein (1.0 × $10^5$/mL) was inoculated onto the leaf disks. After the inoculation, the microplate was placed in a growth chamber (light on for 6 hours, light off for 18 hours, 23°C temperature, 60% humidity). After 1 day, the leaf disks were air-dried to disappear water droplets on the surface of the leaf disk, and the microplate was placed again in the growth chamber for 12 days. Thereafter, a lesion area of soybean rust disease was assessed. As a result, lesion areas in the leaf disk treated with any one of the present compounds 1-2, 2-1, 3-1, 3-2, 4-1, 4-2, 4-3, 4-4, 4-5, 4-6, 5-1, 6-1, 1-1, 1-3, 1-4, 1-5, 1-6, 1-7, 1-8, 1-9, 2-2, 2-3, 3-3, 3-4, 4-1, 4-6, 4-7, 4-8, 4-9, 4-10, 4-11, 4-12, 4-13, 4-14, 4-15, 4-16, 4-17, 4-18, 4-19, 4-20, 4-21, 4-22, 4-23, 4-24, 4-25, 4-26, 4-27, 4-28, 4-29, 5-1, 5-2, 5-3, 5-4, 5-5, and 6-2 as a tested compound at a prescribed concentration of 50 ppm showed 30% or less compared to the lesion areas in an untreated leaf disk.

Test Example 10: Control test against barley net blotch (Pyrenophora teres)

Each of plastic pots was filled with soil and thereto barley (cv; NISHINOHOSHI) seeds were sown and the barleys were grown in a greenhouse for 7 days. Thereafter, the present compound 1-2, 4-2, 4-4, 4-5, 6-1, 1-1, 1-3, 1-4, 1-5, 1-6, 4-1, 4-6, 4-8, 4-9, 4-10, 4-11, 4-12, 4-13, 4-14, 4-15, 4-16, 4-17, 4-18, 4-19, 4-20, 4-21, 4-22, 4-23, 4-24, 4-25, 4-26, 4-27, 4-28, 4-29, 5-1, 5-2, 5-3, or 5-4, each of which was made to a formulation according to the similar method to that of Formulation Example 1, was mixed with water so as to be a prescribed concentration (200 ppm). The resulting mixtures were sprayed to foliar parts so as to adhere adequately on the leaves of the above-mentioned barley. After spraying the mixtures, the barleys were air-dried and after 1 day, an aqueous suspension of the conidia of Pyrenophora teres was spraying-inoculated. After the inoculation, the barleys were placed at 23°C during daytime and 20°C during nighttime under a high humidity for 3 day and then cultivated in a greenhouse for 7 days, and a lesion area was observed. As a result, every of the lesion areas in barleys treated with each of the present compounds showed 30% or less compared to the lesion area in an untreated barley.

Test Example 11: Control test against wheat brown rust (Puccinia recondita)

Each of plastic pots was filled with soil and thereto wheat (cv; SHIROGANE) seeds were sown and the wheats were grown in a greenhouse for 9 days. The present compound 1-2, 2-1, 3-1, 3-2, 4-2, 4-3, 4-4, 4-5, 6-1, 1-1, 1-3, 1-4, 1-5, 1-6, 1-7, 1-8, 1-9, 3-1, 3-2, 3-4, 4-1, 4-6, 4-7, 4-8, 4-9, 4-10, 4-12, 4-13, 4-14, 4-15, 4-16, 4-17, 4-18, 4-19, 4-20, 4-21, 4-22, 4-23, 4-24, 4-25, 4-26, 4-27, 4-28, 4-29, 5-1, 5-2, 5-3, 5-4, 5-5, 6-2, or 2-3 each of which was made to a formulation according to the similar method to that of Formulation Example 1, was mixed with water so as to be 200

ppm, and the mixtures were sprayed to foliar parts so as to adhere adequately on the leaves of the above-mentioned wheat. After spraying the mixtures, the wheats were air-dried and were then cultivated at 20°C under lighting for 5 to 7 days. The conidia of Puccinia recondita were sprinkling-inoculated. After the inoculation, the wheats were placed under a dark and humid condition at 23°C for 1 day and were then cultivated at 20°C under lighting for 8 days, and a lesion area was observed. As a result, every of the lesion areas in wheats treated with each of the present compounds showed 30% or less compared to the lesion area in an untreated wheat.

Test Example 12: Control test against septoria leaf blotch (Septoria tritici)

Each of plastic pots was filled with soil and thereto wheat (cv; Apogee) seeds were sown and the wheats were grown in a greenhouse for 10 days. Thereafter, the present compound 1-2, 2-1, 3-1, 3-2, 4-2, 4-3, 4-4, 4-5, 6-1, 1-1, 1-3, 1-4, 1-5, 1-6, 1-7, 1-8, 1-9, 3-1, 3-2, 3-4, 4-1, 4-6, 4-7, 4-8, 4-9, 4-10, 4-12, 4-13, 4-14, 4-15, 4-16, 4-17, 4-18, 4-19, 4-20, 4-21, 4-22, 4-23, 4-24, 4-25, 4-27, 4-28, 4-29, 5-1, 5-2, 5-3, 5-4, 6-2, or 2-3 each of which was made to a formulation according to the similar method to that of Formulation Example 1, was mixed with water so as to be 200 ppm. The mixtures were sprayed to foliar parts so as to adhere adequately on the leaves of the above-mentioned wheat. After spraying the mixtures, the wheats were air-dried and after 4 days, an aqueous suspension of the conidia of Septoria tritici was spraying-inoculated. After the inoculation, the wheats were placed at 18°C under a high humidity for 3 days and then under lighting for 14 to 18 days, and a lesion area was observed. As a result, every of the lesion areas in wheats treated with each of the present compounds showed 30% or less compared to the lesion area in an untreated wheat.

Test Example 13: Control test against septoria leaf blotch (Septoria tritici)

Each of plastic pots was filled with soil and thereto wheat (cv; Apogee) seeds were sown and the wheats were grown in a greenhouse for 10 days. An aqueous suspension of the conidia of Septoria tritici was spraying-inoculated. After the inoculation, the wheats were placed at 18°C under a high humidity for 3 days. Thereafter, the present compound 1-2, 2-1, 3-1, 4-2, 4-3, 4-4, 4-5, 6-1, 1-1, 1-3, 1-4, 1-5, 1-6, 1-7, 1-8, 1-9, 3-1, 4-1, 4-6, 4-7, 4-10, 4-12, 4-13, 4-14, 4-15, 4-17, 4-18, 4-19, 4-20, 4-21, 4-22, 4-23, 4-24, 4-25, 4-27, 4-28, 4-29, 5-1, 6-2, or 2-3 each of which was made to a formulation according to the similar method to that of Formulation Example 1, was mixed with water so as to be 200 ppm. The mixtures were sprayed to foliar parts so as to adhere adequately on the leaves of the above-mentioned wheat. After spraying the mixtures, the wheats were air-dried and were placed under lighting for 14 to 18 days, and a lesion area was observed. As a result, every of the lesion areas in wheats treated with each of the present compounds showed 30% or less compared to the lesion area in an untreated wheat.

Test Example 14: Control test against tomato late blight (Phytophthora infestans)

Each of plastic pots was filled with soils and thereto tomato (cv; PATIO) seeds were sown and the tomatoes were grown in a greenhouse for 20 days. Thereafter, the present compound 2-1, 3-2, 4-2, 4-3, 4-5, 1-1, 1-3, 1-4, 1-5, 1-6, 1-8, 1-9, 3-2, 4-1, 4-6, 4-7, 4-8, 4-9, 4-12, 4-13, 4-15, 4-16, 4-17, 4-18, 4-19, 4-22, 4-23, 4-24, 4-28, 4-29, 5-1, 5-2, or 5-4, each of which was made to a formulation according to the similar method to that of Formulation Example 1, was mixed with water so as to be 200 ppm. The mixtures were sprayed to foliar parts so as to adhere adequately on the leaves of the above-mentioned tomato. After spraying the mixtures, the tomatoes were air-dried and after 1 day, an aqueous suspension of the spores of Phytophthora infestans were spraying-inoculated. After the inoculation, the tomatoes were placed in a greenhouse of 23°C during daytime and 20°C during nighttime under a high humidity for 1 day, and were then cultivated in a greenhouse for 4 days, and a lesion area was observed. As a result, every of the lesion areas in tomatoes treated with each of the present compounds showed 30% or less compared to the lesion area in an untreated tomato.

Test Example 15: Control test against soybean rust (Phakopsora pachyrhizi)

Each of plastic pots was filled with soil and thereto soybean (cv: Kurosengoku) seeds were sown and the soybeans were grown in a greenhouse for 10 to 14 days. Thereafter, the present compound 1-2, 2-1, 3-1, 3-2, 4-2, 4-3, 4-4, 4-5, 6-1, 1-1, 1-3, 1-4, 1-5, 1-6, 1-7, 1-8, 1-9, 3-1, 3-2, 3-3, 3-4, 4-1, 4-6, 4-7, 4-8, 4-9, 4-10, 4-11, 4-12, 4-13, 4-14, 4-15, 4-16, 4-17, 4-18, 4-19, 4-20, 4-21, 4-22, 4-23, 4-24, 4-25, 4-26, 4-27, 4-28, 4-29, 5-1, 5-2, 5-3, 5-4, 5-5, 6-2, or 2-3 each of which was made to a formulation according to the similar method to that of Formulation Example 1, was mixed with water so as to be 200 ppm. The resulting mixtures were sprayed to foliar parts so as to adhere adequately on the leaves of the above-mentioned soybean. After spraying the mixtures, the soybeans were air-dried and after 2 to 5 days, an aqueous suspension of the conidia of Phakopsora pachyrhizi was spraying-inoculated. After the inoculation,

the soybeans were placed in a greenhouse of 23°C during daytime and 20°C during nighttime under a high humidity for 1 to 2 days, and were then cultivated in the greenhouse for 12 days, and a lesion area was observed. As a result, every of the lesion areas in soybean treated with each of the present compounds showed 30% or less compared to the lesion area in an untreated soybean.

Test Example 16: Control test against soybean rust (Phakopsora pachyrhizi)

Each of plastic pots was filled with soil and thereto soybean (cv: Kurosengoku) seeds were sown and the soybeans were grown in a greenhouse for 10 days, and an aqueous suspension containing the conidia of Phakopsora pachyrhizi was spraying-inoculated. After the inoculation, the soybeans were placed in a greenhouse of 23°C during daytime and 20°C during nighttime under a high humidity for 1 day, and were then cultivated in the greenhouse for 2 days, and thereafter, the present compound 1-2, 2-1, 3-1, 3-2, 4-2, 4-3, 4-4, 4-5, 6-1, 1-1, 1-3, 1-5, 1-6, 1-7, 1-8, 1-9, 3-1, 3-2, 3-3, 3-4, 4-1, 4-7, 4-8, 4-9, 4-10, 4-12, 4-14, 4-15, 4-16, 4-17, 4-18, 4-19, 4-20, 4-22, 4-24, 4-29, 5-1, 5-4, or 2-3 each of which was made to a formulation according to the similar method to that of Formulation Example 1, was mixed with water so as to be 200 ppm, and the resulting mixtures were sprayed to foliar parts so as to adhere adequately on the leaves of the above-mentioned soybean. After spraying the mixtures, the soybeans were air-dried and cultivated in a greenhouse for 8 days, and a lesion area was then observed. As a result, every of the lesion areas in soybean treated with each of the present compounds showed 30% or less compared to the lesion area in an untreated soybean.

Test Example 17: Control test against soybean leaf spot (Cercospora sojina)

Each of plastic pots was filled with soil and thereto soybean (cv: Tachinagaha) seeds were sown and the soybeans were grown in a greenhouse for 13 days. Thereafter, the present compound 1-2, 2-1, 3-1, 3-2, 4-2, 4-3, 4-4, 4-5, 6-1, 1-7, 1-8, 1-9, 3-1, 3-2, 4-14, 4-16, 4-17, 4-19, 4-20, 4-21, 4-22, 4-23, 4-24, 4-25, 4-26, 4-27, 4-28, 4-29, 5-4, 5-5, 6-2, or 2-3 each of which was made to a formulation according to the similar method to that of Formulation Example 1, was mixed with water so as to be 200 ppm. The resulting mixtures were sprayed to foliar parts so as to adhere adequately on the leaves of the above-mentioned soybean. After spraying the mixtures, the soybeans were air-dried and after 1 day, an aqueous suspension of the conidia of Cercospora sojina was spraying-inoculated. After the inoculation, the soybeans were placed in a greenhouse of 23°C during daytime and 20°C during nighttime under a high humidity for 3 days, and were then cultivated in the greenhouse for 16 days, and a lesion area was observed. As a result, every of the lesion areas in soybean treated with each of the present compounds showed 30% or less compared to the lesion area in an untreated soybean.

Test Example 18: Control test against tomato early blight (Alternaria solani)

Each of plastic pots was filled with soils and thereto tomato (cv; PATIO) seeds were sown and the tomatoes were grown in a greenhouse for 20 days. Thereafter, the present compound 2-1, 3-1, 3-2, 4-2, 4-3, 6-1, 1-5, 1-6, 1-7, 1-8, 1-9, 2-2, 3-1, 3-2, 3-3, 4-1, 4-6, 4-7, 4-8, 4-9, 4-10, 4-11, 4-12, 4-13, 4-14, 4-15, 4-16, 4-18, 4-19, 4-20, 4-21, 4-22, 4-23, 4-24, 4-25, 4-26, 4-28, 4-29, 4-30, 5-1, 5-2, 5-3, 5-4, 5-5, 6-2, or 2-3 each of which was made to a formulation according to the similar method to that of Formulation Example 1, was mixed with water so as to be 200 ppm. The resulting mixtures were sprayed to foliar parts so as to adhere adequately on the leaves of the above-mentioned tomato. After spraying the mixtures, the tomatoes were air-dried and after 1 day, an aqueous suspension of the conidia of Alternaria solani were spraying-inoculated. After the inoculation, the tomatoes were placed at 18°C under a high humidity for 6 days, and a lesion area was observed. As a result, every of the lesion areas in tomatoes treated with each of the present compounds showed 300 or less compared to the lesion area in an untreated tomato.

Test Example 19: Control test against kidney bean stem rot (Sclerotinia sclerotiorum)

Each of plastic pots was filled with soil and thereto Kidney bean (cv; NAGAUZURA SAITO) seeds were sown and the kidney beans were grown in a greenhouse for 8 days. Thereafter, the present compound 1-2, 2-1, 3-2, 4-2, 4-3, 4-4, 4-5, 6-1, 1-3, 1-4, 1-5, 1-6, 1-7, 1-8, 1-9, 3-2, 3-3, 3-4, 4-1, 4-6, 4-7, 4-9, 4-10, 4-12, 4-13, 4-14, 4-15, 4-17, 4-18, 4-19, 4-20, 4-21, 4-22, 4-23, 4-28, 4-29, 5-1, or 5-2, each of which was made to a formulation according to the similar method to that of Formulation Example 1, was mixed with water so as to be 200 ppm. The resulting mixtures were sprayed to the foliar parts so as to adhere adequately on the leaves of the above-mentioned kidney bean. After spraying the mixtures, the kidney beans were air-dried and a PDA medium containing hyphae of Sclerotinia sclerotiorum was placed on the leaves of the kidney bean. After the inoculation, all kidney beans were placed under a high humidity during only night and after 4 days, a lesion area was observed. As a result, every of the lesion areas in kidney beans treated with each of the present compounds showed 300 or less compared to the lesion area in an untreated kidney beans.

Test Method 20: Tests against cotton aphids

The test compounds is made to a formulation according to a similar method to that described in the Formulation Example 1, and thereto is added water containing 0.03 v/v % of a spreader to prepare a diluted solution containing a prescribed concentration of the test compound.
Cucumber (*Cucumis sativus*) seedling (on the developmental stage of the second true leaf) is planted in a container and approximately 30 cotton aphids (*Aphis gossypii*) (all stages of life) are released onto the leaves of the cucumber. After 1 day, the diluted solutions are sprayed into the seedling in a ratio of 10 mL/seedling. Further, after 5 days, the number of the surviving insects is examined and the controlling value is calculated by the following equation.

$$\text{Controlling value (\%)} = \{1-(Cb \times Tai)/(Cai \times Tb)\} \times 100$$

wherein the symbols in the formula represent the following descriptions.

Cb: Number of the test insects in untreated group;
Cai: Number of the surviving insects at the time of the investigation in untreated group;
Tb: Number of the test insects in treated group;
Tai: Number of the surviving insects at the time of the investigation in treated group.

Test Example 20

The test was conducted by making the prescribed concentration 500 ppm and using the below-mentioned compounds of the present invention as a test compound according to the test method 20. As a result of the test, the below-mentioned compounds of the present invention showed 90 % or greater as the controlling value.
Compound of the present invention: 1-2, 2-1, 3-1, 3-2, 4-2, 4-3, 6-1, 1-1, 1-6, 1-7, 1-9, 4-1, 4-7, 4-9, 4-10, 4-11, 4-12, 4-15, 4-16, 4-17, 4-18, 4-19, 4-22, 4-23, 4-24, 4-25, 4-29, 5-1, and 6-2.

Test Method 21: Tests against brown planthoppers

The test compounds are made to a formulation according to a similar method to that described in the Formulation Example 1, and thereto is added water containing 0.03 v/v % of Shindain (registered trademark) to prepare a diluted solution containing a prescribed concentration of the test compound.
Rice (Oryza sativa) seedling (on the developmental stage of the second true leaf) is planted in a container, and the diluted solutions are sprayed into the seedling in a ratio of 10 mL/seedling. Thereafter, 20 of 3rd instar larvae of brown planthoppers (*Nilaparvata lugens*) are released onto the rice leaves. After 6 days, the morality is calculated by the following equation.

$$\text{Morality (\%)} = \{1- \text{the number of the surviving insects}/20\} \times 100$$

Test Example 21

The test was conducted by making the prescribed concentration 500 ppm and using the below-mentioned compounds of the present invention as a test compound according to the test method 21. As a result of the test, the below-mentioned compound of the present invention showed 90 % or greater as the mortality.
Compound of the present invention: 1-2, 2-1, 3-1, 3-2, 6-1, 1-6, 1-7, 1-9, 2-2, 3-3, 4-7, 4-14, 4-15, 4-16, 4-17, 4-18, 4-19, 4-22, 4-29, and 6-2

Test Method 22: Tests against diamondback moth

Test compounds are made to a formulation according to a similar method to that described in the Formulation example 1, and thereto is added water containing 0.03 v/v% of Shindain (registered trademark) to prepare a diluted solution containing a prescribed concentration of the test compound.
Cabbage (*Brassicae oleracea*) seedling (on the developmental stage of the second to third true leaf) is planted in a cup, and the diluted solutions are sprayed into the seedling at a ratio of 20 mL/seedling. Thereafter, the stem and

leaf thereof is cut out and then is installed into a cup that is covered with filter paper on the bed of the cup. Five (5) diamondback moth (*Plutella xylostella*) at the second instar larval stage are released into the cup. After 5 days, the number of the surviving insects is counted, and the mortality of insects is calculated by the following equation.

$$\text{Mortality (\%)} = (1 - \text{Number of surviving insects}/5) \times 100$$

Test Example 22

The test was conducted by making the prescribed concentration 500 ppm and using the below-mentioned compounds of the present invention as a test compound according to the test method 22. As a result of the test, the below-mentioned compound of the present invention showed 80 % or greater as the mortality.

Compound of the present invention: 3-1, 3-2, 4-2, 6-1, 4-7, 4-15, 4-18, 4-13, 1-7, 1-8, 1-9, 4-14, 4-17, 4-29, 4-22, 4-23, 4-24, and 4-25

Test Method 23: Tests against Common red spider mite

The test compounds is made to a formulation according to a similar method to that described in the Formulation Example 1, and thereto is added water containing 0.03 v/v % of Shindain (registered trademark) to prepare a diluted solution containing a prescribed concentration of the test compound.

Kidney bean (*Phaseolus vulgaris*) seedling (on the developmental stage of the first true leaf) is planted in a container and approximately 40 adult female of common red spider mites (*Tetranychus urticae*) are released onto the leaves of the kidney bean. After 1 day, the diluted solutions are sprayed into the seedling in a ratio of 10 mL/seedling. Further, after 13 days, the number of the surviving insects is examined and the controlling value is calculated by the following equation.

$$\text{Controlling value (\%)} = \{1-(Cb\times Tai)/(Cai\times Tb)\}\times 100$$

wherein the symbols in the formula represent the following descriptions.

Cb: Number of the test insects in untreated group;
Cai: Number of the surviving insects at the time of the investigation in untreated group;
Tb: Number of the test insects in treated group;
Tai: Number of the surviving insects at the time of the investigation in treated group;

Test Example 23

The test was conducted by making the prescribed concentration 500 ppm and using the below-mentioned compounds of the present invention as a test compound according to the test method 23. As a result of the test, the below-mentioned compound of the present invention showed 90 % or greater as the controlling value.

Compound of the present invention: 1-2, 2-1, 3-1, 3-2, 4-2, 6-1, 1-3, 1-6, 2-2, 2-3, 3-3, 4-7, 4-9, 4-10, 4-11, 4-15, 4-16, 4-17, 4-18, 4-19, 4-21, 4-22, 4-24, 4-25, 4-27, 4-29, and 5-5.

Industrial Applicability

The compound of the present invention has efficacies for controlling pests and can be used to control pests.

## Claims

1. A compound represented by formula (I):

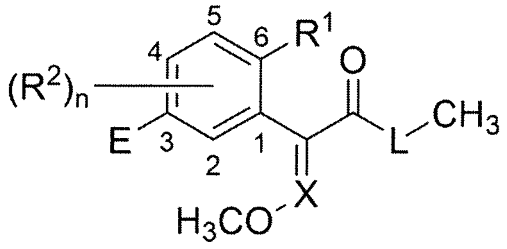

( I )

[wherein

a combination of X and L represents

a combination wherein X represents a nitrogen atom, and L represents an oxygen atom or NH; or
a combination wherein X represents CH, and L represents an oxygen atom,

n is 1 or 2,
E represents a C6-C10 aryl group which may be optionally substituted with one or more substituents selected from Group A, a five- to ten- membered aromatic heterocyclic group which may be optionally substituted with one or more substituents selected from Group B, a C3-C7 cycloalkenyl group which may be optionally substituted with one or more substituents selected from Group C, $R^3$-O-N=C($R^4$)-, $R^5$-C≡C-, or $R^6$O-,
$R^1$ and $R^2$ are identical to or different from each other and each represents a C1-C3 chain hydrocarbon group which may be optionally substituted with one or more halogen atoms, a methoxy group, a cyclopropyl group, or a halogen atom,
$R^3$ represents a C1-C6 chain hydrocarbon group which may be optionally substituted with one or more substituents selected from Group G, a C3-C6 cycloalkyl group which may be optionally substituted with one or more substituents selected from Group E, a C6-C10 aryl group, a five- to ten-membered aromatic heterocyclic group {the C6-C10 aryl group, and the five- to ten- membered aromatic heterocyclic group may be optionally substituted with one or more substituents selected from Group F}, or $R^7CH_2$-,
$R^4$ represents a C1-C3 chain hydrocarbon group which may be optionally substituted with one or more halogen atoms, or a hydrogen atom,
$R^5$ represents a C1-C6 chain hydrocarbon group which may be optionally substituted with one or more substituents selected from Group D, a C3-C6 cycloalkyl group which may be optionally substituted with one or more substituents selected from Group E, a phenyl group, or a five- to six-membered aromatic heterocyclic group {the phenyl group, and the five- to six- membered aromatic heterocyclic group may be optionally substituted with one or more substituents selected from Group F},
$R^6$ represents a methyl group which is substituted with one or more substituents selected from Group G, a C2-C6 chain hydrocarbon group which may be optionally substituted with one or more substituents selected from Group D, or a C3-C4 cycloalkyl group which may be optionally substituted with one or more substituents selected from Group E,
$R^7$ represents a phenyl group, or a five- to six-membered aromatic heterocyclic group {the phenyl group, and the five- to six- membered aromatic heterocyclic group may be optionally substituted with one or more substituents selected from Group F},
Group A is a group consisting of a C1-C6 chain hydrocarbon group, a C1-C3 alkylthio group {the C1-C6 chain hydrocarbon group, and the C1-C3 alkylthio group may be optionally substituted with one or more substituents selected from Group G}, a C3-C6 cycloalkyl group which may be optionally substituted with one or more substituents selected from Group E, $OR^{10}$, C(O)$R^8$, C(O)O$R^8$, C(O)N$R^8R^9$, N$R^8R^9$, C($R^{11}$)=N-O$R^{12}$, a phenyl group, a five- to six- membered aromatic heterocyclic group {the phenyl group, and the five-to six- membered aromatic heterocyclic group may be optionally substituted with one or more substituents selected from Group F}, a halogen atom, a cyano group, and a nitro group,
$R^8$ and $R^9$ are identical to or different from each other and each represents a C1-C6 chain hydrocarbon group which may be optionally substituted with one or more substituents selected from Group G, a C3-C6 cycloalkyl group which may be optionally substituted with one or more substituents selected from Group E, a phenyl group, a five- to six-membered aromatic heterocyclic group {the phenyl group, and the five- to six- membered aromatic heterocyclic group may be optionally substituted with one or more substituents selected from Group F}, or a hydrogen atom,
$R^{10}$ represents a C1-C6 chain hydrocarbon group which may be optionally substituted with one or more substituents selected from Group G, a C3-C6 cycloalkyl group which may be optionally substituted with one or more

substituents selected from Group E, a phenyl group, or a five- to six-membered aromatic heterocyclic group {the phenyl group, and the five- to six- membered aromatic heterocyclic group may be optionally substituted with one or more substituents selected from Group F},

$R^{11}$ represents a C1-C3 chain hydrocarbon group which may be optionally substituted with one or more halogen atoms, or a hydrogen atom,

$R^{12}$ represents a C1-C3 chain hydrocarbon group which may be optionally substituted with one or more substituents selected from Group D, a C3-C6 cycloalkyl group which may be optionally substituted with one or more substituents selected from Group E, a C6-C10 aryl group, and a five- to ten- membered aromatic heterocyclic group {the C6-C10 aryl group, and the five- to ten- membered aromatic heterocyclic group may be optionally substituted with one or more substituents selected from Group F},

Group B is a group consisting of a C3-C6 cycloalkyl group which may be optionally substituted with one or more substituents selected from Group E, a C1-C6 chain hydrocarbon group, a C1-C3 alkylthio group {the C1-C6 chain hydrocarbon group, and the C1-C3 alkylthio group may be optionally substituted with one or more substituents selected from Group G}, a phenyl group, a five- to six- membered aromatic heterocyclic group {the phenyl group, and the five- to six-membered aromatic heterocyclic group may be optionally substituted with one or more substituents selected from Group F}, $OR^{13}$, $C(O)R^8$, $C(O)OR^8$, $C(O)NR^8R^9$, $NR^8R^9$, $C(R^{11})=N-OR^{12}$, a halogen atom, a cyano group, and a nitro group,

$R^{13}$ represents a C3-C6 cycloalkyl group which may be optionally substituted with one or more substituents selected from Group E, a C1-C6 chain hydrocarbon group {the C1-C6 chain hydrocarbon group may be optionally substituted with one or more substituents selected from a C3-C6 cycloalkyl group, and a C1-C3 alkoxy group}, a phenyl group, a five- to six- membered aromatic heterocyclic group {the phenyl group, and the five- to six-membered aromatic heterocyclic group may be optionally substituted with one or more substituents selected from Group F}, or a trifluoromethyl group,

Group C is a group consisting of a C1-C4 chain hydrocarbon group, a C1-C3 alkoxy group {the C1-C4 chain hydrocarbon group, and the C1-C3 alkoxy group may be optionally substituted with one or more halogen atoms}, a halogen atom, a cyano group, an oxo group, and ($=NOR^{13}$),

Group D is a group consisting of a C1-C6 alkoxy group, a C1-C6 alkylthio group {the C1-C6 alkoxy group and the C1-C6 alkylthio group may be optionally substituted with one or more substituents selected from Group G}, a C3-C6 cycloalkyl group, a three- to eight- membered non-aromatic heterocyclic group {the C3-C6 cycloalkyl group, and the three- to eight-membered non-aromatic heterocyclic group may be optionally substituted with one or more substituents selected from Group E}, a C6-C10 aryl group, a five- to ten- membered aromatic heterocyclic group {the C6-C10 aryl group, and the five- to ten- membered aromatic heterocyclic group may be optionally substituted with one or more substituents selected from Group F}, a halogen atom, a cyano group, a nitro group, and a hydroxy group,

Group E is a group consisting of a C1-C3 chain hydrocarbon group, a C1-C3 alkoxy group, a C3-C6 cycloalkyl group {the C1-C3 chain hydrocarbon group, the C1-C3 alkoxy group, and the C3-C6 cycloalkyl group may be optionally substituted with one or more halogen atoms}, an oxo group, a halogen atom, a cyano group, and ($=NOR^4$),

Group F is a group consisting of a C1-C3 chain hydrocarbon group, a C3-C6 cycloalkyl group, a C1-C3 alkoxy group, a C1-C3 alkylthio group {the C1-C3 chain hydrocarbon group, the C3-C6 cycloalkyl group, the C1-C3 alkoxy group, and the C1-C3 alkylthio group may be optionally substituted with one or more substituents selected from Group G}, a halogen atom, a cyano group, a nitro group, and a hydroxy group,

Group G is a group consisting of a C3-C6 cycloalkyl group, a C1-C3 alkoxy group, and a halogen atom],

or its N-oxide or salts thereof.

**2.** The compound according to claim 1, its N-oxide or salts thereof, wherein

n is 1,

$R^1$ and $R^2$ are identical to or different from each other and each represents a C1-C3 alkyl group which may be optionally substituted with one or more halogen atoms, a methoxy group, or a halogen atom,

$R^2$ binds at the 4-position or the 5-position,

E represents a phenyl group, a five- to six- membered aromatic heterocyclic group {the phenyl group, and the five-to six- membered aromatic heterocyclic group may be optionally substituted with one or more substituents selected from Group H}, $R^3-O-N=C(R^4)-$, $R^5-C\equiv C-$, $R^6O-$, or a C5-C6 cycloalkenyl group,

$R^3$ represents a C1-C6 chain hydrocarbon group which may be optionally substituted with one or more substituents selected from Group G, a C3-C6 cycloalkyl group which may be optionally substituted with one or more halogen atoms, or $R^7CH_2-$,

$R^4$ represents a C1-C3 chain hydrocarbon group, or a hydrogen atom,
$R^5$ represents a C1-C6 chain hydrocarbon group, or a C3-C6 cycloalkyl group {the C1-C6 chain hydrocarbon group, and the C3-C6 cycloalkyl group may be optionally substituted with one or more halogen atoms},
$R^6$ represents a C2-C6 chain hydrocarbon group which may be optionally substituted with one or more substituents selected from Group I, or a C3-C4 cycloalkyl group,
$R^7$ represents a phenyl group or a five- to six- membered aromatic heterocyclic group {the phenyl group, and the five-to six- membered aromatic heterocyclic group may be optionally substituted with one or more substituents selected from Group J},
Group H is a group consisting of a C1-C3 chain hydrocarbon group which may be optionally substituted with one or more halogen atoms, a C1-C3 alkoxy group, a trifluoromethoxy group, a C3-C4 cycloalkyl group, a halogen atom, a cyano group, and a nitro group,
Group I is a group consisting of a C3-C6 cycloalkyl group, a C1-C3 alkoxy group, a phenyl group {the C1-C3 alkoxy group and the phenyl group may be optionally substituted with one or more halogen atoms}, and a halogen atom,
Group J is a group consisting of a C1-C3 chain hydrocarbon group, a C1-C3 alkoxy group {the C1-C3 chain hydrocarbon group, and the C1-C3 alkoxy group may be optionally substituted with one or more halogen atoms}, a C3-C6 cycloalkyl group, a halogen atom, a cyano group, and a nitro group.

3. The compound according to claim 2, or its N-oxide or salts thereof, wherein E represents a phenyl group, a pyridyl group, a thienyl group, a thiazolyl group, a pyrazolyl group {the phenyl group, the pyridyl group, the thienyl group, the thiazolyl group, and the pyrazolyl group may be optionally substituted with one or more substituents selected from Group H}, $R^5$-C≡C-, $R^6$O-, or a C5-C6 cycloalkenyl group.

4. An agricultural composition which comprises the compound according to any one of claims 1 to 3 or salts thereof, and an inert carrier.

5. A composition which comprises one or more ingredients selected from the group consisting of the following Groups (a), (b), (c) and (d), as well as the compound according to any one of claims 1 to 3 or its N-oxide or salts thereof:

Group (a): a group consisting of insecticidal ingredients, miticidal ingredients, and nematicidal ingredients;
Group (b): fungicidal ingredients;
Group (c): plant growth modulating ingredients; and
Group (d): repellent ingredients.

6. A method for controlling pests which comprises applying an effective amount of the compound according to any one of claims 1 to 3 or its N-oxide or salts thereof or an effective amount of the composition according to claim 5 to a plant or soil.

7. A method for controlling soybean rust fungus having an amino acid substitution of F129L on a mitochondrial cytochrome b protein, which comprises applying an effective amount of the compound according to any one of claims 1 to 3, or its N-oxide or salts thereof, or an effective amount of the composition according to claim 5 to a soybean or soil where the soybean grows.

8. Use of the compound according to any one of claims 1 to 3, or its N-oxide or salts thereof, or the composition according to claim 5 for controlling a pest.

9. A seed or vegetative reproductive organ carrying an effective amount of the compound according to any one of claims 1 to 3 or its N-oxide or salt thereof or an effective amount of the composition according to claim 5.

10. A compound represented by formula (II):

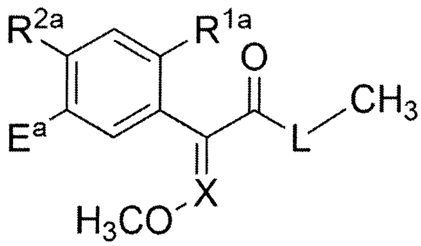

( II )

[wherein

a combination of X and L represents

a combination wherein X represents a nitrogen atom, and L represents an oxygen atom or NH; or
a combination wherein X represents CH, and L represents an oxygen atom,

$E^a$ represents $R^{12a}C(O)$ -, $R^{12a}C(=N-OH)$ -, a hydroxy group, $B(OH)_2$, a 4,4,5,5-tetramethyl-1,3,2-dioxaborolan-2-yl group, or a halogen atom,
$R^{1a}$ and $R^{2a}$ are identical to or different from each other and each represents a methyl group, or a halogen atom, and
$R^{12a}$ represents a C1-C3 alkyl group, or a hydrogen atom].

## INTERNATIONAL SEARCH REPORT

International application No.
**PCT/JP2022/008031**

**A. CLASSIFICATION OF SUBJECT MATTER**

***C07C 69/612***(2006.01)i; ***A01N 25/00***(2006.01)i; ***A01N 37/36***(2006.01)i; ***A01N 37/44***(2006.01)i; ***A01N 43/10***(2006.01)i; ***A01N 43/40***(2006.01)i; ***A01N 43/56***(2006.01)i; ***A01N 43/78***(2006.01)i; ***A01N 47/02***(2006.01)i; ***A01N 65/12***(2009.01)i; ***A01P 3/00***(2006.01)i; ***A01P 7/02***(2006.01)i; ***A01P 7/04***(2006.01)i; ***A61K 31/216***(2006.01)i; ***A61K 31/381***(2006.01)i; ***A61K 31/415***(2006.01)i; ***A61K 31/427***(2006.01)i; ***A61K 31/4418***(2006.01)i; ***A61P 31/04***(2006.01)i; ***A61P 33/14***(2006.01)i; ***C07C 69/618***(2006.01)i; ***C07C 69/65***(2006.01)i; ***C07C 251/38***(2006.01)i; ***C07C 251/48***(2006.01)i; ***C07C 251/52***(2006.01)i; ***C07D 213/26***(2006.01)i; ***C07D 213/61***(2006.01)i; ***C07D 231/12***(2006.01)i; ***C07D 277/22***(2006.01)i; ***C07D 333/28***(2006.01)i

FI: C07C69/612 CSP; A01N37/36; A01N43/40 101A; A01P3/00; A01P7/04; A01P7/02; A61K31/216; A61P33/14; A61P31/04; C07D213/61; A61K31/4418; C07C251/48; C07C251/52; C07C69/618; C07C69/65; A01N37/44; A01N47/02; A01N43/78 B; A01N43/10 B; A01N43/56 B; A01N65/12; A01N25/00 102; C07D213/26; C07D277/22; C07D333/28; A61K31/427; A61K31/381; A61K31/415; C07D231/12 Z; C07C251/38 CSP

According to International Patent Classification (IPC) or to both national classification and IPC

**B. FIELDS SEARCHED**

Minimum documentation searched (classification system followed by classification symbols)

C07C69/612; A01N25/00; A01N37/36; A01N37/44; A01N43/10; A01N43/40; A01N43/56; A01N43/78; A01N47/02; A01N65/12; A01P3/00; A01P7/02; A01P7/04; A61K31/216; A61K31/381; A61K31/415; A61K31/427; A61K31/4418; A61P31/04; A61P33/14; C07C69/618; C07C69/65; C07C251/38; C07C251/48; C07C251/52; C07D213/26; C07D213/61; C07D231/12; C07D277/22; C07D333/28

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2019/189287 A1 (SUMITOMO CHEMICAL COMPANY, LIMITED) 03 October 2019 (2019-10-03)<br>entire text, all drawings, particularly, claims, formula (I), paragraphs [0006]-[0007], [0057], [0063], examples | 1-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 April 2022** | **26 April 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No. **PCT/JP2022/008031**

**C. DOCUMENTS CONSIDERED TO BE RELEVANT**

| Category* | Citation of document, with indication, where appropriate, of the relevant passages | Relevant to claim No. |
|---|---|---|
| A | JIANG, Wentao et al. Synthesis and Fungicidal Activity of Methyl 2-Methoxyimino-2-polysubstituted-phenylacetates. Chin. J. Org. Chem. 2014, vol. 34, pages 774-782, DOI: 10.6023/cjoc201309004<br>entire text, all drawings | 1-10 |
| A | JP 2010-523570 A (E.I. DU PONT DE NEMOURS AND COMPANY) 15 July 2010 (2010-07-15)<br>entire text, all drawings | 1-10 |
| A | WO 99/28305 A1 (E.I. DU PONT DE NEMOURS AND COMPANY) 10 June 1999 (1999-06-10)<br>entire text, all drawings | 1-10 |
| P, X | WO 2021/153794 A1 (SUMITOMO CHEMICAL COMPANY, LIMITED) 05 August 2021 (2021-08-05)<br>entire text, all drawings, particularly, claims, chemical formulas 93, 101 | 10 |

Form PCT/ISA/210 (second sheet) (January 2015)

**INTERNATIONAL SEARCH REPORT**
**Information on patent family members**

International application No.
**PCT/JP2022/008031**

| Patent document cited in search report | Publication date (day/month/year) | Patent family member(s) | Publication date (day/month/year) |
|---|---|---|---|
| WO 2019/189287 A1 | 03 October 2019 | US 2021/0022338 A1<br>entire text, all drawings<br>EP 3778551 A1<br>CN 111902392 A | |
| JP 2010-523570 A | 15 July 2010 | US 2010/0120714 A1<br>entire text, all drawings<br>CN 101686688 A<br>KR 10-2010-0015788 A | |
| WO 99/28305 A1 | 10 June 1999 | (Family: none) | |
| WO 2021/153794 A1 | 05 August 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2021029651 A
- JP 2021124464 A
- EP 0422597 A2
- WO 98043949 A1
- US 20180009778 A1
- WO 2016121970 A1
- WO 2016123253 A1

### Non-patent literature cited in the description

- *Angew. Chem. Int. Ed,* 2013, vol. 52, 8611-8615
- FAO Plant Production and Protection Papers-271-276. *the FAO/WHO Joint Meeting on Pesticide Specifications,* 2016, ISSN 0259-2517